# EUROPEAN PATENT APPLICATION

(11) **EP 4 813 979 A1**
(43) Date of publication of application: **30.09.2026**
(21) Application number: 24893492.9
(22) Date of filing: 20.11.2024
(51) Int. Cl.: C07D 401/14, C07F 9/53, A61K 31/506, A61K 31/675, A61P 35/00, A61P 29/00, A61P 9/00, A61P 37/00

(54) **COMPOUND FOR EGFR PROTEIN DEGRADATION AND USE THEREOF**

(30) Priority: 20.11.2023 CN 202311548377; 23.08.2024 CN 202411168908; 22.10.2024 CN 202411478209
(71) Applicant: TYK Medicines, Inc., Huzhou, Zhejiang 313100 (CN)
(72) Inventor: LIANG, Apeng, Huzhou, Zhejiang 313100 (CN); HAN, Shunxun, Huzhou, Zhejiang 313100 (CN); CHEN, Shaoqing, Huzhou, Zhejiang 313100 (CN); WU, Yusheng, Huzhou, Zhejiang 313100 (CN); LI, Jun, Huzhou, Zhejiang 313100 (CN); DONG, Shengli, Huzhou, Zhejiang 313100 (CN)
(74) Representative: dompatent
(86) International application number: PCT/CN2024/133328
(87) International publication number: WO 2025/108336

(57) **Abstract**

The present invention relates to a compound for EGFR protein degradation and a use thereof. Specifically, the compound of the present invention has a structure represented by formula III, wherein the definition of each group and substituent is as described in the description Also disclosed in the invention are a preparation method for the compound and a use of same in the prevention and/or treatment of EGFR-related diseases.

## Description

### THCHNICAL FIFLD

The present invention relates to the field of pharmaceutical technology and specifically, the present invention relates to compounds for the degradation of the EGFR protein and their uses.

### BACKGROUND

Receptor tyrosine kinases of the HER family act as mediators of cell growth, differentiation, and survival. This receptor family comprises four distinct members: the epidermal growth factor receptor (EGFR, ErbB1, or HER1), HER2 (ErbB2), HER3 (ErbB3), and HER4 (ErbB4). Upon ligand binding, these receptors form homodimers or heterodimers; subsequent activation of their intrinsic tyrosine kinase activity leads to receptor autophosphorylation and the activation of downstream signaling molecules. It has been demonstrated that EGFR activation caused by overexpression or mutation plays a role in regulating various types of human cancers, including colorectal cancer, pancreatic cancer, glioma, head and neck cancer, and lung cancer, particularly non-small cell lung cancer (NSCLC). Over the years, numerous EGFR-targeted agents have been developed, and three generations of drugs are now in clinical use.

In clinical practice, patients generally develop the EGFR T790M resistance mutation 8 to 12 months after starting first- or second-generation EGFR inhibitors, causing the drugs to lose their therapeutic efficacy. Although third-generation EGFR inhibitors, such as Osimertinib and Amitinib, which were subsequently introduced, can effectively overcome resistance caused by the EGFR T790M mutation, resistance-associated mutations such as EGFR C797S still emerge after a period of treatment, leading to disease progression.

The frequent emergence of mutation-driven resistance during treatment with EGFR small-molecule tyrosine kinase inhibitors has become a pressing clinical challenge. Although some EGFR allosteric inhibitor compounds, such as EA1045, have been reported to overcome C797S resistance, their clinical efficacy remains limited. In recent years, several patents (WO2019149922, WO2021127561) have reported a series of PROTAC-type compounds capable of overcoming C797S resistance by degrading the EGFR protein, establishing a new research direction.

In summary, although some progress has been made with EGFR allosteric inhibitors and EGFR degraders, the field still requires the development of additional EGFR-modulating drugs with greater clinical value for the treatment of diseases caused by EGFR dysregulation, particularly in the context of EGFR-positive non-small cell lung cancer.

### SUMMARY OF THE INVENTION

One purpose of the present invention is to provide a compound as shown in Formula I.

Another purpose of the present invention is to provide a use of the compound shown in Formula I in the prevention and/or treatment of EGFR-related diseases.

In the first aspect of the present invention, provided is a compound shown in formula I, or a pharmaceutically acceptable salt, a stereoisomer, a tautomer, a hydrate, a solvate, an isotope, or a prodrug thereof, 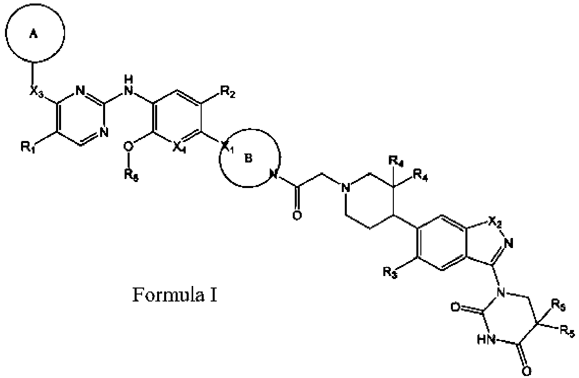 wherein,
X₃ is selected from none, NH, or NR;
X₂ is selected from NR, O, or S;
each R is independently selected from C₁₋₆ alkyl or halogenated C₁₋₆ alkyl;
X₁ is selected from CH or N;
X₄ is selected from CH or N;
ring B 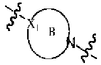 is selected from the group consisting of substituted or unsubstituted 6-7-membered heterocyclylene, and substituted or unsubstituted 7-9-membered spiro heterocyclylene ; the "substituted" refers to one or more hydrogens of the group being substituted by substituents selected from the group consisting of H, halogen, amino, nitro, hydroxy, cyano, C₁₋₆ alkyl, C₁₋₆ alkoxy, C₃₋₆ cycloalkyl, halogenated C₁₋₆ alkyl, and C₁₋₆ hydroxyalkyl;
ring A is selected from the group consisting of substituted or unsubstituted C₆₋₁₀ aryl; substituted or unsubstituted 6-10 membered heteroaryl; the substituted refers to 0-1 hydrogen of the group being substituted by R₆, and m hydrogens are substituted by R₇; m is 0, 1, or 2;
wherein,
R₆ is selected from the group consisting of C₁₋₆ alkyl, C₁₋₆ hydroxyalkyl, halogenated C₁₋₆ alkyl, -P(O)RaRb, -C(O)R, -C(O)NHR, -C(O)NRaRb, -OC(O)-OR, -OC(O)NHR, -OC(O)NRaRb, -S(O)₂R, -NR-S(O)₂R, -NR-C(O)-OR, -NH-C(O)-OR, and C₁₋₄ alkyl-substituted or unsubstituted 4-7-membered heterocyclyl-O-;
Ra and Rb each independently are C₁₋₆ alkyl or C₃₋₆ cycloalkyl, or Ra and Rb with the heteroatom to which they are attached together form a 5-7-membered heterocycle;
R₇ is selected from the group consisting of H, halogen, amino, nitro, hydroxy, cyano, C₁₋₆ alkyl, C₁₋₆ alkoxy, C₃₋₆ cycloalkyl, halogenated C₁₋₆ alkyl, and C₁₋₆ hydroxyalkyl;
R₁ is selected from halogen;
R₂ is selected from H, substituted or unsubstituted C₁₋₆ alkyl, or substituted or unsubstituted 2-6-membered heteroalkyl; wherein the heteroalkyl contains at least one heteroatom selected from the group consisting of O, N, and S, and wherein the "substituted" refers to one or more hydrogen atoms on the group being substituted by substituents selected from the group consisting of cyano, nitro, hydroxy, amino, and halogen;
R₃ is selected from halogen;
each R₄ is independently H or halogen;
each R₅ is independently H, halogen, or C₁₋₆ alkyl; or two R₅ groups with the carbon atom to which they are attached together form a C₃₋₆ cycloalkyl;
R₈ is selected from C₁₋₆ alkyl, or halogenated C₁₋₆ alkyl.

In another preferred embodiment, R₆ is located at the adjacent position to the binding site.

In another preferred embodiment, ring A is selected from the group consisting of 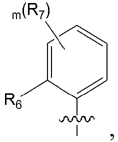 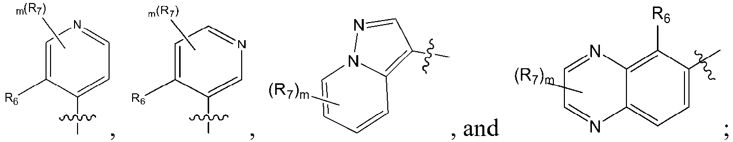 wherein, R₆, R₇, and m are as described in the first aspect of the present invention.

In another preferred embodiment, ring B 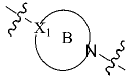 is substituent or unsubstituted groups selected from the group consisting of 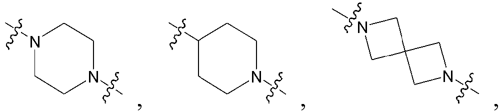 and 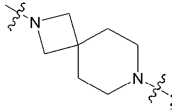 ; wherein, the "substituted" refers to one or more hydrogen atoms of the groups being substituted by a substituent selected from the group consisting of H, halogen, amino, nitro, hydroxy, cyano, C₁₋₆ alkyl, C₁₋₆ alkoxy, C₃₋₆ cycloalkyl, halogenated C₁₋₆ alkyl, and C₁₋₆ hydroxyalkyl.

In another preferred embodiment, ring B 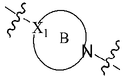 is substituted with a substituent selected from the group consisting of H, halogen, C₁₋₆ alkyl, C₁₋₆ alkoxy, and C₃₋₆ cycloalkyl.

In another preferred embodiment, R₆ is selected from the group consisting of C₁₋₆ alkyl, C₁₋₆ hydroxyalkyl, -P(O)RaRb, -C(O)NHR, -C(O)NRaRb, -OC(O)NHR, -OC(O)NRaRb, -S(O)₂R, -NR-S(O)₂R, -NR-C(O)-OR, and -NH-C(O)-OR;

Ra and Rb are each independently C₁₋₄ alkyl, or C₃₋₆ cycloalkyl; alternatively, Ra and Rb with the heteroatom to which they are attached together form a 5-membered heterocycle,

In another preferred embodiment, the compound has the structure shown in Formula II-1: 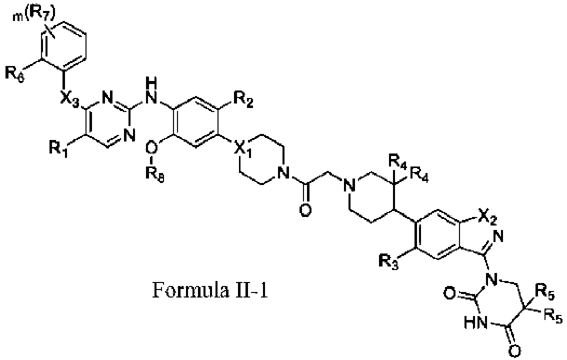 wherein,
X₁ is selected from CH or N;
X₂ is selected from NR;
R is selected from C₁₋₃ alkyl or halogenated C₁₋₃ alkyl (preferably CF₃);
X₃ is selected from none, or NH;
R₁ is selected from halogen;
R₂ is selected from H, substituted or unsubstituted C₁₋₆ alkyl, or substituted or unsubstituted 2-4-membered heteroalkyl; wherein, the heteroalkyl contains at least one heteroatom selected from the group consisting of O, N, and S; and the "substituted" refers to one or more hydrogen atoms of the groups being substituted by substituents selected from the group consisting of cyano, nitro, hydroxyl, amino, and halogen;
R₃ is selected from halogen;
each R₄ is independently H or a halogen;
each R₅ is independently H, a halogen, or a C₁₋₃ alkyl group; or two R₅ with the carbon atom to which they are attached together form a C₃₋₄ cycloalkyl;
R₆ is selected from the group consisting of 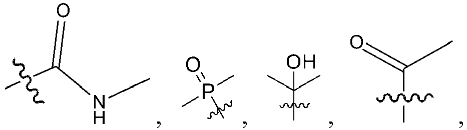 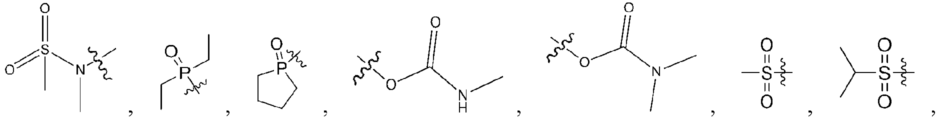 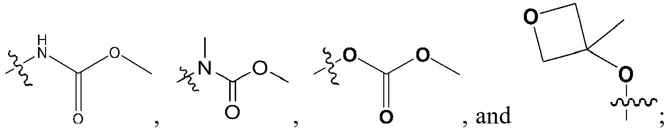
R₇ is selected from the group consisting of H, C₃₋₆ cycloalkyl, halogen, C₁₋₆ alkyl, and C₁₋₆ alkoxy;
R₈ is selected from the group consisting of C₁₋₄ alkyl, and halogenated C₁₋₄ alkyl;
m is 0, 1, or 2.

In another preferred embodiment, R₄ and R₅ are not both halogens.

In another preferred embodiment, R₄ is F and R₅ is H.

In another preferred embodiment, R₄ is H and R₅ is F.

In another preferred embodiment, R₂ is ethyl.

In another preferred embodiment, R₁ is Cl or Br.

In another preferred embodiment, R₇ is H, Br, cyclopropyl, methoxy, ethyl, methyl, F, Cl, or isopropyl.

In the second aspect of the present invention, it provides a compound, the compound is a compound of Formula II, or a pharmaceutically acceptable salt, a stereoisomer, a tautomer, a hydrate, a solvate, a isotopically labeled compound, or a prodrug thereof, 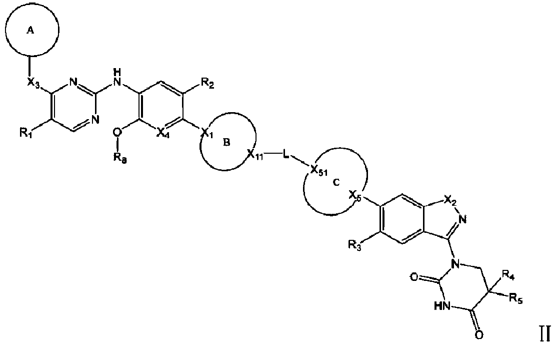 wherein,
X₃ is selected from the group consisting of none, NH, NR, and O;
X₂ is selected from the group consisting of NR, O, and S;
each R is independently selected from the group consisting of C1-6 alkyl, and C6-10 aryl;
X₁ is selected from the group consisting of CH, and N;
X₁₁ is selected from the group consisting of CH, and N;
X₄ is selected from the group consisting of N, and CR';
X₅ is selected from the group consisting of CH, N, and C;
X₅₁ is selected from the group consisting of C-(OH), N, and C;
ring B 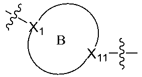 is selected from the group consisting of substituted or unsubstituted 6-7-membered heteromonocycloalkylene containing 1 to 3 heteroatoms selected from N, O or S, substituted or unsubstituted 7-9-membered bridged heterocycloalkylene containing 1 to 3 heteroatoms selected from N, O or S, and substituted or unsubstituted 7-10-membered spiro heterocycloalkylene containing 1 to 3 heteroatoms selected from N, O or S; the "substituted" refers to one or more hydrogens of the groups being substituted by substituents selected from the group consisting of halogen, amino, nitro, hydroxy, cyano, C₁₋₆ alkyl, halogenated C₁₋₆ alkyl, hydroxy-substituted C₁₋₆ alkyl, C₁₋₆ alkoxy, and C₃₋₆ cycloalkyl;
ring C 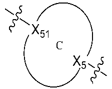 is selected from the group consisting of substituted or unsubstituted 6-7-membered heteromonocycloalkylene containing 1-3 heteroatoms selected from N, O or S, substituted or unsubstituted 7-9-membered spiro heterocycloalkylene containing 1-3 heteroatoms selected from N, O or S, substituted or unsubstituted 4-10-membered bridged heterocycloalkylene containing 1 to 3 heteroatoms selected from N, O or S, and substituted or unsubstituted 5-7-membered heteroarylene containing 1-3 heteroatoms selected from N, O or S; the "substituted" refers to one or more hydrogens of the groups being substituted by substituents selected from the group consisting of halogen, amino, nitro, hydroxy, cyano, C₁₋₆ alkyl, halogenated C₁₋₆ alkyl, hydroxy-substituted C₁₋₆ alkyl, C₁₋₆ alkoxy, and C₃₋₆ cycloalkyl;
ring A is selected from the group consisting of substituted or unsubstituted C₆₋₁₀ aryl, and substituted or unsubstituted 5-10-membered heteroaryl containing 1 to 3 heteroatoms selected from N, O or S; the "substituted" refers to one hydrogen atom of the group being substituted by R₆ and m hydrogen atoms being substituted by R₇;
R₆ is selected from the group consisting of C₁₋₆ alkyl, hydroxy-substituted C₁₋₆ alkyl, -P(O)RaRb, 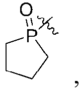 -C(O)Ra, -C(O)NRaRb, -OC(O)NRaRb, -S(O)₂Ra, -NRa-S(O)₂Rb, -NRa-C(O)-ORa, 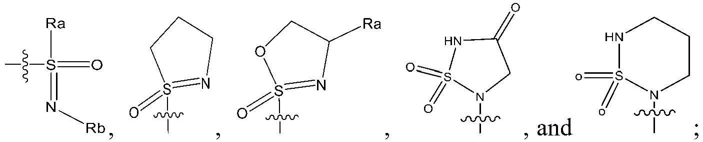
Ra and Rb are each independently selected from the group consisting of H and C₁₋₆ alkyl;
R₇ is selected from the group consisting of H, halogen, amino, nitro, hydroxy, cyano, C₁₋₆ alkyl, halogenated C₁₋₆ alkyl, hydroxy-substituted C₁₋₆ alkyl, C₁₋₆ alkoxy, and C₃₋₆ cycloalkyl;
R₁ is halogen;
R₂ is selected from the group consisting of H, halogen, C₁₋₆ alkyl, halogenated C₁₋₆ alkyl, C₁₋₆ alkoxy, halogenated C₁₋₆ alkoxy, C₃₋₆ cycloalkyl, -(C=O)-O-C₁₋₆ alkyl, and -(C=O)-NH-C₁₋₆ alkyl;
R₃ is a halogen;
R₄ is selected from the group consisting of H and halogen;
R₅ is selected from the group consisting of H and halogen;
R₈ is selected from the group consisting of C₁₋₆ alkyl, and halogenated C₁₋₆ alkyl; or R₈ and R' with the atoms to which they are attached together form a 5-6-membered heterocyclic group containing one O atom,
L is selected from the groups consisting of 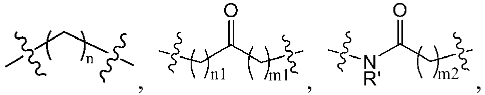 and 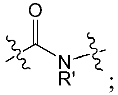
each R' is independently selected from the group consisting of H and C1-6 alkyl;
m is selected from the group consisting of 0, 1, and 2;
n is selected from the group consisting of 0, 1, and 2;
n1 is selected from the group consisting of 0, 1, and 2;
m1 is selected from the group consisting of 0, 1, and 2;
m2 is selected from the group consisting of 0, 1, and 2.

In another preferred embodiment, X₃ is selected from the group consisting of none, NH, NR, and O.

In another preferred embodiment, X₃ is NH.

In another preferred embodiment, X₂ is selected from the group consisting of NR, O, and S.

In another preferred embodiment, X₂ is -N(C₁₋₆-alkyl)-.

In another preferred embodiment, X₁ is selected from the group consisting of CH and N.

In another preferred embodiment, X₁ is N.

In another preferred embodiment, X₁₁ is selected from the group consisting of CH, and N.

In another preferred embodiment, X₁₁ is N.

In another preferred embodiment, X₄ is selected from the group consisting of N, and CR'.

In another preferred embodiment, X₄ is CH.

In another preferred embodiment, X₅ is selected from the group consisting of CH, N, and C.

In another preferred embodiment, X₅ is selected from the group consisting of CH, and C.

In another preferred embodiment, X₅₁ is selected from the group consisting of C-(OH), N, and C.

In another preferred embodiment, X₅₁ is N.

In another preferred embodiment, ring A is 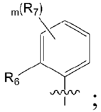 wherein, R₆, R₇, and m are as defined above.

In another preferred embodiment, R₆ is selected from the group consisting of C₁₋₆ alkyl, hydroxy-substituted C₁₋₆ alkyl, -P(O)RaRb, -C(O)NRaRb, -OC(O)NRaRb, -S(O)₂Ra, -NRa-S(O)₂Rb, -NRa-C(O)-ORa,

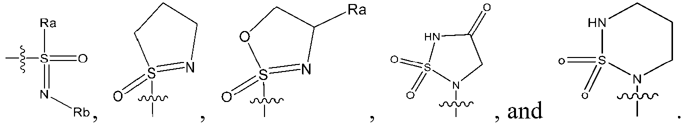

In another preferred embodiment, R₆ is selected from the group consisting of -P(O)RaRb, and - C(O)NRaRb.

In another preferred embodiment, Ra and Rb are each independently selected from the group consisting of H, C1-6 alkyl, and C3-6 cycloalkyl.

In another preferred embodiment, Ra is selected from the group consisting of H, and C1-6 alkyl.

In another preferred embodiment, Rb is C₁₋₆ alkyl.

In another preferred embodiment, R₂ is selected from the group consisting of H, halogen, C₁₋₆ alkyl, halogenated C₁₋₆ alkyl, C₁₋₆ alkoxy, halogenated C₁₋₆ alkoxy, C₃₋₆ cycloalkyl, -(C=O)-O-C₁₋₆ alkyl, and -(C=O)-NH-C₁₋₆ alkyl.

In another preferred embodiment, R₂ is selected from the group consisting of C₁₋₆ alkyl, and halogenated C₁₋₆ alkyl.

In another preferred embodiment, R₂ is C₁₋₆ alkyl.

In another preferred embodiment, R₈ is selected from the group consisting of C₁₋₆ alkyl and halogenated C₁₋₆ alkyl; or R₈ and R' with the atoms to which they are attached together form a 5-6-membered heterocyclic group containing one oxygen atom.

In another preferred embodiment, R₈ is selected from the group consisting of C1-6 alkyl and halogenated C1-6 alkyl.

In another preferred embodiment, R₈ is a C₁₋₆ alkyl group.

In another preferred embodiment, ring B 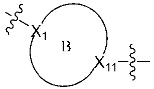 is substituent or unsubstituted groups selected from the group consisting of 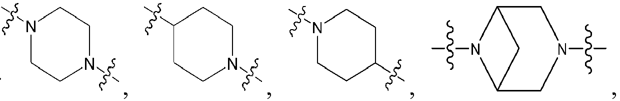 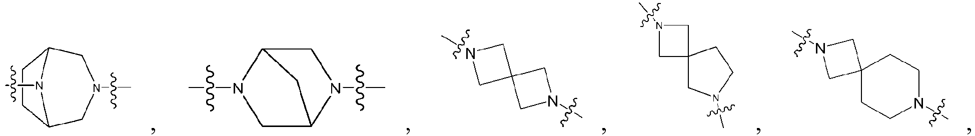 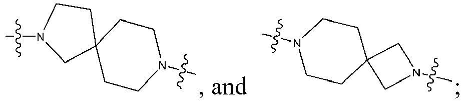 wherein, the "substituted" refers to one hydrogen atom of the group being substituted by substituents selected from the group consisting of halogen, amino, nitro, hydroxy, cyano, C₁₋₆ alkyl, halogenated C₁₋₆ alkyl, hydroxy-substituted C₁₋₆ alkyl, C₁₋₆ alkoxy, and C₃₋₆ cycloalkyl.

In another preferred embodiment, ring C 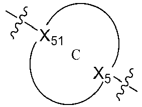 is selected from the group consisting of

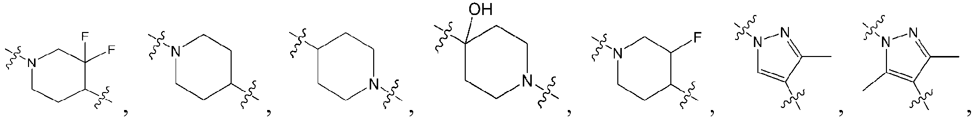

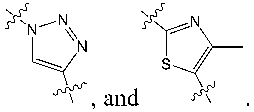

In another preferred embodiment, L is selected from the group consisting of

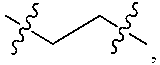

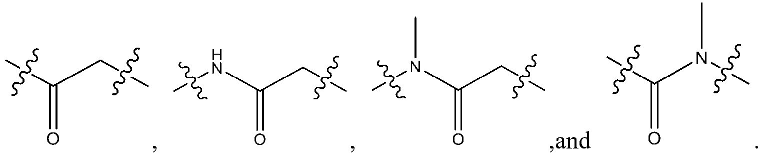

In another preferred embodiment, the compound is selected from the group consisting of compounds of Formula II', compounds of Formula III', and compounds of Formula IV': 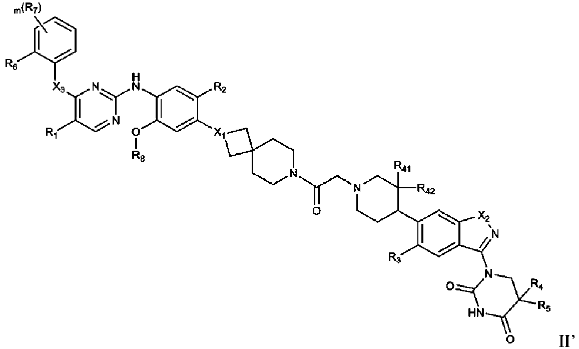 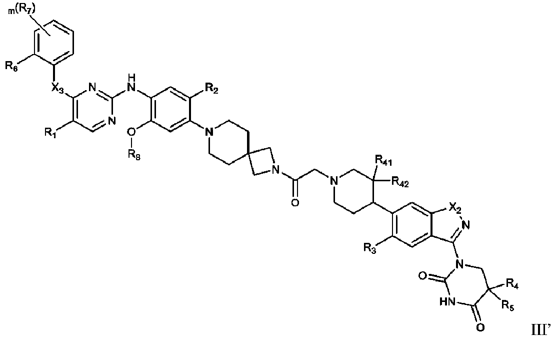 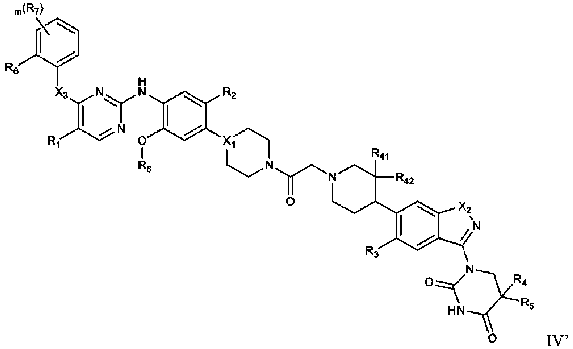 wherein,
X₁ is selected from the group consisting of CH, and N;
X₂ is NR;
R is a C₁₋₃ alkyl group;
X₃ is selected from the group consisting of none and NH;
R₁ is a halogen;
R₂ is selected from the group consisting of H, halogen, C₁₋₆ alkyl, halogenated C₁₋₆ alkyl, C₁₋₆ alkoxy, halogenated C₁₋₆ alkoxy, C₃₋₆ cycloalkyl, -(C=O)-O-C₁₋₆ alkyl, and -(C=O)-NH-C₁₋₆ alkyl;
R₃ is a halogen;
R₄₁ is selected from the group consisting of H, and halogen;
R₄₂ is selected from the group consisting of H, and halogen;
R₄ is selected from the group consisting of H, and halogen;
R₅ is selected from the group consisting of H, and halogen;
R₆ is selected from the group consisting of 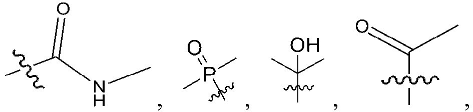 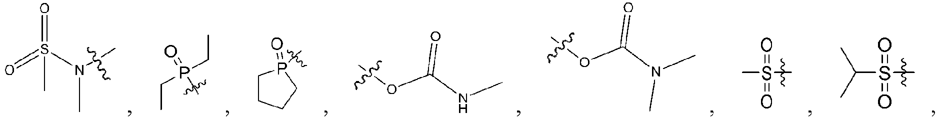 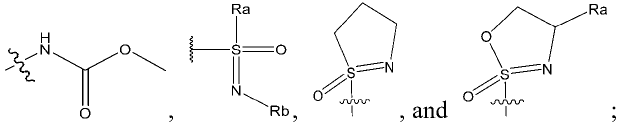
Ra and Rb are each independently selected from the group consisting of H, and C1-6 alkyl;
R₇ is selected from the group consisting of H, C3-6 cycloalkyl, halogen, C1-6 alkyl, and C1-6 alkoxy;
R₈ is selected from the group consisting of C1-6 alkyl, and halogenated C1-6 alkyl;
m is selected from the group consisting of 0, 1, and 2.

In the third aspect of the present invention, it provides a compound as shown in Formula III, or a pharmaceutically acceptable salt, a stereoisomer, a tautomer, a hydrate, a solvate, an isotope, or a prodrug thereof, 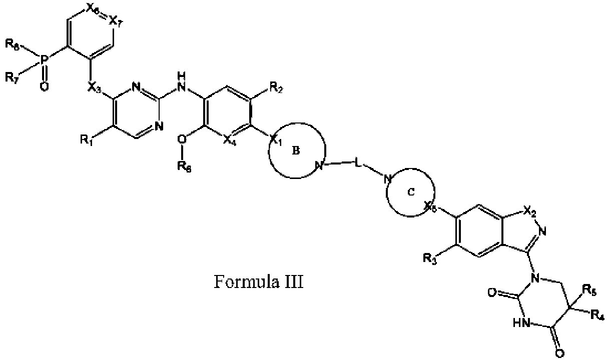 in the formula,
X₁ and X₅ are each independently selected from CH, N, or C;
X₂ is selected from NR, O, or S;
X₃ is selected from none(bond), NH, NR, or O;
X₄, X₆, and X₇ are each independently selected from the group consisting of CR and N;
each R is independently selected from the group consisting of H, halogen, hydroxy, amino, nitro, C₁₋₆ alkyl, C₁₋₆ alkoxy, halogenated C₁₋₆ alkyl, halogenated C₁₋₆ alkoxy, C₃₋₇ cycloalkyl, C₃₋₇ cycloalkyloxy, C₆₋₁₀ aryl, and C₆₋₁₀ aryloxy;
L is 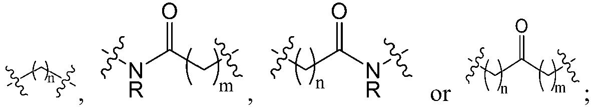 m and n are each independently 0, 1, 2, 3 or 4;
R₇ and R₈ with the P atom to which they are attached together form Cy1, and Cy1 is selected from the group consisting of saturated or partially unsaturated 4-7-membered ring containing P=O, except for P, the ring further comprises 0-3 heteroatoms independently selected from nitrogen, oxygen, or sulfur; and the ring is optionally substituted by one or more substituents R^{a}; or
R₇ and R₈ are each independently selected from the group consisting of C₁₋₆ alkyl, halogenated C₁₋₆ alkyl, and C₁₋₆ hydroxyalkyl;
R^{a} is selected from the group consisting of hydrogen, deuterium, halogen, C₁₋₈ alkyl, C₂₋₈ alkenyl, C₂₋₈ alkynyl, C₃₋₈ cycloalkyl, 3-8-membered heterocyclyl, C₆₋₁₀ aryl, 5-12-membered heteroaryl, -CN, -OR^{b}, -COR^{b}, -COOR^{b}, CONR^{b}R^{c}, -NR^{b}R^{c}, -NR^{b}COR^{c}, and -NR^{b}COOR^{c}, wherein, the alkyl, alkenyl, alkynyl, cycloalkyl, heterocyclic, aryl, or heteroaryl groups optionally be substituted by R^{d};
R^{b}, R^{c} and R^{d} are independently selected from the group consisting of hydrogen, hydroxyl, C₁₋₈ alkyl, C₂₋₈ alkenyl, C₂₋₈ alkynyl, and C₃₋₈ cycloalkyl;
R₁ is selected form halogen;
R₂ is selected from H, C₁₋₆ haloalkyl, C₁₋₆ alkyl, C₃₋₈ cycloalkyl, C₄₋₈ heterocycloalkyl, -(C=O)-O-C₁₋₆ alkyl, -(C=O)-NH-C₁₋₆ alkyl, halogen, -(CH₂)ₚ-C₁₋₄-alkyloxy, -(CH₂)ₚ-halogenated C₁₋₄-alkyloxy, -(CH₂)ₚ-CN, C1-6 alkyl substituted or unsubstituted 5-6-membered heteroaryl containing 1-3 heteroatoms selected from N, O or S or 2-6-membered heteroalkyl; wherein, the heteroalkyl or heterocycloalkyl contains one or more heteroatoms independently selected from the group consisting of O, N, and S; p is 0, 1, 2, or 3;
R₃ is selected from the group consisting of H, halogen, cyano, amino, nitro, hydroxy, C₁₋₄ alkyl, halogenated C₁₋₄ alkyl, C₁₋₄ alkoxy, halogenated C₁₋₄ alkoxy, and C₁₋₄ hydroxyalkyl;
R₄ is selected from the group consisting of H, halogen, cyano, amino, nitro, hydroxy, C₁₋₄ alkyl, halogenated C₁₋₄ alkyl, C₁₋₄ alkoxy, halogenated C₁₋₄ alkoxy, and C₁₋₄ hydroxyalkyl;
R₅ is selected from the group consisting of H, halogen, cyano, amino, nitro, hydroxy, C₁₋₄ alkyl, halogenated C₁₋₄ alkyl, C₁₋₄ alkoxy, halogenated C₁₋₄ alkoxy, and C₁₋₄ hydroxyalkyl;
or, R₄ and R₅ form a C₃₋₆ carbocycle or a 3-7-membered heterocycle, wherein the heteroatoms of the heterocycle are selected from O, N, or S;
R₆ is selected from the group consisting of C₁₋₆ alkyl, halogenated C₁₋₆ alkyl, and C₁₋₆ hydroxyalkyl; or, X₄ is CR, wherein the R of X₄ and R₆ form a saturated or partially saturated 5-6-membered heterocyclic group containing one oxygen atom;
ring B 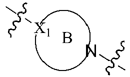 is selected from the group consisting of substituted or unsubstituted 6-7-membered heterocyclylene, substituted or unsubstituted 7-12-membered spiro heterocyclylene, and substituted or unsubstituted 7-12-membered bridged heterocyclene; the "substituted" refers to one or more hydrogen atoms of the group are substituted by substituents selected from the group consisting of H, D, halogen, amino, oxo (=O), nitro, hydroxy, cyano, C₁₋₆ alkyl, C₁₋₆ alkoxy, C₃₋₆ cycloalkyl, halogenated C₁₋₆ alkyl, and C₁₋₆ hydroxyalkyl, or any two hydrogen atoms connected to form a bond or -(CH₂)_{q}-, wherein, q is 1, 2, 3, 4, or 5 (e.g. compound TB-081, TB-056, etc.);
ring C 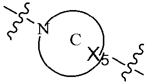 is selected from the group consisting of substituted or unsubstituted 6-7-membered heterocyclylene, substituted or unsubstituted 5-6-membered heteroarylene, and substituted or unsubstituted 7-12-membered spiro heterocyclylene; the "substituted" refers to one or more hydrogens of the group being substituted by substituents selected from the group consisting of H, halogen, amino, oxo (=O), nitro, hydroxy, cyano, C1-6 alkyl, C1-6 alkoxy, C3-6 cycloalkyl, halogenated C1-6 alkyl, and C1-6 hydroxyalkyl, or any two hydrogen atoms connected to form a bond or -(CH₂)_{q}-, wherein, q is 1, 2, 3, 4, or 5;
wherein, the heterocyclylene, heteroarylene, spiro heterocyclylene, and bridged heterocyclene each independently contain 1-3 heteroatoms selected from N, S, or O.

In another preferred embodiment, R is selected from the group consisting of C₁₋₆ alkyl, C₁₋₆ alkoxy, halogenated C₁₋₆ alkyl, halogenated C₁₋₆ alkoxy, C₃₋₇ cycloalkyl, C₃₋₇ cycloalkyloxy, C₆₋₁₀ aryl, and C₆₋₁₀ aryloxy.

In another preferred embodiment, R^{a} is selected from the group consisting of hydrogen, halogen, C₁₋₄ alkyl, C₂₋₄ alkenyl, C₂₋₄ alkynyl, C₃₋₆ cycloalkyl, 3-6-membered heterocyclyl, phenyl, 5-6-membered heteroaryl, -CN, -OR^{b}, -COR^{b}, -COOR^{b}, CONR^{b}R^{c}, -NR^{b}R^{c}, -NR^{b}COR^{c}, and -NR^{b}COOR^{c}, wherein the alkyl, alkenyl, alkynyl, cycloalkyl, heterocyclic, aryl, or heteroaryl may optionally be substituted by R^{d};

R^{b}, R^{c}, and R^{d} are each independently selected from the group consisting of hydrogen, hydroxyl, C1-4 alkyl, C2-4 alkenyl, C2-4 alkynyl, and C3-6 cycloalkyl.

In another preferred embodiment, L is 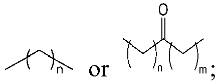 m and n are each independently 0, 1, or 2.

In another preferred embodiment, R₆ is selected from the group consisting of C₁₋₆ alkyl, and halogenated C₁₋₆ alkyl.

In another preferred embodiment, R₃ is selected from the group consisting of H and halogen.

In another preferred embodiment, R₄ is selected from the group consisting of H and halogen.

In another preferred embodiment, R₅ is selected from the group consisting of H and halogen.

In another preferred embodiment, ring B 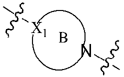 is selected from the group consisting of substituted or unsubstituted 6-7-membered heterocyclylene, and substituted or unsubstituted 7-10-membered spiro heterocyclylene; more preferably, ring B 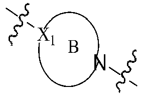 is substituted or unsubstituted groups selected from the group consisting of 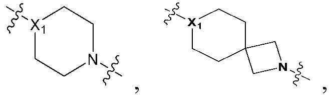 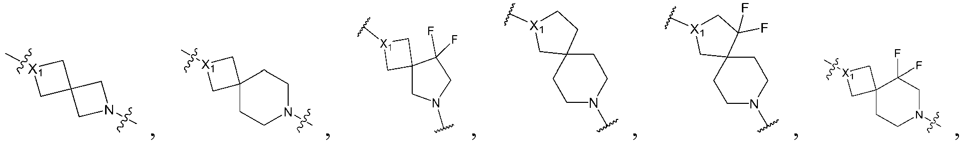 and 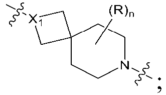 wherein, the "substituted" refers to one or more hydrogens of the group being substituted by substituents selected from the group consisting of H, halogen, amino, oxo (=O), nitro, hydroxy, cyano, C₁₋₆ alkyl, C₁₋₆ alkoxy, C₃₋₆ cycloalkyl, halogenated C₁₋₆ alkyl, and C₁₋₆ hydroxyalkyl, or any two hydrogen atoms connected to form a bond or a -(CH₂)_{q}- , wherein q is 1, 2, 3, 4, or 5.

In another preferred embodiment, ring C 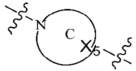 is substituted or unsubstituted groups selected from the group consisting of 6-7-membered heterocyclylene, 5-6-membered heteroarylene, and a 7-10-membered spiro heterocyclylene; preferably, ring C 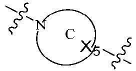 is substituted or unsubstituted groups selected from the group consisting of 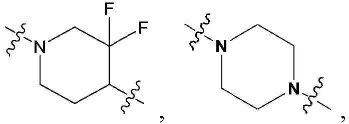 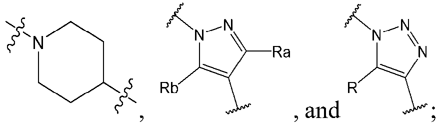 wherein, the "substituted" refers to one or more hydrogens of the group being substituted by substituents selected from the group consisting of H, halogen, amino, oxo (=O), nitro, hydroxy, cyano, C₁₋₆ alkyl, C₁₋₆ alkoxy, C₃₋₆ cycloalkyl, halogenated C₁₋₆ alkyl, and C₁₋₆ hydroxyalkyl, or any two hydrogen atoms connected to form a bond or -(CH₂)_{q}-, wherein q is 1, 2, 3, 4, or 5; R^{a}, R^{b}, and R are as described in the third aspect of the present invention.

In another preferred embodiment, the compound has the structure shown in formula 2: 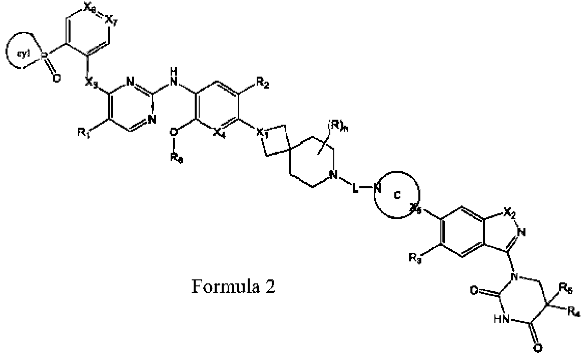 in the formula,
X₂, X₃, X₄, X₅, X₆, X₇, L, Cy1, ring C, R₁, R₂, R₃, R₄, R₅, and R₆ are as defined in the third aspect of the present invention.

In another preferred embodiment, the compound has the structure shown in Formula 2: 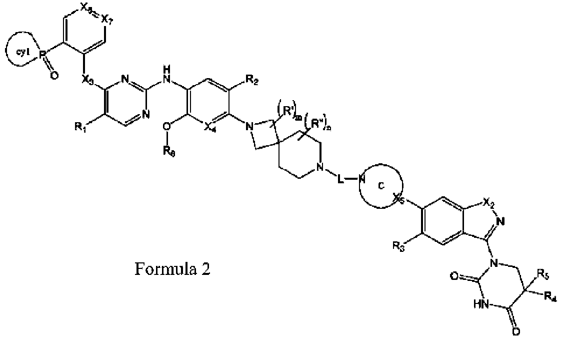 in the formula,
R' and R" are each independently selected from the group consisting of D, halogen, oxo (=O), cyano, C₁₋₆ alkyl, C₁₋₆ alkoxy, C₃₋₆ cycloalkyl, halogenated C₁₋₆ alkyl, and C₁₋₆ hydroxyalkyl;
or any two R' or two R" connected to form a bond or a -(CH₂)_{q}-, wherein q is 1, 2, 3, 4, or 5;
or two R' or two R" attached to the same carbon atom with the carbon atom to which they are attached together form a 3-6-membered saturated carbon ring;
m and n are each independently selected from the group consisting of 0, 1, 2, and 3;
X₂, X₃, X₄, X₅, X₆, X₇, L, Cy1, Ring C, R₁, R₂, R₃, R₄, R₅, and R₆ are as defined in the third aspect of the present invention.

In another preferred embodiment, 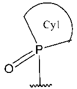 is selected from the group consisting of

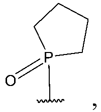

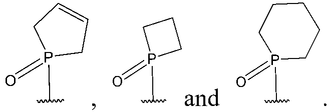

In another preferred embodiment, X₁ is N.

In another preferred embodiment, X₅ is CH.

In another preferred embodiment, the compound has the structure shown in Formula 3: 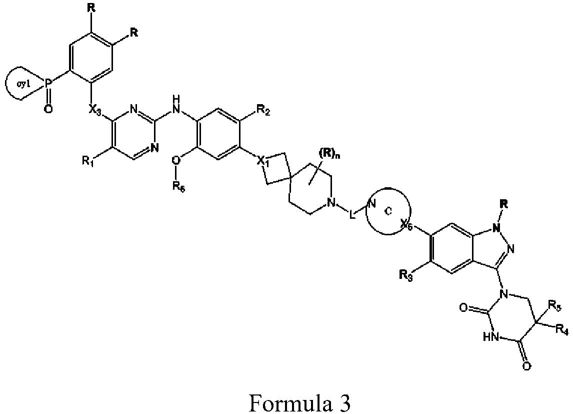 in the formula,
X₁, X₃, X₅, L, Cy1, ring C, R₁, R₂, R₃, R₄, R₅, R₆, and R are as defined in the third aspect of the present invention.

In another preferred embodiment, the compound has the structure shown in Formula 4: 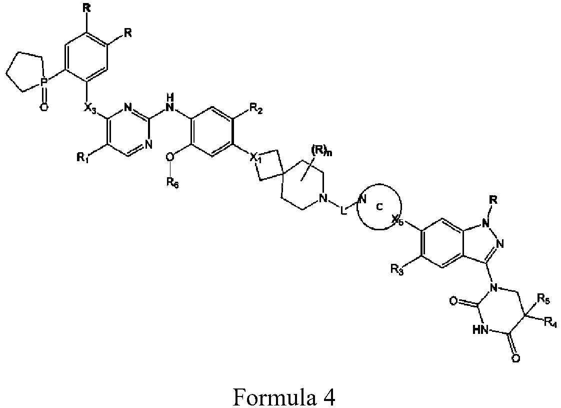 in the formula,
X₃, X₅, L, ring C, R₁, R₂, R₃, R₄, R₅, R₆, and R are as defined in the third aspect of the present invention.

In another preferred embodiment, ring C 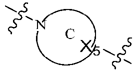 is substituted or unsubstituted groups selected from the group consisting of 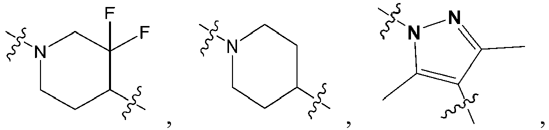 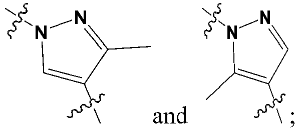 wherein, the "substituted" refers to one or more hydrogen atoms of the group being substituted by substituents selected from the group consisting of H, halogen, amino, oxo (=O), nitro, hydroxy, cyano, C₁₋₆ alkyl, C₁₋₆ alkoxy, and C₃₋₆ cycloalkyl, halogenated C₁₋₆ alkyl, and C₁₋₆ hydroxyalkyl, or any two hydrogen atoms connected to form a bond or -(CH₂)_{q}-, wherein q is 1, 2, 3, 4, or 5; R^{a}, R^{b}, and R are as described in the third aspect of the present invention.

In another preferred embodiment, X₂ is selected from NR, O, or S; R is selected from the group consisting of CH₃, phenyl, isopropyl, and cyclopropyl.

In another preferred embodiment, R₄ and R₅ are each independently selected from the group consisting of H.

In another preferred embodiment, L is selected from the group consisting of ethylene, and

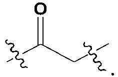

In another preferred embodiment, X₄ is selected from CH.

In another preferred embodiment, R₂ is selected from ethyl.

In another preferred embodiment, R₆ is selected from methyl.

In another preferred embodiment, R₁ is selected from Cl, or Br.

In another preferred embodiment, X₃ is selected from NH.

In another preferred embodiment, X₆ is selected from CR; R is selected from methoxy, cyclopropyl, or cyclopropoxy.

In another preferred embodiment, X₇ is selected from CH.

In another preferred embodiment, R₃ is selected from H, or F.

In the fourth aspect of the present invention, provided is a compound, or a pharmaceutically acceptable salt, a stereoisomer, a tautomer, a hydrate, a solvate, an isotope, or a prodrug thereof, wherein the compound is selected from the group consisting of:

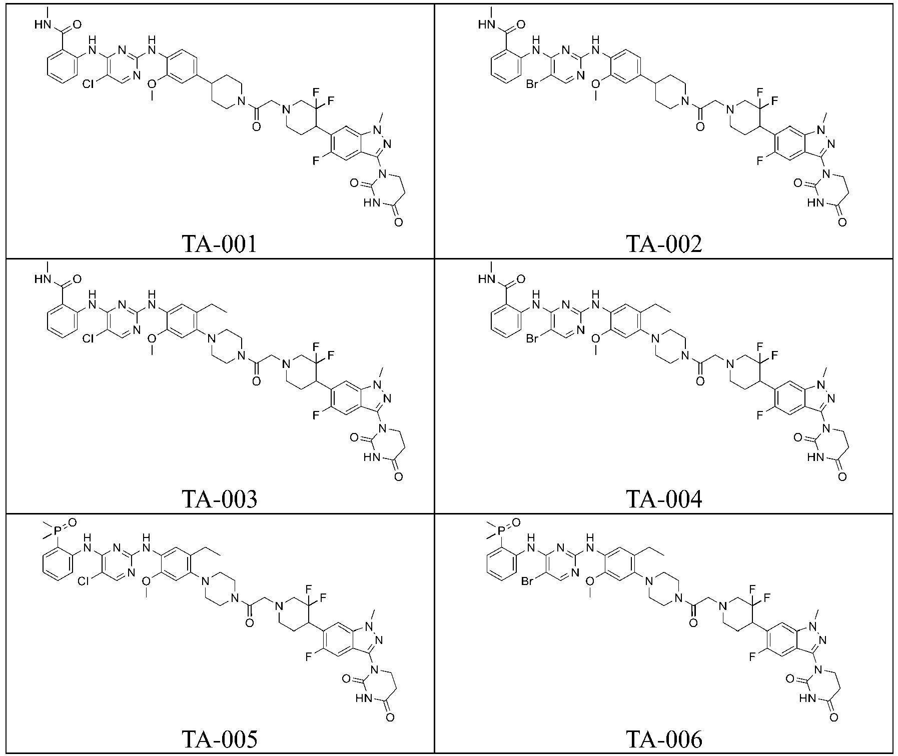

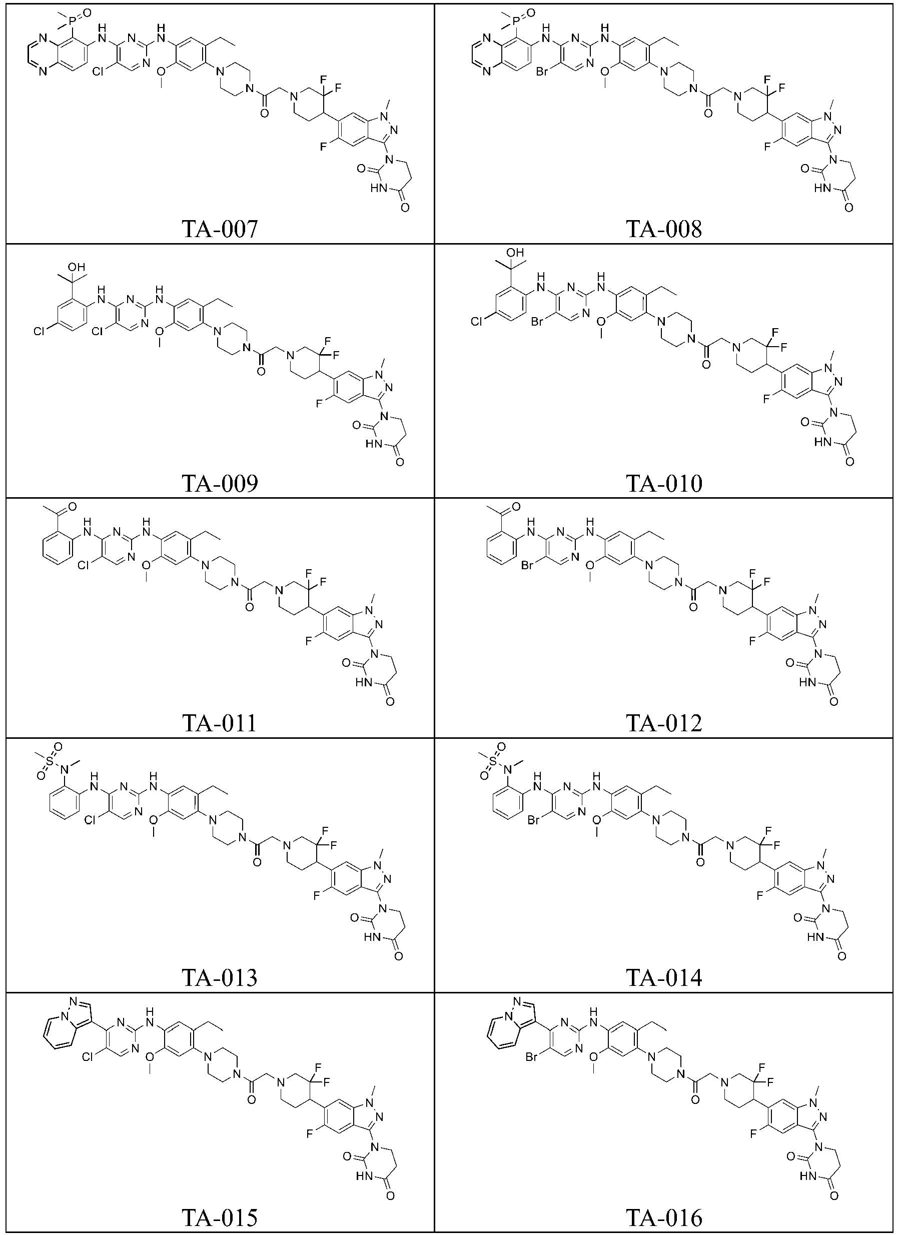

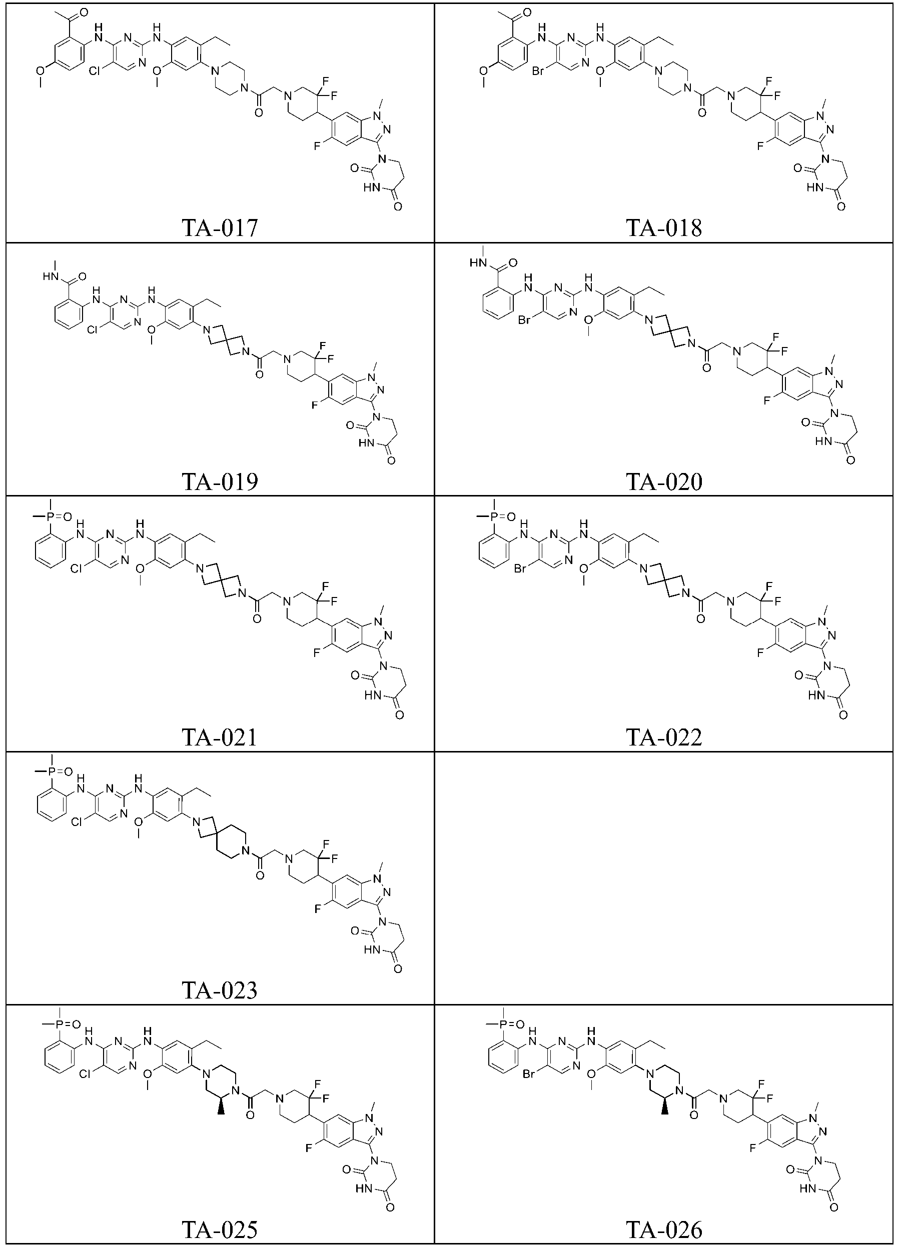

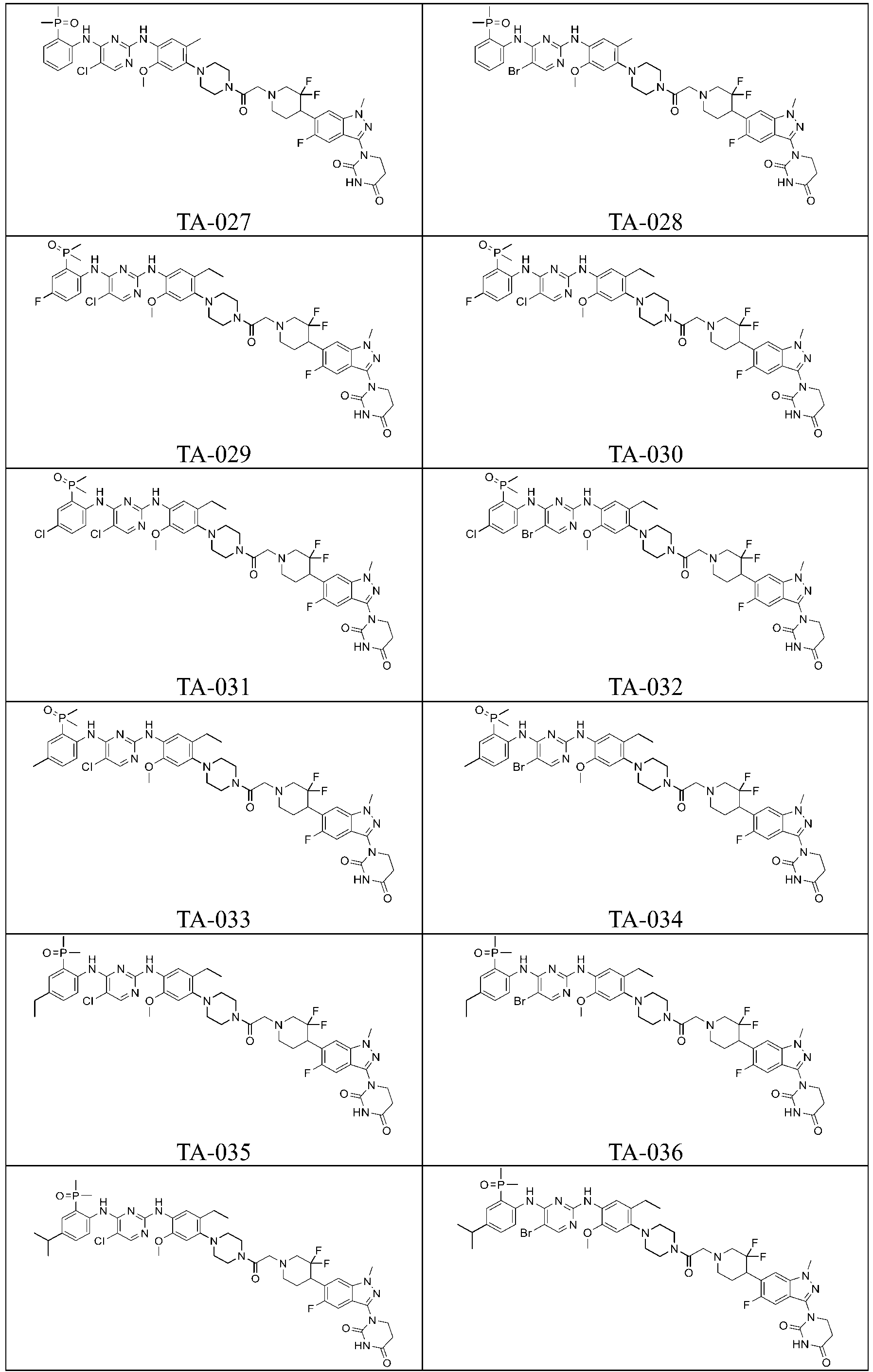

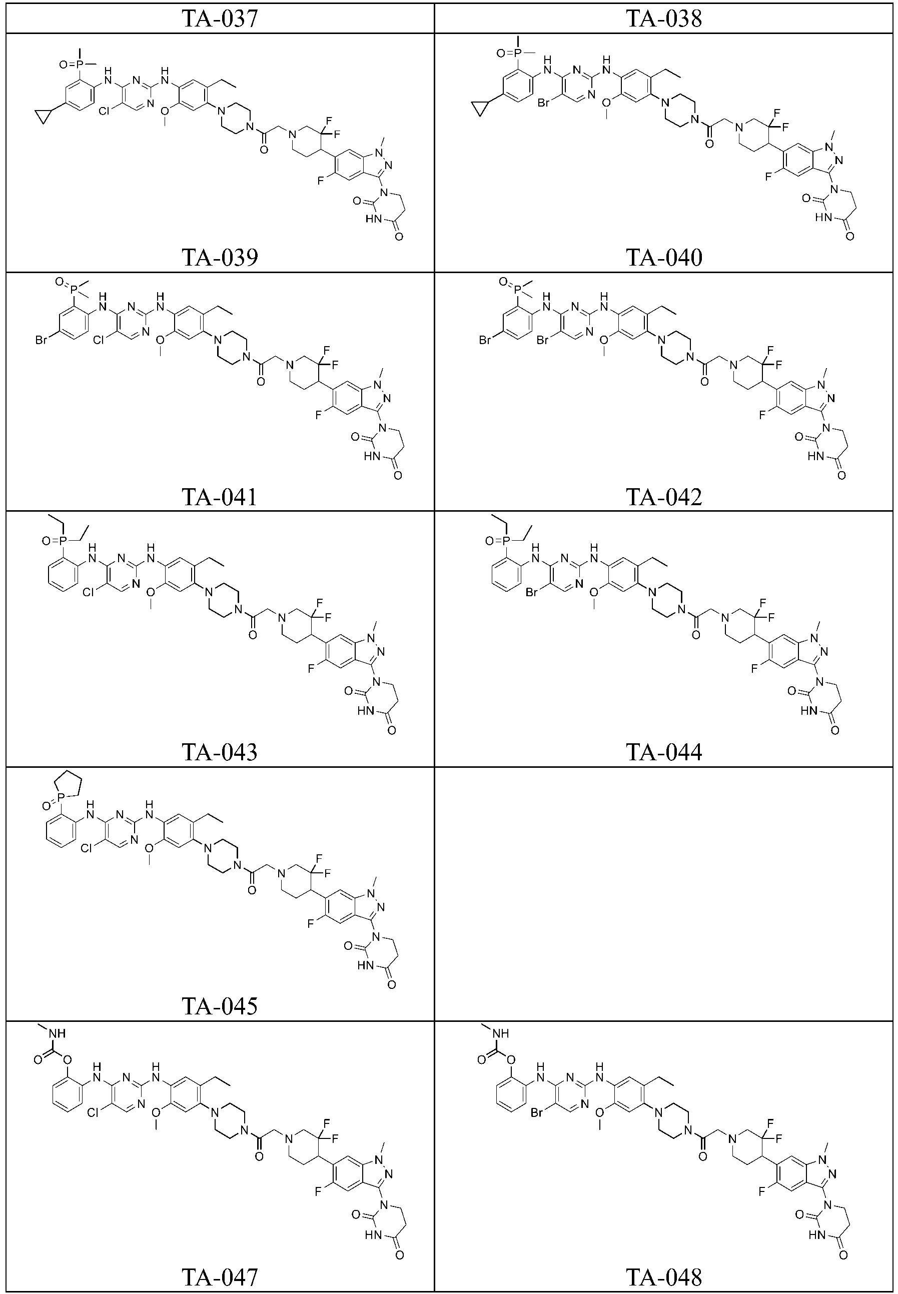

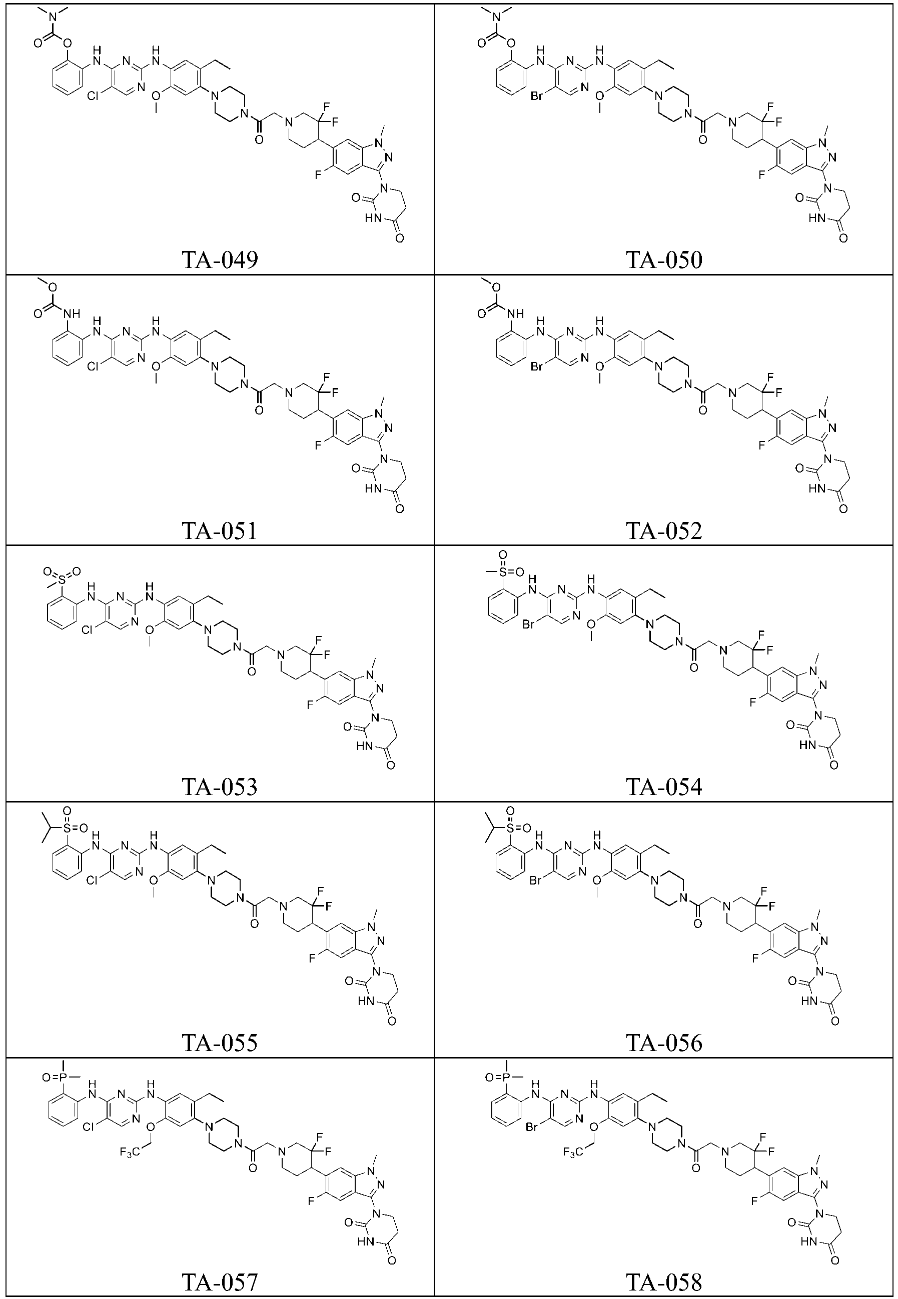

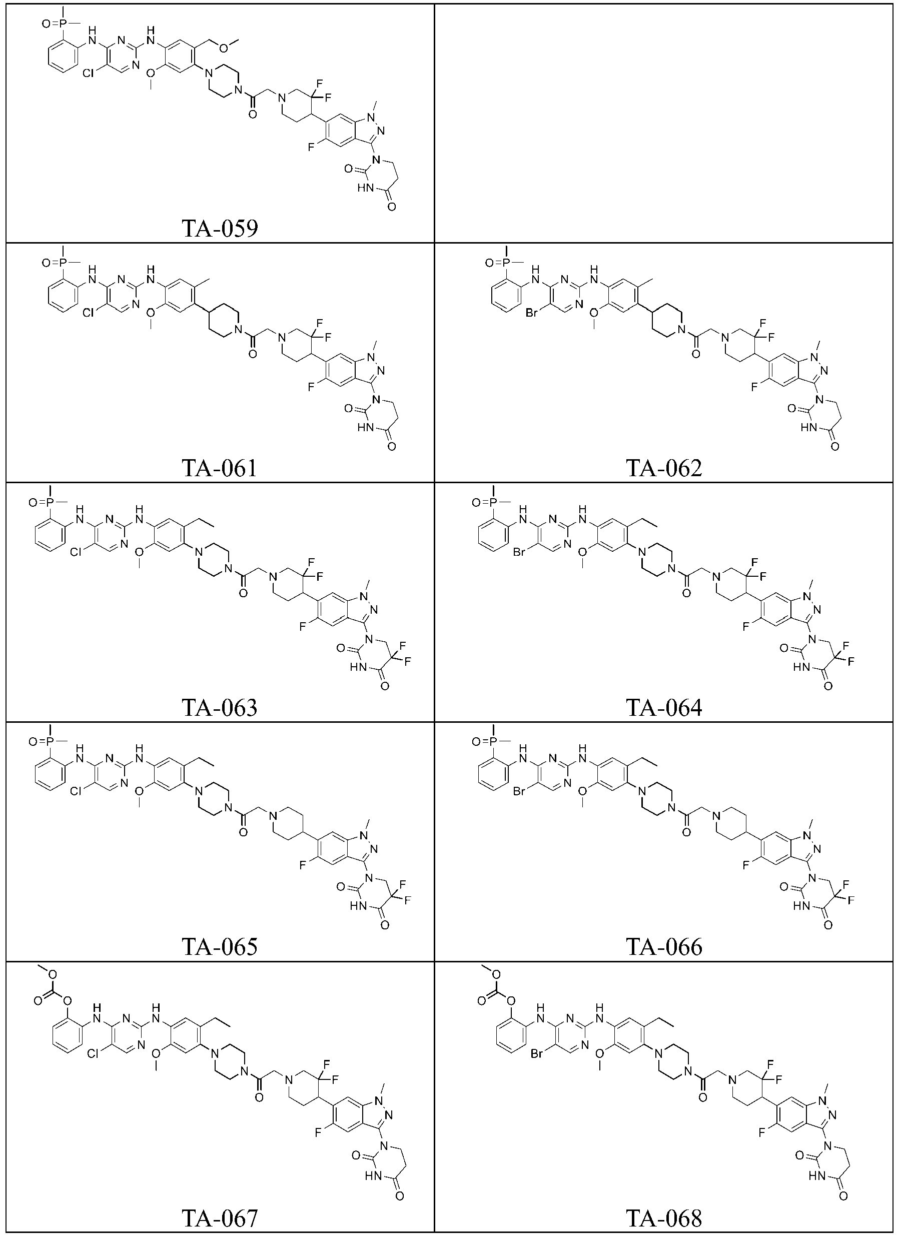

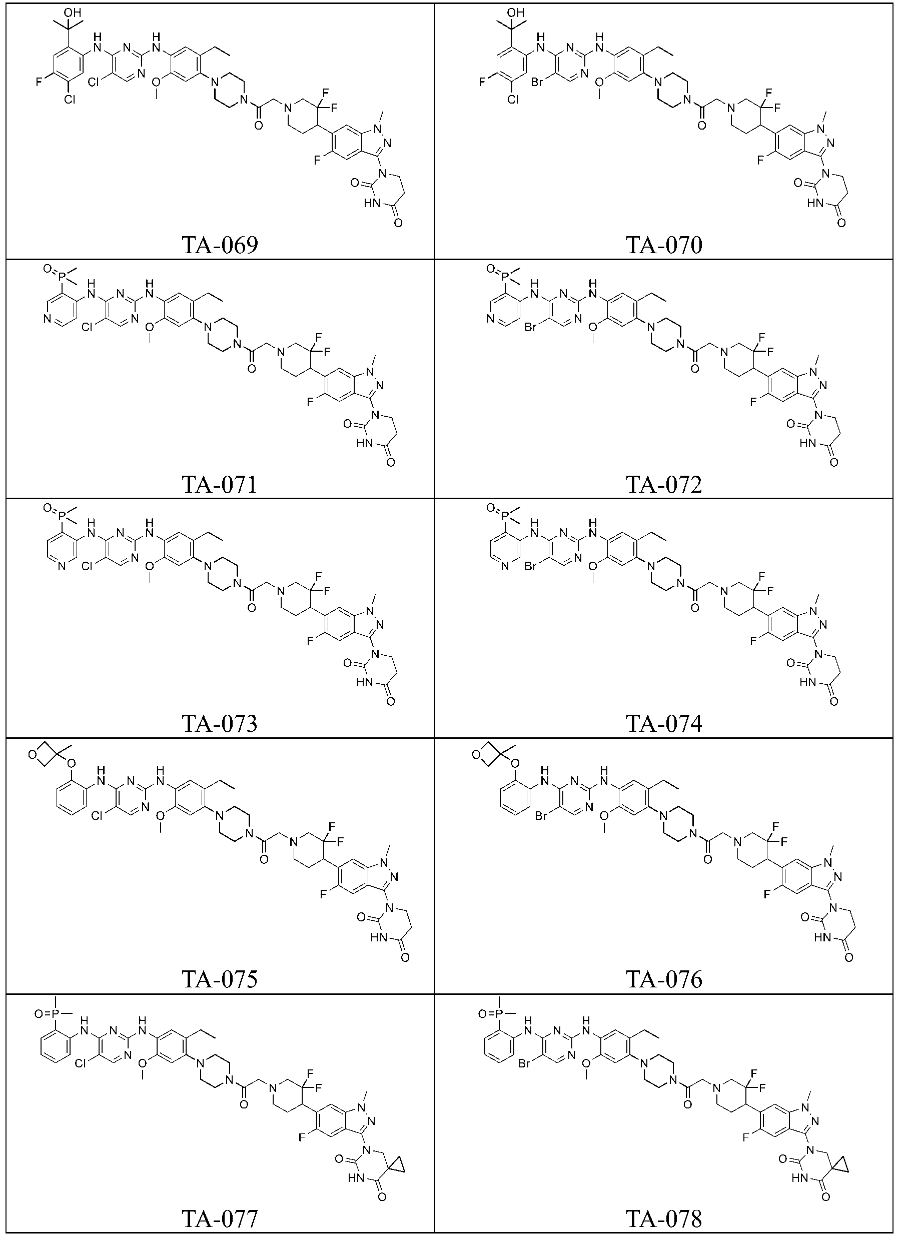

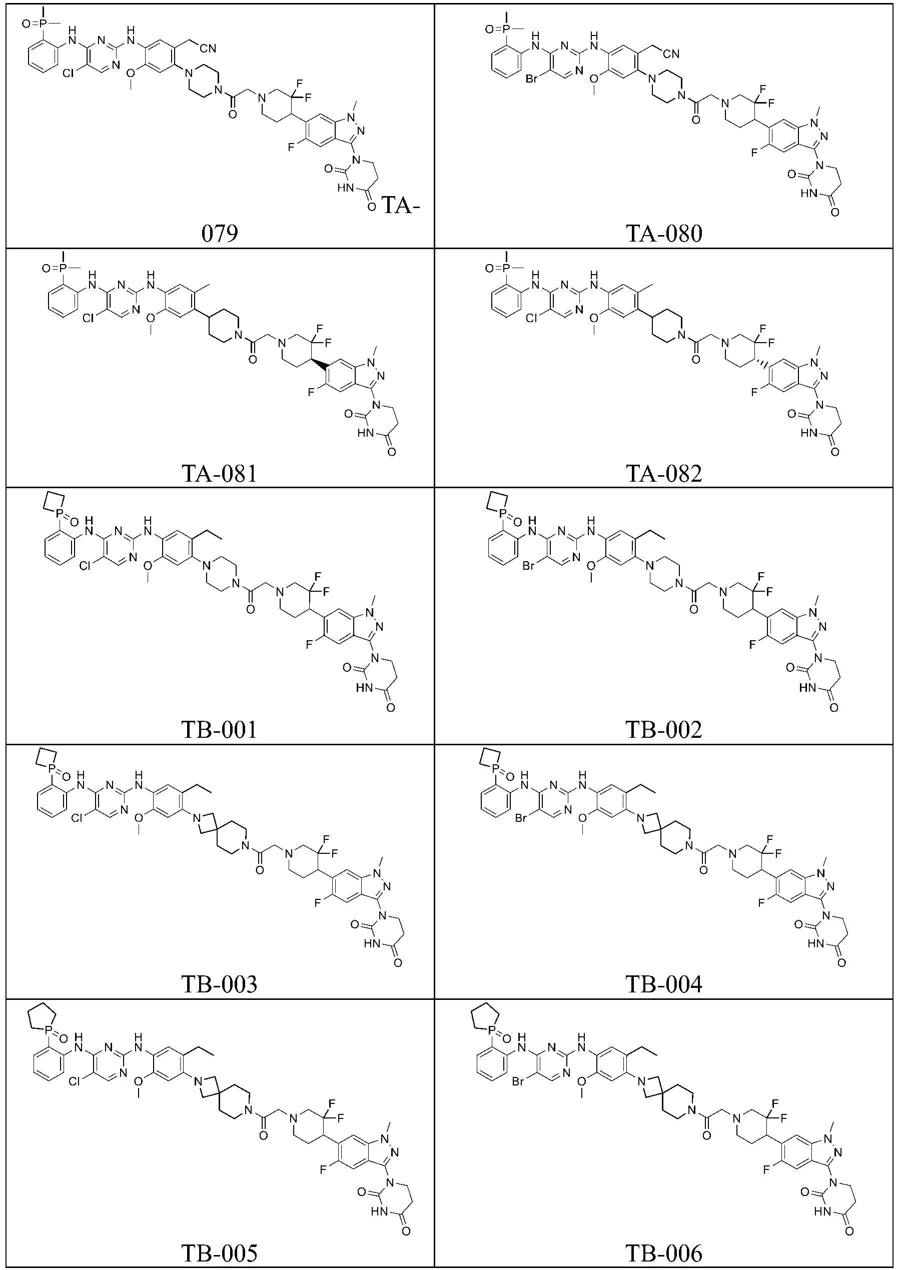

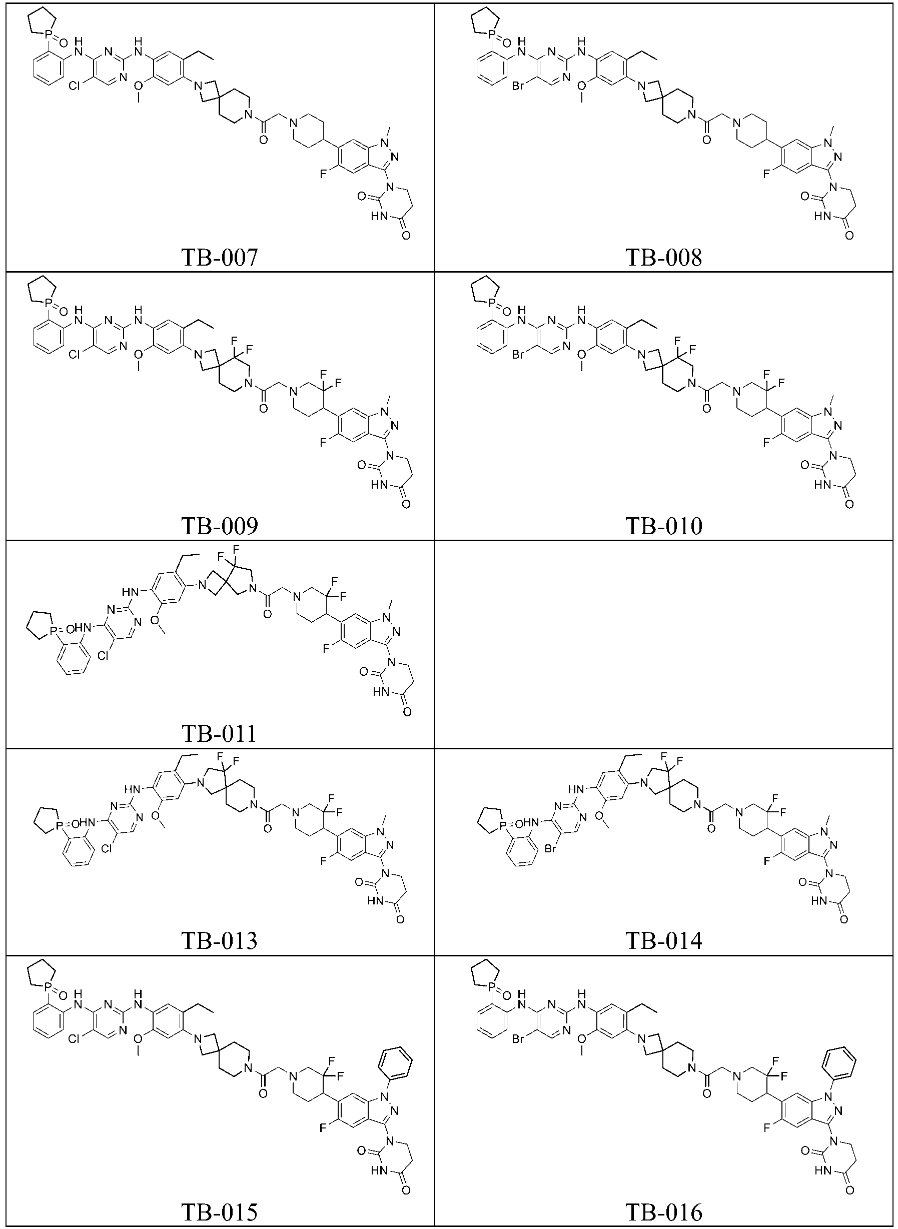

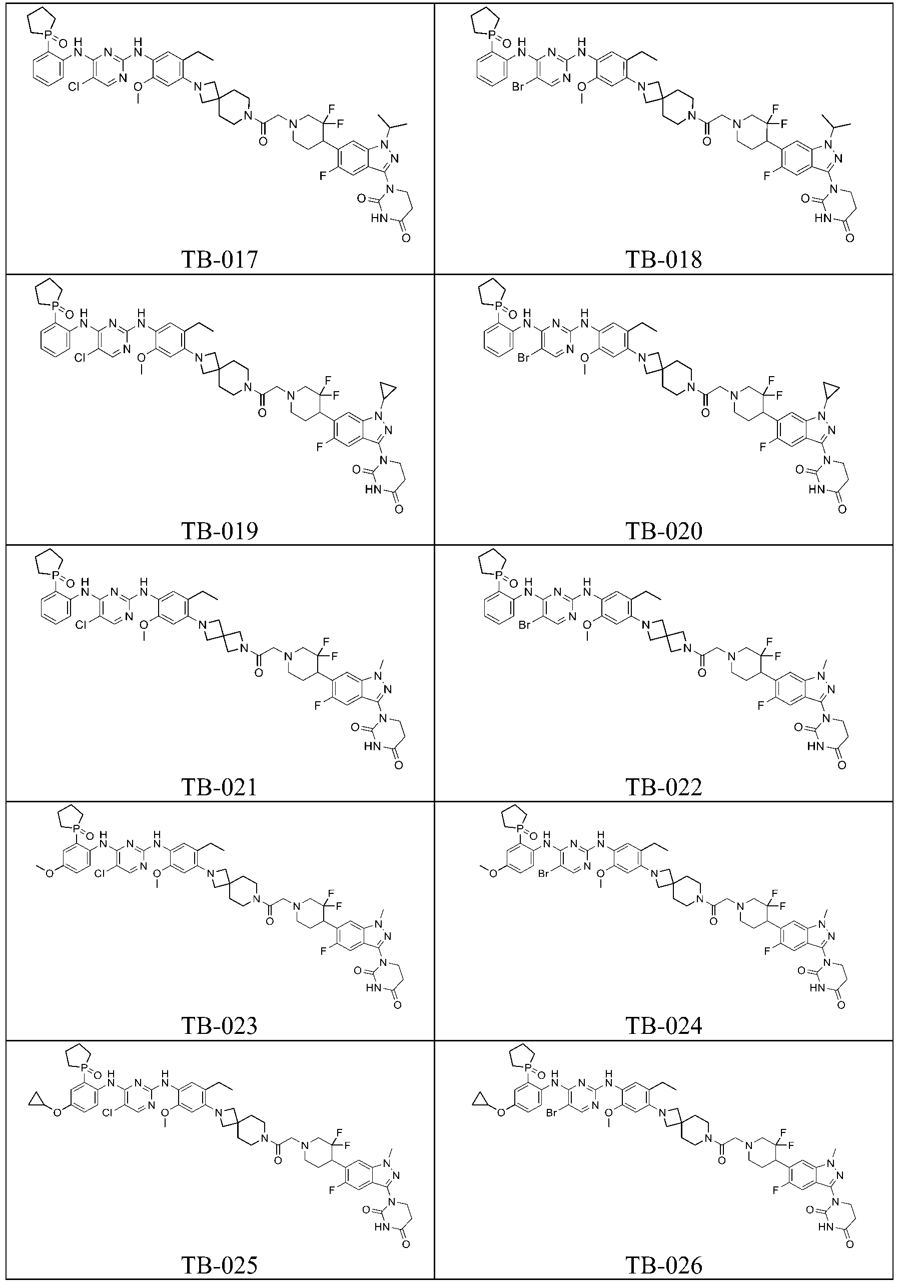

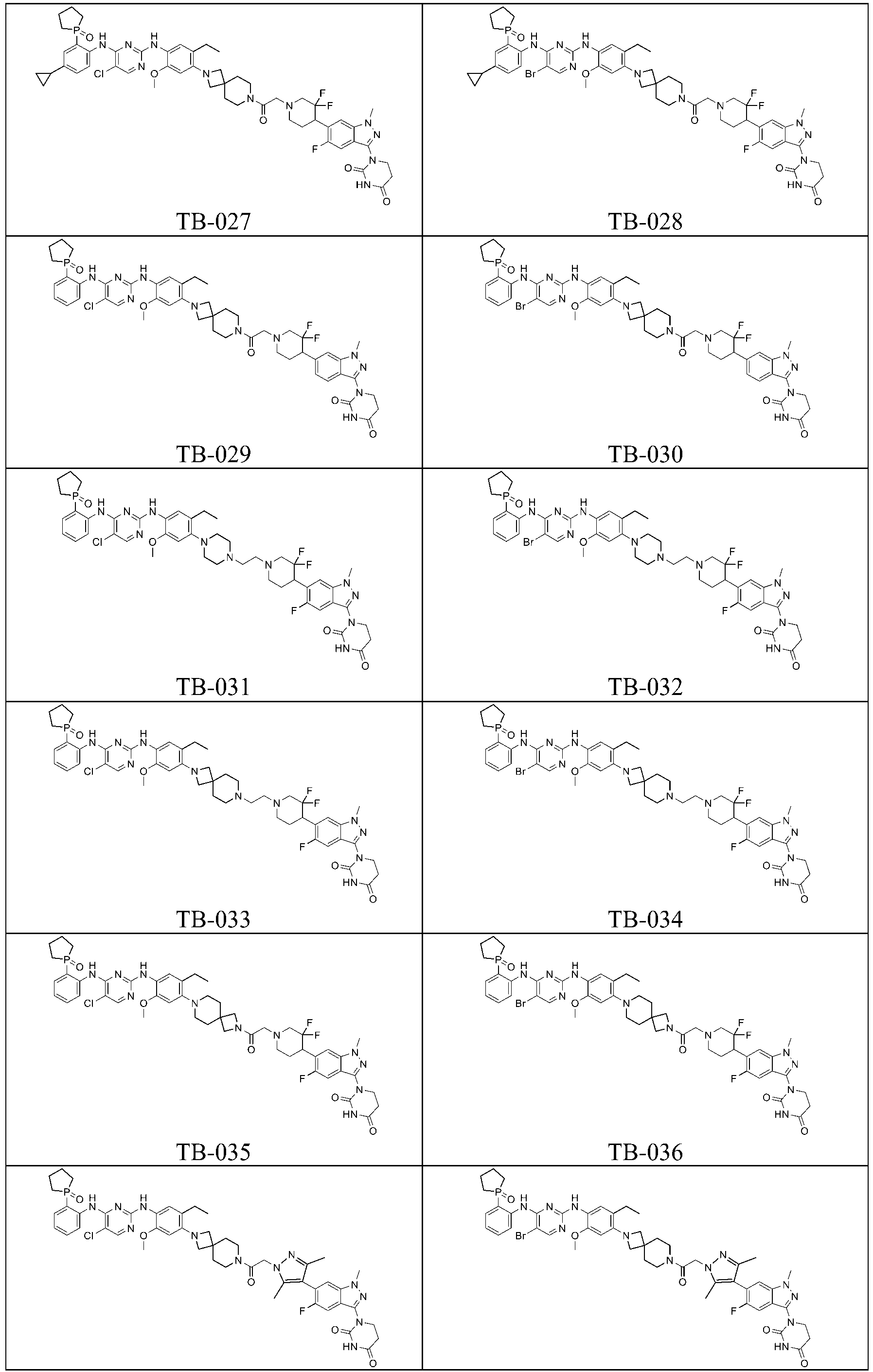

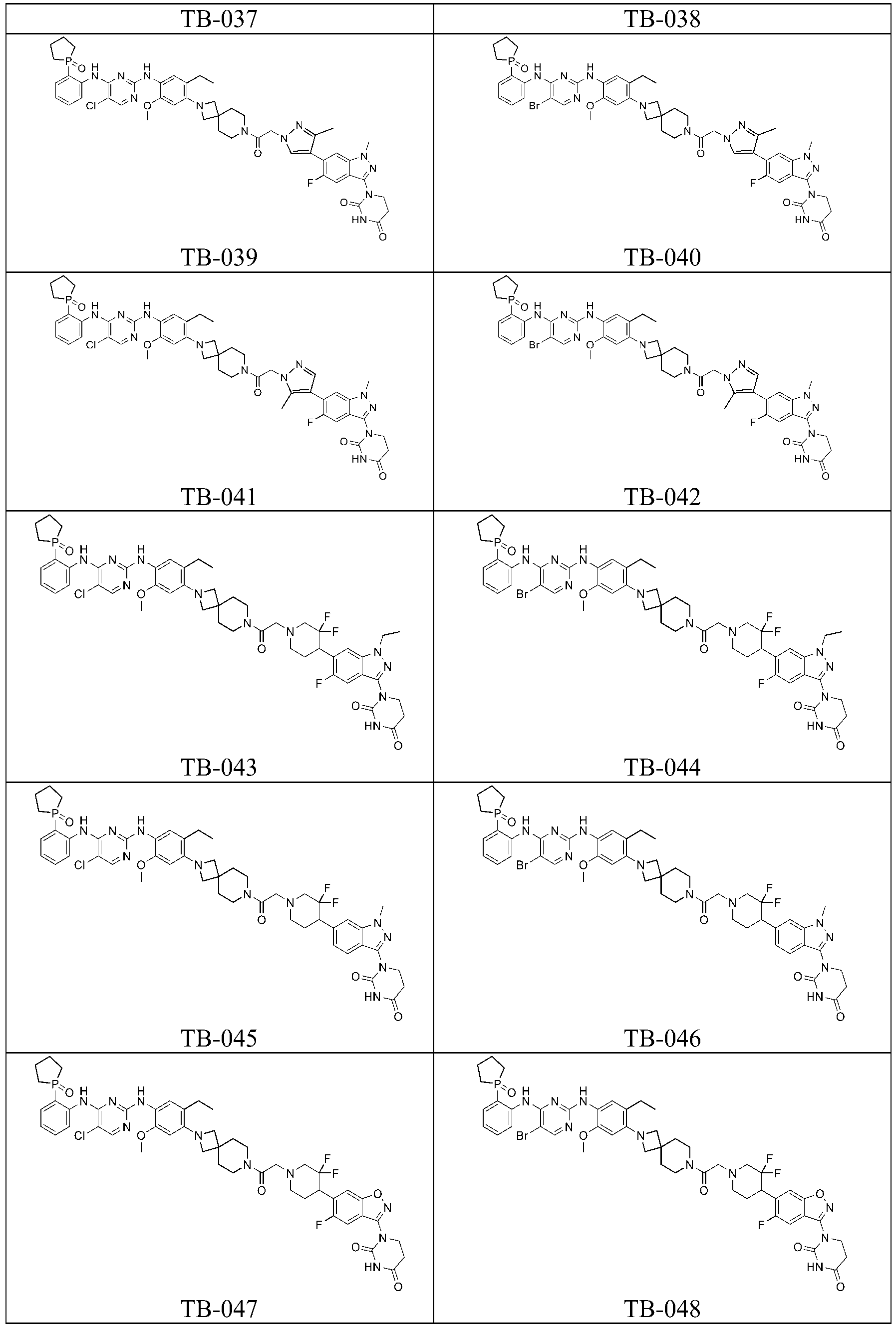

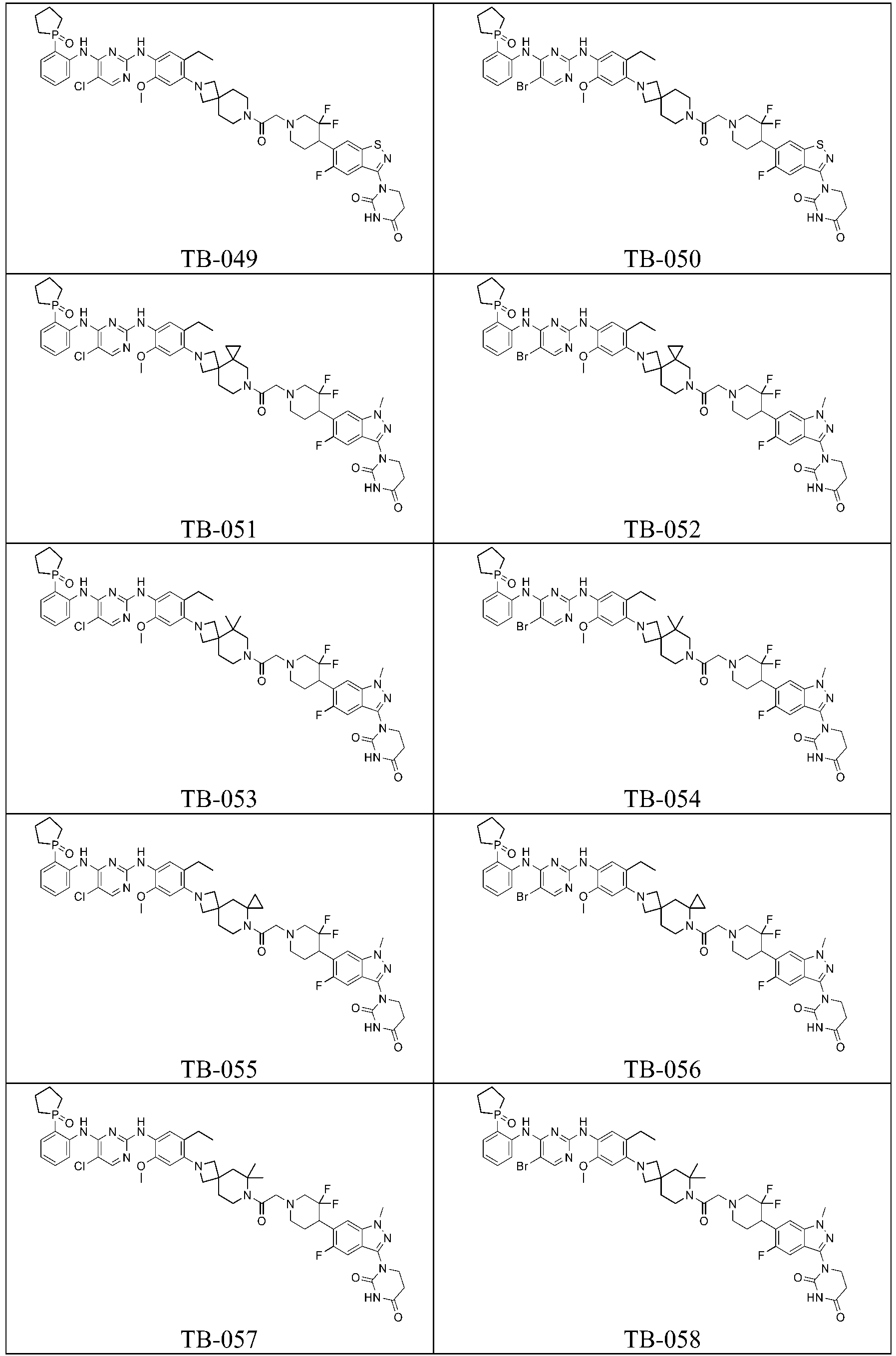

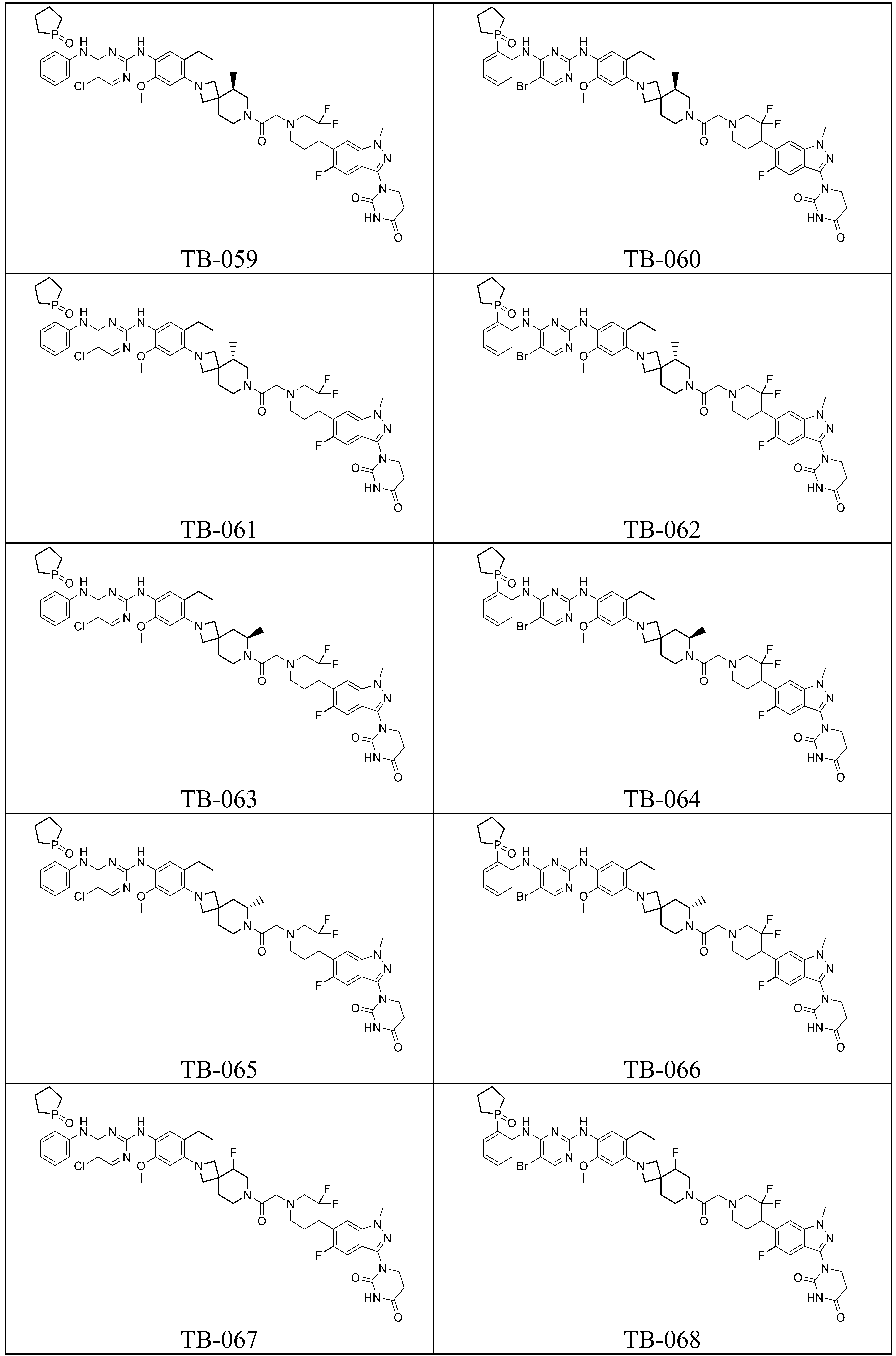

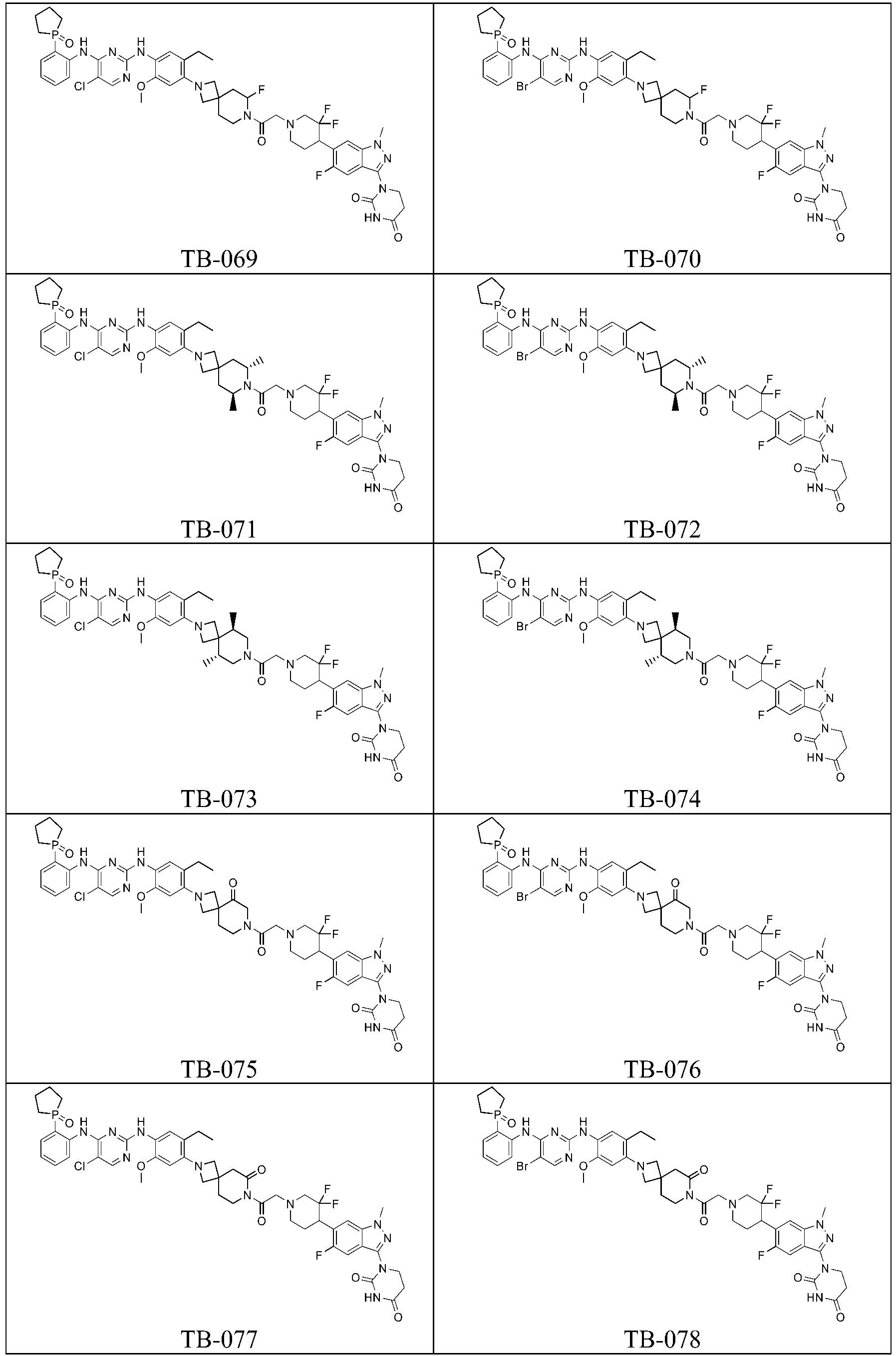

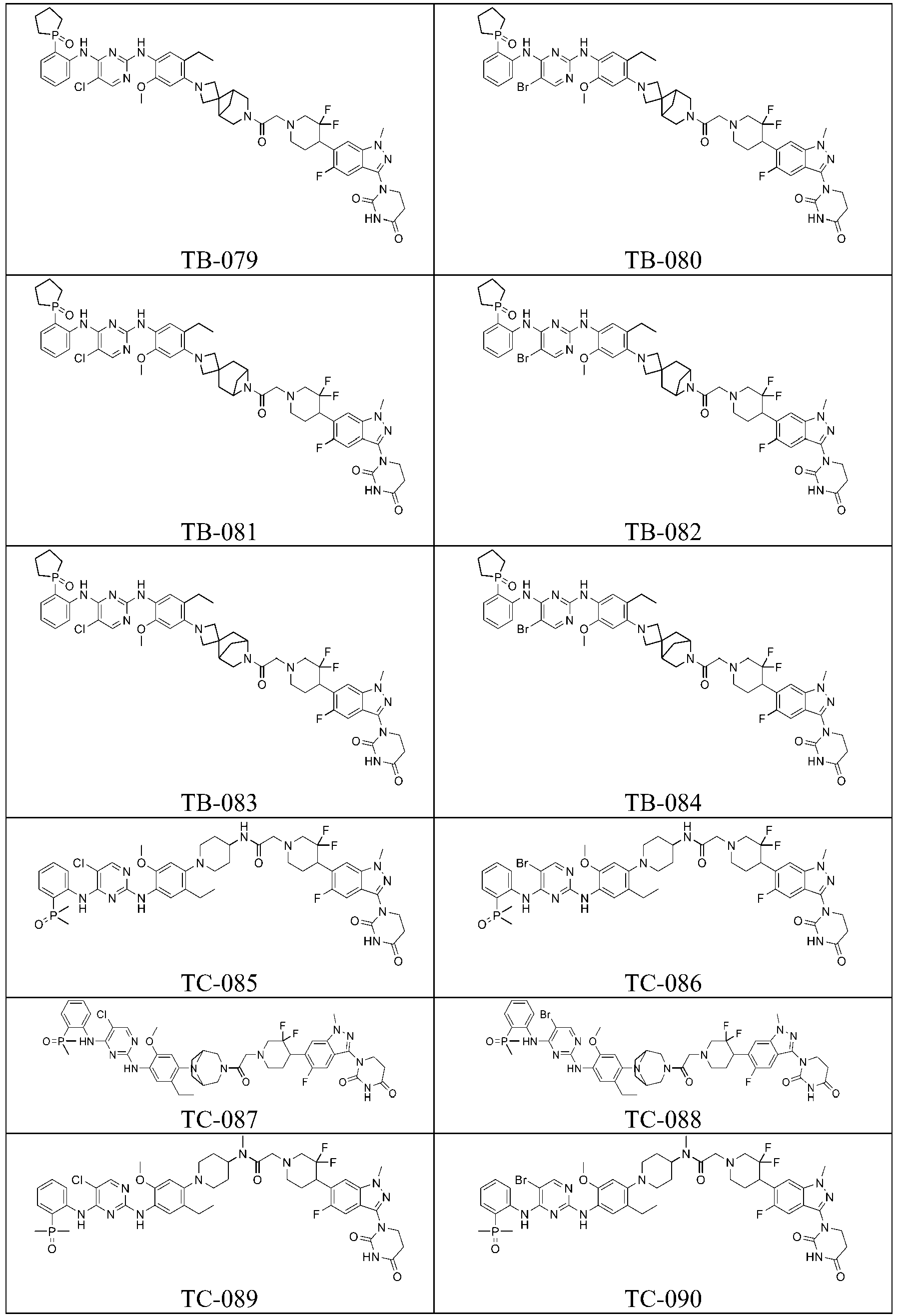

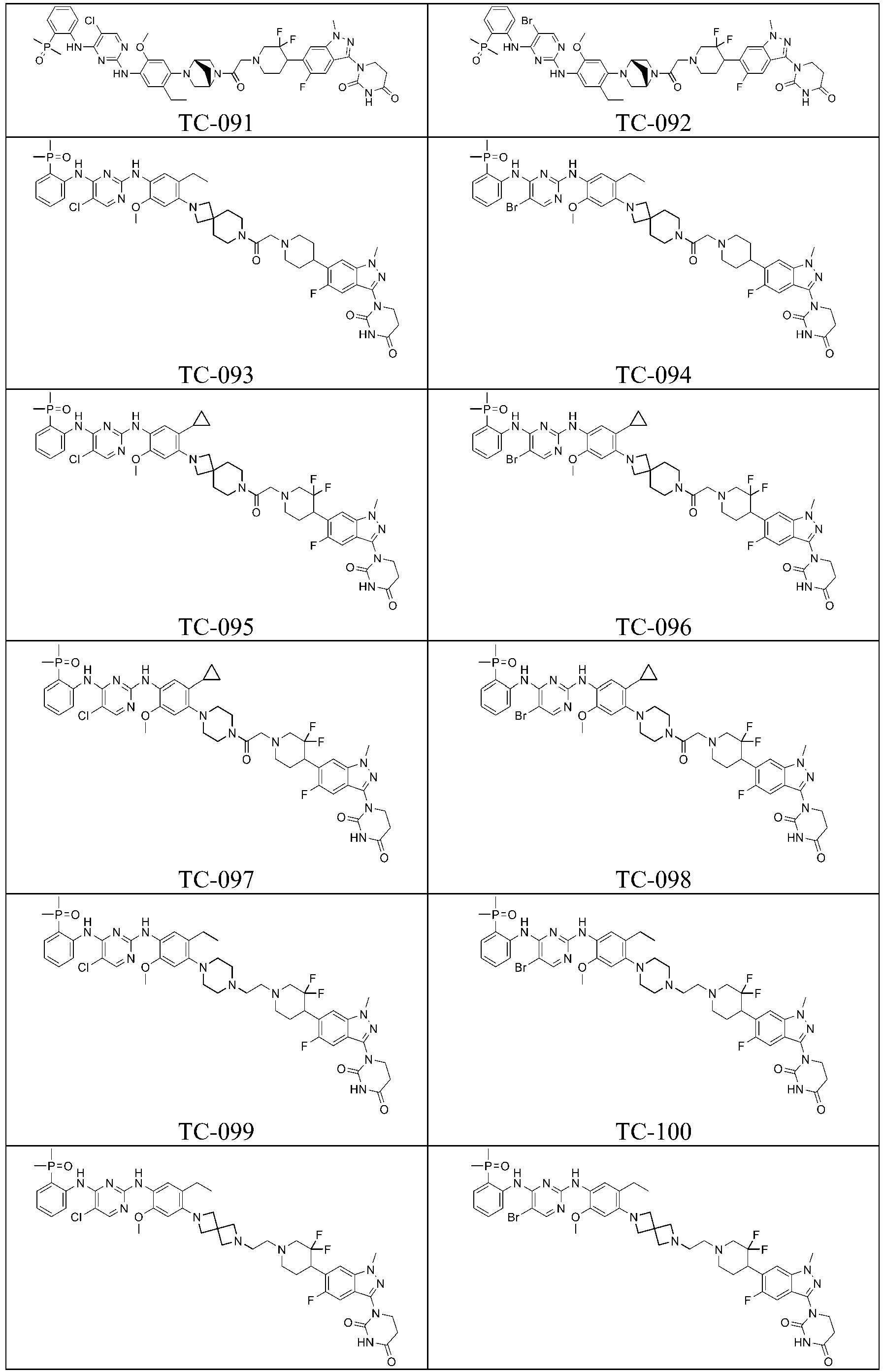

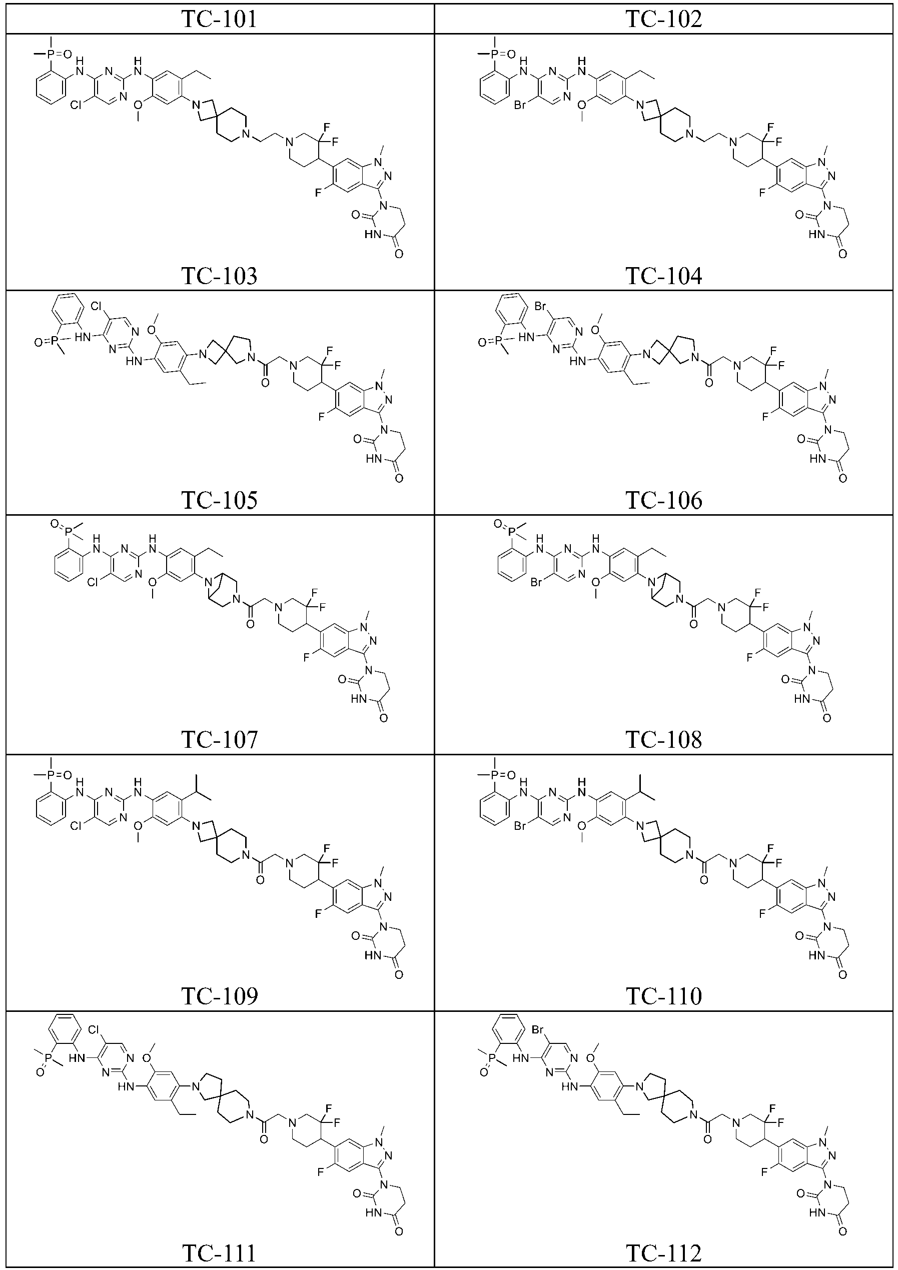

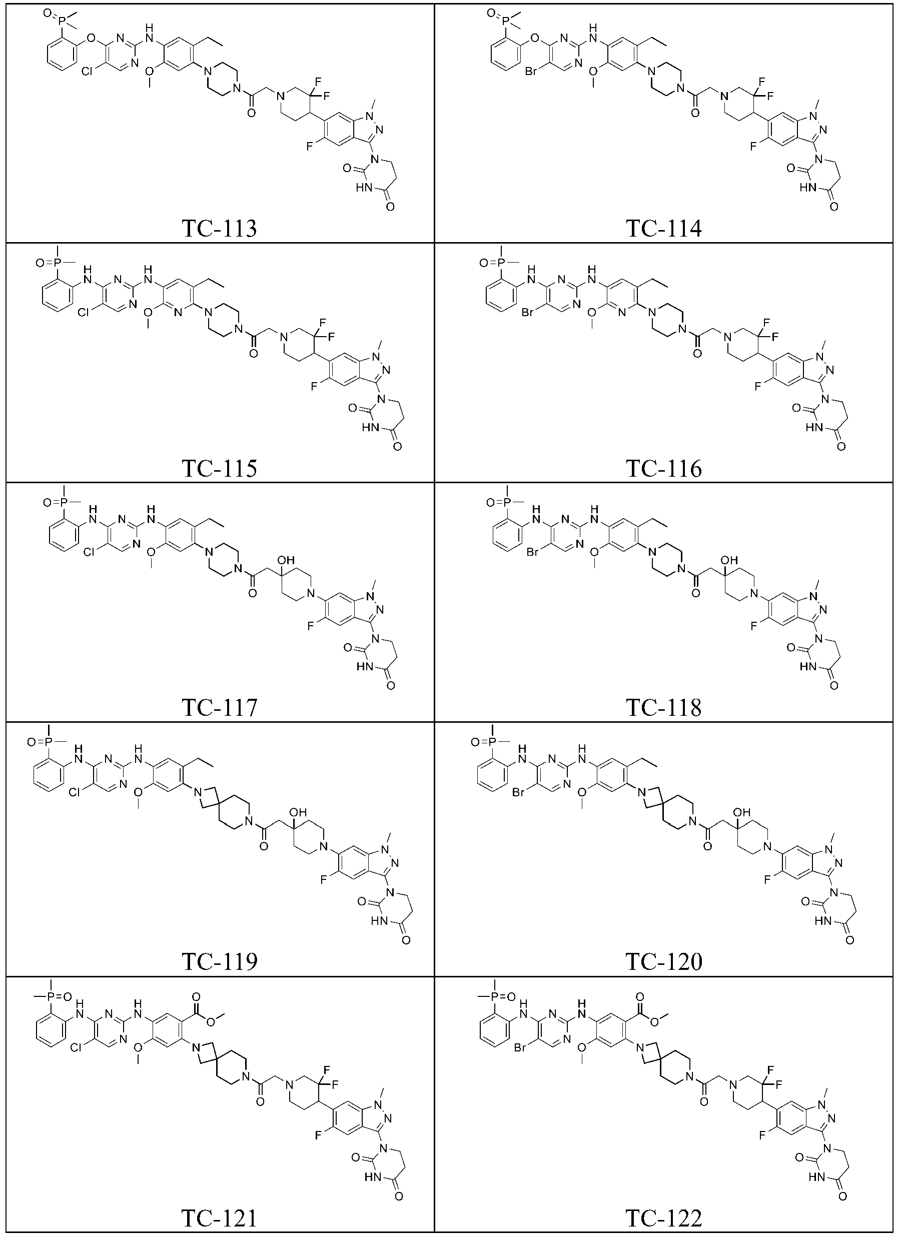

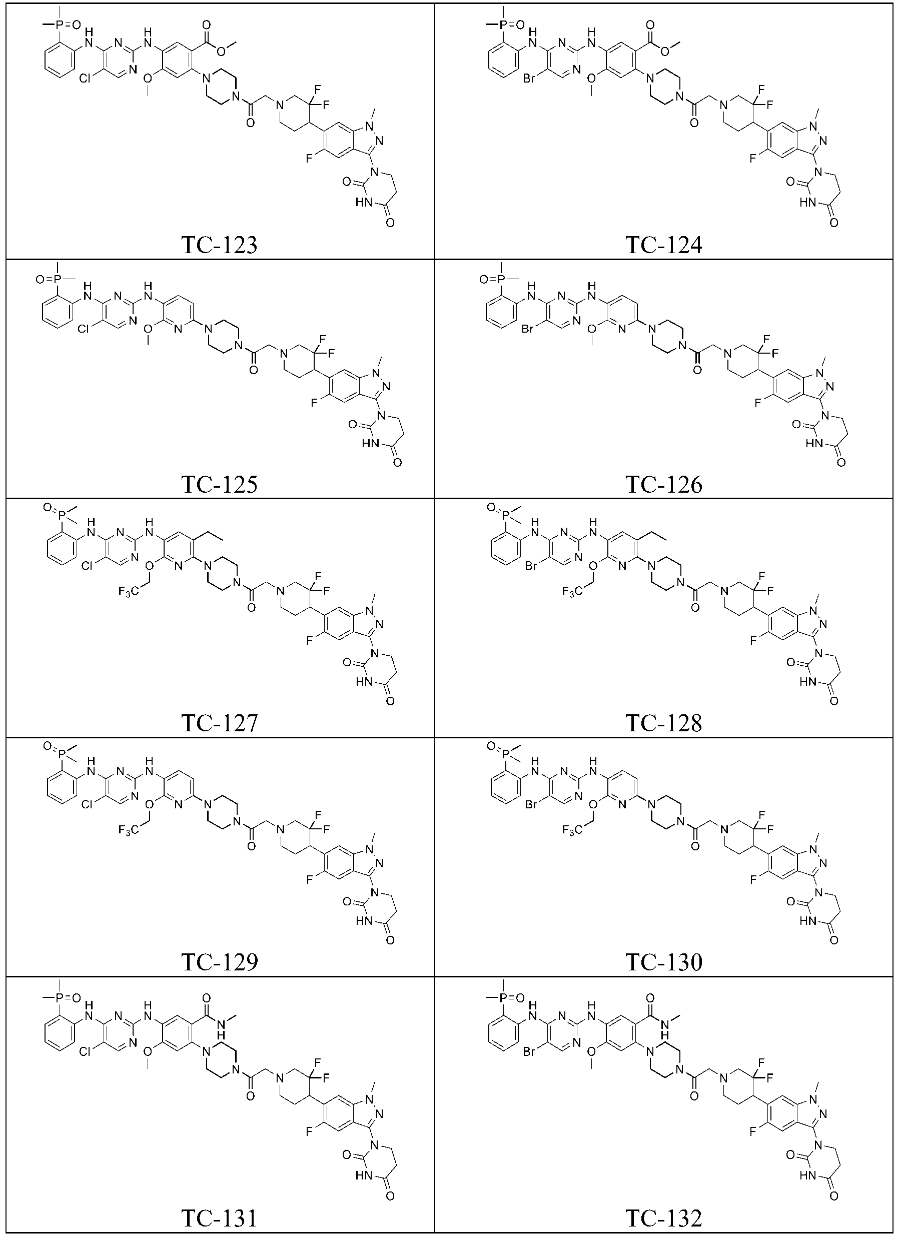

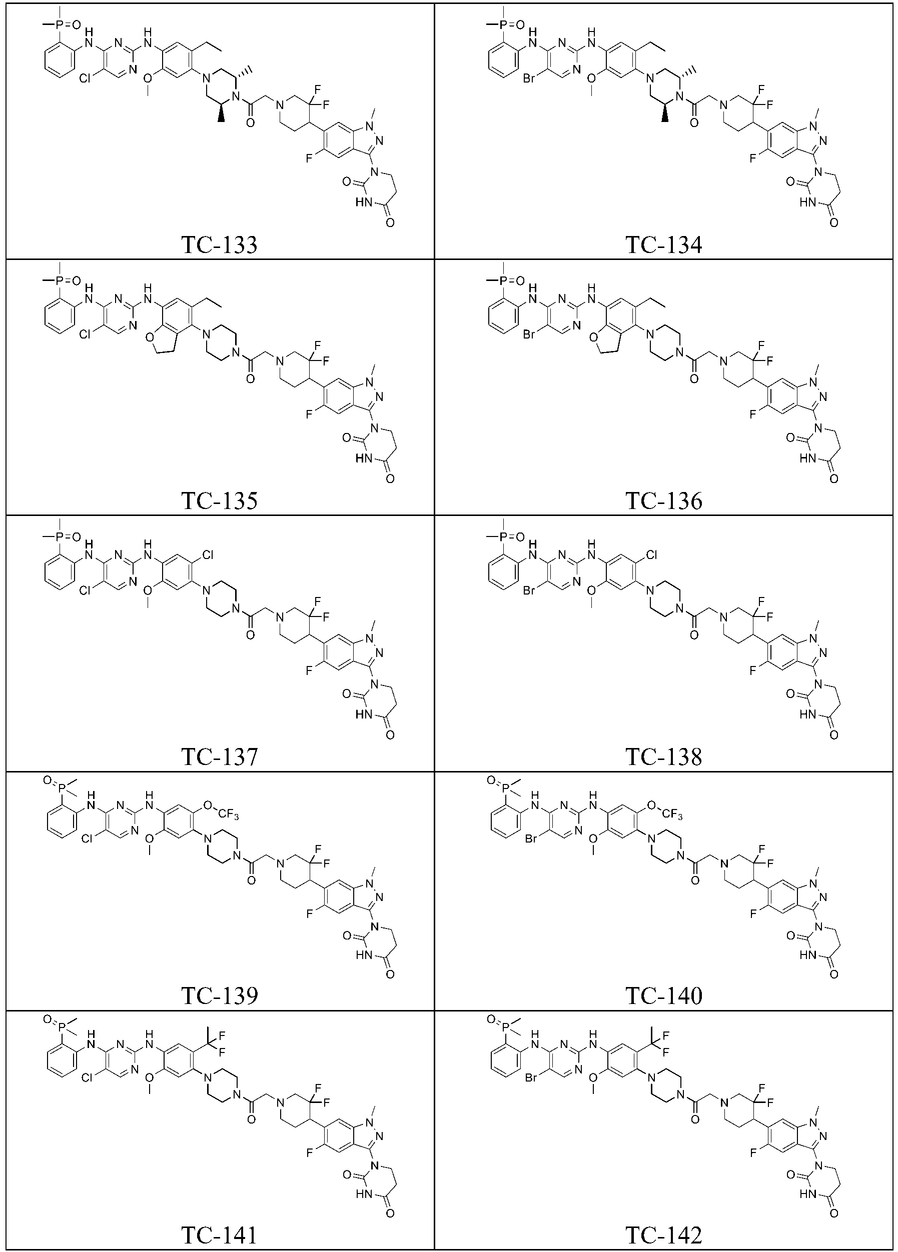

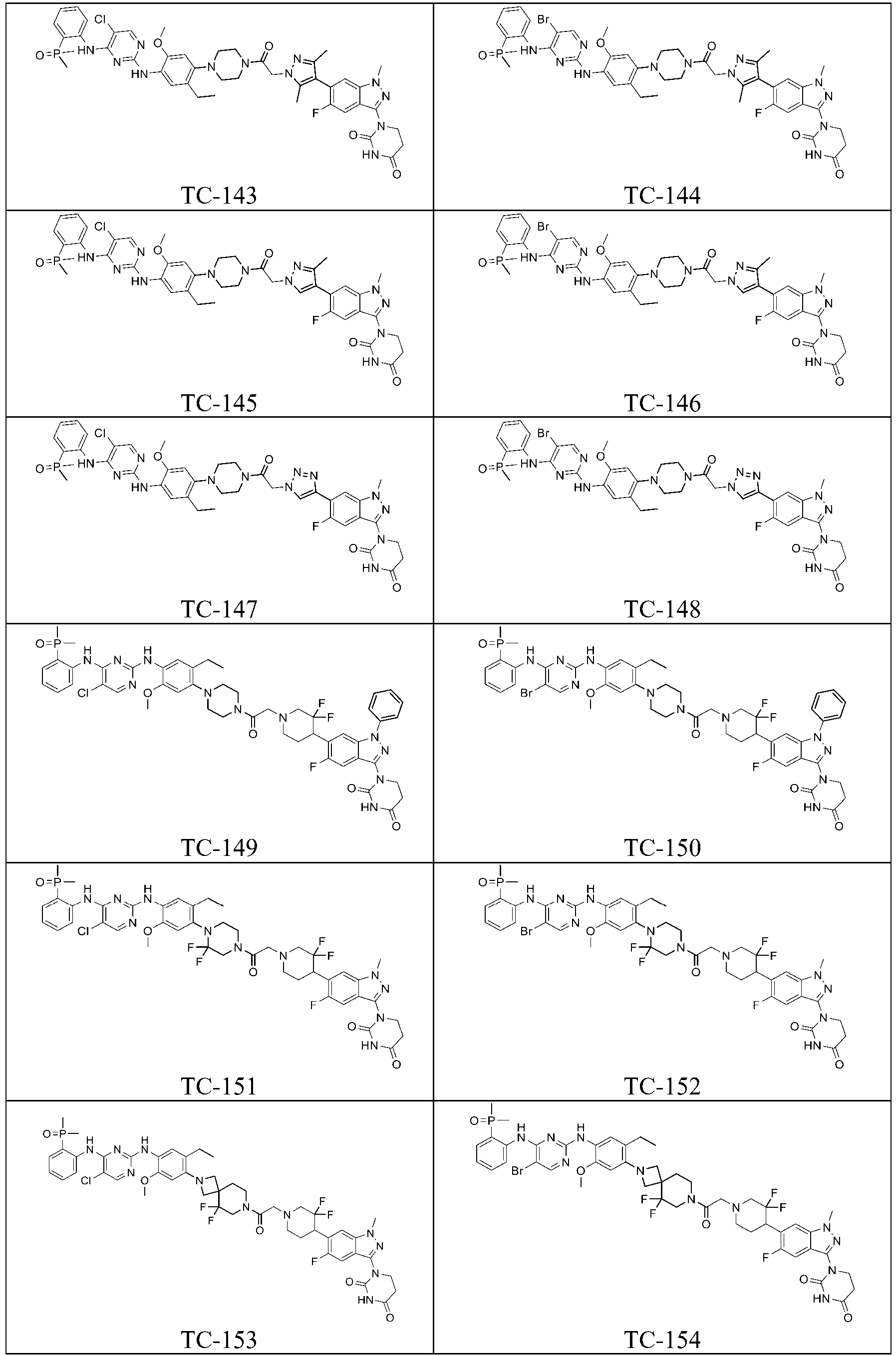

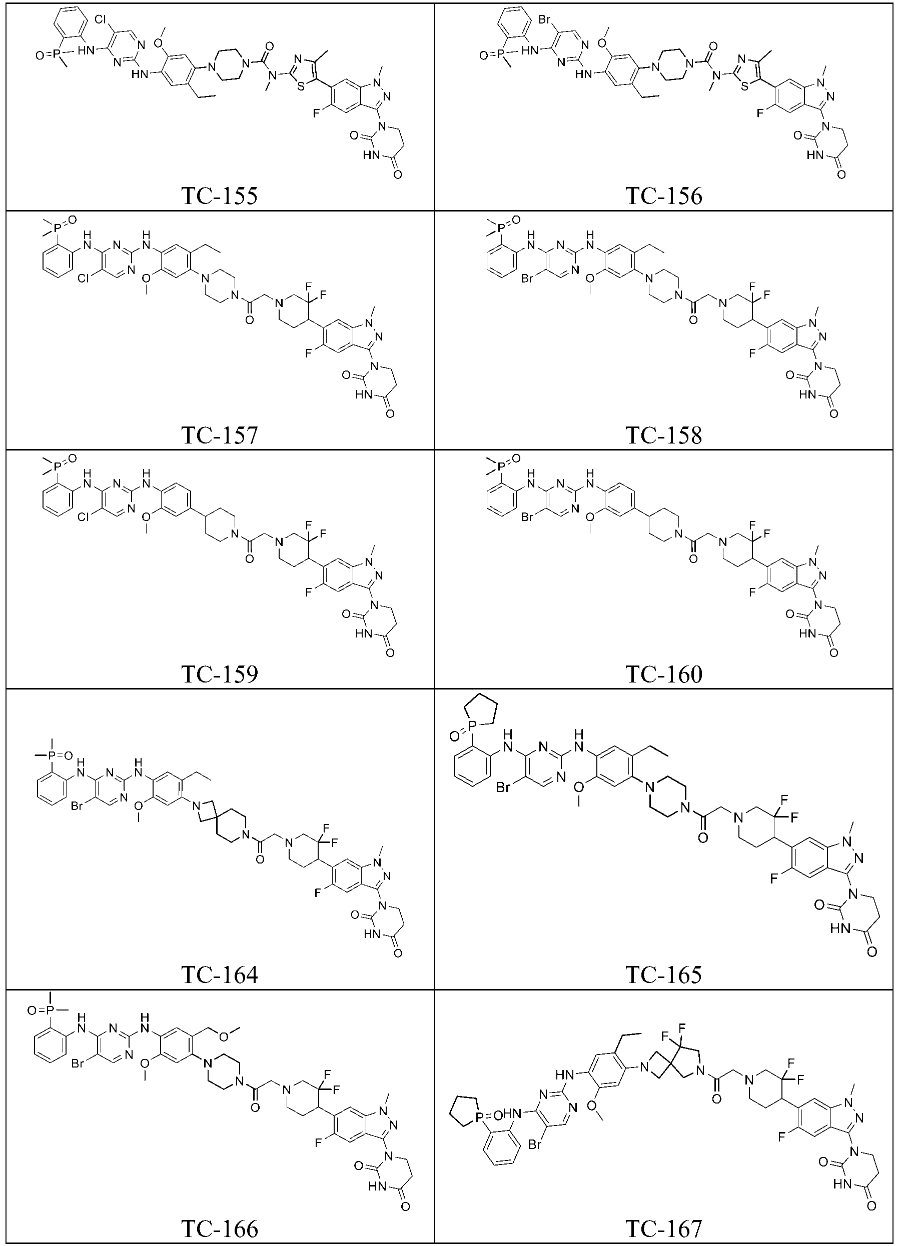

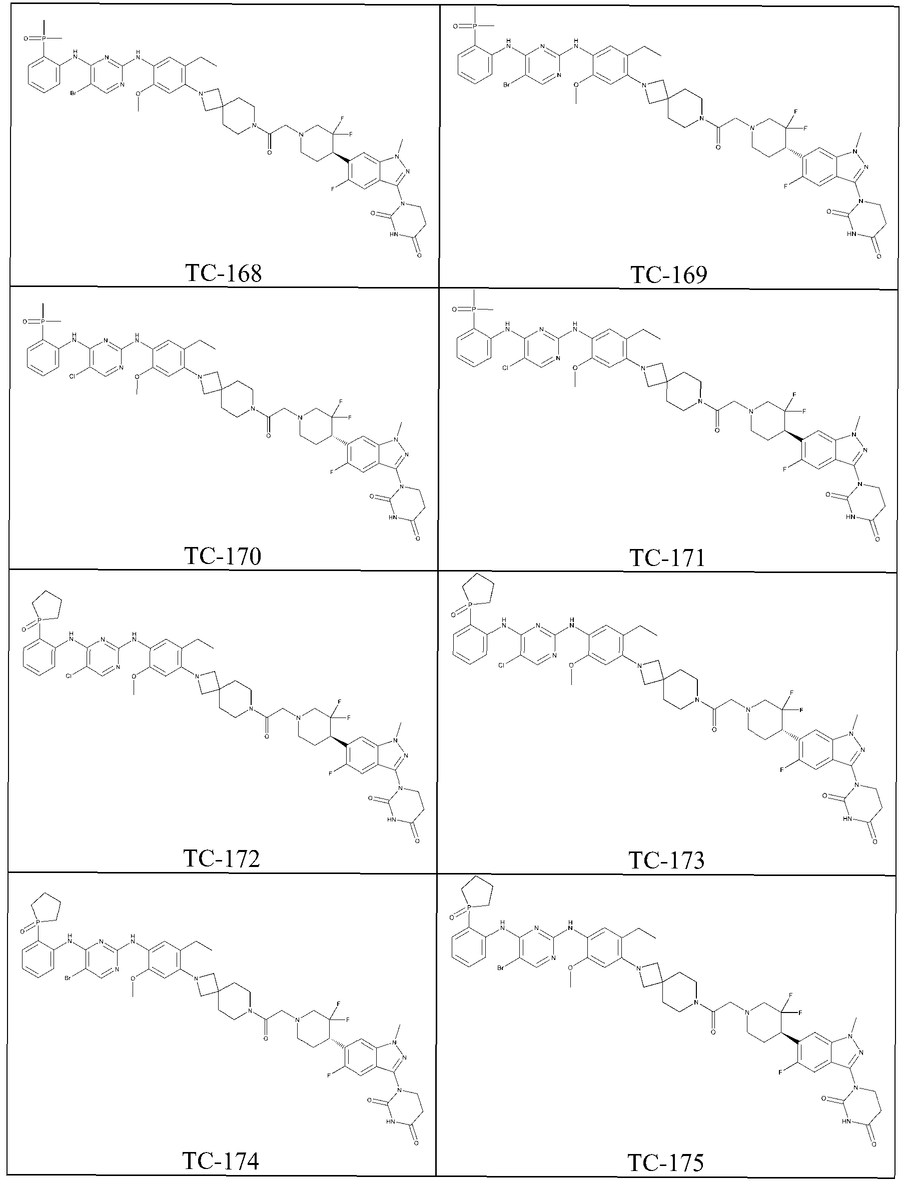

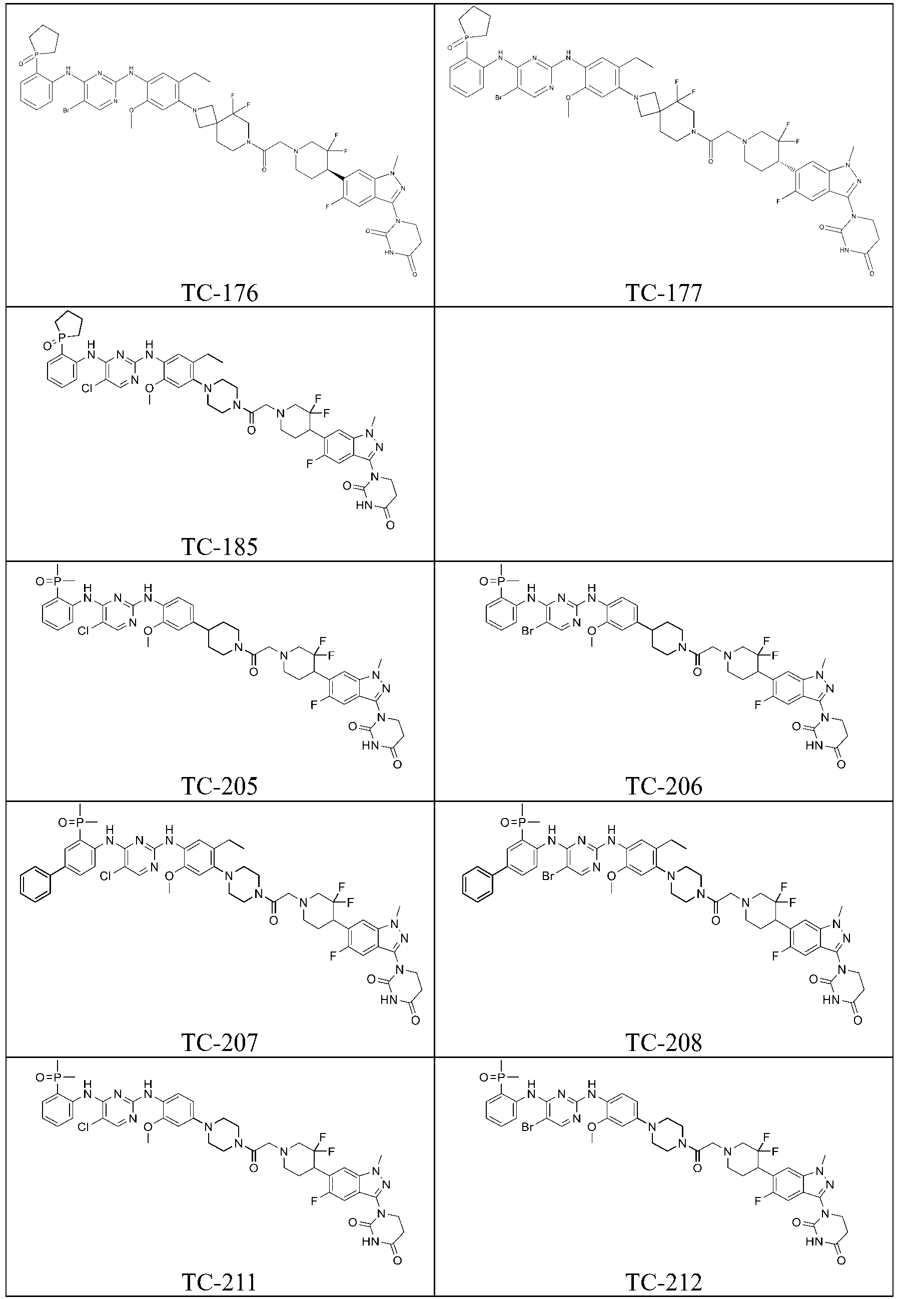

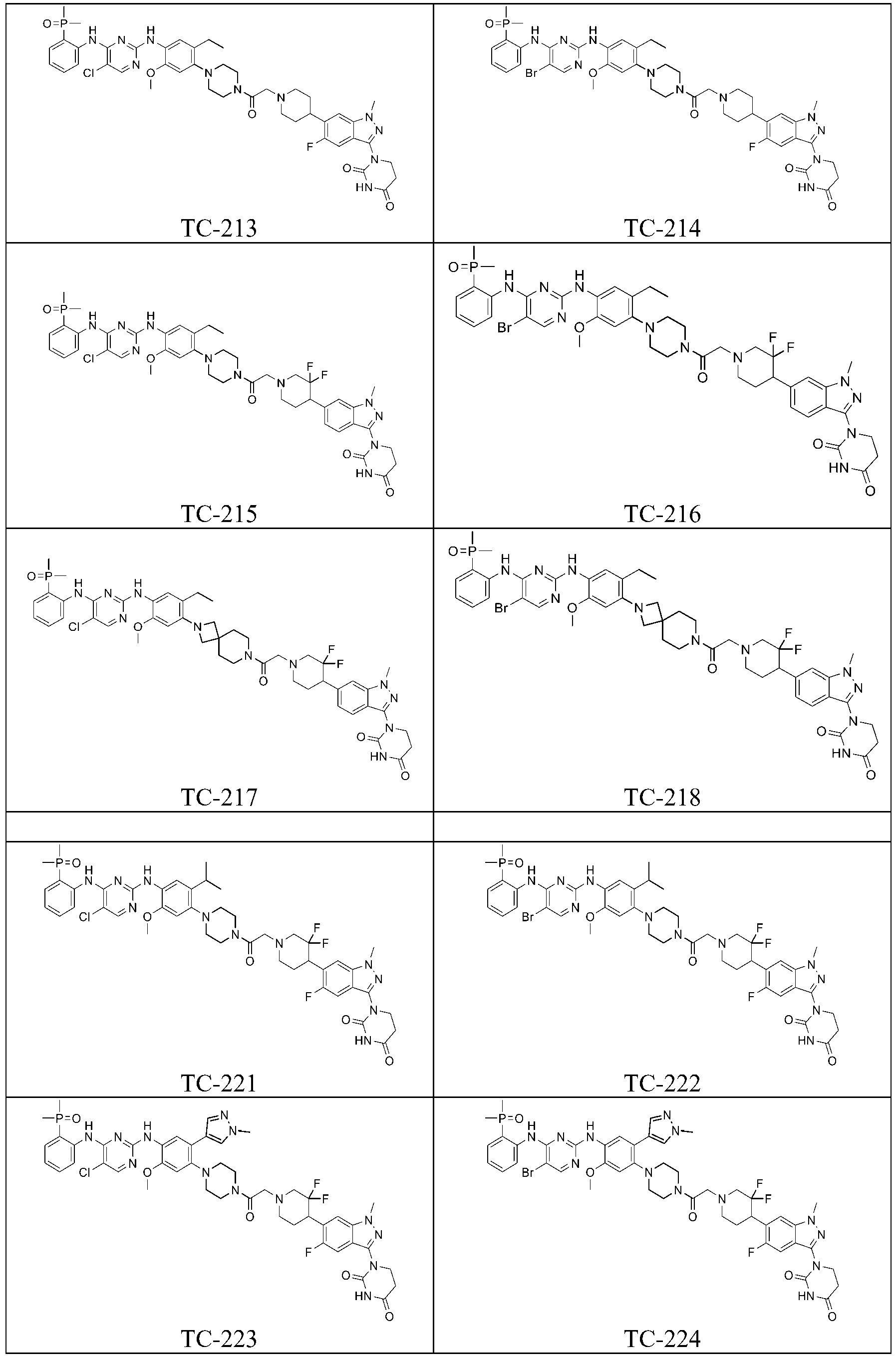

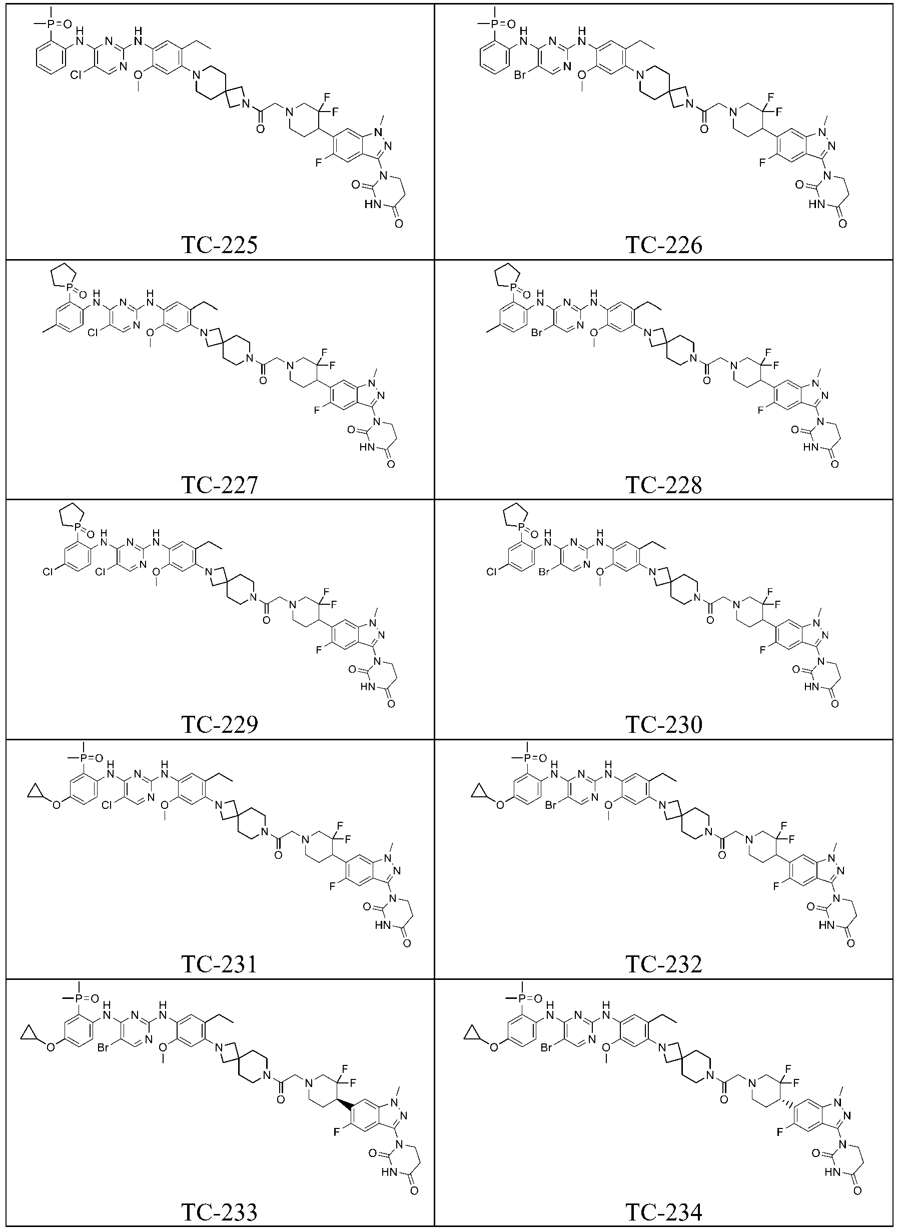

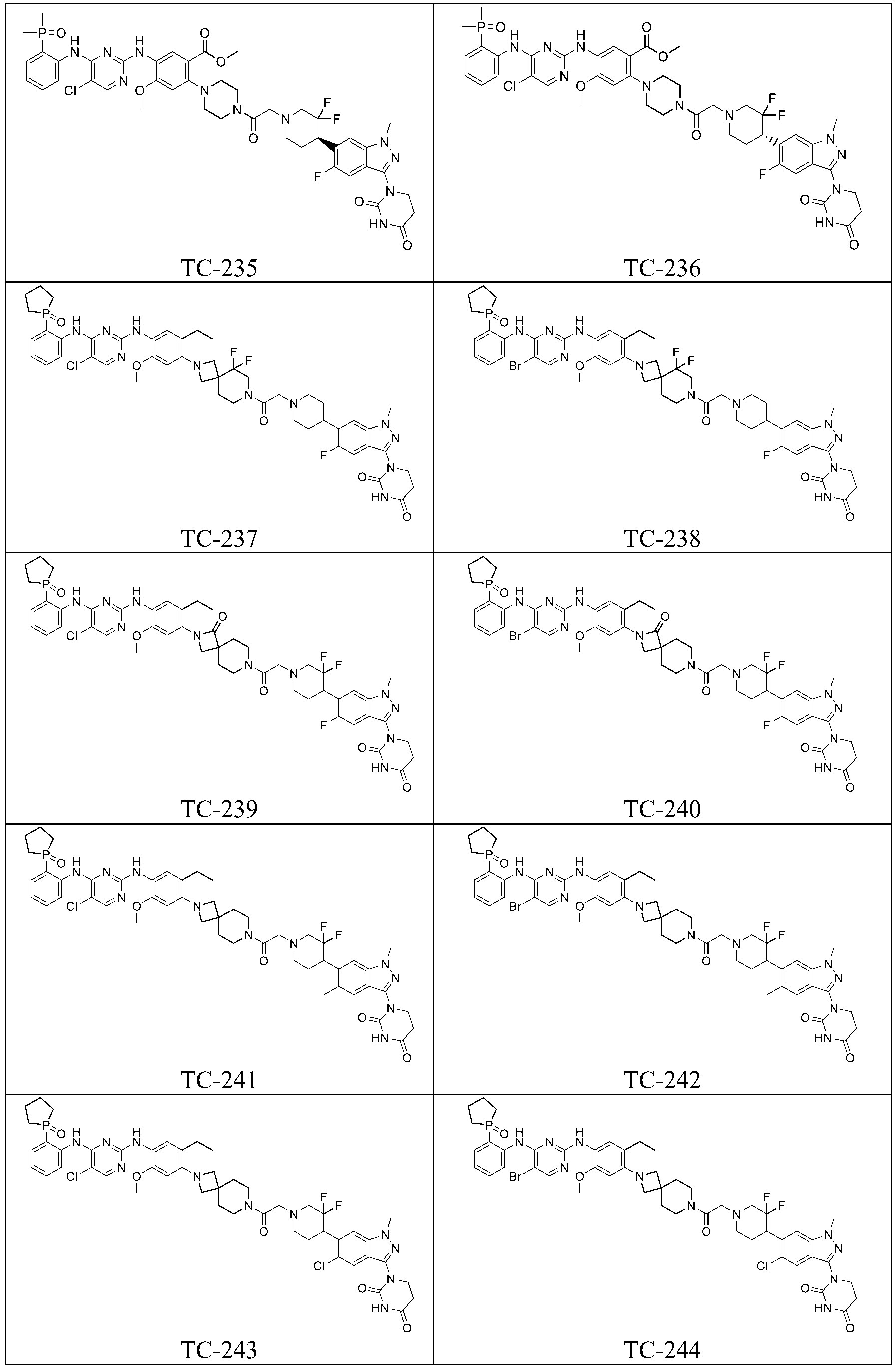

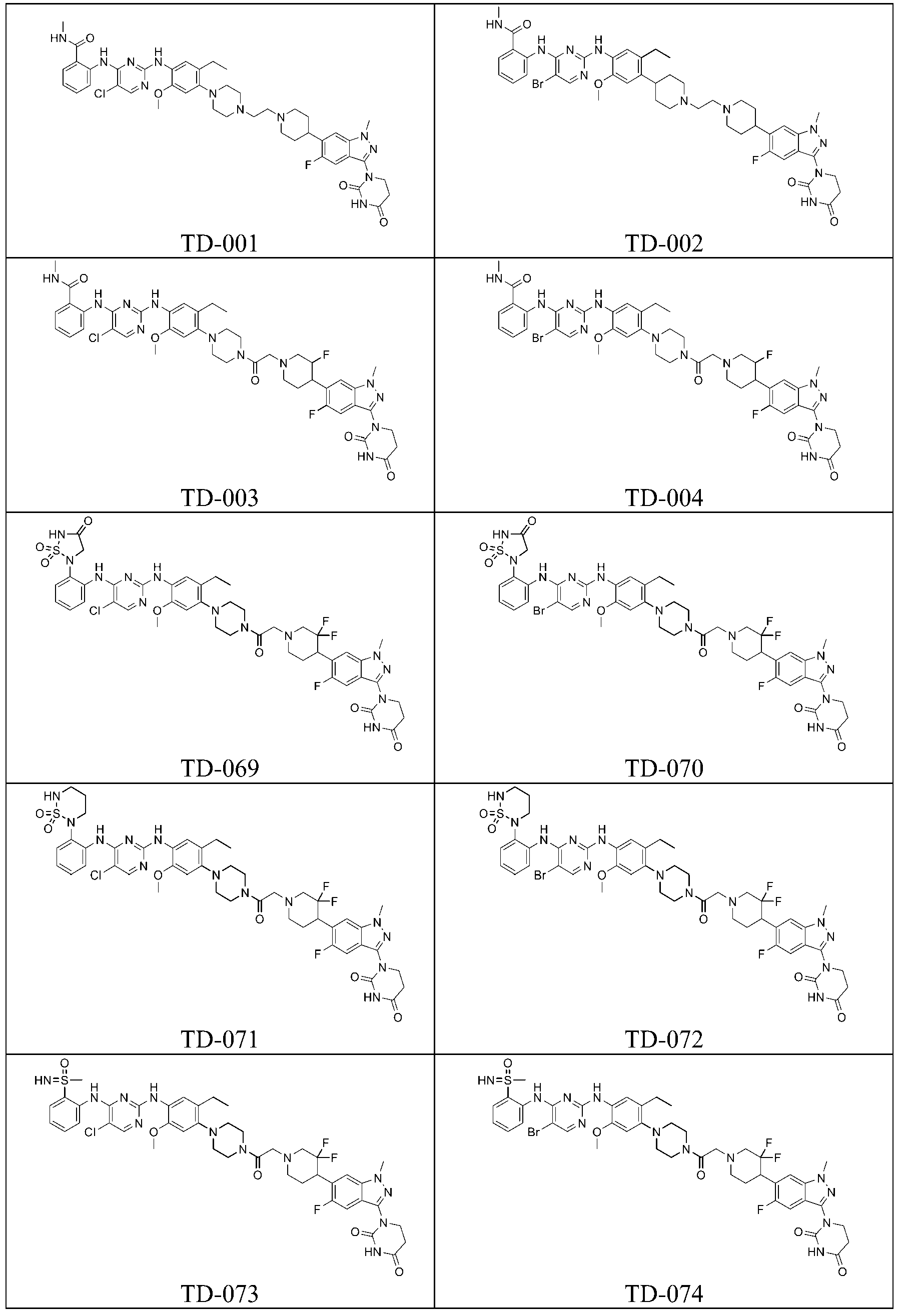

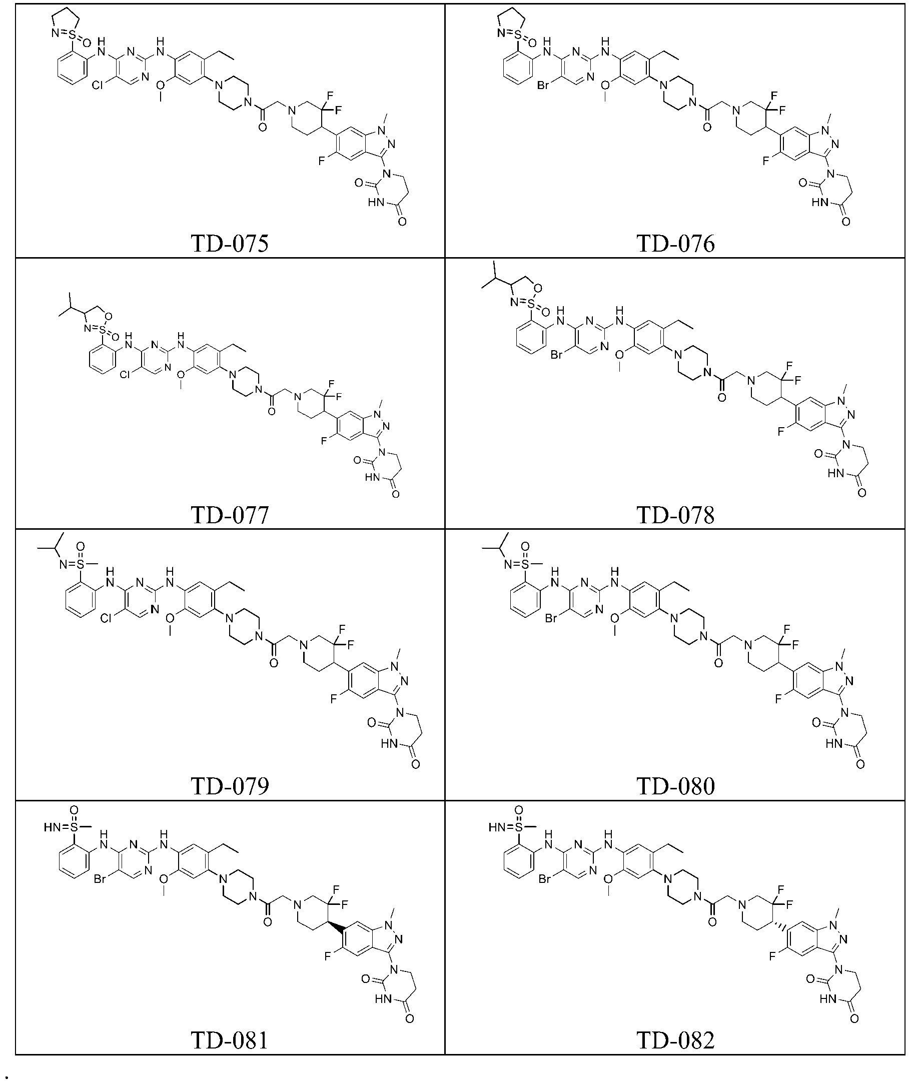

In the fifth aspect of the present invention, provided is a pharmaceutical composition, comprising (a) a therapeutically effective amount of the compound described in the first, second, third, or fourth aspects of the present invention as an active ingredient, and (b) a pharmaceutically acceptable carrier.

In another preferred embodiment, component (a) constitutes 0.001-99.99 wt% of the total weight of the pharmaceutical composition or formulation; more preferably 0.01-99.9 wt%; and most preferably 0.05-90 wt%.

In another preferred embodiment, the dosage form of the pharmaceutical composition or formulation is an injection, tablet, capsule, pill, suspension, or emulsion.

In another preferred embodiment, the dosage form of the pharmaceutical composition is an injection, tablet, capsule, pill, suspension, or emulsion; more preferably, an injection.

In the sixth aspect of the present invention, provided is a pharmaceutical composition, comprising:
(a1) a first active ingredient: a therapeutically effective amount of the compound described in the first, second, third, or fourth aspects of the present invention;
(a2) a second active ingredient selected from the group consisting of gefitinib, erlotinib, icotinib, lapatinib, XL647, NVP-AEE-788, ARRY-334543, vandetanib, PF00299804, cetuximab, panitumumab, pertuzumab, zarumumab, nitumumab, MDX-214, CDX-110, IMC-11F8, CNF2024, tanospiramycin, aspiramycin, IPI-504, and NVP-AUY922; and
(b) a pharmaceutically acceptable carrier.

In a seventh aspect of the present invention, provided is a use of the compounds described in the first, second, third, and fourth aspects of the present invention, the use is selected from the group consisting of:
1) the preparation of a medicament for modulating abnormal EGFR kinase activity;
2) the preparation of a medicament for the prevention and/or treatment of diseases associated with abnormal EGFR kinase activity or mutations (preferably, diseases associated with EGFR resistance mutations); and/or
3) the preparation of a medicament for the degradation of the EGFR protein.

In another preferred embodiment, the abnormal EGFR kinase activity refers to EGFR kinase positivity.

In another preferred embodiment, the abnormal EGFR kinase activity refers to EGFR kinase overexpression.

In another preferred embodiment, the EGFR resistance mutations are selected from the group consisting of EGFRT790M, EGFRC797S, DEL19, L858R, DEL19/T790M, DEL10/T790M/C797S, and L858R/T790M/C797S.

In another preferred embodiment, the disease associated with abnormal or mutated EGFR kinase activity is selected from the group consisting of inflammation, cancer, cardiovascular disease, infection, immune disorders, metabolic disorders, and combinations thereof.

In another preferred embodiment, the cancer is selected from the group consisting of lung cancer (including lung adenocarcinoma and non-small cell lung cancer), breast cancer, prostate cancer, colorectal cancer, liver cancer, pancreatic cancer, ovarian cancer, leukemia, neuroblastoma, gastric cancer, renal cancer, esophageal cancer, uterine cancer, glioma, and head and neck cancer; preferably non-small cell lung cancer.

In another preferred embodiment, the EGFR-related disease is non-small cell lung cancer having an EGFR mutation selected from the group consisting of T790M/L858R, T790M/L858R/C797S, L858R, and L858R/C797S.

In the eighth aspect of the present invention, provided is a method for preventing and/or treating diseases associated with EGFR resistance mutations, comprising steps:
1) determining the EGFR kinase activation mutation status of a subject in need;
2) when the subject's EGFR activation mutation status meets the treatment criteria, administering the compound described in the first, second, third, or fourth aspects of the present invention to the subject in need.

In another preferred embodiment, the "determination of the patient's EGFR activating mutation status" is performed using the cobas EGFR Mutation Test v2.

In another preferred embodiment, the disease associated with EGFR resistance mutations is non-small cell lung cancer.

In the ninth aspect of the present invention, provided is a method for preventing and/or treating diseases associated with abnormal EGFR kinase activity or mutations, comprising administering a therapeutically effective amount of the compound described in the first, second, third, or fourth aspects of the present invention, or the pharmaceutical composition described in the fifth or sixth aspects of the present invention, to a subject in need thereof.

In another preferred embodiment, the method is non-diagnostic and non-therapeutic.

In another preferred embodiment, the method is in vitro.

It should be understood that in the present invention, any of the technical features specifically described above and below (such as in the Examples) can be combined with each other, so as to constitute new or preferred technical solutions. Due to space limitations, it will not redundantly be described one by one herein.

### DETAILED DESCRIPTION OF THE INVENTION

After extensive and in-depth research, the present inventors unexpectedly developed a compound exhibiting excellent EGFR-degrading properties. The compound has the structure shown in Formula I and demonstrates excellent inhibitory and/or therapeutic effects against EGFR-related diseases (particularly those associated with EGFR resistance mutations); furthermore, the compound exhibits excellent pharmacokinetic properties. On above basis, the inventors have completed the present invention.

### TERMS

In the present invention, unless otherwise indicated, the terms used have the ordinary meaning known to the skilled person in the art.

When a substituent is described using a conventional chemical formula written from left to right, the substituent also includes the chemically equivalent substituent obtained when the structural formula is written from right to left. For example, -CH₂O- is equivalent to -OCH₂-.

"Alkyl (alone or as part of another group)" refers to a monovalent, straight-chain or branched saturated hydrocarbon group consisting of solely of carbon and hydrogen atoms and containing 1 to 12 carbon atoms. Alkyl groups are preferably C1-C6 alkyl groups (i.e., containing 1, 2, 3, 4, 5, or 6 carbon atoms) . Examples of alkyl groups include, but are not limited to, methyl, ethyl, propyl, isopropyl, isobutyl, sec-butyl, tert-butyl, pentyl, n-hexyl, octyl, and dodecyl. In this application, "alkyl" is also intended to include substituted alkyl groups, i.e., alkyl groups in which one or more positions are substituted, particularly with 1 to 4 substituents, which may be located at any position. "Halogenated alkyl" refers to an alkyl group as defined herein in which one or more hydrogen atoms are replaced by the same or different halogens. Examples of halogenated alkyl groups include -CH₂Cl, -CH₂CF₃, -CH₂CCl₃, perfluoroalkyl groups (e.g., -CF₃), and the like.

"Alkylene" refers to a divalent alkyl group, such as -CH₂-, -CH₂CH₂-, and -CH₂CH₂CH₂-.

"Alkoxy (alone or as part of another group)" refers to an alkyl group bearing an oxygen atom, having an alkyl-O structure, wherein the alkyl group is defined as described above. Preferably, the alkoxy group is a C1~C6 alkoxy group. Alkoxy groups include, but are not limited to, methoxy, ethoxy, propoxy, and tert-butoxy. "Halogenated alkoxy" refers to the group -OR, where R is a halogenated alkyl group as defined herein. Examples of halogenated alkoxy groups include, but are not limited to, trifluoromethoxy, difluoromethoxy, and 2,2,2-trifluoroethoxy.

"Alkenyl (alone or as part of another group)" refers to an aliphatic group containing at least one double bond, typically having 2 to 20 carbon atoms. In the present invention, "C2-C6 alkenyl" refers to an alkenyl group containing 2, 3, 4, 5, or 6 carbon atoms. Alkenyl groups include, but are not limited to, vinyl, propenyl, butenyl, 1-methyl-2-buten-1-yl, and the like. In the present invention, alkenyl includes substituted alkenyl.

"Alkenylene" refers to an alkenyl group with two bonded carbon atoms. For example, "Vinylene" denotes the group -CH=CH-. Alkenylene may be unsubstituted or substituted with one or more substituents.

"Alkynyl (either alone or as part of another group)" refers to a linear or branched hydrocarbon chain containing two or more carbon atoms and characterized by having one or more triple bonds, typically comprising 2 to 20 carbon atoms. In the present invention, "C2-6 alkynyl" refers to an alkynyl group having 2, 3, 4, 5, or 6 carbon atoms. alkynyl groups include, but are not limited to, ethynyl, propyn-1-yl, and 3-hexyn-1-yl. One of the carbon atoms in the triple bond may optionally serve as a linkage point for an alkynyl group substituent. In the present invention, alkynyl groups also include substituted alkynyl.

"Alkynylene" refers to an alkynyl group with two bonding sites. For example, "ethynylene" denotes the group: -C≡C-. Alkynylene groups may be unsubstituted or substituted with one or more substituents.

"Cycloalkyl" refers to a monovalent saturated carbon ring group consisting of a single or double ring, having 3-12, preferably 3-10, and more preferably 3-8 ring atoms. The cycloalkanyl group may optionally be substituted by one or more substituents, wherein each substituent is independently a hydroxyl group, an alkyl group, an alkoxy group, a halogen atom, a halogenated alkyl group, an amino group, a monoalkylamino group, or a dialkylamino group. Examples of cycloalkyl groups include, but are not limited to, cyclopropyl, cyclobutyl, cyclopentyl, cyclohexyl, and cycloheptyl.

"Cycloalkyloxy" refers to the group -OR, where R is a cycloalkyl group as defined herein. Exemplary cycloalkyloxy groups include cyclopropyl oxy, cyclobutyl oxy, cyclopentyl oxy, cyclohexyl oxy, and the like. "Cycloalkylalkyl" refers to a group in which the cycloalkyl and alkyl moieties are as disclosed herein, such as (cycloalkyl)alkyl. "Cycloalkylalkyl" is bonded to the parent molecule via the cycloalkyl moiety.

"Heteroaryl" refers to a mononuclear (e.g., 5- or 6-membered), bicyclic (e.g., 8-10-membered), or tricyclic group containing 5-12 ring atoms, wherein at least one ring atom is a heteroatom selected from N, O or S, and the remaining ring atoms are carbon atoms. It should be understood that the point of attachment of the heterocyclic aryl group must be located on the aromatic ring. Examples of heteroaryl include, but are not limited to: imidazolyl, oxazolyl, isoxazolyl, thiazolyl, isothiazolyl, oxadiazolyl, thiadiazolyl, pyrazinyl, thienyl, furyl, pyranyl, pyridyl, pyrrolyl, pyrazolyl, pyrimidinyl, quinolinyl, isoquinolinyl, benzofuryl, benzothienyl, benzothiopyranyl, benzimidazolyl, benzoxazolyl, benzoxadiazolyl, benzothiazolyl, benzothiadiazolyl, benzopyranyl, indolyl, isoindolyl, triazolyl, triazinyl, quinoxalinyl, purinyl, quinazolinyl, quinolizinyl, naphthidinyl, pteridinyl, carbazolyl, azepinyl, diazepinyl, acridinyl and the like; the heteroarylene refers to a heteroaryl group having two attachment sites..

"Heterocyclic system" refers to monocyclic, bicyclic, and polycyclic systems in which at least one ring is saturated or partially unsaturated (but not aromatic) and contains at least one heteroatom. Heterocyclic systems can be linked to side chains attached to any heteroatom or carbon atom, resulting in stable structures, and any ring atom may be optionally substituted.

"Heterocyclyl" refers to a monovalent group within a heterocyclic system, typically denoting a stable monocyclic ring (e.g., 3-8 members, i.e., 3, 4, 5, 6, 7, or 8 members), a bicyclic ring (e.g., 5-12 members, i.e., 5, 6, 7, 8, 9, 10, 11, or 12 members), or a polycyclic ring (e.g., 7-14 members, i.e., 7, 8, 9, 10, 11, 12, 13, or 14), including fused, spiral, and/or bridged ring structures, which are saturated or partially unsaturated and contain carbon atoms and 1, 2, 3, or 4 heteroatoms independently selected from N, O, or S. Representative heterocyclyl groups include the following ring systems, wherein (1) each ring is non-aromatic and at least one ring contains a heteroatom, for example, tetrahydrofuryl, tetrahydropyranyl, tetrahydrothienyl, pyrrolidinyl, pyrrolidonyl, piperidinyl, pyrrolinyl, decahydroquinolinyl, oxazolidinyl, piperazinyl, dioxanyl, dioxolanyl, diazepinyl, oxazepinyl, thiazepinyl, morpholinyl and quinuclidinyl; (2) at least one ring is non-aromatic and contains a heteroatom, and at least one other ring is an aromatic carbocyclic ring, for example, 1,2,3,4-tetrahydroquinolinyl and 1,2,3,4-tetrahydroisoquinolinyl; and (3) at least one ring is non-aromatic and contains a heteroatom, and at least one other ring is aromatic and contains a heteroatom, for example, 3,4-dihydro-1H-pyrano[4,3-c]pyridine and 1,2,3,4-tetrahydro-2,6-dihydronaphthyridine.A heterocycloalkylene group refers to a heterocyclic group having two attachment sites. In the present invention, preferred heterocycloalkylene groups are bicyclic rings in which one ring is a heteroaryl and is linked to other moieties in the general formula via the heteroaryl. In the present invention, the preferred heterocycloalkylene groups are 5-6 membered monocyclic heterocycloalkylene groups or 8-10 membered bicyclic heterocycloalkylene groups.

"heterocycloalkyl" refers to an alkyl group substituted by a heterocyclyl, where the definitions of heterocyclyl and alkyl are as described above.

When a substituent is a non-terminal substituent, it is the corresponding ylene form of the group; for example, alkyl corresponds to alkylene, cycloalkyl corresponds to cycloalkylene, heterocyclyl corresponds to heterocycloalkylene, and alkoxy corresponds to alkoxyalkylene.In the present invention, the groups mentioned above may be substituted or unsubstituted, such as alkyl, alkoxy, cycloalkyl, heteroalkyl, aryl, heteroaryl, cycloheteroalkyl, alkenyl, alkynyl, heterocycle, and heterocyclyl.

In the present invention, the term "substituted" refers to one or more hydrogen atoms on a particular group being substituted by a particular substituent. The particular substituent is a substituent described above accordingly, or a substituent in the embodiments. Unless indicated specifically, a substituted group may have a substituent selected from a particular group at any substitutable site of the group, and substituents can be identical or different at each site. It should be understood by the person skilled in the art that the combinations of substituents expected in the present invention are ones that are stable or chemically realizable.

Typical substituents include, but are not limited to, one or more of the following groups: hydrogen, deuterium, halogens (e.g., monohalogenated or polyhalogenated substituents, such as trifluoromethyl or alkyl groups containing Cl₃), cyano, nitro, oxo (e.g., =O), trifluoromethyl, trifluoromethoxy, cycloalkyl, alkenyl, alkynyl, heterocyclic, aromatic, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein, Ra may independently represent hydrogen, deuterium, alkyl, cycloalkyl, alkenyl, alkynyl, heterocycle, or aromatic ring; Rb, Rc, and Rd may independently represent hydrogen, deuterium, alkyl, cycloalkyl, heterocycle, or aromatic ring, or Rb and Rc with the N atom may together form a heterocycle; Re may independently represent hydrogen, alkyl, cycloalkyl, alkenyl, alkynyl, heterocycle, or aromatic ring. The aforementioned typical substituents, such as alkyl, cycloalkyl, alkenyl, cycloalkenyl, alkynyl, heterocycle, or aromatic groups, may be optionally substituted. Such substituents include, for example (but are not limited to): halogen, hydroxyl, cyano, carboxyl (-COOH), C1-C6 alkyl, C2-C6 alkenyl, C2-C6 alkynyl, C3-C8 cycloalkyl, 3-12-membered heterocyclic groups, aryl, heteroaryl, C1-C8-aldehyde, C2-C10-acetyl, C2-C10-ester, amino, C1-C6-alkoxy, C1-C10-sulfonyl, and C1-C6-ureido groups, etc.

"Cyan" refers to the -CN group.

"Nitro" refers to -NO₂₂.

"Hydroxy" refers to -OH.

"Amino" refers to -NH₂ or RNH-, wherein R represents a ketone carbonyl group, sulfonyl group, sulfonamide group, Ra-C(=O)-, RaRbN-C(=O)-, etc., and Ra and Rb represent alkyl, cycloalkyl, aryl, or heteroaryl groups, etc.

"Halogen (halogenated)" refers to any halogen group, such as -F, -Cl, -Br, or -I.

"Deuterated compound" refers to a compound obtained by replacing one or more hydrogen atoms (H) with deuterium atoms (D).

In the present invention, the term "multiple" independently refers to 2, 3, 4, or 5.

### Compound of Formula I

The present invention provides a compound, the compound shown in Formula III, or a pharmaceutically acceptable salt, a stereoisomer, a tautomer, a hydrate, a solvate, an isotopically labelled compound, or a prodrug thereof, 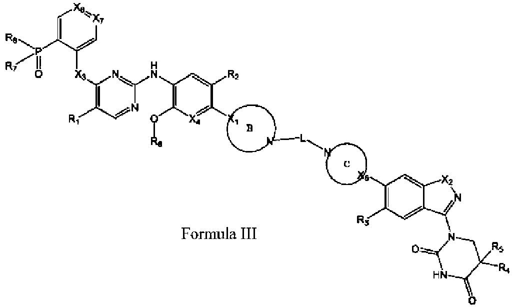 wherein, the groups are as defined in the third aspect of the present invention.

In one embodiment, any one of each groups of the compound is independently a corresponding group in the specific compound.

As used herein, the term "pharmaceutically acceptable salt" refers to a salt of a compound of the invention formed with an acid or base suitable for use as a drug. Pharmaceutically acceptable salts include inorganic and organic salts. A preferably class of salts are salts formed by the compounds of the present invention with acids. Acids suitable for forming salts include, but are not limited to: inorganic acids such as hydrochloric acid, hydrobromic acid, hydrofluoric acid, sulfuric acid, nitric acid, phosphoric acid, and the like; organic acids such as formic acid, acetic acid, trifluoroacetic acid, propionic acid, oxalic acid, malonic acid, succinic acid, fumaric acid, maleic acid, lactic acid, malic acid, tartaric acid, citric acid, picric acid, benzoic acid, methanesulfonic acid, ethylsulfonic acid, p-toluenesulfonic acid, benzene sulfonic acid, naphthalenesulfonic acid, and the likes; and amino acids such as proline, phenylalanine, aspartic acid and glutamic acid, etc.

Another preferred class of salts are salts formed by the compounds of the present invention with bases, such as alkali metal salts (e.g., sodium salt or potassium salt), alkaline earth metal salts (e.g., magnesium salt or calcium salt), and ammonium salts (e.g., lower alkanolammonium salts and other pharmaceutically acceptable amine salts), for example, methylamine salt, ethylamine salt, propylamine salt, dimethylamine salt, trimethylamine salt, diethylamine salt, triethylamine salt, tert-butylamine salt, ethylenediamine salt, hydroxyethylamine salt, dihydroxyethylamine salt, trihydroxyethylamine salt, as well as amine salts respectively formed from morpholine, piperazine and lysine.

The term "solvate" refers to a complex formed when the compound of the present invention coordinates with solvent molecules in a specific ratio. "Hydrate" refers to a complex formed when the compound of the present invention coordinates with water.

In addition, the compounds of the present invention also include prodrugs of the compounds shown in Formula I. The term "prodrug" refers to a compound that itself may be biologically active or inactive, but which, when administered by an appropriate method, undergoes metabolism or a chemical reaction in the human body to convert into a compound of Formula I, or a salt or solution composed of a compound of Formula I. The prodrugs include (but are not limited to) carboxylates, carbonates, phosphates, nitrates, sulfates, sulfonates, sulfinates, amino compounds, carbamates, azo compounds, phosphoramides, glucosides, ethers, and acetal forms.

The compounds, salts, or solvates of the present invention may exist in tautomeric forms (e.g., amides and imine ethers). All such tautomers are included within the scope of the present invention.

All stereoisomers of the compounds (e.g., those resulting from asymmetric carbon atoms subject to various substituents), including their enantiomeric and diastereomeric forms, fall within the scope of the present invention. The individual stereoisomers of the compounds of the present invention may not coexist with other isomers (e.g., as a pure or substantially pure optical isomer exhibiting specific activity), or they may exist as mixtures, such as racemates, or as mixtures formed with all or some of the other stereoisomers. The chiral centers of the present invention have two configurations, S or R, as defined by the International Union of Pure and Applied Chemistry (IUPAC) in 1974. The racemic form may be resolved by physical methods, such as stepwise recrystallization, or by derivatization and recrystallization of the diastereomers, or by chiral column chromatography. Individual enantiomers may be obtained from the racemic mixture by suitable methods, including but not limited to conventional methods such as salification with an optically active acid followed by recrystallization.

The compounds of the present invention, obtained sequentially through preparation, separation, and purification, wherein the weight percentage of the compound is equal to or greater than 90%, for example, equal to or greater than 95%, or equal to or greater than 99% ("highly pure" compounds), are listed in the description. Such "highly pure" compounds of the present invention are also considered part of the present invention.

All configurational isomers of the compounds of the present invention are included within the scope of the invention, whether in the form of a mixture, a pure compound, or a highly pure compound. The definition of the compounds of the present invention includes both cis (Z) and trans I alkene isomers, as well as cis and trans isomers of carbon rings and heterocycles.

Throughout this specification, functional groups and substituents may be selected to provide stable fragments and compounds.

Definitions of specific functional groups and chemical terminology are provided in detail below. For the purposes of this invention, chemical elements are defined in accordance with the Periodic Table of the Elements, CAS version, Handbook of Chemistry and Physics, 75th Ed. Definitions of specific functional groups are also described therein. In addition, the fundamental principles of organic chemistry, as well as specific functional groups and reactivity, are described in "Organic Chemistry" by Thomas Sorrell, University Science Books, Sausalito: 1999, the entirety of which is incorporated by reference.

Certain compounds of the present invention may exist in specific geometric or stereoisomeric forms. The present invention encompasses all compounds, including their cis and trans isomers, R and S enantiomers, diastereomers, (D)-isomers, (L)-isomers, racemic mixtures, and other mixtures. Furthermore, the asymmetric carbon atom may bear substituents, such as alkyl groups. All isomers and mixtures thereof are included within the scope of the present invention.

According to the present invention, the ratio of isomers in a mixture of isomers may vary. For example, in a mixture containing only two isomers, the following combinations are possible: 50:50, 60:40, 70:30, 80:20, 90:10, 95:5, 96:4, 97:3, 98:2, 99:1, or 100:0; all such ratios of isomers fall within the scope of the present invention. Similar ratios readily understood by those skilled in the art, as well as ratios for more complex mixtures of isomers, are also within the scope of the present invention.

The present invention also encompasses isotopically labeled compounds equivalent to the original compounds disclosed herein. However, in practice, one or more atoms are typically replaced by atoms having a different atomic weight or mass number. Examples of isotopes of the compounds of the present invention include isotopes of hydrogen, carbon, nitrogen, oxygen, phosphorus, sulfur, fluorine, and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively. Compounds of the present invention, or their enantiomers, diastereomers, isomers, or pharmaceutically acceptable salts or solvates, wherein the compounds contain the aforementioned isotopes or other isotopic atoms, are all within the scope of the present invention. Certain isotopically labeled compounds described in this invention, including radioactive isotopes such as ³H and ¹⁴C, are useful in experiments involving the tissue distribution of drugs and substrates. Tritium (³H) and carbon-14 (¹⁴C) are relatively easy to prepare and detect, making them the preferred isotopes. Furthermore, heavier isotope substitutes such as deuterium (²H) offer advantages in certain therapies due to their excellent metabolic stability, such as increasing the half-life in vivo or reducing the dosage; therefore, they may be preferred in certain situations. Isotope-labeled compounds can be prepared using general methods by replacing non-isotopic reagents with readily available isotope-labeling reagents, following the procedures disclosed in the examples.

To synthesize a specific enantiomer of a compound of the present invention, it may be prepared via asymmetric synthesis or derivatized with a chiral auxiliary, followed by separation of the resulting racemic mixture and removal of the chiral auxiliary to obtain the pure enantiomer. Alternatively, if the molecule contains a basic functional group, such as an amino group, or an acidic functional group, such as a carboxyl group, it can be converted into a diastereomeric salt with a suitable optically active acid or base, and then separated by conventional methods such as recrystallization or chromatography to obtain the pure enantiomer.

As described herein, the compounds of the present invention may be substituted with any number of substituents or functional groups to broaden their scope. Generally, the term "substituted" whether appearing before or after the term "optionally," in the general formula for substituents in the formulation of the present invention refers to the substitution of hydrogen atoms with the specified substituents. When multiple positions in a specific structure are substituted with multiple specific substituents, the substituents at each position may be the same or different. The term "substituted" as used herein encompasses all organic compounds capable of substitution. Broadly speaking, permissible substituents include non-cyclic, cyclic, branched, and unbranched; carbon-based and heterocyclic; and aromatic and non-aromatic organic compounds. In the present invention, a heteroatom such as nitrogen may be substituted with a hydrogen substituent or any of the permissible organic compounds described above to complete its valence. Furthermore, the present invention is not intended to limit the permissible organic compounds in any way. The present invention considers combinations of substituents and variable groups to be suitable for the treatment of diseases in the form of stable compounds. The term "stable" herein refers to a compound that remains stable for a sufficiently long period to maintain the integrity of its structure, and preferably remains effective for a sufficiently long period; it is used herein for the aforementioned purposes.

The metabolites of the compounds and their pharmaceutically acceptable salts described in this application, as well as prodrugs that can be converted in vivo into the compounds and their pharmaceutically acceptable salts described in this application, are also included in the claims of this application.

It should be understood that the various embodiments of the present invention specifically describe methods for preparing the compound of formula I of the present invention; however, these specific methods do not limit the scope of the present invention. The compounds of the present invention may also be conveniently prepared by combining various synthetic methods described herein or known in the art, and such combinations can be readily carried out by those skilled in the art.

Typically, unless otherwise specified, the raw materials and reagents used in the preparation process of the compounds of the present invention are commercially available.

Generally, in the preparation process, the reactions are carried out under an inert gas atmosphere in an appropriate solvent at temperature 0 to 150 °C, with reaction times typically 2-24 hours.

### Pharmaceutical Compositions and Administration Methods

The present invention provides a pharmaceutical composition, wherein comprising the compound, or a pharmaceutically acceptable salt, a stereoisomer, a tautomer, a hydrate, a solvate, an isotope, or a prodrug thereof, and a pharmaceutically acceptable carrier.

Given the excellent antitumor activity of the compounds of the present invention, the compounds of the present invention and their various polymorphs, pharmaceutically acceptable inorganic or organic salts, hydrates, or solvates, as well as pharmaceutical compositions containing the compounds of the present invention as the principal active ingredient, may be used for the treatment, prevention, and alleviation of tumor-related diseases.

The pharmaceutical composition of the present invention comprises a safe and effective amount of a compound of the present invention or a pharmacologically acceptable salt thereof, and a pharmacologically acceptable excipient or carrier. In which, "safe and effective amount" is meant that the amount of the compound is sufficient to significantly improve the condition without causing serious side effects. Generally, the pharmaceutical composition contains 1-2000 mg the compound of the present invention/dose, more preferably, 10-1000 mg the compound of the present invention/dose. Preferably, the "dose" is a capsule or tablet.

"Pharmaceutically acceptable carrier" means one or more compatible solid or liquid fillers or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agent (such as sodium dodecyl sulfate), coloring agents, flavoring agents, stabilizers, antioxidants, preservatives, pyrogen-free water, etc..

The pharmaceutical composition is an injection, a capsule, a tablet, a pill, a powder, or a granule.

The dosage forms of the pharmaceutical composition of the present invention include (but are not limited to): injections, tablets, capsules, aerosols, suppositories, films, pellets, topical ointments, controlled-release or sustained-release formulations, and nanformulations.

Solid dosage forms for oral administration include capsules, tablets, pills, powders and granules. In these solid dosage forms, the active ingredient is mixed with at least one conventional inert excipient (or carrier), such as sodium citrate or dicalcium phosphate, or mixed with any of the following components: (a) fillers or compatibilizer, such as starch, lactose, sucrose, glucose, mannitol and silicic acid; (b) binders, such as hydroxymethyl cellulose, alginate, gelatin, polyvinylpyrrolidone, sucrose and arabic gum; (c) humectants, such as glycerol; (d) disintegrating agents such as agar, calcium carbonate, potato starch or tapioca starch, alginic acid, certain composite silicates, and sodium carbonate; (e) retarding solvents, such as wax, (f) absorption accelerators, such as quaternary ammonium compound; (g) wetting agents, such as cetyl alcohol and glyceryl monostearate; (h) adsorbents, such as kaolin; and (i) lubricants, such as talc, calcium stearate, magnesium stearate, solid polyethylene glycol, sodium dodecyl sulfate or mixture thereof. In capsules, tablets and pills, the dosage forms may also contain buffering agents.

Solid dosage forms such as tablets, dragees, capsules, pills and granules can be prepared with coatings and shells such as enteric coatings and other materials known in the art. They may contain opacifying agents and the release of the active compound or compound in such compositions may be released in a portion of the digestive tract in a delayed manner. Examples of embedding components that can be employed are polymeric materials and waxy materials. If necessary, the active compound may also be in microencapsulated form with one or more of the above-mentioned excipients.

Liquid dosage forms for oral administration include pharmaceutically acceptable emulsions, solutions, suspensions, syrups or tinctures. In addition to the active compound, the liquid dosage form may contain inert diluents conventionally used in the art, such as water or other solvents, solubilizers and emulsifiers, such as ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures of these substances.

In addition to these inert diluents, the compositions may contain adjuvants such as wetting agents, emulsifying and suspending agents, sweetening agents, flavoring agents and spices.

In addition to the active compound, the suspension may contain suspending agent, such as ethoxylated isooctadecanol, polyoxyethylene sorbitol and dehydrated sorbitan ester, microcrystalline cellulose, aluminum methoxide and agar, or the mixture thereof etc..

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

Dosage forms for the compounds of the invention for topical administration include ointments, powders, patches, sprays and inhalants. The active ingredient is mixed under sterile conditions with a physiologically acceptable carrier and any preservatives, buffers, or propellants which may be required if necessary.

The compounds of the invention can be administered alone or in combination with other pharmaceutically acceptable compounds (e.g., Anticancer drugs).

The treatment method of the present invention can be administered alone or in combination with other treatment means or therapeutic drugs.

The compounds of Formula I may be used in combination with other known drugs for treating or improving similar conditions. When administered in combination, the original drug's administration method and dosage may remain unchanged, while the compound of Formula I is administered concurrently or subsequently. When the compound of Formula I is administered concurrently with one or more other drugs, it is preferable to use a pharmaceutical composition containing both one or more known drugs and the compound of Formula I. Drug combination therapy also includes administering the compound of Formula I and one or more other known drugs during overlapping time periods. When the compound of Formula I is used in combination with one or more other drugs, the dose of the compound of Formula I or the known drug(s) may be lower than the dose when administered alone.

Drugs or active ingredients that can be used in combination with the compounds described in Formula I include, but are not limited to: gefitinib, erlotinib, icotinib, lapatinib, XL647, NVP-AEE-788, ARRY-334543, vandetanib, PF00299804, cetuximab, panitumumab, pertuzumab, zarumumab, nitumumab, MDX-214, CDX-110, IMC-11F8, CNF2024, tanospiramycin, aspiramycin, IPI-504, and NVP-AUY922.

When the pharmaceutical composition is used, a safe and effective amount of the compound of the present invention is administered to a mammal(such as a human or a mouse) in need of treatment, wherein the dosage at the time of administration is the pharmaceutically effective dosage, for people having a body weight of 60kg, the daily dose is usually 1~2000mg, preferably 50~1000mg. Of course, specific doses should also consider factors such as the administration route, the health of the patient, etc., which are within the skill of the skilled physician.

**Compared with the prior art, the present invention has the following main advantages:**
(1) The compounds of the present invention exhibit excellent EGFR-degrading activity;
(2) The compounds of the present invention exhibit excellent inhibitory and/or therapeutic effects against EGFR-related diseases, particularly those involving EGFR resistance mutations; such diseases include, but are not limited to the diseases selected from the group consisting of H1975 (T790M/L858R), HCC827(19DEL), and PC-9(19DEL);
(3) The compounds of the present invention exhibit excellent pharmacokinetic properties;
(4) The compounds of the present invention are suitable for the prevention and/or treatment of diseases selected from the group consisting of EGFR-sensitive mutated cancers and diseases that have developed secondary resistance during current EGFR therapy;
(5) The compounds of the present invention exhibit excellent safety;
(6) The compounds of the present invention exhibit excellent degradation selectivity toward wild-type and mutated EGFR proteins, with very high degradation efficiency for mutated EGFR proteins.

The present invention will be further illustrated below with reference to the specific examples. It should be understood that these examples are only to illustrate the invention but not to limit the scope of the invention. Experimental methods in which the specific conditions are not specified in the following examples are usually in accordance with conventional conditions such as the conditions described in Sambrook et al., Molecular Cloning: Laboratory Manual (New York: Cold Spring Harbor Laboratory Press, 1989), or in accordance with the conditions recommended by the manufacturer. Unless indicated otherwise, percentage and parts are calculated by weight.

Unless otherwise defined, all professional and scientific terminology used in the text have the same meanings as known to the skilled in the art. In addition, any methods and materials similar or equal with the recorded content can apply to the methods of the invention. The preferred embodiments and the materials described herein are only for demonstration purposes.

### Example 1-1: Synthesis of Compound TA-003

### Synthesis of Intermediate Compound M1

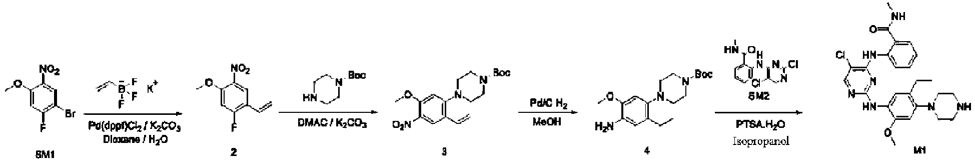

### 1. Synthesis of Compound 2

In a 250 mL three-neck flask, 10 g SM1, 10.7 g boron trifluoride, 2.9 g Pd(dppf)Cl₂, and 8.2 g potassium carbonate were dissolved in 50 mL 1,4-dioxane and 10 mL H₂O. Heated to 100 °C after N₂ gas protection, the reaction was proceeded overnight, and monitored by TLC until complete. The reaction solution was cooled to room temperature, vacuum filtered to remove insoluble impurities, and the filtrate was added water and extracted with EA. The organic phase was washed once with saturated NaCl solution and dried over, directly spun-dried and mixed as the sample, 14.4g compound 2 was obtained through column chromatography. LC-MS [M+1]: 198.

### 2. Synthesis of Compound 3

In a 100 mL three-neck flask, 2.7 g compound 2, 4.3 g 1-Boc-piperazine, and 9.4 g potassium carbonate were dissolved in 27 mL DMAC. Heated to 100 °C after N₂ gas protection, the reaction was proceeded overnight, TLC indicated that the raw materials had disappeared. The reaction solution was cooled to room temperature, vacuum filtered to remove insoluble impurities, the filtrate was added water and extracted with EA. The organic phase was washed once with saturated NaCl solution and dried over, directly spun-dried and mixed as the sample, 3.8 g Compound 3 was obtained through column chromatography. LC-MS [M+1]: 364.

### 3. Synthesis of Compound 4

In a 100 mL reaction flask, 3.8 g compound 3 and 1.5 g palladium carbon were dissolved in 35 mL methanol. The reaction was proceeded overnight at room temperature after H₂ gas protection. TLC indicated that the raw materials had disappeared. The reaction solution was vacuum filtered to remove insoluble impurities, the filtrate was directly spun-dried and mixed as the sample, 2.9 g compound 4 was obtained through column chromatography. LC-MS [M+1]: 336.

### 4. Synthesis of Intermediate M1

In a 100 mL reaction tube, 300 mg compound 4, 265 mg SM2, and 220 mg p-toluenesulfonic acid monohydrate were dissolved in 3 mL isopropanol. Heated to 90 °C after N₂ gas protection, the reaction was proceeded overnight, and monitored by TLC until complete. The reaction was quenched with a small amount of saturated sodium bicarbonate solution, extracted with ethyl acetate, dried over with anhydrous sodium sulfate, spun-dried and 331 mg intermediate M1 was obtained through column chromatography. LC-MS [M+1]: 496.

### Synthesis of the intermediate compound M2

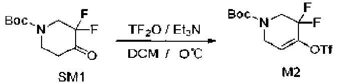

In a 250 mL three-neck flask, 8.3 g SM1 were dissolved in 100 mL dichloromethane, triethylamine was added after N₂ gas protection, cooled to 0 °C in an ice bath, and TF₂O was slowly added dropwise, the reaction was proceeded overnight at room temperature, monitored the reaction until complete by TLC. The reaction solution was added water and extracted with EA. The organic phase was washed once with saturated NaCl solution, spun-dried and mixed as sample, 2 g intermediate M2 was obtained through column chromatography.

### Synthesis of the intermediate compound M3

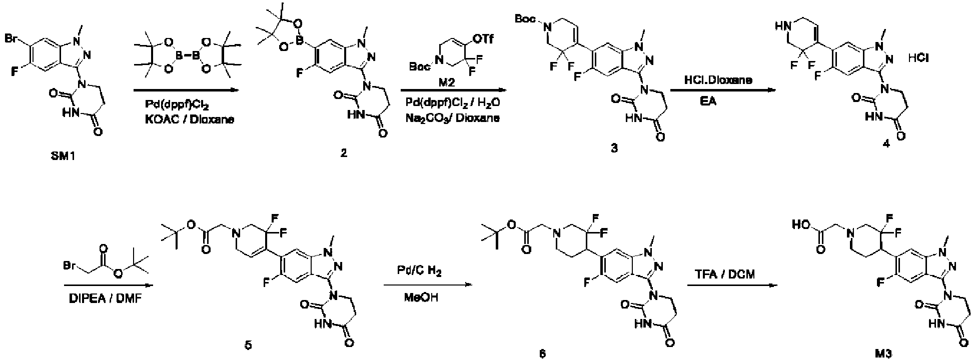

### 1. Synthesis of Compound 2

In a 100 mL three-neck flask, 2 g SM1, 2.2 g bis(pinacolato)diboron, 2.4 g pd(dppf)Cl₂, and 1.7 g potassium acetate were dissolved in 30 mL 1,4-dioxane. Heated to 85°C after N₂ gas protection, the reaction was proceeded overnight, and monitored by TLC until complete. The reaction solution was cooled to room temperature, vacuum filtered to remove insoluble impurities, and the filtrate was added water and extracted with EA. The organic phase was washed once with saturated NaCl solution, dried over, and directly spun-dried and mixed as sample, 2 g compound 2 was obtained through column chromatography. LC-MS [M+1]: 389.

### 2. Synthesis of Compound 3

In a 250 mL three-neck flask, 2 g compound 2, 2 g intermediate M2, 1.5 g sodium carbonate, and 376 mg pd(dppf)Cl₂ were dissolved in 40 mL 1,4-dioxane and 10 mL water. Heated to 55°C after N₂ gas protection and reacted for 2 h, TLC indicated the raw materials were disappeared. The reaction solution was cooled to room temperature, vacuum filtered to remove insoluble impurities, the filtrate was added water and extracted with dichloromethane. The organic phase was washed once with saturated NaCl solution, dried over, and directly spun-dried and mixed as sample, 1.8 g compound 3 was obtained through column chromatography. LC-MS[M+1]: 480.

### 3. Synthesis of Compound 4

In a 100 mL round-bottomed flask, 1.8 g compound 3 were dissolved in 18 mL ethyl acetate, and 1,4-dioxane hydrochloride was added, and reacted at room temperature overnight. TLC indicated the raw materials were disappeared. The reaction sulution was vacuum filtered to obtain 1 g filtrate cake, which was compound 4. LC-MS [M+1]: 380.

### 4. Synthesis of Compound 5

In a 100 mL three-neck flask, 1 g compound 4 was dissolved in 10 mL DMF. 2 mL N, N-diisopropylethylamine was added after N₂ gas protection, and was stirred under an ice bath for 10 min, 0.4 mL tert-butyl bromoacetate was added, and reacted overnight at room temperature, monitored by TLC until complete. The reaction solution was added water and extracted with ethyl acetate, dried over with anhydrous sodium sulfate, spun-dried, and 1.5 g compound 5 was obtained through column chromatography. LC-MS [M+1]: 494.

### 5. Synthesis of Compound 6

In a 100 mL reaction flask, 1.5 g compound 5 and 228 mg palladium carbon were dissolved in 30 mL methanol. The reaction was proceeded overnight at room temperature after H₂ gas protection. TLC indicated the raw materials were disappeared. The reaction solution was vacuum filtered to remove insoluble impurities, the filtrate was directly spun-dried and mixed as sample, 200 mg compound 6 was obtained through column chromatography. LC-MS [M+1]: 496.

### 6. Synthesis of Intermediate M3

In a 100 mL reaction tube, 200 g compound 6 was dissolved in 1 mL dichloromethane, and 0.2 mL trifluoroacetic acid was added, the reaction was proceeded overnight at room temperature. TLC indicated the raw materials were disappeared. The reaction solution was concentrated to remove the solvent, 283 mg intermediate M3 was obtained. LC-MS [M+1]: 440.

### Synthesis of the compound TA-003

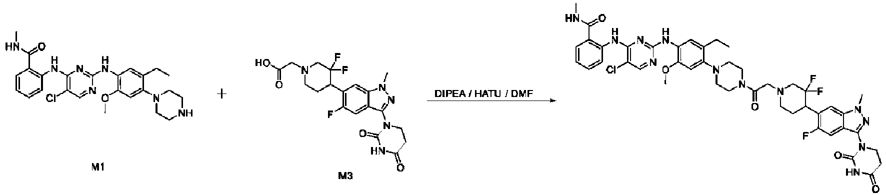

In a 100 mL reaction tube, 98 mg compound M1 and 139 mg compound M3 were dissolved in 1 mL N, N-dimethylformamide, cooled to 0°C in an ice-water bath, N, N-diisopropylethylamine and HATU were added under stirring and reacted for 1 hour in an ice-water bath under nitrogen protection, then warmed to room temperature naturally, proceeded overnight, and monitored by TLC until complete. Extracted with dichloromethane and water, the organic phase was dried over, and 80 mg compound TA-003 was obtained through column chromatography. LC-MS [M+1]: 917.8.

¹H NMR (400 MHz, DMSO) δ 11.62 (s, 1H), 10.60 (s, 1H), 8.73 (d, *J* = 4.4 Hz, 1H), 8.57 (d, *J* = 7.0 Hz, 1H), 8.23 (s, 1H), 8.14 (s, 1H), 7.69 (t, *J=* 5.8 Hz, 2H), 7.50 (s, 1H), 7.43 (d, *J=* 10.4 Hz, 1H), 7.31 (t, *J =* 7.5 Hz, 1H), 7.05 (t, *J =* 7.5 Hz, 1H), 6.82 (s, 1H), 3.99 (s, 3H), 3.91 (t, *J* = 6.7 Hz, 2H), 3.75 (s, 6H), 3.59 (s, 2H), 3.49 (d, *J* = 15.4 Hz, 3H), 3.06 (d, *J* = 10.3 Hz, 1H), 2.92 (s, 1H), 2.89 (s, 1H), 2.83 (d, *J* = 5.1 Hz, 2H), 2.80 (d, *J* = 4.4 Hz, 3H), 2.75 (dd, *J* = 12.1, 5.6 Hz, 3H), 2.63 (dd, *J* = 14.7, 7.3 Hz, 2H), 2.58 - 2.53 (m, 1H), 2.39 (d, *J* = 12.0 Hz, 1H), 1.86 (d, *J* = 11.9 Hz, 1H), 1.10 (t, *J* = 7.4 Hz, 3H).

Referring to the synthesis method of intermediate compound M1 and the synthesis method of compound TA-003, the following compounds were synthesized respectively:

| Compound No. | compound structure | characterization data |
|---|---|---|
| TA-004 | 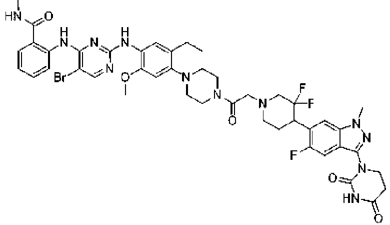 | LC-MS[M+1]: 963.3 |
| TA-019 | 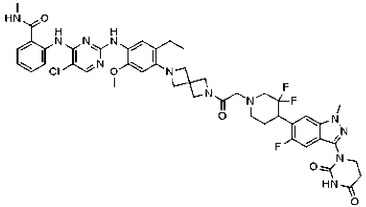 | LC-MS[M+1]: 929.7 |

### Example 1-2: Synthesis of Compound TA-005

### Synthesis of Intermediate Compound M1

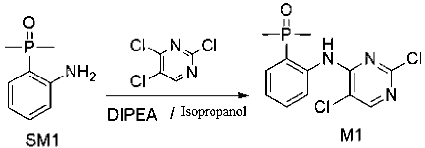

In a 100 mL single-necked flask, 500 mg (2-aminophenyl) dimethylphosphine oxide were dissolved in 10 mL isopropanol, and then 0.7 mL 2,4,5-trichloropyrimidine and 2.9 mL DIPEA were added in sequence. Heated to 100 °C after N₂ gas protection, and proceeded overnight, monitored by TLC until complete, the reaction solution was directly spun-dried and mixed as sample, 1 g intermediate M1 was obtained through column chromatography. LC-MS [M+1]: 317.

### Synthesis of the intermediate compound M2

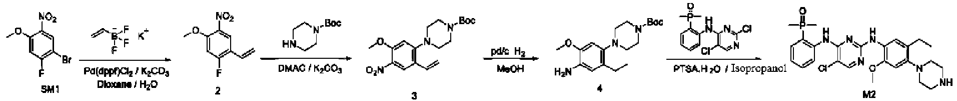

### 1. Synthesis of Compound 2

In a 250 mL three-neck flask, 8.4 g SM1, 9 g boron trifluoride, 2.4 g Pd(dppf)Cl₂, and 6.9 g potassium carbonate were dissolved in 42 mL 1,4-dioxane and 8.4 mL water. Heated to 100 °C after N₂ gas protection,, and proceeded overnight, monitored by TLC until complete. The reaction mixture was cooled to room temperature, vacuum filtered to remove insoluble impurities, and the filtrate was added water and extracted with EA. The organic phase was washed once with saturated NaCl solution, dried over, and directly spun-dried and mixed as sample, 5.3 g compound 2 was obtained through column chromatography. LC-MS [M+1]: 198.

### 2. Synthesis of Compound 3

In a 250 mL three-neck flask, 4.8 g Compound 2, 6.9 g 1-Boc-piperazine, and 16.8 g potassium carbonate were dissolved in 48 mL DMAC. Heated to 100 °C after N₂ gas protection, and proceeded overnight, TLC indicated the raw materials were disappeared. The reaction solution was cooled to room temperature, vacuum filtered to remove insoluble impurities, the filtrate was added water and extracted with EA, the organic phase was washed once with saturated NaCl solution, dried over, and directly spun-dried and mixed as sample, 6.5 g compound 3 was obtained by column chromatography. LC-MS [M+1]: 364.

### 3. Synthesis of Compound 4

In a 100 mL reaction flask, 6.5 g compound 3 and 1.3 g palladium carbon were dissolved in 70 mL methanol. The reaction was proceeded overnight at room temperature after H₂ gas protection. TLC indicated the raw materials were disappeared. The reaction solution was vacuum filtered to remove insoluble impurities. The filtrate was directly spun-dried and mixed as sample, 5.6 g compound 4 was obtained through column chromatography. LC-MS [M+1]: 336.

### 4. Synthesis of Intermediate M2

In a 100 mL reaction tube, 200 mg compound 4, 188 mg intermediate M1, and 147 mg p-toluenesulfonic acid monohydrate were dissolved in 2 mL isopropanol. Heated to 90 °C after N₂ gas protection, and proceeded overnight, monitored by TLC until complete, quenched with a small amount of saturated sodium bicarbonate solution, extracted with ethyl acetate, dried over with anhydrous sodium sulfate, spun-dried, and 136 mg intermediate M2 was obtained through column chromatography. LC-MS [M+1]: 515.

### Synthesis of the compound TA-005

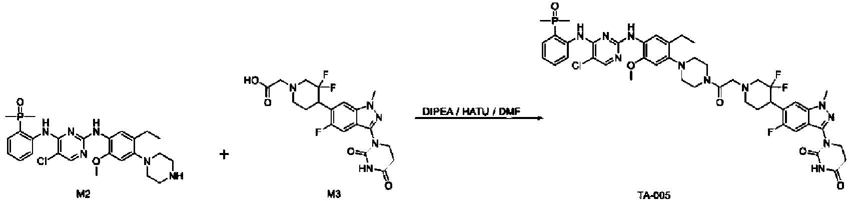

In a 100 mL reaction tube, 105 mg compound M2 and 144 mg intermediate compound M3 of Example 1-1 were dissolved in 1 mL N, N-dimethylformamide. Cooled to 0°C in an ice-water bath, and N, N-diisopropylethylamine and HATU was added under stirring. Reacted in an ice-water bath under nitrogen protection for 1 hour, warmed to room temperature naturally, and proceeded overnight, monitored by TLC until complete. Extracted with dichloromethane and water, the organic phase was dried over, and 18 mg compound TA-005 was obtained by column chromatography. LC-MS [M+1]: 936.

¹H NMR (400 MHz, DMSO) δ 11.13 (s, 1H), 10.59 (s, 1H), 8.42 (s, 1H), 8.18 (s, 1H), 8.11 (s, 1H), 7.69 (d, *J* = 5.3 Hz, 1H), 7.53 - 7.41 (m, 3H), 7.31 (d, *J* = 7.8 Hz, 1H), 7.05 (t, *J* = 7.3 Hz, 1H), 6.81 (s, 1H), 3.98 (s, 3H), 3.91 (t, *J* = 6.7 Hz, 2H), 3.75 (s, 4H), 3.25 (s, 2H), 3.06 (d, *J* = 9.3 Hz, 2H), 2.90 (s, 2H), 2.83 (s, 2H), 2.76 (t, *J* = 6.7 Hz, 3H), 2.59 (dt, *J* = 17.8, 8.8 Hz, 3H), 2.43 - 2.30 (m, 2H), 2.03 - 1.93 (m, 1H), 1.88 (s, 1H), 1.76 (d, *J* = 13.5 Hz, 6H), 1.35 - 1.28 (m, 1H), 1.20 - 1.14 (m, 1H), 1.09 (t, *J* = 7.5 Hz, 3H).

Referring to the method described in Example 1-2, the following compounds were synthesized:

| Compound No. | compound structure | characterization data |
|---|---|---|
| TA-006 | 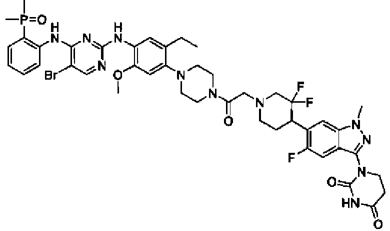 | LC-MS[M+1]: 982.6 |
| TA-009 | 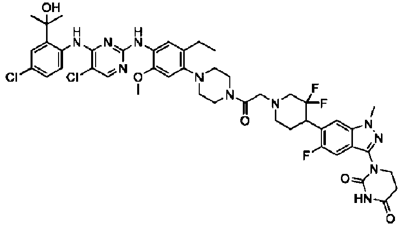 | LC-MS[M+1]: 952.6 |
| TA-011 | 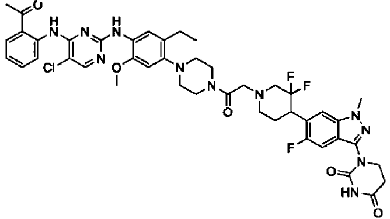 | LC-MS[M+1]: 902.6 |
| TA-013 | 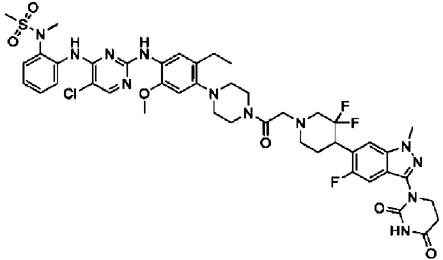 | LC-MS[M+1]: 967.7 |
| TA-017 | 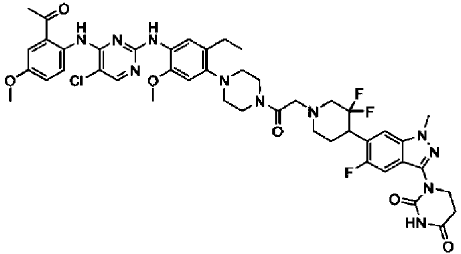 | LC-MS[M+1]: 932.7 |
| TA-023 | 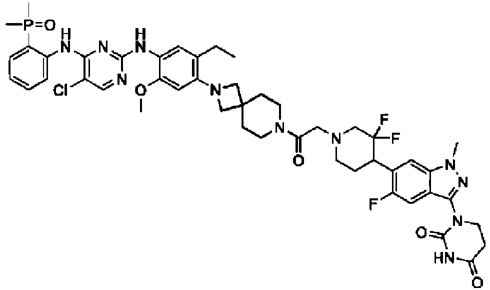 | LC-MS[M+1]: 976.7 |
| TA-025 | 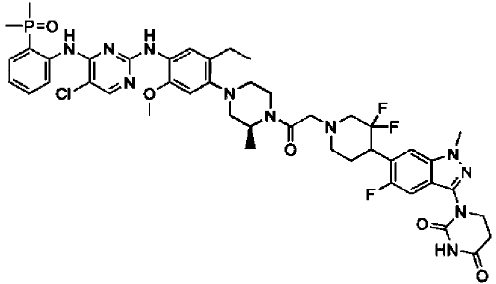 | LC-MS[M+1]: 950.7 |

The chiral compounds obtained through high-pressure preparative chromatography were as follows:

| Compound No. | compound structure | characterization data |
|---|---|---|
| TC-170 | 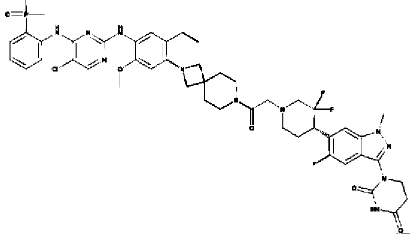 | LC-MS[M+1]: 976.7 |
| TC-171 | 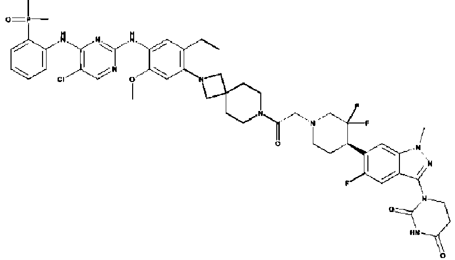 | LC-MS[M+1]: 976.7 |

### Example 1-3: Synthesis of compound TC-159

### Synthesis of intermediate compound M1

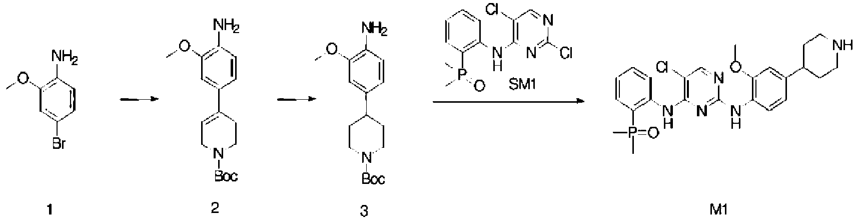

### 1. Synthesis of Compound 2

In a 50 mL three-necked flask, 500 mg Compound 1, 917 mg N-Boc-1,2,5,6-tetrahydropyridine-4-boronic acid pinacol ester, 787 mg sodium carbonate, and 181 mg pd(dppf)Cl₂ were dissolved in 10 mL 1, 4-dioxane and 1 mL H₂O. Heated to 80°C after N₂ gas protection and proceeded overnight. TLC indicated the raw materials were disappeared. The reaction solution was cooled to room temperature, vacuum filtered to remove insoluble impurities, and the filtrate was added water and extracted with dichloromethane. The organic phase was washed once with saturated NaCl solution, dried over, spun-dried and mixed as sample. 300 mg compound 2 was obtained through column chromatography. LC-MS [M+1]: 305

### 2. Synthesis of Compound 3

In a 50 mg stoppered vial, 300 mg Compound 2, 15 mg palladium carbon catalyst, and 4 mL methanol was added, maintained the pressure after exchanging the hydrogen balloon three times. Proceed overnight at room temperature, complete reaction was confirmed by TLC, vacuum filtered through diatomaceous earth pad to remove the palladium carbon. The supernatant was spun-dried to obtain 100 mg Compound 3.

### 3. Synthesis of Intermediate M1

In a 100-mL reaction flask, 103 mg compound SM1, 100 mg compound 3, and 81 mg p-toluenesulfonic acid monohydrate were dissolved in 2 mL isopropanol. Heated to 90°C after N₂ gas protection, and proceeded overnight, monitored by TLC until complete, quenched with a small amount of saturated sodium bicarbonate solution, extracted with ethyl acetate, dried over with anhydrous sodium sulfate, spun-dried, and 50 mg intermediate M1 was obtained by column chromatography. LC-MS [M+1]: 486.

### Synthesis of the compound TC-159

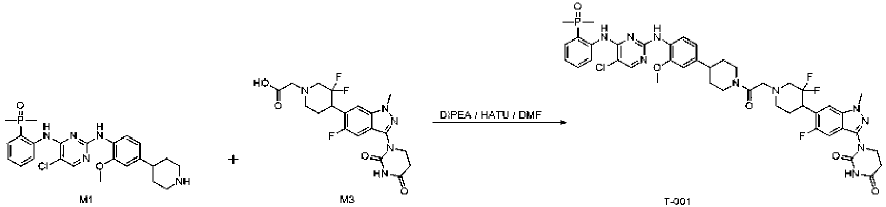

Referring to the synthesis method of TA-003 in Example 1-1, compound TC-159 was synthesized using intermediate M3 from Example 1-1. LC-MS [M+1]: 907.6.

### Example 1-4

### Synthesis of intermediate compound M1

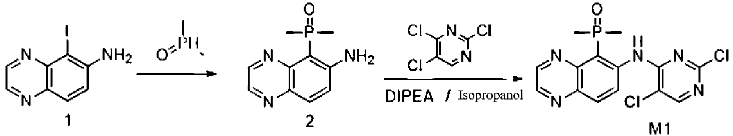

### 1. Synthesis of Compound 2

In a 100 mL round-bottomed flask, 735 mg SM1, 317 mg dimethylphosphinic oxide, 12 mL DMF, and 3 mL water was mixed, then purged with nitrogen, and then 862 mg potassium phosphate, 60 mg palladium acetate, and 151 mg Xantphos were added under nitrogen protection, then proceeded overnight at 120°C. TLC indicated the reaction was complete, then extracted with ethyl acetate and purified by column chromatography, 283 mg compound 2 was obtained.

### 2. Synthesis of Intermediate Compound M1

In a 100 mL single-necked flask, 140 mg compound 2 were dissolved in 3 mL isopropanol, then 0.14 mL 2,4,5-trichloropyrimidine and 0.6 mL DIPEA were added sequentially. Heated to 100 °C after N₂ gas protection, and proceeded overnight, monitored by TLC until complete. The reaction solution was directly spun-dried and mixed as sample, 81 mg intermediate M1 was obtained by column chromatography. LC-MS [M+1]: 368.

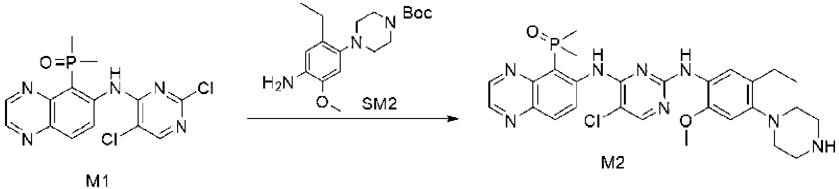

### Synthesis of Intermediate Compound M2

In a 100-mL reaction tube, 81 mg compound M1, 74 mg intermediate SM2, and 54 mg p-toluenesulfonic acid monohydrate were dissolved in 2 mL isopropyl alcohol. Heated to 90°C after N₂ gas protection, proceeded overnight, monitored by TLC until complete, then quenched with a small amount of saturated sodium bicarbonate solution, extracted with ethyl acetate, dried over with anhydrous sodium sulfate, spun-dried, and 76 mg intermediate M2 was obtained through column chromatography. LC-MS [M+1]: 567.

### Synthesis of the compound TA-007

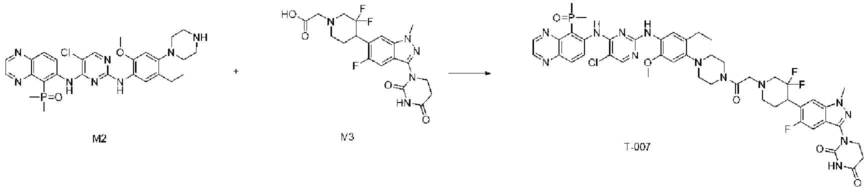

Referring to the synthesis method of TA-003 in Example 1-1, compound TA-007 was synthesized using intermediate M3 from Example 1-1. LC-MS [M+1]: 988.7.

### Example 1-5: Synthesis of compound TA-015

### Synthesis of intermediate compound M1

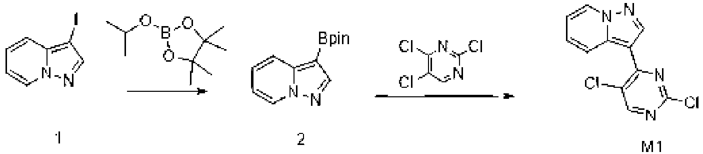

### 1. Synthesis of Compound 2

In a 250 mL round-bottomed flask, 3.8 g compound 1, 5.2 mL isopropyl pinacolate, and 95 mL tetrahydrofuran was stirred and dissolved. Cooled to 0°C in an ice-water bath for 10 minutes, then 12 mL isopropylmagnesium chloride was slowly added dropwise. After reacted at room temperature for 2 hours, quenched with water, extracted with ethyl acetate, and purified by column chromatography to obtain 3.4 g Compound 2.

### 2. Synthesis of Intermediate M1

In a 250-mL three-neck flask, 3.4 g compound 2, 2.53 g trichloropyrimidine, 2.94 g sodium carbonate, 85 mL acetonitrile, and 17 mL water were added, after stirred until homogeneous, 1 g tetraphenylphosphinepalladium was added under nitrogen protection, and proceeded overnight at 80°C. After purified by column chromatography, 1.7 g intermediate M1 was obtained.

### Synthesis of intermediate compound M2

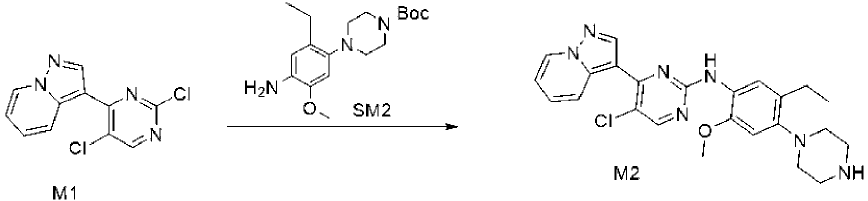

Referring to the synthesis method of intermediate, M2 in Example 3 M2 was synthesized. LC-MS [M+1]: 463.

### Synthesis of compound TA-015

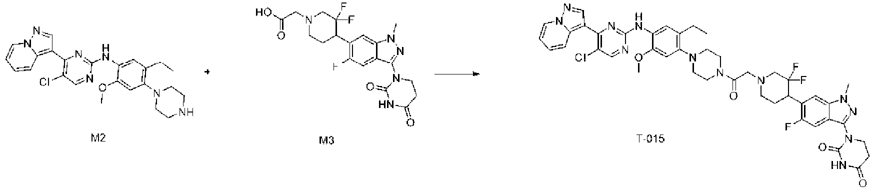

Referring to the synthesis method of TA-003 in Example 1-1, TA-015 was synthesized. LC-MS [M+1]: 855.6.

### Example 1-6: Synthesis of compound TA-039

### Synthesis of intermediate compound M1

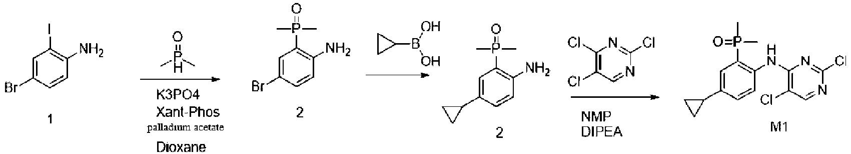

### 1. Synthesis of Compound 2

In a 100ml round-bottomed flask, 1g Compound 1, 393mg dimethylphosphine oxide, and 20ml dioxane was added, after mixed well the nitrogen was purged, 1,066mg potassium phosphate, 38mg palladium acetate, and 97mg Xantphos were added under the nitrogen protection, and proceeded overnight at 80°C. TLC indicated the reaction was complete. After extraction with ethyl acetate, purified by a column to obtain 705mg Compound 2.

### 2. Synthesis of Compound 3

In a 100ml round-bottomed flask, 500 mg Compound 2, 520 mg cyclopropylboronic acid, 5 mL dioxolane, and 1 mL water were added, after mixed well the nitrogen was purged, 1.7 g potassium phosphate, 45 mg palladium acetate, and 113 mg tricyclohexylphosphine were added under nitrogen protection, proceeded overnight at 80°C. TLC indicated the reaction was complete. After extraction with ethyl acetate, purified by a column to obtain 151 mg Compound 3.

### 3. Synthesis of Intermediate M1

In a 250mL three-neck flask, 100 mg compound 3, 176 mg trichloropyrimidine, 199 mg potassium carbonate, and 2 mL DMF were added, mixed well followed by the nitrogen protection, proceeded overnight at 100°C under nitrogen protection, purified by column chromatography to obtain 93 mg intermediate M1.

### Synthesis of compound TA-039

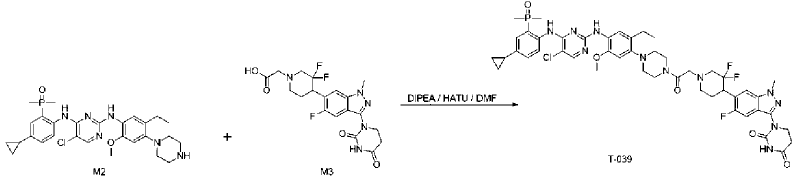

Referring to the synthesis method of TA-003 in Example 1-1, compound TA-039 was synthesized using intermediate M3 from Example 1-1. LC-MS [M+1]: 977.

Referring to the methods of Examples 1-6, the following compounds were synthesized:

| Compound No. | compound structure | characterization data |
|---|---|---|
| TA-031 | 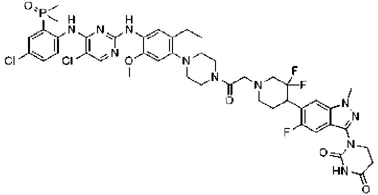 | LC-MS[M+1]: 971 |
| TA-035 | 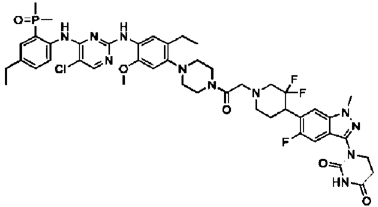 | LC-MS[M+1]: 965 |
| TA-041 | 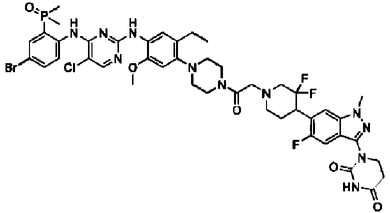 | LC-MS[M+1]: 1016 |

### Comparative Example 1-1: Synthesis of Comparative Example Compound C1

### Synthesis of intermediate compound M1

The synthesis route as followed:

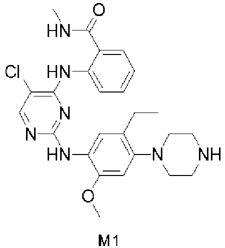

Intermediate M1 was synthesized by referring to the synthesis method of Intermediate M1 in Example 1-1.

### Synthesis of intermediate compound M2

### The synthesis route as followed:

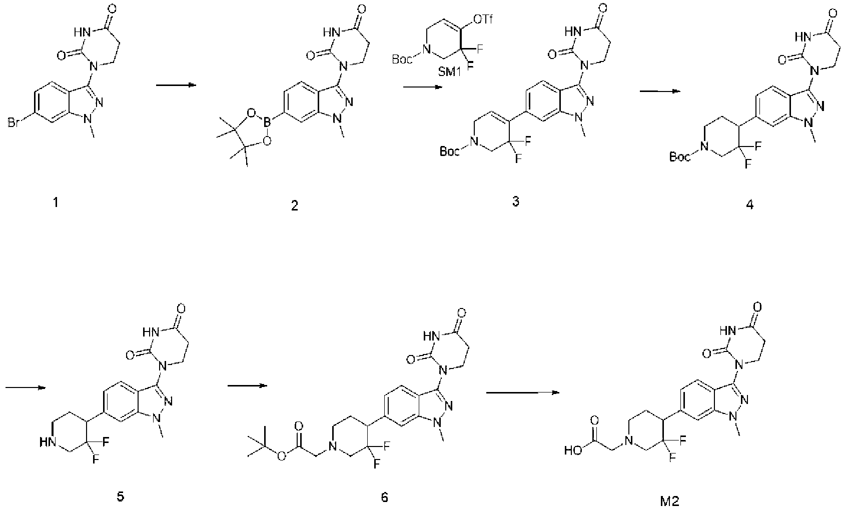

### 1. Synthesis of Compound 2

In a 100mL round-bottomed flask, compound 1 (1.0 g, 1.0 eq), Bis(pinacolato)diboron (1.18 g, 1.2 eq), 15 mL 1,4-dioxane, potassium acetate (911 mg, 3.0 eq), and PdCl₂(dppf) (113 mg, 0.12 eq) were mixed well. After purging with nitrogen, heated to 85°C and proceeded overnight, monitored by TLC until complete. Extracted with ethyl acetate, dried over with anhydrous sodium sulfate, and spun-dried, and separated by column chromatography to obtain 1.2 g compound 2. LC-MS [M+1]: 372.

### 2. Synthesis of Compound 3

In a 100mL three-neck flask, 400 mg Compound 2 (1.2 equivalents), 484 mg SM1 (1.0 equivalents), 345 mg potassium carbonate (3.0 equivalents), 10 mL 1,4-dioxane, and 2.5 mL water were mixed well under nitrogen protection. 80 mg [1,1'-Bis(diphenylphosphino)ferrocene] dichloropalladium(II) (0.12 equivalents) were added in sequence, ted to 55°C, and reacted for 2 hours, TLC indicated the reaction was complete, vacuum filtered through a diatomaceous earth pad, the mother liquor was collected, the solvent was removed from the mother liquor by rotary evaporation, 30 mL water was added, and extracted three times with 15 mL ethyl acetate. The organic phases were combined, mixed, and passed through a column, 500 mg compound 3 was obtained by column chromatography. LC-MS [M+1]: 462.

### 3. Synthesis of Compound 4

In a 50mL single-necked flask, compound 3 (500 mg, 1.0 eq), palladium carbon catalyst (50 mg, 5 wt%), and 5 mL methanol were mixed well. Maintained the pressure after purging three times with hydrogen gas, and reacted at room temperature for 20 hours. TLC indicated the raw materials were reacted completely. Vacuum filtered through a diatomaceous earth pad, the mother liquor was collected, and 400 mg compound 4 was obtained.

### 4. Synthesis of Compound 5

In a 100 mL single-necked flask, compound 4 was added and 10 mL tetrahydrofuran was added to dissolve. Cooled in an ice-water bath and 4 M hydrochloric acid solution of dioxolone was added dropwise. After the addition was complete, reacted at 40°C for 2 hours. The solvent was removed by rotary evaporation, and 330 mg compound 5 was obtained. LC-MS [M+1]: 364.

### 5. Synthesis of Compound 6

In a 100-mL single-necked flask, 330 mg compound 5, 4 mL N, N-dimethylformamide, 1.2 mL N, N-diisopropylethylamine (5.0 eq), and 195 mg tert-butyl bromoacetate (1.1 eq) were added. Stirred at room temperature overnight. TLC indicated the reaction was complete. Extracted with ethyl acetate and water, the organic phase was dried over, vacuum filtered, and 370 mg compound 6 was obtained by column chromatography, LC-MS [M+1]: 479.

### 6. Synthesis of Intermediate M2

In 100-mL round-bottomed flask, 350 mg compound 6 and 3 mL dichloromethane were added. 2 mL trifluoroacetic acid was added dropwise, and stirred at room temperature overnight. TLC indicated the reaction was complete. The solvent was removed by rotary evaporation, 150 mg M2 was obtained, LC-MS [M+1]: 420.

### Comparative Example 1-1 Synthesis of compound C1

### The synthetic route as followed:

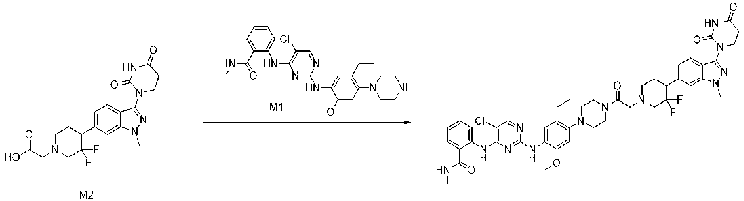

In a 50mL single-necked flask, compounds M2 (150 mg, 1.0 eq) and M1 (176 mg, 1.0 eq) were dissolved in 4 mL N, N-dimethylformamide, cooled to 0°C in an ice-water bath, and N, N-diisopropylethylamine and HATU (176 mg, 1.3 eq) were added under stirring. Reacted in an ice-water bath under nitrogen protection for 1 hour, warmed to room temperature. TLC indicated the reaction was complete. Extracted with ethyl acetate and water; the organic phase was dried over, vacuum filtered, and compound C1 was obtained by column chromatography, LC-MS [M+1]: 899.6.

1H NMR (400 MHz, DMSO-d6) δ11.69 (s, 1H), 10.64 (s, 1H), 8.80 (s,1H), 8.70 - 8.54 (m, 1H), 8.29 (s,1H), 8.21 (s, 1H), 7.76 (d, J = 7.8 Hz,1H), 7.71 - 7.52 (m, 3H), 7.38 (s,1H), 7.13 (q, J = 8.5, 7.6 Hz, 2H),6.88 (s, 1H), 4.12 - 3.93 (m, 6H),3.79 (d, J = 18.4 Hz, 5H), 3.68 (s,3H), 3.53 - 3.51 (m, 1H), 3.20 (d, J =7.6 Hz, 2H), 3.10 (s, 1H), 2.98 (s,2H), 2.93 - 2.78 (m, 8H), 2.69 (d, J =7.4 Hz, 1H), 2.33 (d, J = 12.9 Hz,1H), 2.05 (s, 1H), 1.94 (d, J = 13.0Hz, 1H), 1.16 (t, J = 7.6 Hz, 3H).

### Example of intermediate system preparation:

### Synthesis of intermediate INT-1

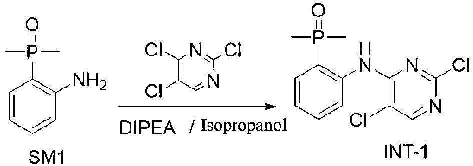

In a 100 mL single-necked flask, 500 mg (2-aminophenyl)dimethylphosphine oxide was dissolved in 10 mL isopropanol. 0.7 mL 2,4,5-trichloropyrimidine and 2.9 mL DIPEA were added in sequence. Heated to 100 °C after N₂ gas protection, proceeded overnight, and monitored the reaction by TLC until complete. The reaction solution directly spun-dried and mixed as sample, 1 g intermediate INT-1 was obtained through column chromatography. LC-MS [M+1]: 317.

### Synthesis of intermediate compound INT-2

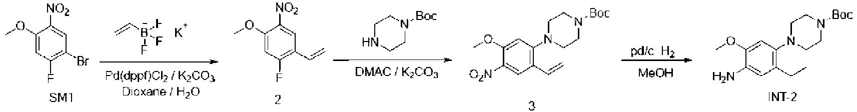

### 1. Synthesis of Compound 2

In a 250 mL three-neck flask, 8.4 g SM1, 9 g boron trifluoride, 2.4 g Pd(dppf)Cl₂, and 6.9 g potassium carbonate were dissolved in 42 mL 1,4-dioxane and 8.4 mL water. Heated to 100 °C after the N₂ gas protection, proceeded overnight, monitored by TLC until complete. The reaction solution was cooled to room temperature, vacuum filtered to remove insoluble impurities, and the filtrate was added water and extracted with EA. The organic phase was washed once with saturated NaCl solution, dried over, and directly spun-dried and mixed as sample. 5.3 g compound 2 was obtained through column chromatography. LC-MS [M+1]: 198.

### 2. Synthesis of Compound 3

In a 250 mL three-neck flask, 4.8 g Compound 2, 6.9 g 1-Boc-piperazine, and 16.8 g potassium carbonate were dissolved in 48 mL DMAC. Heated to 100°C after the N₂ gas protection and proceeded overnight. TLC indicated the raw materials were disappeared. The reaction solution was cooled to room temperature, vacuum filtered to remove insoluble impurities, and the filtrate was added water and extracted with EA. The organic phase was washed once with saturated NaCl solution, dried over, and directly spun-dried and mixed as sample. 6.5 g compound 3 was obtained by column chromatography. LC-MS [M+1]: 364.

### 3. Synthesis of Intermediate INT-2

In a 100 mL reaction flask, 6.5 g compound 3 and 1.3 g palladium on carbon were dissolved in 70 mL methanol. Proceeded overnight at room temperature after the N₂ gas protection. TLC indicated the raw materials were disappeared. The reaction solution was vacuum filtered to remove insoluble impurities. The filtrate was directly spun-dried and mixed as sample, 5.6 g intermediate INT-2 was obtained by column chromatography. LC-MS [M+1]: 336.

### Synthesis of intermediate INT-3

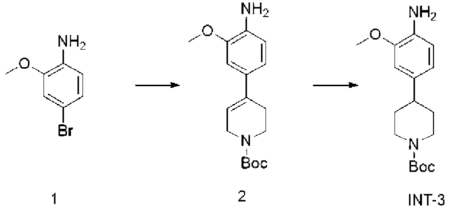

### 1. Synthesis of Compound 2

In a 50 mL three-necked flask, 500 mg Compound 1, 917 mg N-Boc-1,2,5,6-tetrahydropyridine-4-boronic acid pinacol ester, 787 mg sodium carbonate, and 181 mg Pd(dppf)Cl₂ were dissolved in 10 mL 1, 4-dioxane and 1 mL H₂O. Heated to 80°C after the N₂ gas protection and proceeded overnight. TLC indicated the raw materials were disappeared. Cooled to room temperature, vacuum filtered to remove insoluble impurities, and the filtrate was added water and extracted with dichloromethane. The organic phase was washed once with saturated NaCl solution, dried over, and directly spun-dried and mixed as sample. 300 mg compound 2 was obtained through column chromatography. LC-MS [M+1]: 305

### 2. Synthesis of Intermediate IN-3

In a 50 mg round-bottomed flask, 300 mg compound 2, 15 mg palladium on carbon catalyst, and 4 mL methanol were added and mixed well, maintained the atmosphere after replacing gas three times using a hydrogen balloon, proceeded overnight at room temperature; TLC confirmed the reaction was complete. the palladium carbon was vacuum filtered and removed through diatomaceous earth pad, the mother liquor was spun-dried to obtain 100 mg intermediate IN-3.

### Synthesis of intermediate compound INT-4

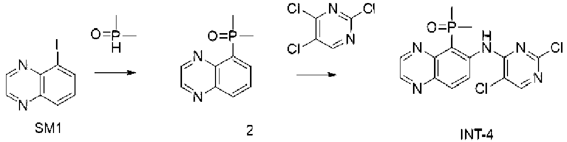

### 1. Synthesis of Compound 2

In a 100mL round-bottomed flask, 735 mg SM1, 317 mg dimethylphosphinic oxide, 12 mL DMF, and 3 mL water were added and mixed well, after purging with nitrogen, 862 mg potassium phosphate, 60 mg palladium acetate, 151 mg Xantphos were added under nitrogen protection. Proceeded overnight at 120°C. TLC indicated the reaction was complete. Extracted with ethyl acetate and column purification, 283 mg compound 2 was obtained.

### 2. Synthesis of Intermediate Compound INT-4

In a 100 mL single-necked flask, dissolved 140 mg Compound 2 in 3 mL isopropanol. Added 0.14 mL 2,4,5-trichloropyrimidine and 0.6 mL DIPEA sequentially. Under N₂ protection, heated to 100°C and reacted overnight. The reaction was Monitored by TLC until complete. The reaction mixture was centrifuged directly and mixed as the sample, and 81 mg intermediate INT-4 was obtained by column chromatography. LC-MS [M+1]: 368.

### Synthesis of intermediate compound INT-5

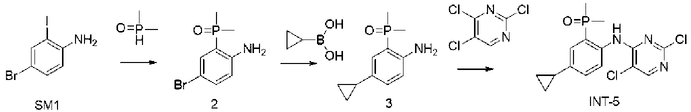

### 1. Synthesis of Compound 2

In a 100ml round-bottomed flask 1g compound 1, 393mg dimethylphosphine oxide, and 20ml dioxane were added and mixed well, after purging with nitrogen, 1,066 mg potassium phosphate, 38 mg palladium acetate, and 97 mg Xantphos were added under the nitrogen protection, and proceeded overnight at 80°C. TLC indicated the reaction was complete. Extracted with ethyl acetate, purified by a column to obtain 705 mg Compound 2.

### 2. Synthesis of Compound 3

In a 100ml round-bottomed flask, 500 mg Compound 2, 520 mg cyclopropylboronic acid, 5 mL dioxolane, and 1 mL water were added and mixed well, after purging with nitrogen. 1.7 g potassium phosphate, 45 mg palladium acetate, and 113 mg tricyclohexyl phosphine were added under the nitrogen protection, proceeded overnight at 80°C. TLC indicated the reaction was complete. Extraction with ethyl acetate, purified by a column to obtain 151 mg Compound 3.

### 3. Synthesis of Intermediate INT-5

In a 250-mL three-neck flask, 100 mg Compound 3, 176 mg trichloropyrimidine, 199 mg potassium carbonate, and 2 mL DMF were added and stirred to homogenize, protected with nitrogen and proceeded overnight at 100°C. 93 mg Intermediate INT-5 was obtained by column chromatography.

### Synthesis of intermediate compound INT-6

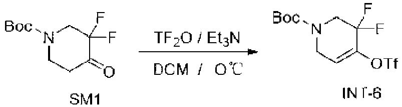

In a 250 mL three-neck flask, 8.3 g SM1 was dissolved in 100 mL dichloromethane, triethylamine was added after the N₂ gas protection, cooled to 0 °C in an ice bath, TF₂O was slowly added dropwise. proceeded overnight at room temperature, monitored the reaction by TLC until complete. The reaction solution was added water and extracted with EA, the organic phase was washed once with saturated NaCl solution, dried over, spun-dried and mixed as sample, and 2 g intermediate INT-6 was obtained by column chromatography.

### Synthesis of intermediate compound INT-7

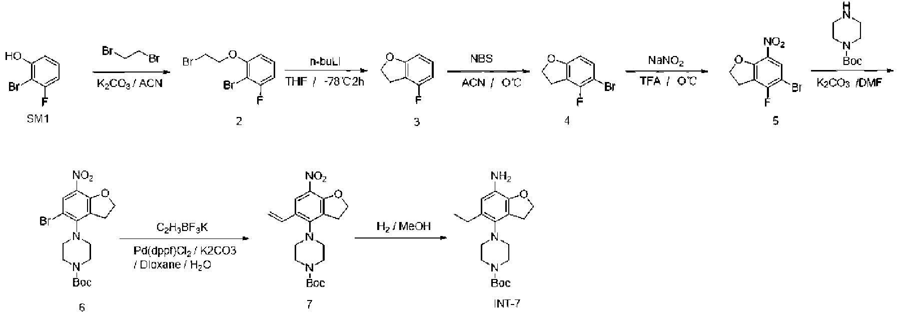

### Synthesis of Compound 2

In a 250 mL round-bottomed flask, 15.2 mL 1,2-dibromoethane, and 14.5 g potassium carbonate were added and dissolved in 67 mL CAN, heated to 85°C and proceeded overnight. After TLC indicated the reaction was complete, the reaction solution was cooled to room temperature, then spun-dried and passed through a column to obtain 8.47 g Compound 2.

### Synthesis of Compound 3

3 g Compound 2 was added to a 250mL three-neck flask and dissolved in 38 mL THF, and then protected with nitrogen, cooled to -78°C in a -80°C environment, and 4 mL n-butyllithium was slowly added dropwise. After the addition is complete, the reaction was continued at -78°C for 2 h, then stirred at room temperature overnight. After TLC indicated the reaction was complete, quenched with ammonium chloride in an ice bath. The organic solvent was removed by centrifugation, and extracted with ethyl acetate. The organic phase was dried over and passed through a column to obtain 1.2 g colorless, oily Compound 3.

### Synthesis of Compound 4

1.2 g Compound 3 was added in a 100-mL round-bottomed flask and dissolved in 12 mL ACN, and protected with nitrogen, and stirred on an ice bath for 10 min. 1.7 g NBS was added and stirred at room temperature for 2 h. the reaction was complete, 1.6 g compound 4 was obtained after purification by column chromatography.

### Synthesis of Compound 5

In a 100 mL round-bottomed flask, 1.6 g compound 4 was dissolved in 13 mL TFA, stirred on an ice bath for 10 min under nitrogen protection, then sodium nitrite was added and warmed to room temperature naturally, the reaction proceeded overnight. After TLC indicated the reaction was complete, the solution was concentrated to remove TFA. The residue solution was quenched with saturated sodium bicarbonate solution, extracted with ethyl acetate, and the organic phase was dried over and concentrated., 874 mg compound 5 was obtained after purification by column chromatography.

### Synthesis of Compound 6

874 mg compound 5, 212 mg N-Boc-piperazine, and 210 mg potassium carbonate were added to a 10 mL reaction tube, dissolved in 4 mL DMF, and protected with nitrogen. Heated to 65°C and proceeded overnight. After TLC indicated the reaction was complete, the reaction solution was cooled to room temperature, added water and extracted with ethyl acetate. The organic phase was washed twice with saturated saline solution, dried over and concentrated, and 189 mg compound 6 was obtained after purification by column chromatography.

### Synthesis of Compound 7

189 mg compound 6, 118 mg C₂H₃BF₃K, 91 mg potassium carbonate, and 32 mg Pd(dppf)Cl₂ were added to a 10 mL reaction tube, dissolved with 2 mL 1,4-dioxane, then 0.4 mL water was added, and heated to 100°C and proceeded for 4 h under nitrogen protection. After TLC indicated the reaction was complete, the reaction solution was cooled to room temperature and 135 mg compound 7 was obtained after purification by column chromatography.

### Synthesis of Intermediate INT-7

In a 10 mL reaction tube, 135 mg compound 7 and 13 mg palladium carbon were added and dissolve with 3 mL methanol, then proceeded overnight at room temperature under hydrogen protection. After TLC indicated the reaction was complete, vacuum filtered to remove the palladium carbon, and the filtrate was concentrated to obtain 137 mg INT-7.

### Synthesis of intermediate compound INT-8

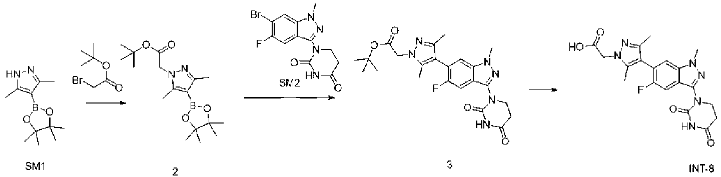

### Synthesis of Compound 2

1 g SM1, 0.83 g tert-butyl bromoacetate, 2.2 g cesium carbonate, and 10 mL acetonitrile was added to a 100 mL round-bottomed flask. Heated to 55°C under nitrogen protection, and reacted for 7 hours. After TLC indicated the reaction was complete, the solvent was removed by rotary evaporation, mixed sample and passed through column to obtain 4 g pale yellow oil compound 2.

### Synthesis of Compound 3

200 mg SM2, 257 mg Compound 2, 381 mg cesium carbonate, 64 mg Pd(dppf)Cl₂, 10 mL 1,4-dioxane, and 1 mL water was added to a 100mL single-necked flask. Heated to 90°C under nitrogen protection and reacted overnight. TLC indicated the reaction was complete, 100mg compound 3 was obtained by directly column chromatography. LC-MS [M+1]: 471

### Synthesis of Compound INT-8

In a 50mL single-necked flask, 100 mg Compound 3 was dissolved in 4 mL dichloromethane. 1 mL trifluoroacetic acid was slowly added dropwise and reacted at room temperature for 3 hours. The reaction solution was spun-dried to obtain the crude product for the next step.

### Synthesis of intermediate compound INT-9

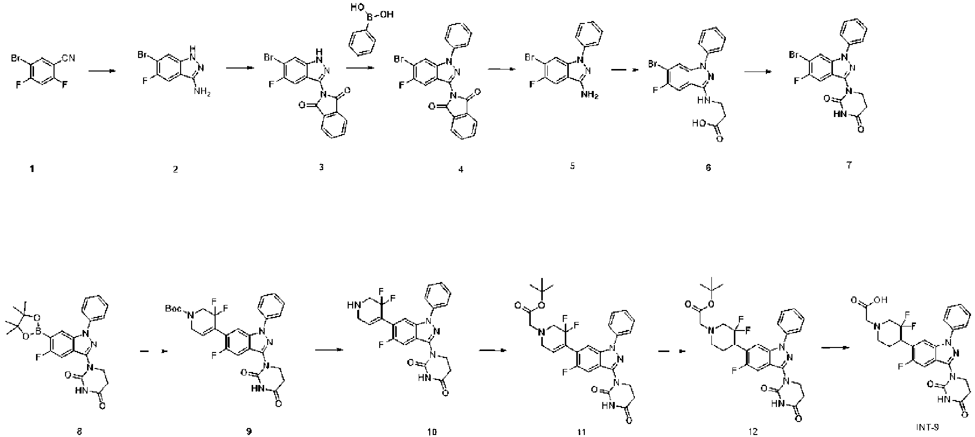

### Synthesis of Compound 2

In a 250 mL round-bottomed flask, 5 g compound 1 and 5 mL ammonia solution were heated to 120°C in 10 mL n-butanol and reacted overnight. After TLC indicated the reaction was complete, the reaction solution was added water and extracted with ethyl acetate. The organic phases were combined, spun-dried and passed through a column to obtain 3.8 g compound 2.

### Synthesis of Compound 3

In a 100 mL round-bottomed flask, 1 g Compound 2 and 640 mg phthalic anhydride were mixed. Heated to 170°C and stirred for 6 hours in the molten state. Stopped heating and cooled to room temperature. 20 mL a 1:1 mixture of dichloromethane and methanol was added, and triturated for 30 minutes at room temperature. the filtrate was collected by vacuum filtration to obtain 1.3 g a white solid.

### Synthesis of Compound 4

1 g Compound 3, 510 mg phenylboronic acid, 750 mg copper acetate, 0.5 mL pyridine, and 50 mL dichloromethane were added to a 100 mL single-necked flask. After thorough mixing, purged three times with oxygen balloon. Proceeded overnight under an oxygen atmosphere. After TLC indicated the reaction was complete, the mother liquor was collected by vacuum filtration, 200mg compound 4 was obtained after rotary evaporation and purification by column chromatography. LC-MS: 436

### Synthesis of Compound 5

200 mg Compound 4, 10 mL ammonia water, and 10 mL methanol were added to a 50 mL single-necked flask, and stirred at room temperature overnight. After TLC indicated the reaction was complete, the methanol was removed by rotary evaporation, then the reaction solution was extracted with ethyl acetate. The organic phases were combined and 110 mg a white solid was obtained after purification by column chromatography. LC-MS: 306/308

### Synthesis of Compound 6

150 mg compound 5 and 53 mg acrylic acid thoroughly were mixed well in 5 mL 2 N hydrochloric acid, 16 mg tetrabutylammonium bromide was added. Heated to 100°C under nitrogen protection, and reacted overnight. After TLC indicated the reaction was complete, the reaction was cooled to room temperature and adjusted the pH to 7-8 with a saturated sodium bicarbonate solution in an ice-water bath. Then adjusted the pH to 5-6 with acetic acid. A large amount of solid precipitated, 150 mg filtrate cake was collected by vacuum filtration. LC-MS: 378

### Synthesis of Compound 7

150 mg Compound 6, 56 mg sodium cyanate, and 1.5 mL acetic acid were mixed well. Heated to 60°C and reacted overnight. 1.5 mL 2N hydrochloric acid was added and maintained for 3 hours. Stopped heating, and cooled to room temperature, and the solid was precipitated. The filter cake was collected by vacuum filtration to obtain 80 mg compound 7.

### Synthesis of Compound 8

500 mg compound 7, 632 mg bis(pinacolato)diboron, 1.2 g potassium acetate, and 45 mg Pd(dppf)Cl₂ was uniformly dispersed in 1.4 L 1,4-dioxane. After purging with nitrogen three times, heated to 80°C under nitrogen protection and reacted overnight. After TLC indicated the reaction was complete, directly mixed as sample and separated by column chromatography to obtain 560 mg compound 8 (LC-MS: 451).

### Synthesis of Compound 9

560 mg compound 8, 538 mg M3 from example 2, 404 mg sodium carbonate, and 100 mg Pd(dppf)Cl₂ were uniformly dispersed in 6 mL 1,4-dioxane and 0.6 mL water. After purging with nitrogen, heated to 55°C under nitrogen protection and reacted for 2 hours.

The reaction was terminated when TLC indicated no raw materials remained, stopped the reaction and after the reaction solution was spun-dried, 340 mg compound 9 was obtained after purification by column chromatography. (LC-MS: 542).

### Synthesis of Compound 10

340 mg compound 9 was dissolved in 6 mL ethyl acetate, and 1.6 mL hydrochloric acid/1,4-dioxane was added. Reacted at room temperature for 4 hours. After LC-MS confirmed no raw materials remained, the reaction solution was spun-dried and used in the next step.

### Synthesis of Compound 11

380 mg Compound 10, 53 mg tert-butyl bromoacetate, and 0.2 mL DIPEA were dissolved in 2 mL DMF, reacted at room temperature overnight. After TLC indicated the reaction was complete, the reaction solution was extracted with ethyl acetate, the organic phases were combined and then 350 mg compound 11 was obtained after purification by column chromatography. LC-MS: 556

### Synthesis of Compound 12

300 mg Compound 11 was added to 60 mg palladium carbon catalyst, 3 mL methanol and 3 mL ethyl acetate were mixed thoroughly, purged with hydrogen three times. Sealed reaction in a hydrogen balloon and reacted overnight at 35°C, after TLC indicated the reaction was complete. Vacuum filtered through diatomaceous earth pad, the mother liquor was collected, 170 mg compound 12 was obtained by spun-dry and column chromatography. LC-MS: 558

### Synthesis of Compound INT-9

170 mg compound 12 was dissolved in 2 mL dichloromethane, 1.7 mL trifluoroacetic acid was added dropwise, and reacted at room temperature for 3 hours. After completion, 150 mg Compound INT-9 was obtained by spun-dry.

### Synthesis of intermediate compound INT-10

The synthetic route as followed:

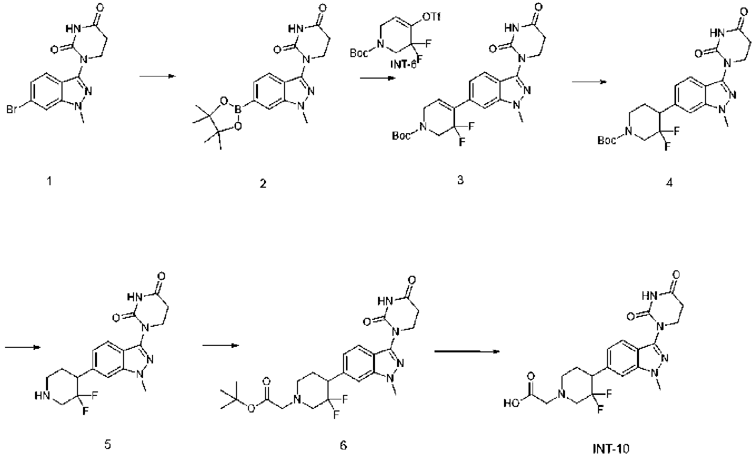

### 1. Synthesis of Compound 2

In a 100 mL round-bottomed flask, Compound 1 (1.0 g, 1.0 eq), pinacol boronic acid (1.18 g, 1.2 eq), 15 mL 1,4-dioxane, potassium acetate (911 mg, 3.0 eq), and PdCl₂(dppf) (113 mg, 0.12 eq) were added. After purging with nitrogen, heated to 85°C and reacted overnight. TLC indicated the reaction was complete. Extracted with ethyl acetate, dried over with anhydrous sodium sulfate, spun-dried, and separated by column chromatography to obtain 1.2 g compound 2. LC-MS [M+1]: 372.

### 2. Synthesis of Compound 3

In a 100 mL three-neck flask, 400 mg Compound 2 (1.2 equivalents), 484 mg SM1 (1.0 equivalents), 345 mg potassium carbonate (3.0 equivalents), 10 mL 1,4-dioxane, and 2.5 mL water were mixed thoroughly under nitrogen protection. Added 80 mg [1,1'-bis(diphenylphosphino) ferrocene] palladium (II) dichloride (0.12 equivalents), and heated to 55°C, reacted for 2 hours. TLC indicated the reaction was complete. Filtered through a diatomaceous earth pad, collected the mother liquor, removed the solvent from the mother liquor by rotary evaporation, then added 30 mL water, and extracted three times with 15 mL ethyl acetate. The organic phases were combined, mixed as sample and passed through a column, 500 mg compound 3 was obtained by column chromatography. LC-MS [M+1]: 462.

### 3. Synthesis of Compound 4

In a 50 mL single-necked flask, 500 mg Compound 3 (1.0 eq), 50 mg palladium carbon catalyst (5 wt%), and 5 mL methanol were added and mixed well. Maintained the pressure after purging three times with hydrogen gas, and proceeded at room temperature for 20 hours. TLC indicated the starting materials. Filtered through a diatomaceous earth pad, and collected the mother liquor to obtain 400 mg Compound 4.

### 4. Synthesis of Compound 5

In a 100 mL single-necked flask, added 400 mg Compound 4 and 10 mL tetrahydrofuran to dissolve. Cooled on an ice-water bath and 4 M hydrochloric acid solution of dioxolone was added dropwise. After completion of the addition, proceeded at 40°C for 2 hours. Removed the solvent by rotary evaporation to obtain 330 mg Compound 5. LC-MS [M+1]: 364.

### 5. Synthesis of Compound 6

330 mg Compound 5, 4 mL N, N-dimethylformamide, 1.2 mL N, N-diisopropylethylamine (5.0 eq), and 195 mg tert-butyl bromoacetate (1.1 eq) were added to a 100mL single-necked flask. Stirred at room temperature overnight. TLC confirmed the reaction was complete. Extracted with ethyl acetate and water, the organic phase was dried over, filtered, and 370 mg compound 6 was obtained by column chromatography., LC-MS [M+1]: 479.

### 6. Synthesis of Intermediate INT-10

In a 100-mL single-necked flask, 350 mg compound 6 and 3 mL dichloromethane were added. Added 2 mL trifluoroacetic acid dropwise and stirred at room temperature overnight. TLC indicated the reaction was complete. The solvent was removed by rotary evaporation to obtain 150 mg INT-10, LC-MS [M+1]: 420.

### Synthesis of intermediate compound INT-11

The synthesis route as followed:

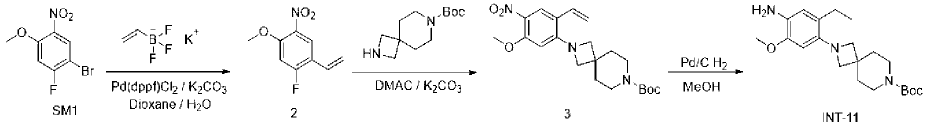

### 1. Synthesis of Compound 2

In a 250 mL three-necked flask, 8.4 g SM1, 9 g boron trifluoride, 2.4 g pd(dppf)Cl₂, and 6.9 g potassium carbonate were dissolved in 42 mL 1,4-dioxane and 8.4 mL H₂O. Heated to 100 °C under N₂ protection, and reacted overnight. The reaction was monitored by TLC until complete. The reaction solution was cooled to room temperature, filtered to remove insoluble impurities, and the filtrate was added water and extracted with EA. The organic phase was washed once with saturated NaCl and dried. Directly spun-dried and mixed as the sample. 5.3 g compound 2 was obtained by column chromatography. LC-MS [M+1]: 198.

### 2. Synthesis of Compound 3

1 g compound 2, 1.7 g tert-butyl 2,7-diazaspiro[3.5]nonane-7-carboxylate, and 2.1 g potassium carbonate were added to a 50 ml single-necked flask. The mixture was dissolved in 10 mL dimethylacetamide and then reacted under nitrogen protection at 110 °C for 5 h. After TLC confirmed the reaction was complete, the reaction solution was cooled to room temperature, extracted with ethyl acetate, the organic phase was washed twice with saturated brine, dried and concentrated, 6 g yellow solid compound 3 was obtained by column chromatography. LC-MS [M+1]: 404.

### 3. Synthesis of intermediate INT-11

6 g compound 3.60 mg palladium carbon was added to a 100 ml single-necked flask, dissolved in 60 mL anhydrous ethanol, and reacted under hydrogen protection at room temperature. After the reaction was complete, the palladium carbon was removed by filtration, and the filtrate was concentrated to obtain 1.9 g intermediate INT-11. LC-MS [M+1]: 376.

### Synthesis of intermediate compound INT-12

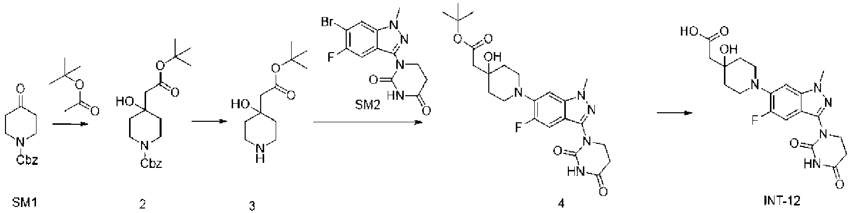

### Synthesis of Compound 2

1.15 g tert-butyl acetate and 20 mL dry tetrahydrofuran were mixed thoroughly and then protected with nitrogen. The mixture was cooled to -78 °C, and 11.8 mL LDA was slowly added dropwise. The reaction was maintained at this temperature for half an hour. Then 3 g SM1 diluted in 20 mL tetrahydrofuran was added dropwise, and maintained at a low temperature for one hour. After the reaction was completed, the reaction solution was quenched with saturated ammonium chloride aqueous solution. Extracted with ethyl acetate, and the organic phase was dried and filtered to obtain 4.38 g compound 2.

### Synthesis of Compound 3

In a 100ml autoclave, 4.38g compound 2 and 1.31g palladium hydroxide on carbon were mixed and then 1,4- dioxane was added and dispersed evenly. After hydrogen purging three times, the reaction was carried out at room temperature overnight. After the reaction was completed, the mother liquor was collected by filtration through a diatomaceous earth pad and spun-dried for later use.

### Synthesis of Compound 4

200 mg compound SM2, 249.3 mg compound 3, 472.5 mg cesium carbonate, and 23 mg PEPPSI-Pd were dispersed in 5 mL 1,4- dioxane. Heated to 100 °C and proceeded overnight under nitrogen protection. After the reaction was completed, directly mixed as sample and spun-dried, passed through a column to obtain 50 mg compound 4 .

### Synthesis of compound INT-12

240 mg compound 4 was dissolved in 3 mL dichloromethane, and a solution of 1,4- dioxane hydrogen chloride was added dropwise. The reaction was carried out at room temperature for 3 hours. The reaction was confirmed to be complete by LC -MS. The solvent was removed by rotary evaporation to obtain the crude compound INT-12.

### Synthesis of intermediate compound INT-13

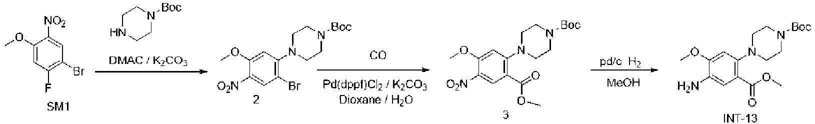

### Synthesis of Compound 2

500 mg SM1, 558 mg boc piperazine, and 552 mg potassium carbonate were added to a 100 ml single-necked flask and dissolved in 10 ml DMAC. Heated to 90 °C and reacted overnight. After TLC confirmed the reaction was complete, the reaction solution was extracted with ethyl acetate and water. The ethyl acetate phase was dried, filtered, then spun-dried and passed through a column to obtain 760 mg compound 2 .

### Synthesis of Compound 3

In a 100 ml single-necked flask, 200 mg compound 2, 56 mg Pd(dppf)Cl₂, 0.7 mL triethylamine, 4 mL DMF, and 4 mL methanol were added. Purged three times with a carbon monoxide balloon, sealed with the balloon, and reacted overnight at 85 °C. After TLC confirmed the reaction was complete, the reaction mixture was extracted with ethyl acetate and water. The organic phases were combined, dried, and spun-dried to obtain 150 mg a yellow oily compound 3 by column chromatography. LC-MS [M+1]: 418

### Synthesis of intermediate INT-13

150 mg compound 3, 10 mg palladium carbon, and 3 mL methanol were added to a 100 ml single-necked flask and mixed. Reacted at 35 °C overnight. After the reaction was completed, the mixture was filtered through a diatomaceous earth pad, and the mother liquor was collected and spun-dried to obtain the crude compound INT-13 .

### Synthesis of intermediate compound INT-14

The synthesis route as followed:

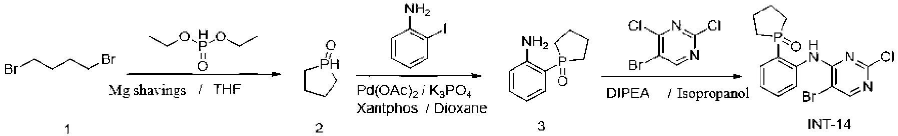

### 1. Synthesis of Compound 2

In a 500ml three-necked flask, 10g magnesium shavings and two grains of iodine were added. After protection with N₂, 150mL tetrahydrofuran was added to dissolve the iodine. 0.1mL 1,4-dibromobutane was added to initiate the reaction. The reaction solution was cooled to 0°C. 12mL 1,4-dibromobutane was dissolved in 40mL tetrahydrofuran and added slowly dropwise, maintained the temperature below 30°C. After the addition was complete, proceeded at room temperature for 2-3 hours. The solution was cooled to 0°C, and 6.8mL diethyl phosphite was added slowly dropwise, maintained the temperature below 20°C. After the addition was complete, proceeded at room temperature. TLC indicated the reaction was complete, the reaction solution was cooled to 0°C, and potassium carbonate solution (30g K2CO3 and 50g H2O) was added slowly dropwise to quench the reaction , maintained the temperature below 10°C. A large amount of solid precipitated. Filtered, and the filtrate was spun-dried and passed through a column chromatography to obtain 1.1g a colorless, transparent liquid compound 2. LC-MS [M+1]: 105.

### 2. Synthesis of Compound 3

In a 100 mL single-necked flask, 1.1 g compound 2, 1.1 g iodoaniline, 1.6 g potassium phosphate, 152 mg 4,5-bis(diphenylphosphine)-9,9-dimethyloxanthracene, and 59 mg palladium acetate were added, and dissolved in 24 mL 1,4-dioxane, reacted under nitrogen protection. Heated to 80 °C and reacted overnight. After TLC indicated the reaction was complete, the reaction solution was cooled to room temperature, filtered, and the filtrate was spun-dried, and passed through a column chromatography column to obtain 611 mg a brown oily compound 3. LC-MS [M+1]: 196.

### 3. Synthesis of intermediate INT-14

300 mg compound 3,649 mg 2,4-dichloro-5-bromopyrimidine and 1.5 mL N,N-diisopropylethylamine were added to a 100 ml single-necked flask and was dissolved in 6 mL isopropanol, heated to 100 °C under nitrogen protection. After the reaction was completed, 309 mg intermediate INT-14 was obtained by column chromatography. LC-MS [M+1]: 388.

### Synthesis of intermediate compound INT-15

The synthesis route as followed:

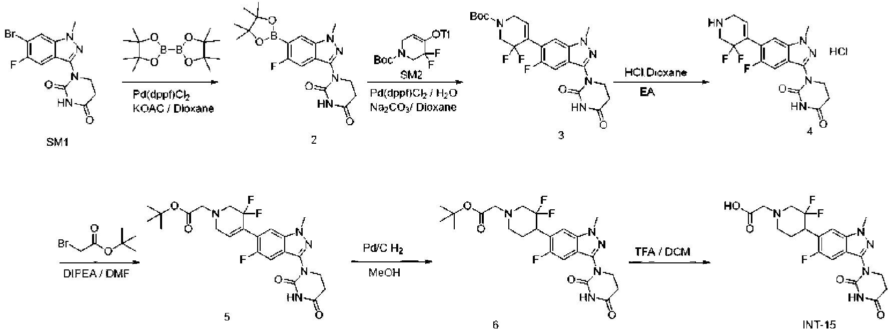

### 1. Synthesis of Compound 2

In a 100 mL three-necked flask, 2 g SM1, 2.2 g pinacol diboronate, 2.4 g pd(dppf)Cl₂, and 1.7 g potassium acetate were dissolved in 30 mL 1,4-dioxane. Under N₂ protection, the solution was heated to 85 °C and reacted overnight. TLC indicated the reaction was complete. The reaction solution was cooled to room temperature, filtered to remove insoluble impurities, and the filtrate was added water and extracted with EA. The organic phase was washed once with saturated NaCl and dried. Directly spun-dried and mixed as sample to obtain 2 g compound 2 by column chromatography. LC-MS [M+1]: 389.

### 2. Synthesis of Compound 3

In a 250 mL three-necked flask, 2 g compound 2, 2 g SM2, 1.5 g sodium carbonate, and 376 mg pd(dppf)Cl₂ were dissolved in 40 mL 1,4-dioxane and 10 mL H2O. Under N₂ protection , heated to 55 °C and reacted for 2 h. TLC showed the raw materials were disappeared. The reaction solution was cooled to room temperature, filtered to remove insoluble impurities, and the filtrate was extracted with water and dichloromethane. The organic phase was washed once with saturated NaCl, dried, spun-dried and mixed as sample. 1.8 g compound 3 was obtained by column chromatography. LC-MS [M+1]: 480.

### 3. Synthesis of Compound 4

1.8 g compound 3 was dissolved in 18 mL ethyl acetate in a 100 mL single-necked flask. 1,4-dioxane hydrochloride was added, and reacted overnight at room temperature. TLC showed the raw materials were disappeared. The reaction solution was filtered to obtain a 1 g filter cake, which was compound 4. LC-MS [M+1]: 380.

### 4. Synthesis of Compound 5

1 g compound 4 was dissolved in 10 mL DMF in a 100 mL three-necked flask. Under N₂ protection, 2 mL N, N-diisopropylethylamine was added, and stirred in an ice bath for 10 min. 0.4 mL tert-butyl bromoacetate was added, and proceeded overnight at room temperature. The reaction was monitored by TLC until complete. The reaction solution was diluted with water, extracted with ethyl acetate, dried over with anhydrous sodium sulfate, and spun-dried. 1.5 g compound 5 was obtained by column chromatography. LC-MS [M+1]: 494.

### 5. Synthesis of Compound 6

1.5 g compound 5 and 228 mg palladium on carbon were dissolved in 30 mL methanol in a 100 mL reaction vessel. The mixture was reacted overnight at room temperature under H₂ protection. TLC showed that the raw materials were disappeared. The reaction solution was filtered to remove insoluble impurities. Spun-dry and mixed as sample, 200 mg compound 6 was obtained by column chromatography. LC-MS [M+1]: 496.

### 6. Synthesis of intermediate INT-15

200 g compound 6 was added to a 100 mL reaction tube and dissolved in 1 mL dichloromethane. After adding 0.2 mL trifluoroacetic acid, and proceeded overnight at room temperature. TLC showed that the raw materials were disappeared. The reaction solution was concentrated to remove the solvent, 283 mg intermediate INT-15 was obtained. LC-MS [M+1]: 440.

### Synthesis of intermediate compound INT-16

The synthesis route as followed:

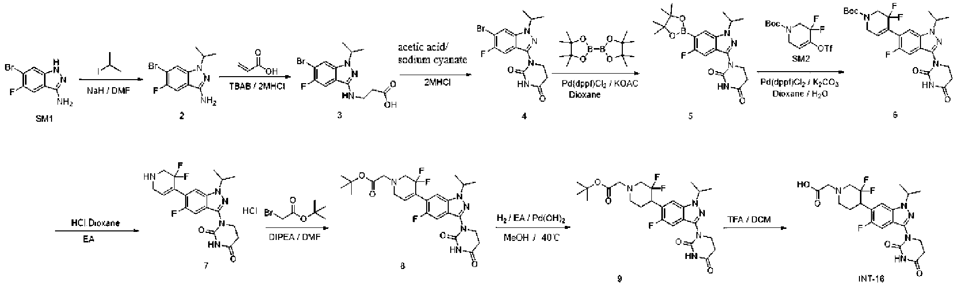

### 1. Synthesis of Compound 2

1 g SM1 was dissolved in 20 mL DMF in a 100 mL three-necked flask. Under nitrogen protection, 260 mg sodium hydride and 810 mg 2-iodopropane were added in an ice bath, and the reaction was carried out at room temperature for 1 h. The reaction was monitored by TLC until complete. The reaction solution was quenched with saturated ammonium chloride, extracted with EA, washed once with saturated NaCl, dried, and directly spun-dried and obtained 776 mg compound 2 by column chromatography. LC-MS [M+1]: 274.

### 2. Synthesis of Compound 3

In a 250 mL three-necked flask, 776 mg compound 2, 0.3 mL acrylic acid, and 92 mg tetrabutylammonium bromide were dissolved in 8 mL 2M hydrochloric acid solution. Under N₂ protection, the solution was heated to 100 °C and reacted overnight. TLC showed the raw materials were disappeared. The reaction solution was cooled to room temperature and quenched with saturated sodium carbonate in an ice bath to a pH of 9. The pH was then adjusted to 3-4 with acetic acid, resulting in the precipitation of a large amount of white solid. After thorough stirring, the mixture was filtered, and the filter cake was dried to obtain 1.2 g compound 3. LC-MS [M+1]: 346.

### 3. Synthesis of Compound 4

1.2 g compound 3 was dissolved in 12 mL acetic acid in a 100 mL single-necked flask, and 453 mg sodium cyanate was added. Heated to 60 °C for 14 h. Then 12 mL 2 M hydrochloric acid solution was added, and the reaction was continued at 60 °C for 3 h. TLC showed the raw materials were disappeared. The reaction solution was cooled to room temperature, quenched with saturated sodium carbonate solution, extracted with ethyl acetate, dried and concentrated the organic phase, and obtained 330 mg compound 4 by column chromatography. LC-MS [M+1]: 369.1.

### 4. Synthesis of Compound 5

In a 100 mL three-necked flask, 330 mg compound 4, 340 mg pinacol diborate, 65 mg pd(dppf)Cl₂, and 262 mg potassium acetate were dissolved in 7 mL 1,4-dioxane. Under N₂ protection, the solution was heated to 85 °C and reacted overnight. The reaction was monitored by TLC until complete. The reaction solution was cooled to room temperature, and insoluble impurities were removed by filtration. The filtrate was spun-dried and mixed as sample. 434 mg compound 5 was obtained by column chromatography. LC-MS [M+1]: 417.

### 5. Synthesis of Compound 6

In a 50 mL single-necked flask, 434 mg compound 5, 421 mg SM2, 316 mg sodium carbonate, and 76 mg pd(dppf)Cl₂ were dissolved in 4 mL 1,4-dioxane and 2 mL H₂O. Under N₂ protection, the solution was heated to 55 °C and reacted for 2 h. TLC showed the starting materials were disappeared. The reaction solution was cooled to room temperature, filtered to remove insoluble impurities, dried, and spun-dried and mixed as sample. 265 mg compound 6 was obtained by column chromatography. LC-MS [M+1]: 508.

### 6. Synthesis of Compound 7

265 mg compound 6 was dissolved in 3 mL ethyl acetate in a 50 mL single-necked flask. 2 mL 1,4-dioxane hydrochloride was added, and the mixture was reacted overnight at room temperature. TLC showed that the raw materials were disappeared. The reaction solution was filtered to obtain a filter cake of 142 mg, which was compound 7. LC-MS [M+1]: 408.

### 7. Synthesis of Compound 8

142 mg compound 7 was dissolved in 3 mL DMF in a 50 mL single-necked flask. Under N2 protection, 0.28 mL N, N-diisopropylethylamine was added, and the mixture was stirred in an ice bath for 10 min. 0.06 mL tert-butyl bromoacetate was added, and the mixture was reacted overnight at room temperature. The reaction was monitored by TLC until complete. The reaction solution was diluted with water, extracted with ethyl acetate, dried over with anhydrous sodium sulfate, and spun-dried to obtain 174 mg compound 8 by column chromatography. LC-MS [M+1]: 522.4.

### 8. Synthesis of Compound 9

In a 50 mL single-necked flask, 174 mg compound 8 and 17 mg palladium on carbon were dissolved in 4 mL methanol and 4 mL ethyl acetate. Under H₂ protection, the mixture was reacted overnight at room temperature. TLC showed that the raw materials were disappeared. The reaction solution was filtered to remove insoluble impurities. The filtrate was directly spun-dried and mixed as sample. 142 mg compound 9 was obtained by column chromatography. LC-MS [M+1]: 524.

### 9. Synthesis of intermediate INT-16

142 mg compound 9 was added to a 10 mL reaction tube and dissolved in 2 mL dichloromethane. 2 mL trifluoroacetic acid was added, and the mixture was reacted overnight at room temperature. TLC showed the starting material had disappeared. The reaction solution was concentrated to remove the solvent, to obtain 265 mg intermediate INT-16. LC-MS [M+1]: 468.2.

### Synthesis of intermediate INT-17

The synthesis route as followed:

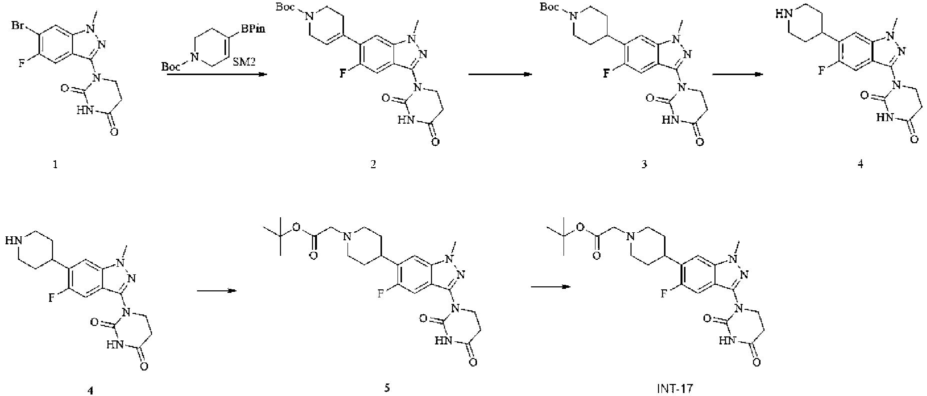

### 1. Synthesis of Compound 2

In a 50 mL single-necked flask, 300 mg compound 1 (1.0 equivalent), 272 mg SM2 (1.0 equivalent), 280 mg sodium carbonate (1.5 equivalent), 6 mL 1,4- dioxane, and 1.5 mL water were mixed thoroughly under nitrogen protection, then 63 mg [1,1'- bis ( diphenylphosphine ) ferrocene ] palladium dichloride (0.1 equivalent) was added, the mixture was purged with nitrogen three times, and the temperature was raised to 100 °C, the reaction proceeded overnight. The reaction was monitored by TLC until complete. The reaction was filtered through a diatomaceous earth filter, and the mother liquor was collected. The solvent was removed by rotary evaporation of the mother liquor, and then 30 mL water was added and extracted three times with 15 mL ethyl acetate. The combined organic phases were mixed as sample and passed through a column chromatography column to obtain 345 mg compound 2.

### 2. Synthesis of Compound 3

345 mg compound 2 and 34.5 mg Pd/C were added to 3.4 mL MeOH in a 50 mL single-necked flask. The mixture was purged three times with hydrogen and reacted at room temperature for 6 h under hydrogen balloon protection. After the reaction was complete, the reaction solution was filtered to remove palladium on carbon. The solvent was removed by rotary evaporation of the filtrate to obtain 345 mg compound 3. LC-MS [M+1] : 446 .

### 3. Synthesis of Compound 4

In a 50 ml single-necked flask, 345 mg compound 3 and 2 mL tetradioxane hydrochloride solution were added to 3 mL THF and reacted at room temperature for 6 h. After the reaction was complete, the solution was directly spun-dried for subsequent reactions. LC-MS [M+1] : 346 .

### 4. Synthesis of Compound 5

690 mg compound 4 was dissolved in 7 mL DMF in a 100 mL three-necked flask. Under N₂ protection, 3.5 mL N, N-diisopropylethylamine was added, and the mixture was stirred in an ice bath for 10 min. 470 mg tert-butyl bromoacetate was added, and the reaction was proceeded overnight at room temperature. The reaction was monitored by TLC until complete. The reaction solution was diluted with water, extracted with ethyl acetate, dried over anhydrous sodium sulfate, and spun-dried to obtain 690 mg compound 5 by column chromatography. LC-MS [M+1] : 460 .

### 5. Synthesis of intermediate INT-17

363 mg compound 6 was added to a 100 mL reaction tube and dissolved in 1 mL dichloromethane. 0.2 mL trifluoroacetic acid was added, and the mixture was reacted overnight at room temperature. TLC showed the raw materials were disappeared. The reaction solution was concentrated to remove the solvent, to obtain 318 mg intermediate INT-17 . LC-MS [M+1] : 404 .

### Synthesis of intermediate INT-18

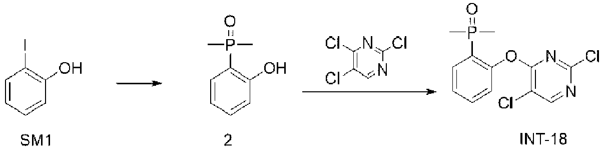

### Synthesis of Compound 2

In a 250 mL three-necked flask, 2.0 g SM1 and 1.06 g dimethylphosphoric acid were dissolved in 20 mL N, N-dimethylformamide. Under N₂ protection, 2.9 g potassium phosphate, 204 mg palladium acetate, and 525 mg Xnatphos were added. After nitrogen purging three times, the reaction was carried out overnight at 115 °C. The reaction was monitored by TLC until complete. The reaction solution was added water and extracted with EA. The organic phase was washed once with saturated NaCl and dried. Spun-dry and mixed as sample, and column chromatography to obtain 350 mg compound 2 .

### Synthesis of intermediate INT-18

300 mg compound 2, 649 mg trichloropyrimidine, and 1.5 mL N, N-diisopropylethylamine were added to a 100 mL single-necked flask and dissolved in 6 mL isopropanol. The mixture was heated to 100 °C under nitrogen protection. After the reaction was complete, 309 mg intermediate INT-18 was obtained by column chromatography. LC-MS [M+1]: 318 .

### Synthesis of intermediate compound INT-19

The synthesis route as followed:

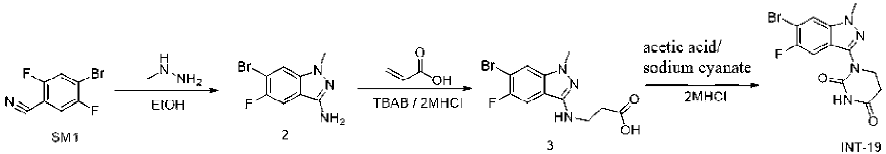

### 1. Synthesis of Compound 2

29 g SM1 was dissolved in 290 mL anhydrous ethanol in a 500 mL three-necked flask under nitrogen protection. 35 mL methylhydrazine was added, and the mixture was heated to 80 °C. The reaction was monitored by TLC until complete. The reaction solution was cooled to room temperature and stirred thoroughly in an ice bath. A large amount of white solid was precipitated. The precipitate was filtered to obtain a filter cake, which was dried to obtain 15.7 g product. The filtrate was further purified by column chromatography to obtain 9 g product, resulting in a total 24.7 g pale yellow solid compound 2. LC-MS [M+1] : 246.1.

### 2. Synthesis of Compound 3

In a 500 mL three-necked flask, 24.7 g compound 2, 10.4 mL acrylic acid, and 3.2 g tetrabutylammonium bromide were dissolved in 250 mL 2M hydrochloric acid solution. The mixture was heated to 100 °C and reacted overnight. TLC showed that the raw materials were disappeared. The reaction solution was cooled to room temperature and quenched with saturated sodium carbonate in an ice bath to a pH of 9. The pH was then adjusted to 3-4 with acetic acid, a large amount of white solid was precipitated. After thorough stirring, the mixture was filtered, and the filter cake was dried to obtain 26 g yellow solid compound 3. LC-MS [M+1] : 316.1 .

### 3. Synthesis of intermediate INT-19

26 g compound 3 was dissolved in 260 mL acetic acid in a 1 L single-necked flask, and 10.6 g sodium cyanate was added. The mixture was heated at 60 °C for 14 h. 260 mL 2 M hydrochloric acid solution was added, and the reaction was continued at 60 °C for 3 h. TLC showed that the raw materials were disappeared. The reaction solution was cooled to room temperature and stirred thoroughly in an ice bath. A large amount of white solid was precipitated. The solid was filtered, and the filter cake was dried to obtain 10.7 g yellow solid INT-19. LC-MS [M+1] : 342 .

### Example 2-1: Synthesis of compound TB-006

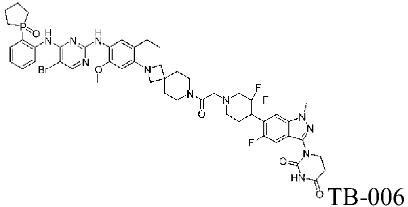

The synthesis route as followed:

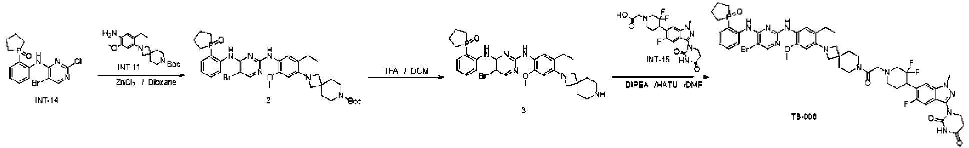

### 1. Synthesis of Compound 2

In a 10 ml reaction tube, 100 mg intermediate INT-14, 97 mg intermediate INT-11, and 46 mg zinc chloride were added sequentially. The solutions were dissolved in 2 mL isopropanol, and the mixture was heated at 105 °C overnight under nitrogen protection. After TCL indicated the reaction was complete, the reaction solution was cooled to room temperature, quenched with saturated sodium bicarbonate, extracted with ethyl acetate, dried and concentrated, and purified by column chromatography to obtain 73 mg compound 2. LC-MS [M+1]: 726.

### 2. Synthesis of Compound 3

1 mL trifluoroacetic acid were added to a 10 ml reaction tube, and dissolved in 2 mL dichloromethane, and reacted at room temperature for 0.5 h. After TCL indicated the reaction was complete, the reaction solution was concentrated to remove the solvent, to obtain 58 mg compound 3. LC-MS [M+1]: 627.

### 3. Synthesis of compound TB-006

In a 10 ml reaction tube, 116 mg intermediate INT-15 was added and dissolved in 2 mL DMF , and then 0.15 mL DIPEA was added. Under nitrogen protection, the mixture was stirred for 10 min in an ice bath.58 mg compound 3 and 45 mg HATU were added, and the reaction was proceeded overnight at room temperature. After TLC indicated the reaction was complete, the reaction solution was diluted with water and extracted with ethyl acetate. The organic phase was washed twice with saturated brine, dried, concentrated, and purified by column chromatography to obtain 17 mg compound TB-006. LC-MS [M+1]: 1047 HPLC: 96 %

¹ H NMR (400 MHz, DMSO) δ 10.58 (s, 2H), 8.30 (s, 1H), 8.14 (s, 1H), 8.03 (s, 1H), 7.70 (d, *J* = 5.2 Hz, 1H), 7.45 (dd, *J* = 18.0, 9.1 Hz, 2H), 7.35 (s, 1H), 7.14 (s, 2H), 6.15 (s, 1H), 4.03 (s, 4H), 3.92 (t, *J =* 6.5 Hz, 3H), 3.74 (s, 3H), 3.68 (s, 5H), 3.02 (s, 2H), 2.81 - 2.73 (m, 3H), 2.68 (s, 1H), 2.45 - 2.35 (m, 3H), 2.34 (s, 1H), 2.08 (s, 2H), 1.99 (s, 5H), 1.84 (s, 5H), 1.74 (s, 2H), 1.28 (d, *J=* 13.0 Hz, 1H), 1.18 (t*, J =* 7.1 Hz, 1H), 1.02 (t*, J* = 7.3 Hz, 3H).

Referring to the synthetic methods of intermediate compound INT-14 and compound TB-006, the following compounds were synthesized:

| Compound no. | Compound Structure | Characterization data |
|---|---|---|
| TB-0 05 | 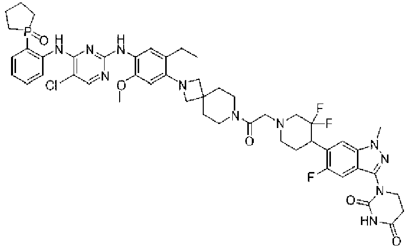 | LC-MS[M+1]: 1002.5 |
| TB-010 | 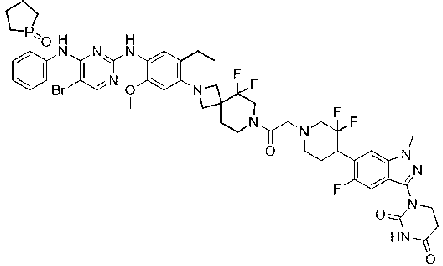 | LC-MS[M+1]: 1084.4 |
| TC-228 | 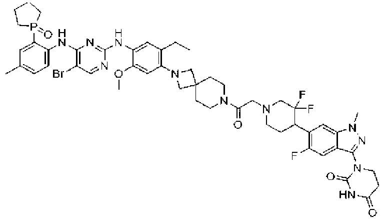 | LC-MS[M+1] : 1060.5 |
| TC-230 | 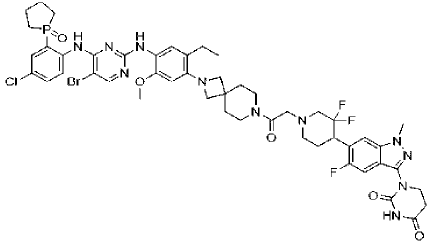 | LC-MS[M+1] : 1082.4 |

The chiral compounds obtained through high-pressure preparative chromatography were as follows:

| Compound no. | Compound Structure | Characterization data |
|---|---|---|
| TC-172 | 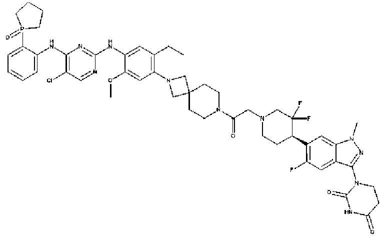 | LC-MS[M+1]: 1002.5 |
| TC-173 | 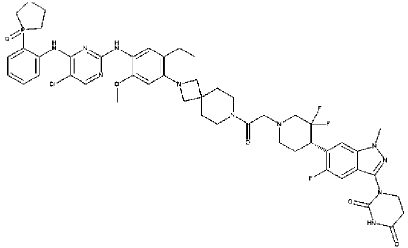 | LC-MS[M+1]: 1002.5 |
| TC-174 | 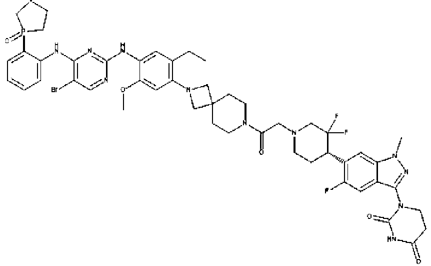 | LC-MS [M+1]: 1047.6 |
| TC-175 | 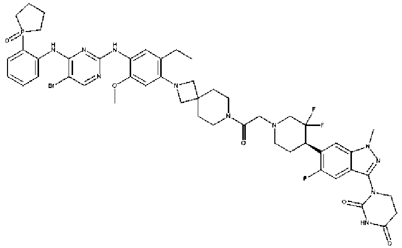 | LC-MS [M+1]: 1047.6 |
| TC-176 | 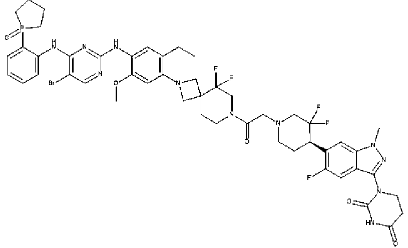 | LC-MS[M+1] : 1084.4 |
| TC-177 | 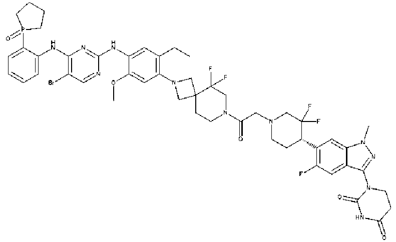 | LC-MS[M+1]: 1084.4 |

### Example 2-2: Synthesis of compound TB - 008

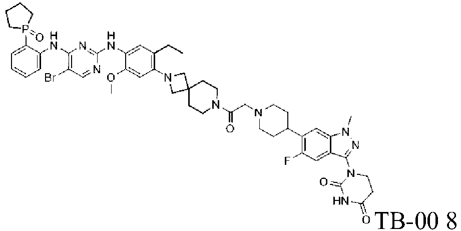

The synthesis route as followed:

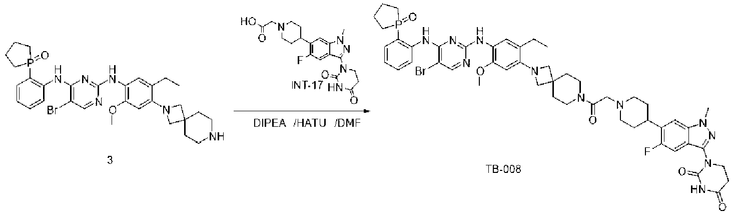

In a 50 mL single-necked flask, 50 mg compound 3 and 75 mg INT-17 were dissolved in 3 mL DMF, followed by the addition of 0.14 mL DIPEA and 40 mg HATU. After purging with N₂ three times, reacted at room temperature for 3 h. TLC indicated the reaction was complete. The reaction solution was extracted with ethyl acetate and water, then evaporated under reduced pressure, spun-dried and mixed as sample to obtain 19 mg compound TB-008 by column chromatography. LC-MS [M+1]: 1012.5. 1H NMR (400 MHz, DMSO-d6) δ 10.62 (s, 1H), 10.57 (s, 1H), 8.31 (s, 1H), 8.14 (s, 1H), 8.04 (s, 1H), 7.57 (d, J = 5.9 Hz, 1H), 7.46 (dd, J = 13.5, 7.7 Hz, 1H), 7.38 (d, J = 10.9 Hz, 2H), 7.14 (s, 2H), 6.14 (s, 1H), 4.01 (s, 3H), 3.90 (t, J = 6.6 Hz, 2H), 3.71 (d, J = 21.6 Hz, 7H), 3.22 (s, 2H), 2.99 (d, J = 10.8 Hz, 3H), 2.85 (s, 2H), 2.75 (t, J = 6.5 Hz, 2H), 2.40 (d, J = 7.5 Hz, 2H), 2.26 - 1.64 (m, 20H), 1.02 (t, J = 7.4 Hz, 3H).

Referring to the synthetic method for compound TB-008, the following compounds were synthesized:

| Compound no. | Compound Structure | Characterization data |
|---|---|---|
| TC-093 | 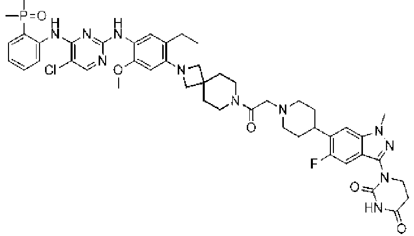 | LC-MS[M+1] : 940.7 |
| TC-213 | 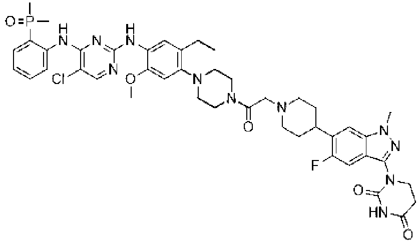 | LC-MS[M+1]: 900.7 |

### Examples 2-3: Synthesis of compound TB-018

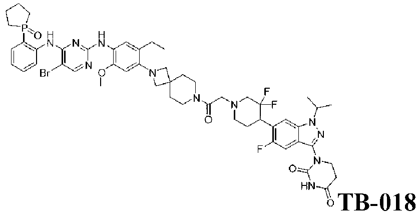

The synthesis route as followed:

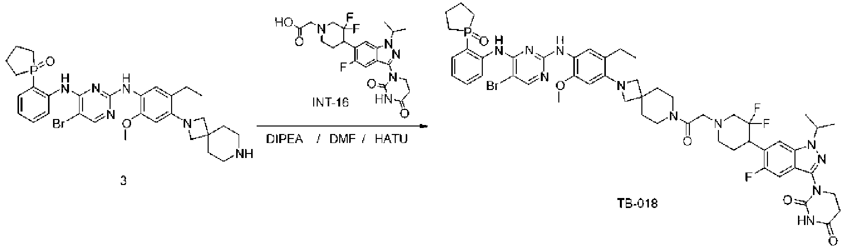

### Synthesis of compound TB-018

78 mg intermediate INT-16 was added to a 10 ml reaction tube, dissolved in 1 ml DMF, and then 0.13 ml DIPEA was added. Under nitrogen protection, the mixture was stirred for 10 min in an ice bath. 50 mg compound 3 and 39 mg HATU were added, and the reaction was proceeded overnight at room temperature. TCL indicated the reaction was complete, the reaction solution was added water and extracted with ethyl acetate. The organic phase was washed twice with saturated brine, dried, concentrated, and purified by column chromatography to obtain 22 mg compound TB-018. LC-MS [M+1]: 1077 HPLC: 99.14 %

¹ H NMR (400 MHz, DMSO) δ 10.60 (d, *J =* 21.0 Hz, 2H), 8.32 (s, 1H), 8.10 (d, *J =* 35.3 Hz, 2H), 7.75 (s, 1H), 7.52 - 7.28 (m, 3H), 7.14 (s, 2H), 6.15 (s, 1H), 5.08 (s, 1H), 3.93 (s, 2H), 3.71 (d, *J* = 22.7 Hz, 7H), 3.51 (s, 5H), 3.02 (s, 1H), 2.72 (d, *J* = 33.1 Hz, 3H), 2.42 (s, 2H), 2.09 (s, 2H), 1.98 (s, 4H), 1.85 (s, 5H), 1.74 (s, 2H), 1.44 (s, 5H), 1.24 (s, 5H), 1.03 (s, 3H), 0.86 (s, 1H).

Referring to the synthetic method for compound TB- 018 , the following compounds were synthesized:

| Compound No. | Compound Structure | Characterization data |
|---|---|---|
| TB -044 | 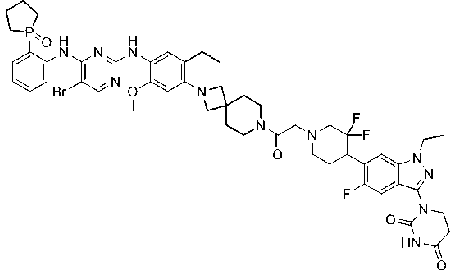 | LC-MS [M+1]: 1062.5 |

### Examples 2-4 :

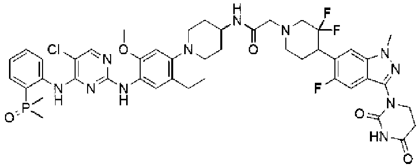

### TC-085

### Synthesis of intermediate compound M2

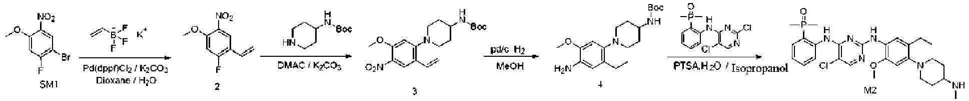

### 1. Synthesis of Compound 2

In a 250 mL three-necked flask, 8.4 g SM1, 9 g potassium vinyltrifluoride borate, 2.4 g pd(dppf)Cl₂, and 6.9 g potassium carbonate were dissolved in 42 mL 1,4- dioxane and 8.4 mL H₂O. Under N₂ protection, the mixture was heated to 100 °C and reacted overnight. TCL indicated the reaction was complete. The reaction solution was cooled to room temperature, filtered to remove insoluble impurities, and the filtrate was added the water and extracted with EA. The organic phase was washed once with saturated NaCl and dried. Directly spun-dried and mixed as sample to obtain 5.3 g compound 2 by column chromatography. LC-MS [M+1]:198.

### 2. Synthesis of Compound 3

In a 250 mL three-necked flask, 0.3 g compound 2, 609 mg 4-tert-butoxycarbonylaminopiperidine, and 631 mg potassium carbonate were dissolved in 10 mL DMAC . Under N₂ protection, the mixture was heated to 100 °C and reacted overnight. TLC showed the raw materials were disappeared. The reaction solution was cooled to room temperature, filtered to remove insoluble impurities, and the filtrate was added water and extracted with EA. The organic phase was washed once with saturated NaCl and dried. Directly spun-dried and mixed as sample to obtain 400 mg compound 3 by column chromatography. LC-MS [M+ 1]:378.

### 3. Synthesis of Compound 4

400 mg compound 3 and 20 mg palladium on carbon were dissolved in 10 mL methanol in a 100 mL single-necked flask. The mixture was reacted overnight at room temperature under H₂ protection. TLC showed that the raw materials were disappeared. The reaction solution was filtered to remove insoluble impurities. The filtrate was directly spun-dried and mixed as sample. 340 mg compound 4 was obtained by column chromatography. LC-MS [M+1]:350.

### 4. Synthesis of intermediate M2

100 mg compound 4, 90 mg intermediate M1, and 70 mg p-toluenesulfonic acid hydrate were dissolved in 2 mL isopropanol in a 100 mL reaction tube. Under N₂ protection, the mixture was heated to 90 °C and reacted overnight. TCL indicated the reaction was complete. The reaction was quenched with a small amount of saturated sodium bicarbonate aqueous solution, extracted with ethyl acetate, dried over with anhydrous sodium sulfate, and spun-dried. 70 mg intermediate M2 was obtained by column chromatography . LC-MS [M+1] : 529 .

### Synthesis of compound TC-085

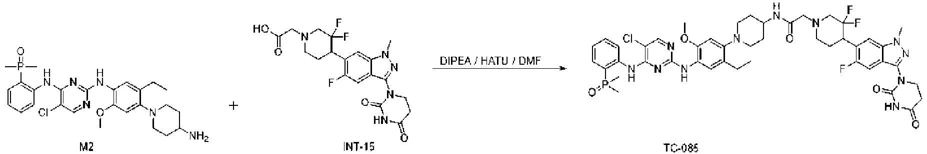

In a 100 mL reaction tube, 70 mg compound M2 and 138 mg intermediate compound INTC-15 were dissolved in 1 mL N, N -dimethylformamide. The mixture was cooled to 0 °C in an ice-water bath, and N, N- diisopropylethylamine and HATU were added with stirring. The reaction was carried out in an ice-water bath for 1 hour under nitrogen protection, and then naturally warmed to room temperature. The reaction was proceeded overnight. TLC indicated the reaction was complete. The mixture was extracted with dichloromethane and water, and the organic phase was dried and 18 mg TC-085 was obtained by column chromatography . LC-MS [M+1] : 950.7.

1H NMR (400 MHz, DMSOd6) δ 11.16 (s, 1H), 10.61 (s, 1H), 8.43 (s, 1H), 8.16 (s, 1H), 8.11 (s, 1H), 7.74 (d, J = 6.4 Hz, 2H), 7.55 (dd, J = 14.1, 7.7 Hz, 1H), 7.44 (d, J = 9.5 Hz, 2H), 7.32 (s, 1H), 7.11 (t, J = 7.2 Hz, 1H), 6.82 (s, 1H), 4.02 (s, 3H), 3.92 (t, J = 6.8 Hz, 2H), 3.81 (s, 1H), 3.72 (s, 3H), 3.56 (s, 1H), 3.18 (s, 2H), 3.07 - 2.92 (m, 3H), 2.88 - 2.71 (m, 5H), 2.56 (t, J = 7.6 Hz, 4H), 1.88 (s, 3H), 1.77 (d, J = 13.5 Hz, 5H), 1.65 (d, J = 11.2 Hz, 2H), 1.07 (t, J = 7.5 Hz, 3H).

Referring to the synthetic methods for intermediate compound M2 and compound TC-085, the following compounds were synthesized:

| Compound no. | Compound Structure | Characterization data |
|---|---|---|
| TC-087 | 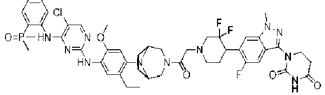 | LC-MS[M+1] : 962.7 |
| TC-089 | 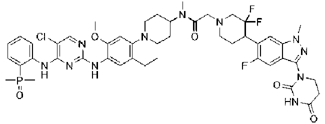 | LC-MS[M+1] : 964.8 |
| TC-091 | 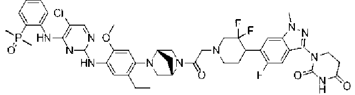 | LC-MS[M+1] : 948.7 |
| TC-097 | 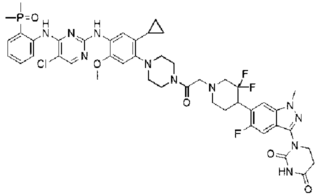 | LC-MS[M+1] : 948.7 |
| TC-105 | 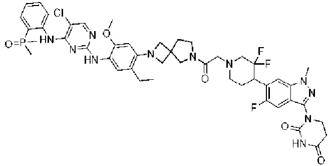 | LC-MS[M+1] : 962.8 |
| TC-107 | 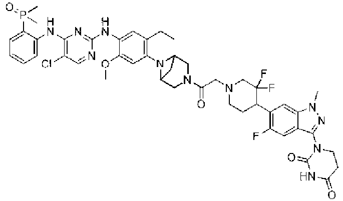 | LC-MS[M+1] : 948.6 |
| TC-111 | 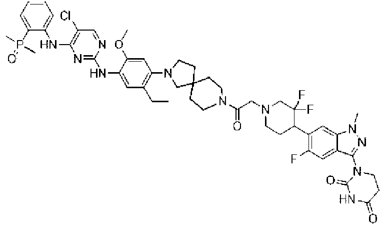 | LC-MS[M+1] : 990.8 |
| TC-115 | 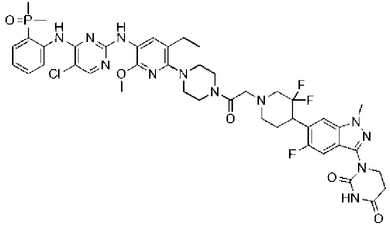 | LC-MS[M+1] : 937.6 |
| TC-125 | 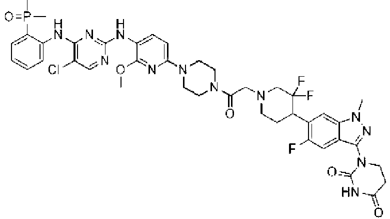 | LC-MS[M+1] : 909.4 |
| TC-127 | 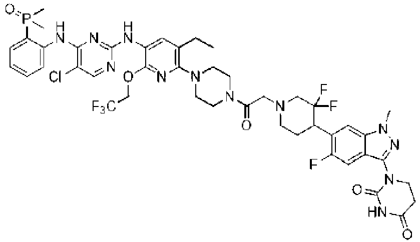 | LC-MS[M+1] : 1005.6 |
| TC-129 | 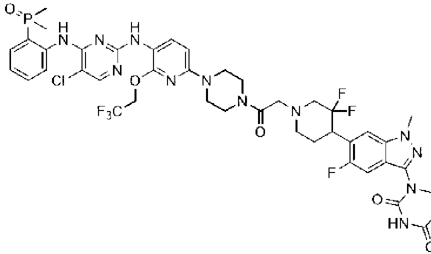 | LC-MS[M+1] : 977.6 |
| TC-133 | 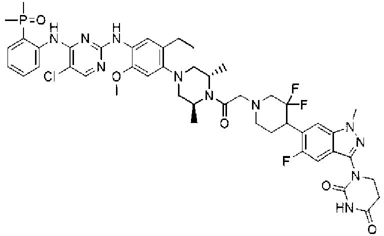 | LC-MS[M+1] : 928.4 |
| TC-138 | 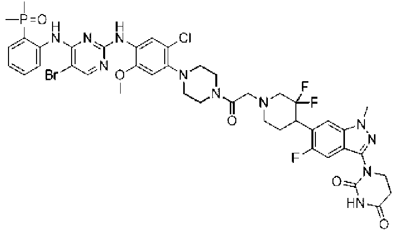 | LC-MS[M+1] : 988.4 |
| TC-157 | 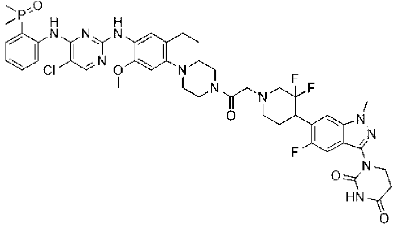 | LC-MS[M+1] : 936.7 |
| TC-158 | 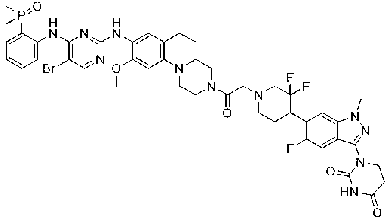 | LC-MS[M+1] : 982.6 |
| TA-021 | 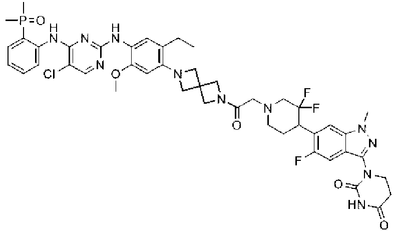 | LC-MS[M+1] : 948.8 |
| TC-164 | 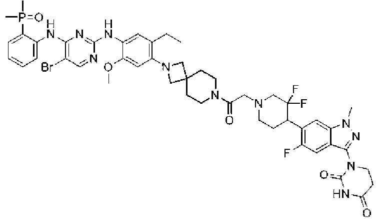 | LC-MS[M+1] : 1022.7 |
| TC-205 | 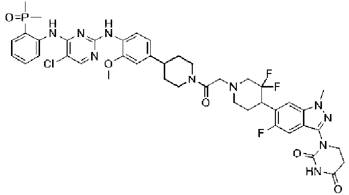 | LC-MS[M+1] : 907.6 |
| TC-207 | 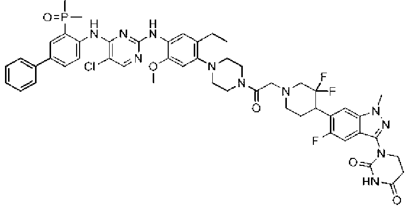 | LC-MS[M+1] : 972.7 |
| TC-211 | 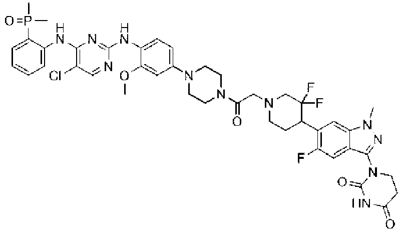 | LC-MS[M+1] : 908.7 |
| TC-221 | 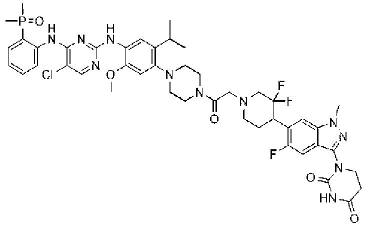 | LC-MS[M+1] : 950.7 |
| TC-224 | 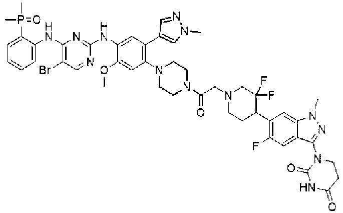 | LC-MS[M+1] : 1034.5 |
| TC-225 | 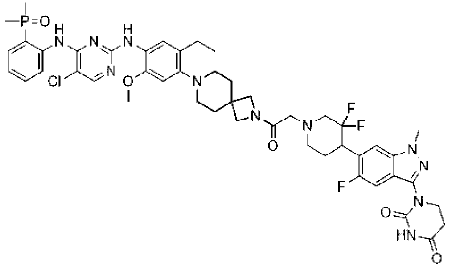 | LC-MS[M+1] : 976.5 |
| TC-226 | 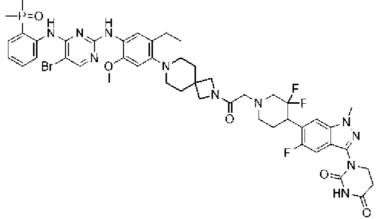 | LC-MS[M+1] : 1022.5 |
| TC-232 | 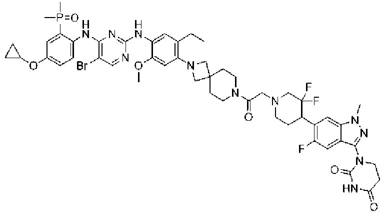 | LC-MS[M+1] : 1078.4 |

The chiral compounds obtained through high-pressure preparative chromatography were as follows:

| Compound no. | Compound Structure | Characterization data |
|---|---|---|
| TC-168 | 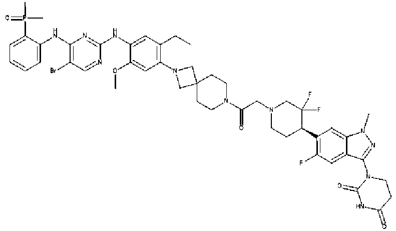 | LC-MS[M+1]: 1022.7 |
| TC-169 | 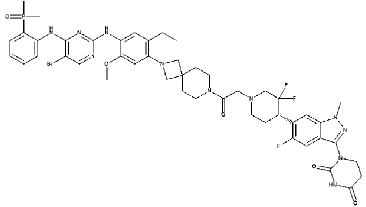 | LC-MS[M+1]: 1002.5 |
| TC-233 | 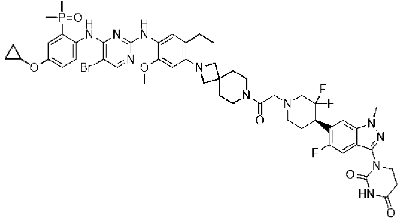 | LC-MS[M+1] : 1078.4 |
| TC-234 | 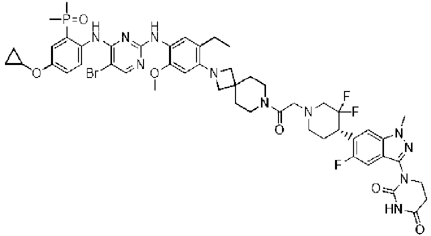 | LC-MS[M+1] : 1078.4 |

### Examples 2-5: Synthesis of compound TC-113

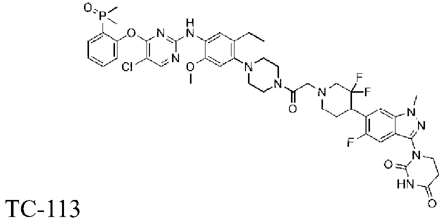

### Synthesis of compound TC-113

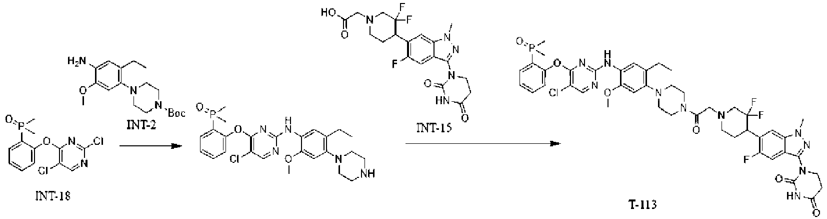

Referring to the synthetic method of compound TB-006 in Example 2-1, compound TC-113 was synthesized: 33 mg , LC-MS [M+1] : 937.5 1H NMR (400 MHz, DMSO-d6) δ 10.60 (s, 1H), 8.47 (s, 1H), 8.30 (s, 1H), 7.93 - 7.83 (m, 1H), 7.71 (d, J = 5.6 Hz, 1H), 7.65 (t, J = 7.6 Hz, 1H), 7.45 (td, J = 11.5, 10.6, 5.5 Hz, 3H), 7.28 (s, 1H), 6.72 (s, 1H), 4.01 (s, 3H), 3.91 (t, J = 6.7 Hz, 2H), 3.69 (d, J = 27.8 Hz, 7H), 3.56 (d, J = 11.7 Hz, 2H), 3.45 (s, 3H), 3.04 (d, J = 10.8 Hz, 1H), 2.77 (dd, J = 19.0, 12.1 Hz, 8H), 2.39 (s, 4H), 1.85 (d, J = 12.1 Hz, 1H), 1.64 (d, J = 13.6 Hz, 6H), 0.95 (s, 3H).

### Examples 2-6: Synthesis of compound TC-117

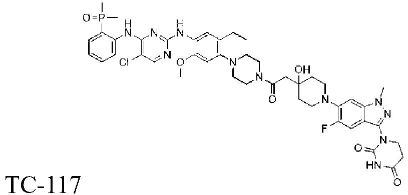

### Synthesis of compound TC-117

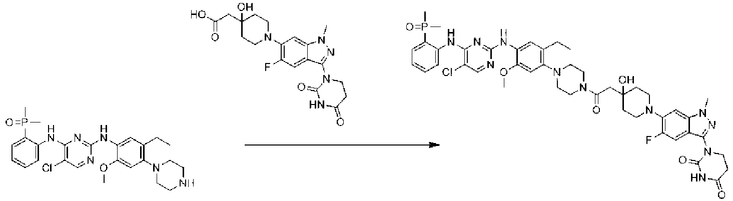

Referring to the synthetic method of compound TB-006 in Example 2-1, compound TC-117 was synthesized.

LC-MS[M+1] : 916.5 1H NMR (400 MHz, DMSO-d6) δ 11.17 (s, 1H), 10.57 (s, 1H), 8.44 (s, 1H), 8.20 (s, 1H), 8.12 (s,1H), 7.59 - 7.51 (m, 1H), 7.47 (s,1H), 7.38 - 7.29 (m, 2H), 7.18 -7.08 (m, 2H), 6.82 (s, 1H), 5.01 (s,1H), 3.95 (s, 3H), 3.89 (t, J = 6.7Hz, 2H), 3.71 (s, 3H), 3.60 - 3.48(m, 1H), 3.19 (d, J = 11.0 Hz, 3H), 3.08 (t, J = 11.0 Hz, 3H), 2.85 ( d,J = 13.2 Hz, 4H), 2.74 (t, J = 6.7Hz, 2H), 2.60 (dd, J = 15.8, 8.3Hz, 5H), 2.00 (q, J = 7.8 Hz, 2H), 1.84 (d, J = 11.2 Hz, 2H), 1.77 ( d,J = 13.6 Hz, 8H), 1.08 (t, J = 7.5Hz, 3H).

Referring to the synthetic method of compound TB-006 in Example 2-1, the following compounds were synthesized:

| Compound no. | Compound Structure | Characterization data |
|---|---|---|
| TC-119 | 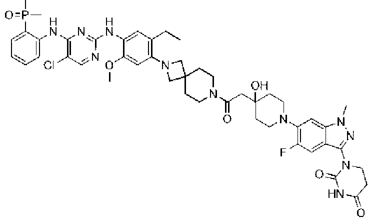 | LC-MS[M+1] : 956.6 |

### Examples 2-7: Synthesis of compound TC-123

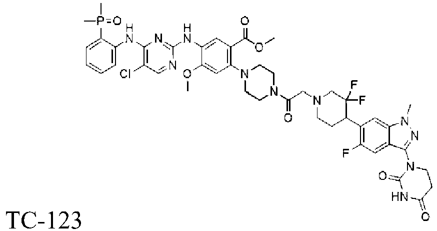

### Synthesis of intermediate compound M1

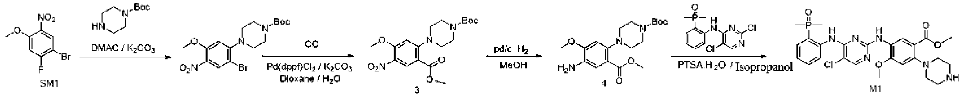

### Synthesis of Compound 2

500 mg SM1, 558 mg boc-piperazine, and 552 mg potassium carbonate were added to a 100 ml single-necked flask and dissolved in 10 ml DMAC. The mixture was heated to 90 °C and reacted overnight. After TLC indicated the reaction was completed, the reaction solution was extracted with ethyl acetate and water. The ethyl acetate phase was dried, filtered, and then spun-dried and passed through a column to obtain 760 mg compound 2 .

### Synthesis of Compound 3

In a 100 ml single-necked flask, 200 mg compound 2, 56 mg Pd(dppf)Cl₂, 0.7 mL triethylamine, 4 mL DMF, and 4 mL methanol were added. The mixture was vented three times with a carbon monoxide balloon, sealed with the balloon, and reacted overnight at 85 °C. After TLC indicated the reaction was complete, the reaction mixture was extracted with ethyl acetate and water. The organic phases were combined, dried and spun-dried to obtain 150 mg a yellow oily compound 3 by column chromatography. LC-MS[M+1]: 418

### Synthesis of Compound 4

150 mg compound 3, 10 mg palladium carbon, and 3 mL methanol were added to a 100 ml single-necked flask and mixed . The mixture was reacted at 35 °C overnight. After the reaction was completed, the mixture was filtered through a diatomaceous earth pad, and the mother liquor was collected and spun-dried to obtain crude compound 4.

### Synthesis of intermediate M1

In a 100 ml single-necked flask, 112 mg intermediate pyrimidine, 130 mg compound 4, 87 mg p-toluenesulfonic acid monohydrate, and 4 mL isopropanol were added and reacted overnight at 90 °C. After the reaction was completed, the mixture was purified by column chromatography to obtain 60 mg intermediate M1.

### Synthesis of compound TC-123

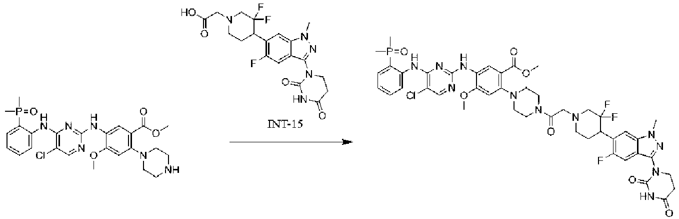

Referring to the synthetic method of TC-123 in Example 2-1, compound TB-006 was synthesized.

LC-MS[M+1] : 966.6 1H NMR (400 MHz, DMSO-d6) δ11.26 (s, 1H), 10.60 (s, 1H), 8.40 (s, 2H), 8.11 (s, 1H), 7.94 (s, 1H),7.71 (d, J = 5.6 Hz, 1H), 7.49 -7.37 (m, 2H), 7.23 (d, J = 9.4 Hz, 1H), 7.01 (d, J = 7.6 Hz, 1H), 6.75(s, 1H), 3.97 (s, 3H), 3.91 (t, J =6.7 Hz, 2H), 3.85 (s, 3H), 3.73 (s, 6H), 3.59 (s, 2H), 3.15 - 2.94 (m,6H), 2.84 - 2.66 (m, 4H), 2.39 (d, J= 12.5 Hz, 2H), 2.00 (d, J = 8.2 Hz,1H), 1.85 (s, 1H), 1.76 (d, J = 13.4Hz, 6H)

Referring to the synthetic methods for intermediate compound M1 and compound TC-123 , the following compounds were synthesized:

| Compound no. | Compound Structure | Characterization data |
|---|---|---|
| TC-131 | 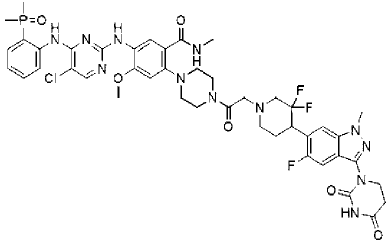 | LC-MS[M+1] : 929.3 |

### Examples 2-8: Synthesis of compound TC-135

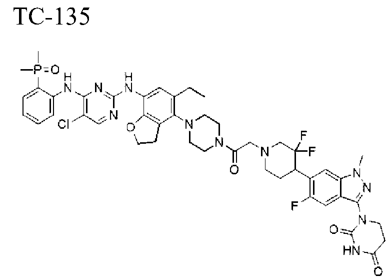

### Synthesis of compound TC-135

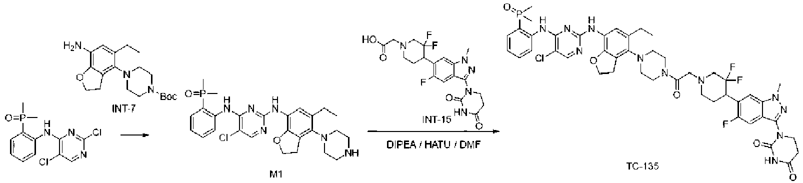

278 mg intermediate INTC-15 was added to a 10 ml reaction tube, dissolved in 2 ml DMF, and then 0.4 ml DIPEA was added. Under nitrogen protection, the mixture was stirred for 10 min in an ice bath. 115 mg intermediate M1 and 107 mg HATU were added, and the reaction was proceeded overnight at room temperature. After TLC indicated the reaction was complete, the reaction solution was added water and extracted with ethyl acetate. The organic phase was washed twice with saturated brine, dried, concentrated, and purified by column chromatography to obtain 10 mg compound TC-135. LC-MS [M+1]: 949.8 HPLC: 95.49%

¹ H NMR (400 MHz, DMSO) δ 11.22 (s, 1H), 10.59 (s, 1H), 8.50 (s, 2H), 8.09 (s, 1H), 7.68 (d, *J* = 4.6 Hz, 1H), 7.48 (dd, *J =* 14.2, 8.3 Hz, 1H), 7.44 (d, *J* = 10.4 Hz, 1H), 7.27 (s, 1H), 7.10 - 7.00 (m, 2H), 4.42 (s, 2H), 4.12 (dd, *J* = 10.5, 5.2 Hz, 1H), 3.98 (s, 2H), 3.92 (t, *J* = 6.8 Hz, 2H), 3.57 (t, *J* = 6.4 Hz, 1H), 3.50 (s, 1H), 3.46 (s, 1H), 3.29 - 3.26 (m, 1H), 3.25 (s, 1H), 3.17 (d, *J* = 5.2 Hz, 2H), 3.06 (d, *J* = 9.7 Hz, 2H), 2.93 (s, 3H), 2.82 - 2.69 (m, 3H), 2.62 (dd, *J* = 14.8, 7.5 Hz, 2H), 2.53 (s, 1H), 2.43 - 2.31 (m, 2H), 2.12 - 1.94 (m, 1H), 1.88 (s, 1H), 1.76 (d, *J* = 13.5 Hz, 5H), 1.24 (s, 1H), 1.12 (t, *J* = 7.4 Hz, 3H), 0.85 (d, *J* = 6.8 Hz, 1H).

### Examples 2-9: Synthesis of compound TC-144

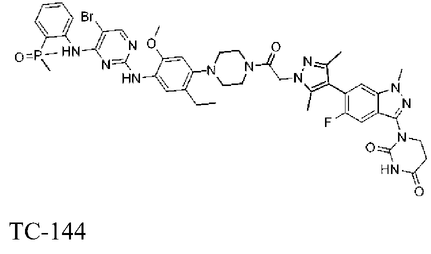

### Synthesis of compound TC-144

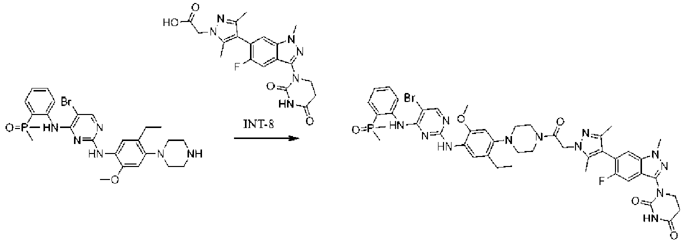

Referring to the synthetic method of TC-144 in Example 2-1, compound TB-006 was synthesized.

LC-MS[M+1] : 957.7 1H NMR (400 MHz, DMSO-d6) δ 10.81 (s, 1H), 10.60 (s, 1H), 8.31 (d, J = 8.2 Hz, 1H), 8.19 (s, 1H), 8.14 (s, 1H), 7.61 - 7.43 (m, 4H), 7.36 (s, 1H), 7.14 (t, J = 7.6 Hz, 1H), 6.83 (s, 1H), 5.16 (s, 2H), 4.03 (d, J = 6.0 Hz, 4H), 3.93 (t, J = 6.6 Hz, 2H), 3.77 (s, 9H), 2.88 (d, J = 25.6 Hz, 5H), 2.77 (t, J = 6.7 Hz, 2H), 2.58 (d, J = 7.4 Hz, 3H), 2.11 (d, J = 10.1 Hz, 6H), 1.99 (s, 2H), 1.76 (d, J = 13.5 Hz, 6H), 1.17 (t, J = 7.1 Hz, 2H), 1.06 (t, J = 7.4 Hz, 3H).

Referring to the synthetic methods of compound TC-144 in Examples 2-9 , the following compounds were synthesized:

| Compound no. | Compound Structure | Characterization data |
|---|---|---|
| TC-146 | 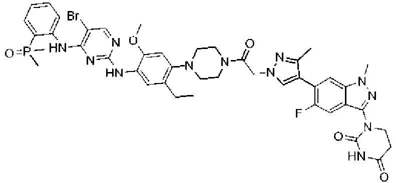 | LC-MS[M+1] : 897.4 |

### Examples 2-10: Synthesis of compound TC-150

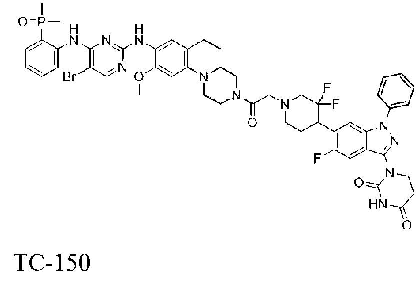

### Synthesis of compound TC-150

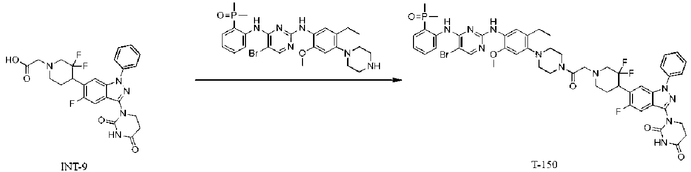

Referring to the synthetic method of TC-150 in Example 2-1, compound TB-006 was synthesized.

LC-MS[M+1] : 1042.4 1H NMR (400 MHz, DMSO-d6) δ 10.86 (s, 1H), 10.72 (s, 1H), 8.31 (s, 1H), 8.19 (s, 1H), 8.15 (s, 1H), 7.77 - 7.64 (m, 3H), 7.64 - 7.44 (m, 5H), 7.43 - 7.26 (m, 2H), 7.08 (s, 1H), 6.78 (s, 1H), 4.05 (t, J = 6.7 Hz, 2H), 3.69 (d, J = 17.4 Hz, 9H), 3.03 (d, J = 10.7 Hz, 2H), 2.94 - 2.70 (m, 8H), 2.31 - 2.10 (m, 3H), 1.93 (s, 2H), 1.74 (d, J = 13.5 Hz, 6H), 1.06 (t, J = 7.4 Hz, 3H).

### Example 2-11: Synthesis of compound TC-215

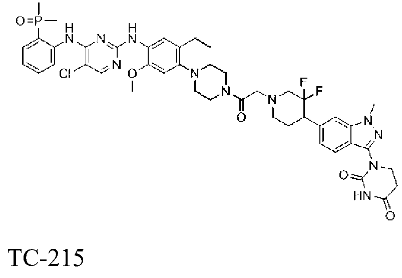

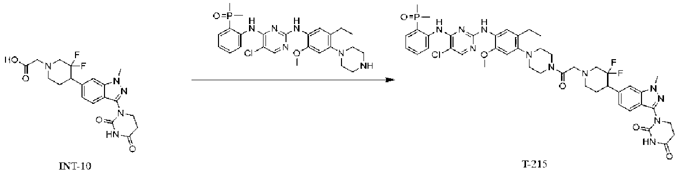

Referring to the synthetic method of TC-150 in Example 2-1, compound TB-006 was synthesized.

LC-MS[M+1] : 918.6 1HNMR (400 MHz, DMSO) δ 11.17 (s, 1H), 10.58 (s, 1H), 8.42 (s, 1H), 8.15 ( d,J = 31.0 Hz, 2H), 7.54 ( dd,J = 30.8, 20.5 Hz, 4H), 7.32 (s, 1H), 7.08 (s, 2H), 6.81 (s, 1H), 3.96 (s, 2H), 3.92 (s, 2H), 3.74 (s, 5H), 3.60 (s, 2H), 3.43 ( d,J = 7.5 Hz, 2H), 3.23 (s, 2H), 3.03 (s, 1H), 2.91 (s, 2H), 2.79 ( d,J = 27.0 Hz, 3H), 2.65 ( d,J = 7.7 Hz, 1H), 2.61 ( d,J = 6.7 Hz, 2H), 2.27 (d,J = 11.1 Hz, 1H), 1.99 (s, 1H), 1.89 (s, 1H), 1.76 ( d,J = 13.3 Hz, 5H), 1.23 (s, 3H), 1.09 (s, 3H).

### Example 2-12: Synthesis of compound TC-099

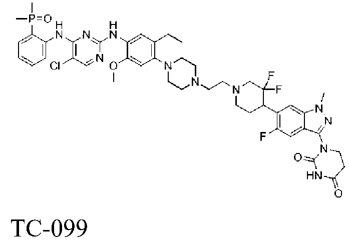

### Synthesis of intermediate 3

The synthesis route as followed:

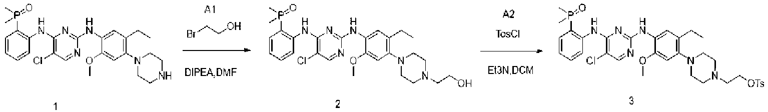

### 1. Synthesis of Compound 2

600 mg compound 1 was weighed and dissolved in 6 mL LDM in a 50 mL three-necked flask. 188 mg Al and 469 mg DIPEA were added. After purging with N₂ three times, the reaction was carried out at 90 °C overnight. TLC showed that a new spot was formed. The reaction solution was added water, extracted with ethyl acetate, dried over with anhydrous sodium sulfate to obtain 448 mg compound 2 by column chromatography.

### 2. Synthesis of intermediate 3

In a 50 mL single-necked flask, 448 mg compound 2 and 252 mg DIPEA were dissolved in 5 mL DCM. 206 mg A2 was dissolved in 2 mL DCM and slowly added dropwise to the reaction solution. After purging with N₂ three times, the reaction was proceeded at room temperature for 2 hours. TLC monitoring was performed until compound 2 no longer decreased. The reaction solution was then directly spun-dried under reduced pressure and mixed as sample, 286 mg intermediate 3 was obtained by column chromatography.

### Synthesis of compound TC-099

The synthesis route as followed:

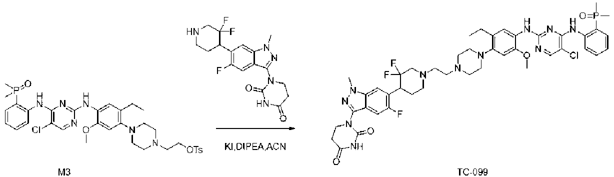

In a 50 ml single-necked flask, 186 mg compound M3 and 119 mg the starting amine (compound 3 in INT-17) were dissolved in 3 mL acetonitrile. 82 mg KI and 159 mg DIPEA were added. After gas exchanged with N2 three times, the mixture was reacted at 75 °C for 3 h. TLC indicated the reaction was complete. The reaction solution was dried over under reduced pressure and mixed as sample. 190 mg compound TC-099 was obtained by column chromatography.

LC-MS[M+1]: 922.7. 1H NMR (400 MHz, DMSO-d6) δ 11.16 (s, 1H), 10.61 (s, 1H), 8.44 (s, 1H), 8.17 (s, 1H), 8.11 (s, 1H), 7.75 (d, J = 5.5 Hz, 1H), 7.55 (dd, J = 14.1, 7.7 Hz, 1H), 7.42 (d, J = 8.7 Hz, 2H), 7.32 (s, 1H), 7.12 (d, J = 8.2 Hz, 1H), 6.81 (s, 1H), 4.03 (s, 3H), 3.92 (t, J = 6.6 Hz, 2H), 3.77 (s, 3H), 3.12 (s, 2H), 2.86 (d, J = 5.6 Hz, 4H), 2.76 (t, J = 6.7 Hz, 3H), 2.71 - 2.63 (m, 4H), 2.37 (s, 3H), 1.77 (d, J = 13.5 Hz, 7H), 1.07 (t, J = 7.5 Hz, 3H)

### Comparative Example 2-1: DZL -1

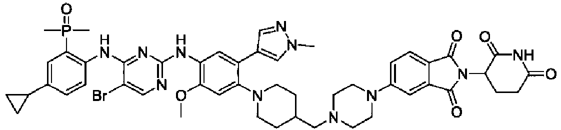

The synthesis method refers to TIBET HAISCO PHARMACEUTICAL CO., LTD. WO2024083183A1

### Comparative Example 2-2: DZL-2

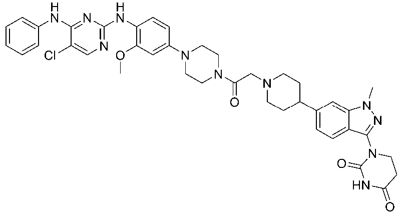

The synthesis method refers to T-14 in TYK MEDICINES, INC. WO2023088385A1.

### Example 3-1: Synthesis of compound TD-001

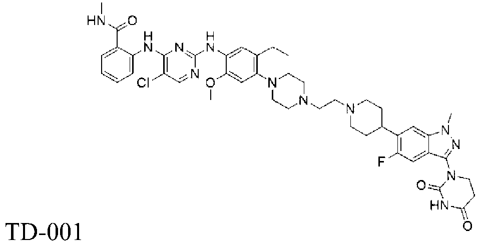

### Synthesis of intermediate compound M1

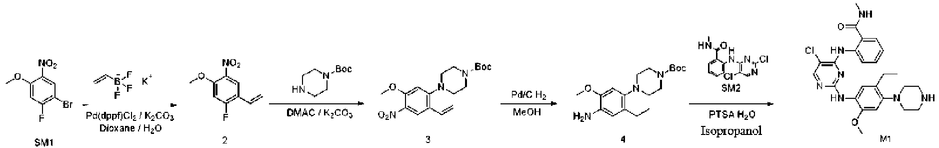

### 1. Synthesis of Compound 2

In a 250 mL three-necked flask, 10 g SM1, 10.7 g vinyl boron trifluoride, 2.9 g pd(dppf)Cl₂, and 8.2 g potassium carbonate were dissolved in 50 mL 1,4-dioxane and 10 mL H₂O. Under N₂ protection, the mixture was heated to 100 °C and reacted overnight. The reaction was monitored by TLC until complete. The reaction solution was cooled to room temperature, filtered to remove insoluble impurities, and the filtrate was added water and extracted with EA. The organic phase was washed once with saturated NaCl and dried. The sample was directly spun-dried and mixed as sample. 14.4 g compound 2 was obtained by column chromatography. LC-MS [M+1]: 198.

### 2. Synthesis of Compound 3

In a 100 mL three-necked flask, 2.7 g compound 2, 4.3 g 1-Boc piperazine, and 9.4 g potassium carbonate were dissolved in 27 mL DMAC. Under N₂ protection, the mixture was heated to 100 °C and reacted overnight. TLC showed that the raw materials were disappeared. The reaction solution was cooled to room temperature, and insoluble impurities were removed by filtration. The filtrate was added water and extracted with EA. The organic phase was washed once with saturated NaCl and dried. The sample was directly spun-dried and mixed as sample. 3.8 g compound 3 was obtained by column chromatography. LC-MS [M+1]: 364.

### 3. Synthesis of Compound 4

3.8 g compound 3 and 1.5 g palladium on carbon were dissolved in 35 mL methanol in a 100 mL reaction vessel. The mixture was reacted overnight at room temperature under H₂ protection. TLC showed that the raw materials were disappeared. The reaction solution was filtered to remove insoluble impurities. The filtrate was directly spun-dried and mixed as sample, 2.9 g compound 4 was obtained by column chromatography. LC-MS [M+1]: 336.

### 4. Synthesis of intermediate M1

In a 100 mL reaction tube, 300 mg compound 4, 265 mg SM2, and 220 mg p-toluenesulfonic acid hydrate were dissolved in 3 mL isopropanol. Under N₂ protection, the mixture was heated to 90 °C and reacted overnight. TLC indicated the reaction was complete. The reaction was quenched with a small amount of saturated sodium bicarbonate aqueous solution, extracted with ethyl acetate, dried over with anhydrous sodium sulfate, spun-dried, and purified by column chromatography to obtain 331 mg intermediate M1. LC-MS [M+1]: 496.

### Synthesis of intermediate compound M2

The synthesis route as followed:

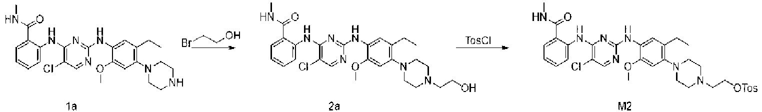

### 1. Synthesis of compound 2a

In a 50 mL three-necked flask, 220 mg compound 1a (1.0 equivalent), 67 mg bromoethanol (1.2 equivalent), 172 mg DIPEA (3.0 equivalent), and 5 mL DMF were mixed thoroughly and refluxed at 90 °C overnight. The reaction was monitored by TLC (DCM: MEOH = 10:1) until complete. The reaction solution was cooled to room temperature and diluted with EA (5 mL). The mixture was extracted three times with 15 mL ethyl acetate. The combined organic phases were stirred and passed through a column chromatography column to obtain 140 mg compound 2a.

### 2. Synthesis of compound M2

In a 50 mL single-necked flask, 140 mg compound 2a, 99 mg (2.0 eq) of TosCl, and 79 mg (3.0 eq) of triethylamine were dissolved in 2.8 mL DCM. The mixture solution was stirred at room temperature and reacted for 16 h. The solvent was removed by rotary evaporation, and 60 mg compound M2 was obtained by column chromatography.

### Synthesis of intermediate M3

The synthesis route as followed:

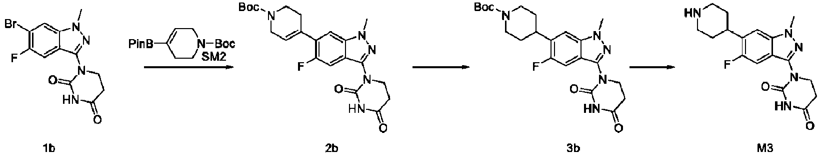

### 1. Synthesis of compound 2b

In a 50 mL single-necked flask, 300 mg compound 1b (1.0 equivalent), 272 mg SM2 (1.0 equivalent), 280 mg sodium carbonate (1.5 equivalent), 6 mL 1,4-dioxane, and 1.5 mL water were mixed thoroughly under nitrogen protection. 63 mg [1,1'-bis(diphenylphosphine) ferrocene] palladium dichloride (0.1 equivalent) was added, the mixture was purged with nitrogen three times, and heated to 100 °C. The reaction was proceeded overnight. TCL indicated the reaction was complete. The mixture was filtered through a diatomaceous earth filter, and the mother liquor was collected. The solvent was removed by rotary evaporation, and the mother liquor was then extracted three times with 30 mL water and 15 mL ethyl acetate. The combined organic phases were stirred and filtered through a column to obtain 345 mg compound 2b.

### 2. Synthesis of compound 3b

345 mg compound 2b and 34.5 mg Pd/C were added to 3.4 mL MeOH in a 50 mL single-necked flask. The mixture was purged three times with hydrogen and reacted at room temperature for 6 h under hydrogen balloon protection. After the reaction was complete, the reaction solution was filtered to remove palladium on carbon. The solvent was removed by rotary evaporation of the filtrate to obtain 345 mg compound 3b. LC-MS [M+1]: 446.

### 3. Synthesis of compound M3

In a 50 ml single-necked flask, 345 mg compound 3b and 2 mL tetradioxane hydrochloride solution were added to 3 mL THF and reacted at room temperature for 6 h. After the reaction was complete, the solution was directly spun-dried for subsequent reactions. LC-MS [M+1]: 346.

### Synthesis of compound TD-001

The synthesis route as followed:

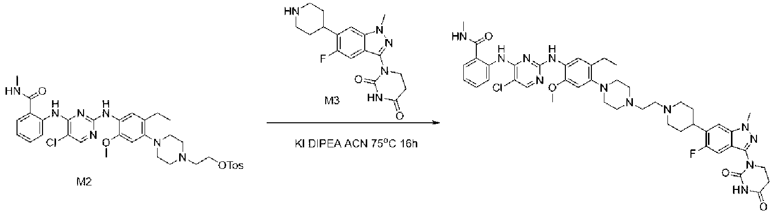

In a 50 mL single-necked flask, 60 mg compounds M2 and 65 mg M3 were dissolved in 1.2 mL acetonitrile. Then, 17.2 mg KI and 33 mg DIPEA were added. After gas exchanged with N₂ three times, the mixture was reacted at 75 °C for 16 h. TLC indicated the reaction was complete. The reaction solution was then evaporated under reduced pressure, stirred to obtain 33 mg the compound by column chromatography.

LC-MS[M+1]: 867.41.

1H NMR (400 MHz, DMSO-d6) δ 11.63 (s,1H), 10.58 (s, 1H), 8.77 (d, J = 5.0 Hz, 1H), 8.57 (d, J = 8.5 Hz, 1H), 8.20 (s, 1H), 8.14 (s,1H), 7.73 (d, J = 7.9 Hz, 1H), 7.57 (d, J = 5.9Hz, 1H), 7.48 (s, 1H), 7.39 (d, J = 10.9 Hz, 1H), 7.31 (t, J = 8.1 Hz, 1H), 7.08 (t, J = 7.6 Hz, 1H),6.81 (s, 1H), 4.01 (s, 3H), 3.91 (t, J = 6.7 Hz,2H), 3.77 (s, 3H), 2.90 (s, 4H), 2.83 - 2.72 (m,5H), 2.58 (q, J = 7.6 Hz, 1H), 1.90 (s, 4H), 1.24(d, J = 8.0 Hz, 4H), 1.08 (t, J = 7.5 Hz, 3H).

Referring to the synthetic methods for intermediate compound M2 and compound TD-00 1 , the following compounds were synthesized:

| Compound no. | Compound Structure | Characterization data |
|---|---|---|
| TD-019 | 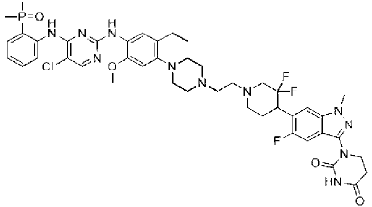 | LC-MS[M+1]: 922.7 |

### Comparative Example 3-1: Synthesis of Compound C1

The synthesis route as followed:

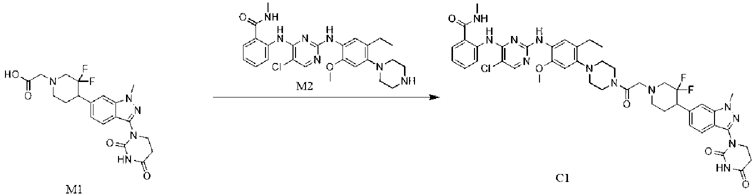

In a 50 ml single-necked flask, compounds M2 (150 mg, 1.0 eq) and M1 (176 mg, 1.0 eq) were dissolved in 4 mL NN-dimethylformamide. The mixture was cooled to 0 °C in an ice-water bath, and NN-diisopropylethylamine and HATU (176 mg, 1.3 eq) were added with stirring. The mixture was reacted in an ice-water bath for 1 hour under nitrogen protection, and then naturally warmed to room temperature. TLC indicated the reaction was complete. The mixture was extracted with ethyl acetate and water, and the organic phase was dried, filtered, and 90 mg the control compound C1 was obtained by column chromatography. LC-MS [M+1]: 899.6.

1H NMR (400 MHz, DMSO-d6) δ11.69 (s, 1H), 10.64 (s, 1H), 8.80 (s,1H), 8.70 - 8.54 (m, 1H), 8.29 (s,1H), 8.21 (s, 1H), 7.76 (d, J = 7.8 Hz,1H), 7.71 - 7.52 (m, 3H), 7.38 (s,1H), 7.13 (q, J = 8.5, 7.6 Hz, 2H),6.88 (s, 1H), 4.12 - 3.93 (m, 6H),3.79 (d, J = 18.4 Hz, 5H), 3.68 (s,3H), 3.53 - 3.51 (m, 1H), 3.20 (d, J =7.6 Hz, 2H), 3.10 (s, 1H), 2.98 (s,2H), 2.93 - 2.78 (m, 8H), 2.69 (d, J =7.4 Hz, 1H), 2.33 (d, J = 12.9 Hz,1H), 2.05 (s, 1H), 1.94 (d, J = 13.0Hz, 1H), 1.16 (t, J = 7.6 Hz, 3H).

### Test of the inhibitory activity of compounds on cell growth

### Experimental Example 1: Cell Anti-proliferation Experiment

### I. Experimental Materials and Equipment:

H1975 has an EGFR: T790M/L858R double mutation, PC-9 has an EGFR exon 19 deletion, and HCC827 has an exon 19 deletion mutation. Baf3-19del- T790M -C797S (Baf3-DTC) is a stable Ba/F3 clone expressing exogenous EGFR gene bearing T790M, C797S, and L858R triple amino acid mutations, purchased from KYinno. Baf3-L858R-T790M - C797S (Baf3-LTC) is a stable Ba/F3 clone expressing exogenous EGFR gene bearing exon19 E746_A750 deletion, T790M, and C797S triple mutations, purchased from KYinno. A549 is a wild-type EGFR, purchased from Nanjing Kebai Biotechnology. A431 is a wild-type EGFR, purchased from Shanghai Xinyu Biotechnology. CellCounting-Lite2.0, Trypsin EDTA, 37°C CO2 incubator, cell counter, EnVision Serial No. 1050454.

### II. Experimental Preparation:

### 1. 96-well plate laying

A) Digested logarithmic phase cells with Trypsin EDTA, added culture medium to stop the reaction, and mixing well with a pipette to prepare a cell suspension.
B) Used Vi-cell to determine cell concentration, and prepared a suspension of 15,000-25,000 cells per milliliter according to the experimental purpose and cell characteristics.
C) After prepared the cell suspension, mixed it gently and added 100 µL to each well, so that the cell density to be tested is 1500-2500 per well.

### 2. Compound treatment

### compound dilution

A) Weighed approximately 2 mg the compound and calculated the required volume of DMSO according to the formula: Compound mass (mg) * Compound purity (%) / Compound molecular weight * 1000.
B) Placed the inoculated cell culture plate into an incubator and incubated for about 24 hours. Then added compounds of varying concentrations.
C) Diluted the 10mM compound stock solution to 50mM with culture medium, and added the 50mM compound solution to the second column of the deep well plate in sequence, and added 375mL culture medium containing 0.5% DMSO to the third to eleventh columns.
D) Gradient dilution: Taked 125 µL solution from the second column and added it to the third column, mix well; then taked 125 µL solution from the third column and added it to the fourth column, repeated this operation until the tenth column.
E) Used a multichannel pipette, aspirated 25 µL the compound from the deep-well plate and added it to a 96-well culture plate. Repeated this process three times for each compound on the 96-well plate. This will ultimately create a concentration gradient of up to 10,000 nM at a 1:4 ratio on the 96-well plate.

### 3. Added CTG and take readings.

A) The effects of the compound were observed under an inverted microscope after the 96-well plate was incubated in an incubator for 72 hours.
B) Added 25 µL CTG solution to each well, placed on a shaker for 10 minutes, and read the OD value of each well.

### 4. Data Analysis

Calculate the % Cell Viability using the following formula: $% Cell Viability = 100 % \times \left(Lum_Sample-Lum_LC\right) / \left(Lum_HC-Lum_LC\right)$
Lum_HC: Cell readings from the 0.1% DMSO control group
Lum_Sample: Cell readings with added compound
Lum_LC: Blank culture medium reading

The IC50 value was obtained by curve fitting using GraphPad Prism 8 software.

As shown in Table 1, where AA≤10 nM; 10nM<A≤100nM; 100nM<B<1000nM; C≥1000nM.

**Table 1**

| compound | H1975 | HCC827 | PC-9 | BaF3-LTC | BAF3-DTC | A549 | A431 |
|---|---|---|---|---|---|---|---|
| TA-003 | AA | C | C | B | B | C | C |
| TA-004 | A | C | C | A | A | C | C |
| TA-005 | A | B | B | AA | AA | B | C |
| TA-006 | A | B | B | AA | AA | C | C |
| TA-013 | A | C | C | A | AA | C | C |
| TA-019 | AA | C | B | A | A | C | C |
| TA-023 | A/14.7 | B/ 592.6 | B/ 460.7 | AA/ 8.4 | B/201.6 | C/1259 | C/1182 |
| TA-026 | A | B | B | AA | A | C | C |
| Comparative Example 1-1 | A | B | B | | | A | B |
| TB-005 | A | B | C | A | A | C | C |
| TB-006 | AA/5 | A/61.4 | B/328 | AA/8.2 | AA/8.9 | C/>5000 | C/>5000 |
| TB-008 | A | A | B | A | A | B | B |
| TB-010 | AA | A | B | A | A | C | C |
| TC-087 | A | B | B | AA | AA | B | B |
| TC-089 | A | B | B | A | A | C | C |
| TC-091 | A | B | B | A | A | C | C |
| TC-093 | A | A | B | A | A | C | C |
| TC-105 | B | A | B | A | A | B | B |
| TC-111 | B | B | B | A | A | B | C |
| TC-115 | B | B | B | A | A | B | C |
| TC-121 | AA | A | B | AA | AA | B | B |
| TC-125 | B | B | B | A | B | B | B |
| TC-127 | B | B | C | A | A | C | C |
| TC-129 | B | B | B | B | B | B | B |
| TC-135 | A | B | C | AA | AA | C | C |
| TC-138 | A | B | B | AA | AA | C | C |
| TC-144 | A | B | B | AA | AA | B | C |
| TC-146 | A | A | B | A | AA | C | C |
| TC-150 | B | B | C | A | A | C | C |
| TC-157 | A/24 | B/812.5 | B/349 | AA/4.1 | AA/4.1 | B/947.8 | C/1648 |
| TC-158 | A | B | B | AA | AA | C | C |
| TA-007 | B | A | B | AA | A | A | A |
| TC-161 | A | B | B | AA | A | C | B |
| TC-164 | AA | A | B | AA | AA | C | C |
| TC-185 | A/ 20.3 | B/ 498.7 | B/ 427.1 | A | A/ 21.6 | C/ >5000 | C/ >5000 |
| TC-211 | B | B | B | A | A | C | C |
| TC-213 | A/35.9 | A/53 | B/505.4 | AA/0.8 | AA/8.9 | C/1773 | C/1341 |
| TC-215 | B/164.4 | B/226.6 | B/646.7 | AA/5.3 | AA/5.6 | B/325.5 | C/1073 |
| TC-224 | A | A | B | AA | AA | B | B |
| TC-225 | B/289.3 | B/576 | B/432 | A/16.7 | A/13.5 | B/961 | C/1610 |
| TC-226 | B | B | B | AA | AA | B | C |
| TC-232 | A | A | B | AA | AA | C | C |
| Comparative Example 2-1 | A/54.8 | B/105.3 | B/204.9 | A/13.6 | A/14.5 | C/>5000 | C/>5000 |
| Comparative Example 2-2 | B/220.1 | B/264.6 | B/612.5 | - | - | B/289.3 | - |
| TD-001 | AA | AA | B | A | A | A | C |
| C 1 | A | B | B | | | A | B |

As shown in Table 1, the compounds of the invention exhibit very good inhibitory effects on H1975(human lung adenocarcinoma cells), BaF3-LTC, and BaF3-DTC, while their inhibitory effect on cells containing exon 19 deletion is secondary. Inhibition of wild-type EGFR is not significant.

Furthermore, based on Table 1, the following conclusions are drawn:
1) Comparing TA-023 and TC-157, it can be seen that the spirocyclic structure used in the selection of ring B has better inhibitory activity against L858 mutation than the piperazine ring, and also improves the inhibition of wild-type EGFR.
2) Comparing TC-225 with TA-023, it can be seen that when the position of the spirocycle changes, the inhibition of mutated EGFR also decreases;
3) Comparing TC-213 and TC-215 with TC-157, it can be seen that adding fluorine atoms at specific positions can increase the inhibitory effect on EGFR and reduce the inhibitory effect on wild-type EGFR.
4) A comparison of TC-185 and TC-157 shows that when a cyclic oxyphosphorus structure is used instead of a dimethylphosphorus structure, the selection effect on wild-type EGFR can be improved.
5) Comparing TB-006 and DZL-1 (control example 2-1), it can be seen that TB-006 has a better inhibitory effect on H1975, BaF3-LTC and BaF3-DTC.
6) Comparing TA-023 with control example 2-2, it can be seen that the new E3 and spirocyclic structure has better inhibitory activity against L858 mutation than the original structure, and has higher selectivity for wild-type EGFR.

### Experimental Example 2: EGFR PROTAC HTRF Experiment

### 1. Instruments and reagents

| reagents | supplier | model |
|---|---|---|
| HTRF TOTAL-EGFR DETECTION kit | Cisbio | 64NG1PEG |
| Consumables | supplier | model |
| HTRF 96 well low volume plate | Cisbio | 66PL96025 |
| 96 -well cell culture plate | Corning | 3599 |
| instrument | supplier | model |
| Vortex mixer | / | QL-861 |
| Microplate centrifuge | / | BE-6100 |
| Microplate thermostatic oscillator | Hangzhou Aosheng Instrument Co., Ltd. | MB100-4A |
| EnVision | PerkinElmer | 1050454 |
| cell | supplier | |
| Baf3-19del-T790M-C797S | KYinno | |
| Baf3-L858R-T790M-C797S | KYinno | |

Among them, Baf3-19del- T790M -C797S (abbreviated as Baf3-DTC) is a stable Ba/F3 clone expressing exogenous EGFR gene bearing T790M, C797S and L858R triple amino acid mutations, purchased from KYinno; Baf3-L858R-T790M - C797S (abbreviated as Baf3-LTC) is a stable Ba/F3 clone expressing exogenous EGFR gene bearing exon19 E746_A750 deletion, T790M, C797S triple mutations, purchased from KYinno.

### 2. Experimental Procedure

### 1) Cell preparation:

| adherent cells | Suspension cells |
|---|---|
| Seeded 50 µL cells into a 96-well cell culture plate and incubated overnight at 37°C. | Seeded 25 µL cells into a 96-well cell culture plate and incubated overnight at 37°C. |
| Typically, 50K cells were seeded per well, but the seeding density can be adjusted depending on the type of cells. | |
| Added 50 µL the compound (2×) to the cells. | Added 5 µL the compound (6×) to the cells. |
| Incubated at 37°C for 16 hours. | |
| Sucked up the clear | No need to inhale the upper body |

### 2) Reagent preparation:

| adherent cells | | Suspension cells |
|---|---|---|
| 1 volume of lysis buffer 4X +3 volumes of distilled water | 1 volume blocking buffer + 99 volumes 1X lysis buffer | 1 volume blocking buffer + 24 volumes lysis buffer ( 4X) |

Dilutded Eu and d2 antibodies 2µl per well with detection buffer 20-fold. Then mixed the two antibody vials thoroughly.

### 3) Testing:

| adherent cells | Suspension cells |
|---|---|
| Added 50 µL lysis buffer (1×) and incubated at room temperature for at least 30 min. | Added 10 µL lysis buffer (4×) and incubated at room temperature for at least 30 min. |
| Added an appropriate amount of lysis buffer and incubated at room temperature with shaking. This could optimize the lysis incubation time, reducing the lysis volume to 25 µL. | |
| Used a pipette to thoroughly mix the lysed cells, then transferred 16 µL from the 96-well cell culture plate to a 96-well low-volume plate. (Added 16 µL lysis buffer to the control wells.) | |
| Added 4 µL the prepared antibody to the detection buffer and sealed the plate with a sealing membrane. Centrifuged the plate thoroughly using a microplate centrifuge. Incubated at room temperature in the dark for 4 hours. The signal reached its maximum after the incubation period and remained stable for 24 hours. Therefore, readings could be taken between the incubation time and 24 hours. | |

### 3. Results and Calculations

Opened EnVision , locate the HTRF program, configured the board, and then read theplate.

The fitted curve, DC50 and Dmax of this compound could be generated using GraphPad Prism .

As shown in Table 2, for DC50, AA≤10nM; 10nM<A≤100nM; 100nM<B<1000nM; C≥1000nM; for Dmax , 70%≤A≤100%; 50%≤B<70%; C<50%; NA is untested.

**Table 2**

| compound | Baf3-LTC | | Baf3-DTC | |
|---|---|---|---|---|
| | DC50 ( nM ) | Dmax (%) | DC50 ( nM ) | Dmax (%) |
| TA-003 | A | A | A | A |
| TA-004 | B | A | B | A |
| TA-005 | AA | A | AA | A |
| TA-006 | AA | A | AA | A |
| TA-007 | AA | A | AA | A |
| TA-009 | B | A | B | A |
| TA-013 | AA | A | AA | A |
| TA-017 | B | A | B | A |
| TA-023 | A | A | A | A |
| TA-025 | A | A | A | A |
| TA-031 | A | A | A | A |
| TA-035 | A | A | A | A |
| TA-045 | A | A | A | A |
| Comparative Example 1-1 | A | A | A | A |
| TB-00 6 | A | A | AA | A |
| TB-010 | AA | A | AA | A |
| TC-157 | AA | A | AA | A |
| TC-158 | AA | A | AA | A |
| TC-161 | AA | A | AA | A |
| TC-164 | AA | A | AA | A |
| TC-207 | A | A | A | A |
| TC-213 | A | A | A | A |
| DZL-1 | A | A | A | A |
| TD-001 | A | B | B | B |

As shown in Table 2, the compounds of the present invention exhibit excellent degradation performance on EGFR cells containing the L858R- T790M -C797S triple mutation and the 19del-T790M -C797S triple mutation.

### Pharmacokinetic studies of compounds

### Experiment 3: Pharmacokinetic Tests in Male SD Rats

### 3.1. Experimental Animals

Three healthy adult male SD rats, 6-8 weeks old, weighing 200-300 grams.

### 3.2. Equipment and Reagents

### 3.2.1. Equipment

Analytical balance, animal weighing scale, magnetic stirrer, gavage syringe, refrigerated centrifuge, single-channel manual pipette, liquid chromatography-mass spectrometry (LC-MS) instrument, etc.

### 3.2.2. Reagents

EDTA-Na₂ anticoagulant, weighed 11.2 g EDTA-Na₂ and placed it in a reagent bottle. Added 100 mL physiological saline and shaked to dissolve completely. After preparation, dispensed into 1.5 mL centrifuge tubes, each containing approximately 20 µL, for whole blood sample collection.

### 3.3. Experimental Procedure

### 3.3.1. Drug Preparation

Accurately weighed approximately 10 mg the sample to be tested, added 10% of the converted total volume of DMSO to dissolve it, and then slowly added 90% of the total volume of 0.5% MC solvent while stirring. Sonicated and vortex to mix thoroughly to obtain a solution of the preparation that is considered to be homogeneous, with a concentration of 1 mg/mL. Prepared fresh immediately before use.

Piped 0.2 mL the sample into a 1.5 mL centrifuge tube and store at -80°C for analysis of the concentration of the drug solution.

### 3.3.2 Animal Preparation

Animals were housed in rat cages and fasted for at least 10 hours starting the day before the experiment, but water was allowed. On the day of the experiment, each animal was weighed and marked on its tail. Blank blood samples were collected before drug administration. Blood was collected via tail vein.

### 3.3.3. Administration

Route of administration: Oral gavage (po);
Dosage concentration: 1 mg/ml;
Dosage: 10 mg/kg;
Dosage volume: 10 mL/kg;
Procedure: Hold the rat upright with your left hand wearing a bite-proof glove, insert the gavage needle into the throat through the mouth, and insert the needle when feeling no obvious resistance. Then inject the drug into the stomach.

### 3.4. Sample Collection

Whole blood (0.1-0.2 ml) was collected from test animals at 0.5 h, 1 h, 2 h, 4 h, 6 h, 8 h, 12 h, and 24 h after drug administration. The samples were placed in EDTA-Na ₂ anticoagulant tubes, inverted 3-4 times to mix, and centrifuged at 4°C, 2000 g for 5 min to separate the supernatant plasma. The plasma was promptly transferred to -80°C for storage until analysis. Blood was collected via tail vein.

### 3.5. Sample Analysis and Data Processing

### 3.5.1. Sample Analysis

A quantitative detection method for the analyte was established using Shimadzu liquid chromatography and Triple Quad^{™} 6500+AB mass spectrometry. The concentration of the parent drug in plasma was analyzed. The analytical results were controlled for variation using quality control samples, with the accuracy of the quality control samples expected to be between 80% and 120%.

### 3.5.2. Data Processing

The main pharmacokinetic parameters were calculated using a non-compartmental model in Winnonlin Phoenix software. These parameters included the area under the curve (AUC(0-t) and AUC(0-∞)), elimination half-life (T_{1/2}), maximum plasma concentration (Cₘₐₓ ), and time to reach maximum plasma concentration (Tₘₐₓ).The results are shown in Table 3.

**Table 3**

| compound | Tmax (h) | Cmax (ng/ml) | AUClast (h*ng/ml) | AUCinf (h*ng/ml) | HL (h) | MRTlast (h) |
|---|---|---|---|---|---|---|
| TA-0 03 | 2.00 | 441 | 3774 | 3799 | 3.22 | 5.63 |
| TB-006 | 3.33 | 1207 | 12427 | 12616 | 3.76 | 6.13 |
| TB-010 | 4.00 | 482 | 5726 | 5805 | 3.59 | 6.60 |
| TC-093 | 4.00 | 665 | 5026 | 4932 | 3.43 | 5.98 |
| TC-099 | 2.67 | 649 | 7838 | 9126 | 7.86 | 7.79 |
| TC-144 | 0.67 | 700 | 4120 | 4136 | 3.21 | 4.23 |
| TC-157 | 3.33 | 768 | 7532 | 5804 | 2.66 | 5.52 |
| TC-158 | 2.67 | 533 | 5590 | 5614 | 2.81 | 6.02 |
| TC-161 | 1.67 | 320 | 1654 | 1663 | 3.38 | 4.05 |
| TA-023 | 2.00 | 580 | 3925 | 3976 | 3.99 | 5.10 |
| TC-213 | 4.00 | 742 | 8072 | 8153 | 3.48 | 6.22 |
| DZL-1 | 4.00 | 699 | 8067 | 8459 | 3.08 | 6.11 |

As shown in Table 3, the compounds of the present invention have good pharmacokinetic effects.

### Experiment 4: Pharmacokinetic Test of CD-1 Mice

### 4.1. Experimental Objective

Plasma was collected from male CD-1 mice after a single gavage administration of the test substance. The plasma was then analyzed to calculate pharmacokinetic parameters.

### 4.2. Information on test samples and standards

The formula for calculating the free base conversion factor : Conversion factor CF = (MW/FW) * purity.

### 4.3. Formulation Preparation

The solvent is 5% DMSO + 10% Solutol HS-15 + 85% Saline.

Formulation preparation process: Accurately weighed the compound powder, added appropriate volumes of DMSO, Solutol and physiological saline in sequence, and vortexed sonicate at each step to finally form a homogeneous formulation solution.

Before administration, collected two samples of the formulation and the remaining formulation after administration, and stored them in an environment of 2-8°C until they were sent out.

### 4.4. Laboratory Animals

Experimental animals were housed in the SPF-grade animal facility of Suzhou Xihua New Drug Development Co., Ltd. (License No.: SYXK(Su)2021-0019). Animals were fed normally for at least 3 days after purchase before the experiment, and each mouse was marked with a tail tag. Animals in the oral administration group were fasted overnight the day before administration, and were resumed feeding 4 hours later, with free access to water. The source and quantity of animals used in this experiment are shown in Table 4.

**Table 4. Sources and Number of Experimental Animals**

| **Species** | **strain** | **source** | **weight** | **Zhou Ling (W)** | **Number of male animals** |
|---|---|---|---|---|---|
| mice | CD-1 | Shanghai Jihui Experimental Animal Breeding Co., Ltd. | 20~25g | 6-8 | 12 |

### 4.5. Experimental Design

1) Patients drank water freely before and after administration;
2) All experiments were conducted using medications based on the animals' actual body weight;
3) Administration method: oral gavage (PO), fasting overnight before administration.

Before administration, checked the state of the administration formulation and ensured its homogeneity by vortexing, stirring or shaking. Calculated the theoretical administration volume for each CD-1 mouse in each group according to the following formula. $\begin{matrix}Theoretical administration volume \left(mL\right) \\ = \frac{Dose \left(mg⋅{kg}^{- 1}\right)}{formulation concentration \left(mg⋅{mL}^{- 1}\right)} \times animal body weight \left(kg\right)\end{matrix}$

### 4.6. Sample Collection and Processing

Sample collection information is shown in Table 5.

**Table 5 Sample Collection Information**

| **Group** | **Animal number** | **Time point** |
|---|---|---|
| A | M1, M2, M3 | 0.5, 2, 6 and 12 h |
| | M4, M5, M6 | Pre, 1, 4, 8 and 24 h |
| B | M7, M8, M9 | 0.5, 2, 6 and 12 h |
| | M10, M11, M12 | Pre, 1, 4, 8 and 24 h |

Remark:
1) Blood collection time window of ±1 minute for blood collection points within 1 hour (excluding blood collection points before drug administration), and blood collection time window of ±5% for other time points; 2) Samples sent out for testing.

40µL blood was collected from the submandibular vein of mice at each time point listed in Table 5. The collected blood was placed in test tubes containing the anticoagulant EDTA-K₂ (15 % EDTA-K₂ solution), stored on moist ice, and centrifuged within 45 min (1,500 g, 2-8 °C, 10 min). Plasma was then collected. The plasma was stored in pre-chilled centrifuge tubes, flash-frozen on dry ice, and subsequently stored in an ultra-low temperature freezer at -60 °C or lower until shipment.

### 4.7. Experimental Observation

Any abnormal reactions observed in the animals during the experiment were recorded in detail.

### 4.8. Sample Analysis and Data Processing

### 4.8.1. Sample Analysis

A quantitative detection method for the analyte was established using Shimadzu liquid chromatography and Triple Quad^{™} 6500+AB mass spectrometry. The concentration of the parent drug in plasma was analyzed. The analytical results were controlled for variation using quality control samples, with the accuracy of the quality control samples expected to be between 80% and 120%.

### 4.8.2. Data Processing

The main pharmacokinetic parameters were calculated using a non-compartmental model in Winnonlin Phoenix software. These parameters included the area under the curve (AUC(0-t) and AUC(0-∞)), elimination half-life (T_{1/2}), maximum plasma concentration (Cₘₐₓ ), and time to reach maximum plasma concentration (Tₘₐₓ).

The results are shown in Table 6.

**Table 6**

| compound | Tmax (h) | Cmax (ng/ml) | AUClast (h*ng/ml) | AUCinf (h*ng/ml) | HL_ (h) | MRTlast (h) |
|---|---|---|---|---|---|---|
| TA-004 | 2.00 | 3167 | 28764 | 28849 | 2.67 | 6.01 |
| TA-003 | 4.00 | 3407 | 20645 | 20683 | 2.45 | 5.11 |
| TA-005 | 1.00 | 5973 | 36300 | 36335 | 2.28 | 4.59 |
| Comparative Example 1-1 | 2.00 | 1743 | 14284 | 14307 | 2.41 | 6.06 |

As shown in Table 6, the compounds of the present invention exhibit excellent pharmacokinetic effects. Compared with Control Example 1-1, they have higher exposure levels and better pharmacokinetic effects.

All literatures mentioned in the present invention are incorporated by reference herein, as though individually incorporated by reference. Additionally, it should be understood that after reading the above teaching, many variations and modifications may be made by the skilled in the art, and these equivalents also fall within the scope as defined by the appended claims.

## Claims

1. A compound shown in Formula I, or a pharmaceutically acceptable salt, a stereoisomer, a tautomer, a hydrate, a solvate, an isotope, or a prodrug thereof, 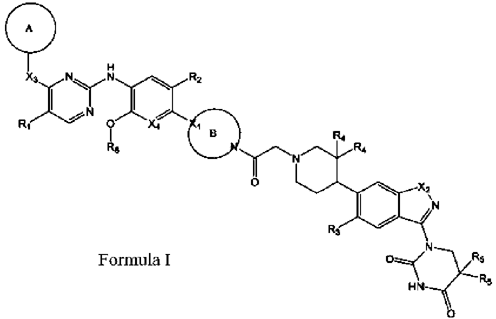 wherein,
X₃ is selected from none, NH, or NR;
X₂ is selected from NR, O, or S;
each R is independently selected from C₁₋₆ alkyl or halogenated C₁₋₆ alkyl;
X₁ is selected from CH or N;
X₄ is selected from CH or N;
ring B 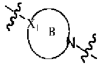 is selected from the group consisting of substituted or unsubstituted 6-7-membered heterocyclylene, and substituted or unsubstituted 7-9-membered spiro heterocyclylene ; the "substituted" refers to one or more hydrogens of the groups being substituted by substituents selected from the group consisting of H, halogen, amino, nitro, hydroxy, cyano, C₁₋₆ alkyl, C₁₋₆ alkoxy, C₃₋₆ cycloalkyl, halogenated C₁₋₆ alkyl, and C₁₋₆ hydroxyalkyl;
ring A is selected from the group consisting of substituted or unsubstituted C₆₋₁₀ aryl and substituted or unsubstituted 6-10 membered heteroaryl; the substituted refers to 0-1 hydrogen of the group being substituted by R₆, and m hydrogens are substituted by R₇; m is 0, 1, or 2;
wherein,
R₆ is selected from the group consisting of C₁₋₆ alkyl, C₁₋₆ hydroxyalkyl, halogenated C₁₋₆ alkyl, -P(O)RaRb, -C(O)R, -C(O)NHR, -C(O)NRaRb, -OC(O)-OR, -OC(O)NHR, -OC(O)NRaRb, -S(O)₂R, -NR-S(O)₂R, -NR-C(O)-OR, -NH-C(O)-OR, and C₁₋₄ alkyl-substituted or unsubstituted 4-7-membered heterocyclyl-O-;
Ra and Rb are each independently C₁₋₆ alkyl or C₃₋₆ cycloalkyl, or Ra and Rb with the heteroatom to which they are attached together form a 5-7-membered heterocycle;
R₇ is selected from the group consisting of H, halogen, amino, nitro, hydroxy, cyano, C₁₋₆ alkyl, C₁₋₆ alkoxy, C₃₋₆ cycloalkyl, halogenated C₁₋₆ alkyl, and C₁₋₆ hydroxyalkyl;
R₁ is selected from halogen;
R₂ is selected from H, substituted or unsubstituted C₁₋₆ alkyl, or substituted or unsubstituted 2-6-membered heteroalkyl; wherein the heteroalkyl contains at least one heteroatom selected from the group consisting of O, N, and S, and the "substituted" refers to one or more hydrogen atoms on the group being substituted by substituents selected from the group consisting of cyano, nitro, hydroxy, amino, and halogen;
R₃ is selected from halogen;
each R₄ is independently H or halogen;
each R₅ is independently H, halogen, or C₁₋₆ alkyl; or two R₅ groups together form a C₃₋₆ cycloalkyl with the carbon atom to which they are attached;
R₈ is selected from C₁₋₆ alkyl, or halogenated C₁₋₆ alkyl.

2. A compound, wherein the compound is a compound of Formula II, or a pharmaceutically acceptable salt, a stereoisomer, a tautomer, a hydrate, a solvate, an isotopically labeled compound, or a prodrug thereof, 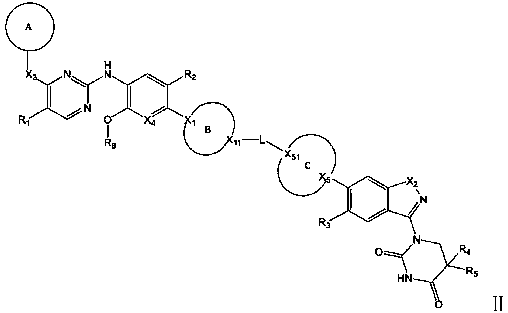 wherein,
X₃ is selected from the group consisting of none, NH, NR, and O;
X₂ is selected from the group consisting of NR, O, and S;
each R is independently selected from the group consisting of C1-6 alkyl, and C6-10 aryl;
X₁ is selected from the group consisting of CH, and N;
X₁₁ is selected from the group consisting of CH, and N;
X₄ is selected from the group consisting of N, and CR';
X₅ is selected from the group consisting of CH, N, and C;
X₅₁ is selected from the group consisting of C-(OH), N, and C;
ring B 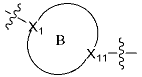 is selected from the group consisting of substituted or unsubstituted 6-7-membered heteromonocycloalkylene containing 1 to 3 heteroatoms selected from N, O or S, substituted or unsubstituted 7-9-membered bridged heterocycloalkylene containing 1 to 3 heteroatoms selected from N, O or S, and substituted or unsubstituted 7-10-membered spiro heterocycloalkylene containing 1 to 3 heteroatoms selected from N, O or S; the "substituted" refers to one or more hydrogens of the groups being substituted by substituents selected from the group consisting of halogen, amino, nitro, hydroxy, cyano, C₁₋₆ alkyl, halogenated C₁₋₆ alkyl, hydroxy-substituted C₁₋₆ alkyl, C₁₋₆ alkoxy, and C₃₋₆ cycloalkyl;
ring C 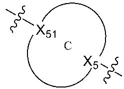 is selected from the group consisting of substituted or unsubstituted 6-7-membered heteromonocycloalkylene containing 1-3 heteroatoms selected from N, O or S, substituted or unsubstituted 7-9-membered spiro heterocycloalkylene containing 1-3 heteroatoms selected from N, O or S, substituted or unsubstituted 4-10-membered bridged heterocycloalkylene containing 1 to 3 heteroatoms selected from N, O or S, and substituted or unsubstituted 5-7-membered heteroarylene containing 1-3 heteroatoms selected from N, O or S; the "substituted" refers to one or more hydrogens of the groups being substituted by a substituent selected from the group consisting of halogen, amino, nitro, hydroxy, cyano, C₁₋₆ alkyl, halogenated C₁₋₆ alkyl, hydroxy-substituted C₁₋₆ alkyl, C₁₋₆ alkoxy, and C₃₋₆ cycloalkyl;
ring A is selected from the group consisting of substituted or unsubstituted C₆₋₁₀ aryl, and substituted or unsubstituted 5-10-membered heteroaryl containing 1 to 3 heteroatoms selected from N, O or S; the "substituted" refers to one hydrogen atom of the group being substituted by R₆ and m hydrogen atoms being substituted by R₇;
R₆ is selected from the group consisting of C₁₋₆ alkyl, hydroxy-substituted C₁₋₆ alkyl, -P(O)RaRb, 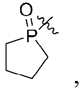 -C(O)Ra, -C(O)NRaRb, -OC(O)NRaRb, -S(O)₂Ra, -NRa-S(O)₂Rb, -NRa-C(O)-ORa, 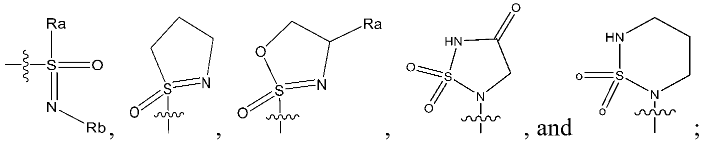
Ra and Rb are each independently selected from the group consisting of H and C₁₋₆ alkyl;
R₇ is selected from the group consisting of H, halogen, amino, nitro, hydroxy, cyano, C₁₋₆ alkyl, halogenated C₁₋₆ alkyl, hydroxy-substituted C₁₋₆ alkyl, C₁₋₆ alkoxy, and C₃₋₆ cycloalkyl;
R₁ is halogen;
R₂ is selected from the group consisting of H, halogen, C₁₋₆ alkyl, halogenated C₁₋₆ alkyl, C₁₋₆ alkoxy, halogenated C₁₋₆ alkoxy, C₃₋₆ cycloalkyl, -(C=O)-O-C₁₋₆ alkyl, and -(C=O)-NH-C₁₋₆ alkyl;
R₃ is a halogen;
R₄ is selected from the group consisting of H and halogen;
R₅ is selected from the group consisting of H and halogen;
R₈ is selected from the group consisting of C₁₋₆ alkyl, and halogenated C₁₋₆ alkyl; or R₈ and R' with the atoms to which they are attached together form a 5-6-membered heterocyclic group containing one O atom,
L is selected from the groups consisting of 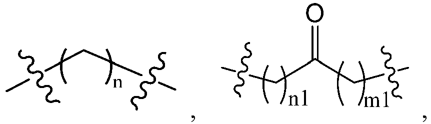 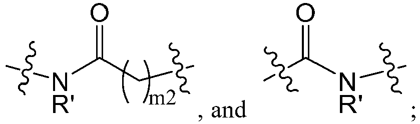
each R' is independently selected from the group consisting of H and C1-6 alkyl;
m is selected from the group consisting of 0, 1, and 2;
n is selected from the group consisting of 0, 1, and 2;
n1 is selected from the group consisting of 0, 1, and 2;
m1 is selected from the group consisting of 0, 1, and 2;
m2 is selected from the group consisting of 0, 1, and 2.

3. A compound as shown in Formula III, or a pharmaceutically acceptable salt, a stereoisomer, a tautomer, a hydrate, a solvate, an isotope, or a prodrug thereof, 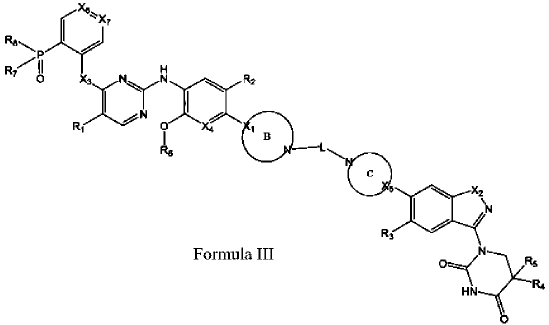 in the formula,
X₁ and X₅ are each independently selected from CH, N, or C;
X₂ is selected from NR, O, or S;
X₃ is selected from none(bond), NH, NR, or O;
X₄, X₆, and X₇ are each independently selected from the group consisting of CR and N;
each R is independently selected from the group consisting of H, halogen, hydroxy, amino, nitro, C₁₋₆ alkyl, C₁₋₆ alkoxy, halogenated C₁₋₆ alkyl, halogenated C₁₋₆ alkoxy, C₃₋₇ cycloalkyl, C₃₋₇ cycloalkyloxy, C₆₋₁₀ aryl, and C₆₋₁₀ aryloxy;
L is 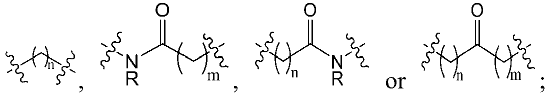
m and n are each independently 0, 1, 2, 3 or 4;
R₇ and R₈ with the P atom to which they are attached together form Cy1, and Cy1 is selected from the group consisting of saturated or partially unsaturated 4-7-membered ring containing P=O, except for P, the ring further comprises 0-3 heteroatoms independently selected from nitrogen, oxygen, or sulfur; and the ring is optionally substituted by one or more substituents R^{a}; or
R₇ and R₈ are each independently selected from the group consisting of C₁₋₆ alkyl, halogenated C₁₋₆ alkyl, and C₁₋₆ hydroxyalkyl;
R^{a} is selected from the group consisting of hydrogen, deuterium, halogen, C₁₋₈ alkyl, C₂₋₈ alkenyl, C₂₋₈ alkynyl, C₃₋₈ cycloalkyl, 3-8-membered heterocyclyl, C₆₋₁₀ aryl, 5-12-membered heteroaryl, -CN, -OR^{b}, -COR^{b}, -COOR^{b}, CONR^{b}R^{c}, -NR^{b}R^{c}, -NR^{b}COR^{c}, and -NR^{b}COOR^{c}, wherein, the alkyl, alkenyl, alkynyl, cycloalkyl, heterocyclic, aryl, or heteroaryl groups optionally be substituted by R^{d};
R^{b}, R^{c} and R^{d} are independently selected from the group consisting of hydrogen, hydroxyl, C₁₋₈ alkyl, C₂₋₈ alkenyl, C₂₋₈ alkynyl, and C₃₋₈ cycloalkyl;
R₁ is selected from halogen;
R₂ is selected from H, C₁₋₆ alkyl, C₁₋₆ haloalkyl, C₃₋₈ cycloalkyl, C₄₋₈ heterocycloalkyl, -(C=O)-O-C₁₋₆ alkyl, -(C=O)-NH-C₁₋₆ alkyl, halogen, -(CH₂)ₚ-C₁₋₄-alkyloxy, -(CH₂)ₚ-halogenated C₁₋₄-alkyloxy, -(CH₂)ₚ-CN, C₁₋₆ alkyl substituted or unsubstituted 5-6-membered heteroaryl containing 1-3 heteroatoms selected from N, O or S or 2-6-membered heteroalkyl; wherein, the heteroalkyl or heterocycloalkyl contains one or more heteroatoms independently selected from the group consisting of O, N, and S; p is 0, 1, 2, or 3;
R₃ is selected from the group consisting of H, halogen, cyano, amino, nitro, hydroxy, C₁₋₄ alkyl, halogenated C₁₋₄ alkyl, C₁₋₄ alkoxy, halogenated C₁₋₄ alkoxy, and C₁₋₄ hydroxyalkyl;
R₄ is selected from the group consisting of H, halogen, cyano, amino, nitro, hydroxy, C₁₋₄ alkyl, halogenated C₁₋₄ alkyl, C₁₋₄ alkoxy, halogenated C₁₋₄ alkoxy, and C₁₋₄ hydroxyalkyl;
R₅ is selected from the group consisting of H, halogen, cyano, amino, nitro, hydroxy, C₁₋₄ alkyl, halogenated C₁₋₄ alkyl, C₁₋₄ alkoxy, halogenated C₁₋₄ alkoxy, and C₁₋₄ hydroxyalkyl;
or, R₄ and R₅ form a C₃₋₆ carbocycle or a 3-7-membered heterocycle, wherein the heteroatoms of the heterocycle are selected from O, N, or S;
R₆ is selected from the group consisting of C₁₋₆ alkyl, halogenated C₁₋₆ alkyl, and C₁₋₆ hydroxyalkyl; or, X₄ is CR, wherein the R of X₄ and R₆ form a saturated or partially saturated 5-6-membered heterocyclyl containing one oxygen atom;
ring B 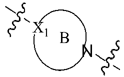
is selected from the group consisting of substituted or unsubstituted 6-7-membered heterocyclylene, substituted or unsubstituted 7-12-membered spiro heterocyclylene, and substituted or unsubstituted 7-12-membered bridged heterocyclylene; the "substituted" refers to one or more hydrogen atoms of the group are substituted by substituents selected from the group consisting of H, D, halogen, oxo(=O), amino, nitro, hydroxy, cyano, C₁₋₆ alkyl, C₁₋₆ alkoxy, C₃₋₆ cycloalkyl, halogenated C₁₋₆ alkyl, C₁₋₆ hydroxyalkyl, or any two hydrogen atoms connected to form a bond or - (CH₂)_{q}-, wherein, q is 1, 2, 3, 4, or 5;
ring C 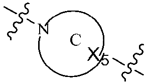
is selected from the group consisting of substituted or unsubstituted 6-7-membered heterocyclylene, substituted or unsubstituted 5-6-membered heteroarylene, and substituted or unsubstituted 7-12-membered spiro heterocyclylene; the "substituted" refers to one or more hydrogens of the group being substituted by a substituent selected from the group consisting of H, halogen, amino, oxo (=O), nitro, hydroxy, cyano, C1-6 alkyl, C1-6 alkoxy, C3-6 cycloalkyl, halogenated C1-6 alkyl, C1-6 hydroxyalkyl, or any two hydrogen atoms linked to form a bond or - (CH₂)_{q}-, wherein, q is 1, 2, 3, 4, or 5;
wherein, the heterocyclylene, heteroarylene, spiro heterocyclylene, and bridged heterocyclene each independently contain 1-3 heteroatoms selected from N, S, or O.

4. The compound according to claim 3, wherein ring B 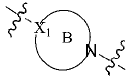 is selected from the group consisting of substituted or unsubstituted 6-7-membered heterocyclylene, and substituted or unsubstituted 7-10-membered spiro heterocyclylene; more preferably, ring B 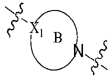 is substituted or unsubstituted group selected from the group consisting of 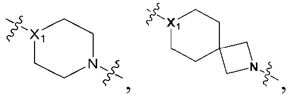 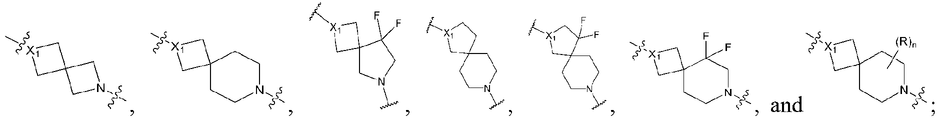 wherein the "substituted" refers to one or more hydrogen atoms of the group are substituted by substituents selected from the group consisting of H, halogen, amino, oxo (=O), nitro, hydroxy, cyano, C₁₋₆ alkyl, C₁₋₆ alkoxy, C₃₋₆ cycloalkyl, halogenated C₁₋₆ alkyl, C₁₋₆ hydroxyalkyl, or any two hydrogen atoms connected to form a bond or -(CH₂)_{q}-, wherein, q is 1, 2, 3, 4, or 5.

5. The compound according to claim 3, wherein ring C 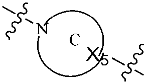 is selected from the substituted or unsubstituted group consisting of a 6-7-membered heterocyclylene, a 5-6-membered heteroarylene, and a 7-10-membered spiro heterocyclylene; more preferably, ring C 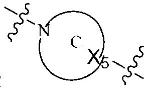 is selected from the substituted or unsubstituted group consisting of 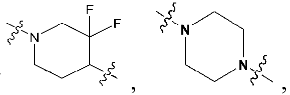 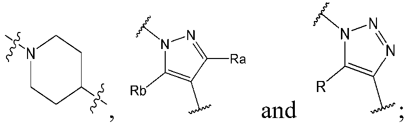 wherein, the "substituted" refers to one or more hydrogen atoms of the group being substituted by a substituent selected from the group consisting of H, halogen, amino, oxo (=O), nitro, hydroxy, cyano, C₁₋₆ alkyl, C₁₋₆ alkoxy, C₃₋₆ cycloalkyl, halogenated C₁₋₆ alkyl, C₁₋₆ hydroxyalkyl, or any two hydrogen atoms connected to form a bond or -(CH₂)_{q}-, wherein, q is 1, 2, 3, 4, or 5; R^{a}, R^{b}, and R are as defined in claim 3.

6. The compound according to claim 3, wherein the compound has the structure as shown in formula 2: 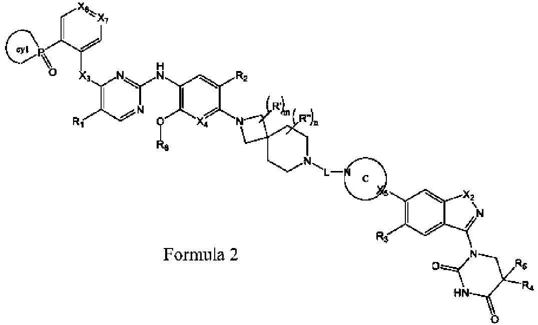 in the formula,
R' and R" are each independently selected from the group consisting of D, halogen, oxo (=O), cyano, C1-6 alkyl, C1-6 alkoxy, C3-6 cycloalkyl, halogeno-C1-6 alkyl, and C1-6 hydroxyalkyl;
or any two R' or two R" form a bond or a -(CH₂)_{q}- group, wherein, q is 1, 2, 3, 4, or 5; or two R' or two R" attached to the same carbon atom with the carbon atoms to which they are attached together form a 3-6-membered saturated carbon ring;
m and n are each independently selected from the group consisting of 0, 1, 2, and 3;
X₂, X₃, X₄, X₅, X₆, X₇, L, Cy1, ring C, R₁, R₂, R₃, R₄, R₅, and R₆ are as defined in claim 3.

7. A compound, or a pharmaceutically acceptable salt, a stereoisomer, a tautomer, a hydrate, a solvate, an isotope, or a prodrug thereof, wherein the compound is selected from the group consisting of:

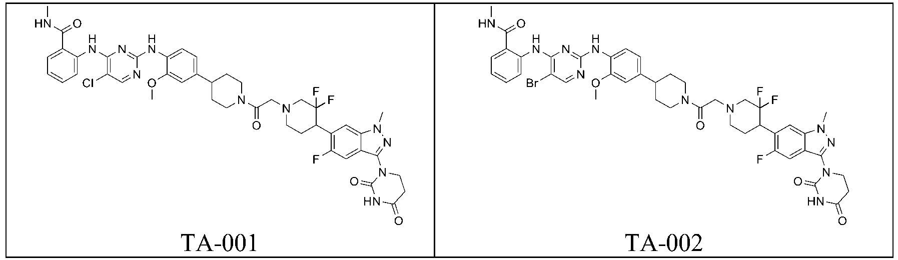

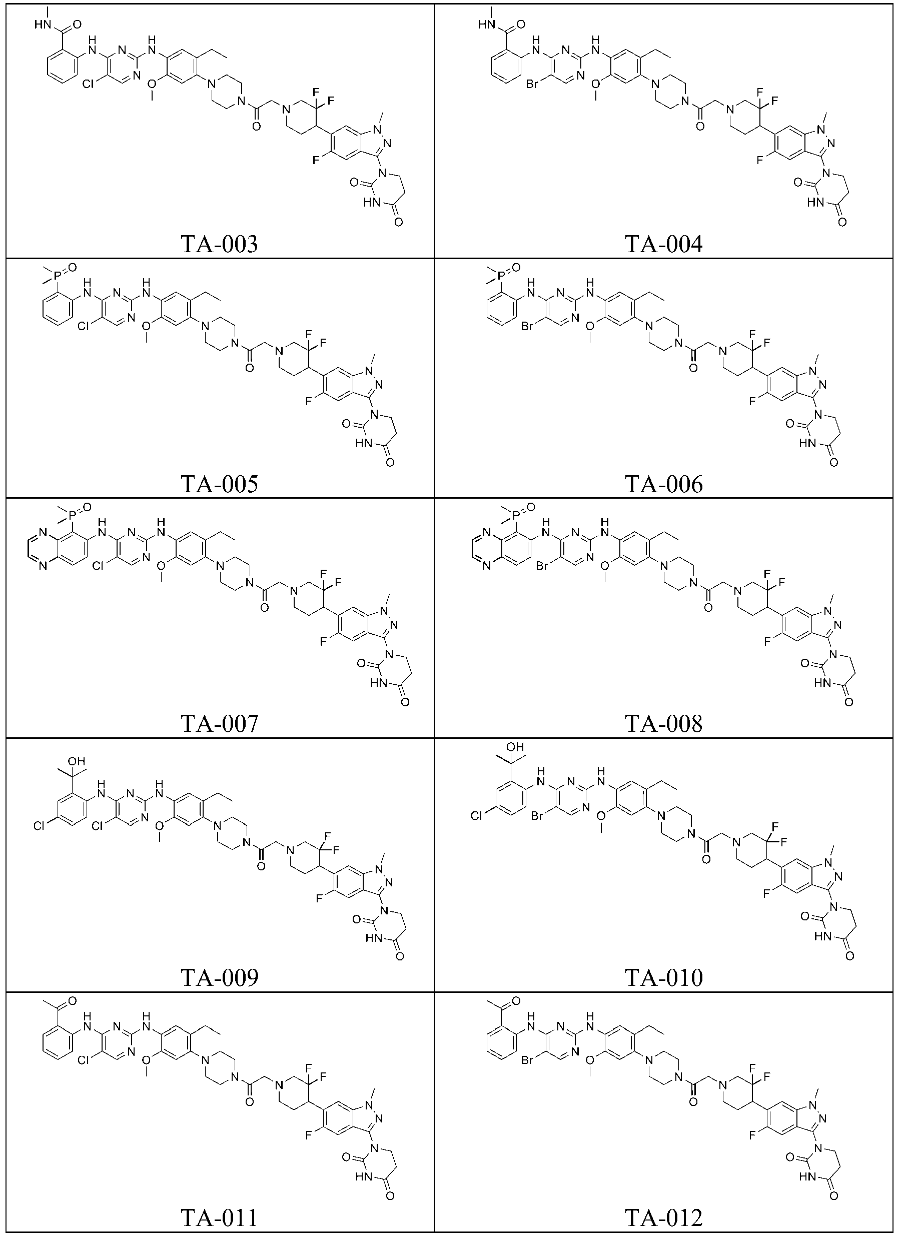

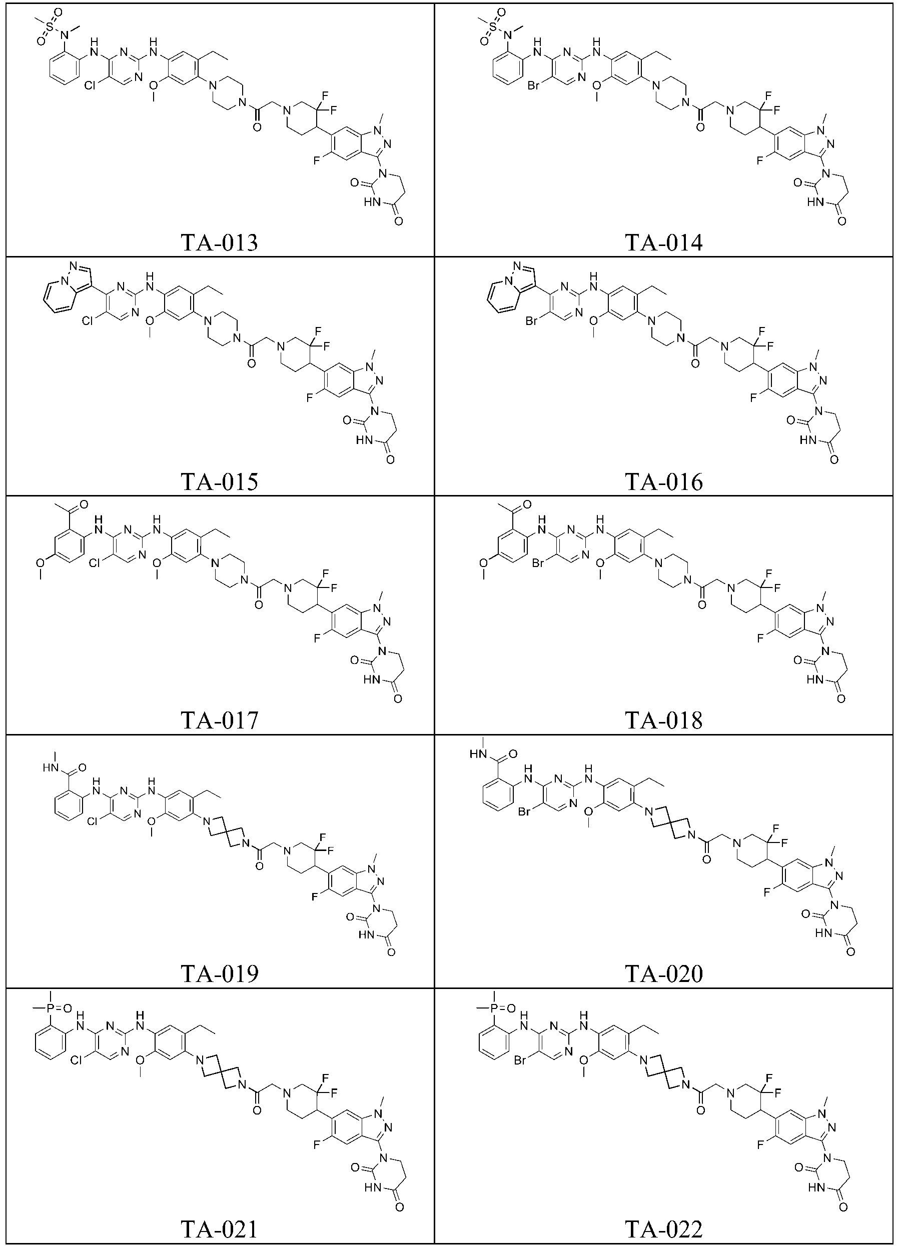

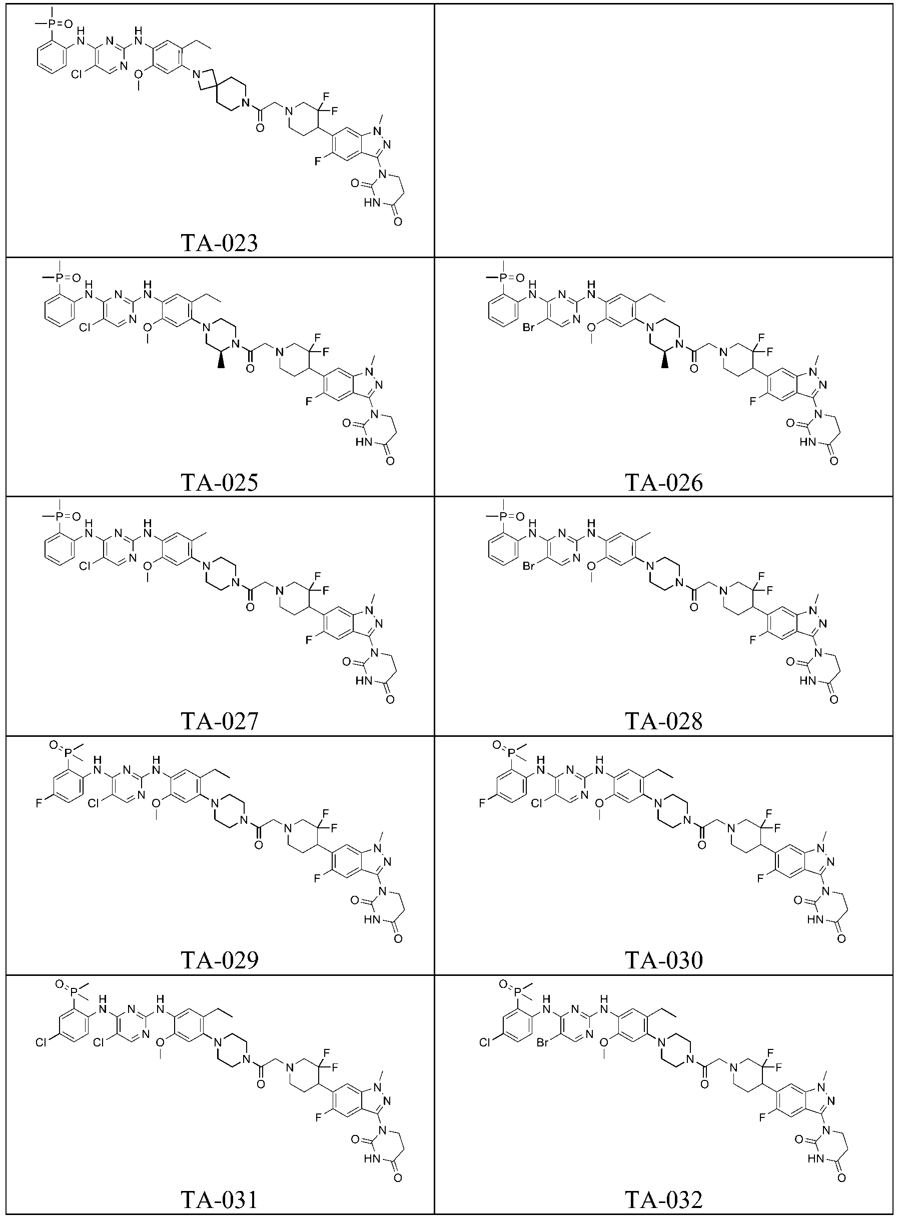

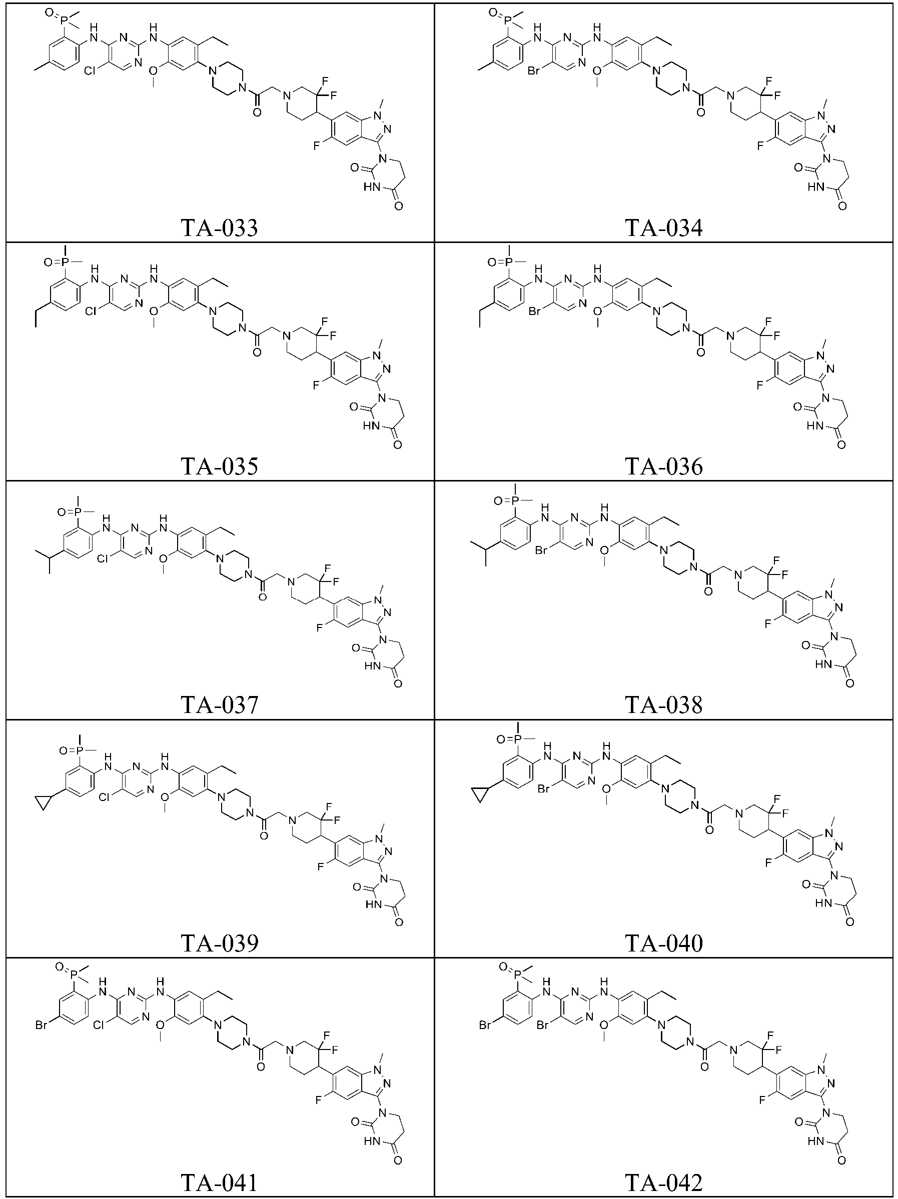

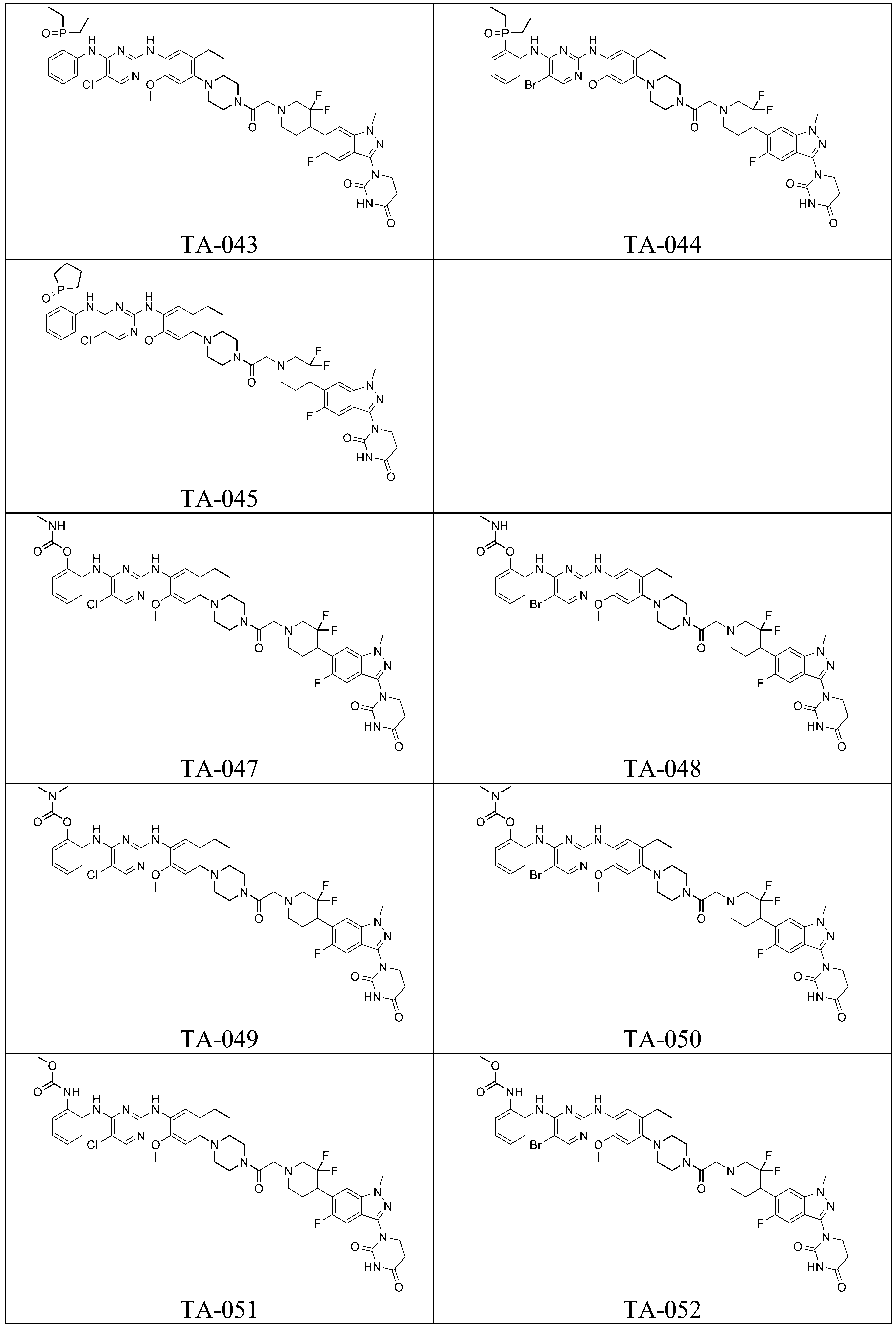

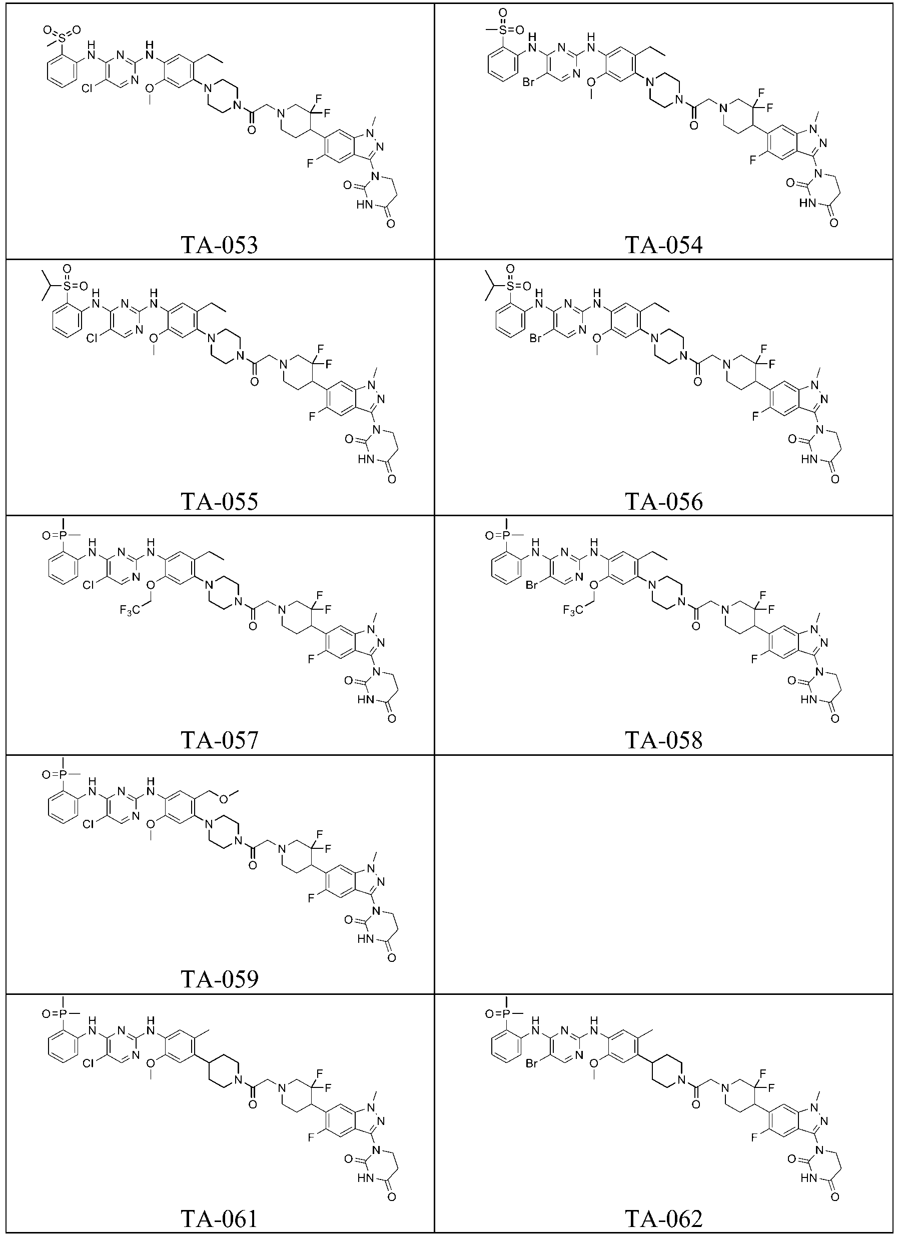

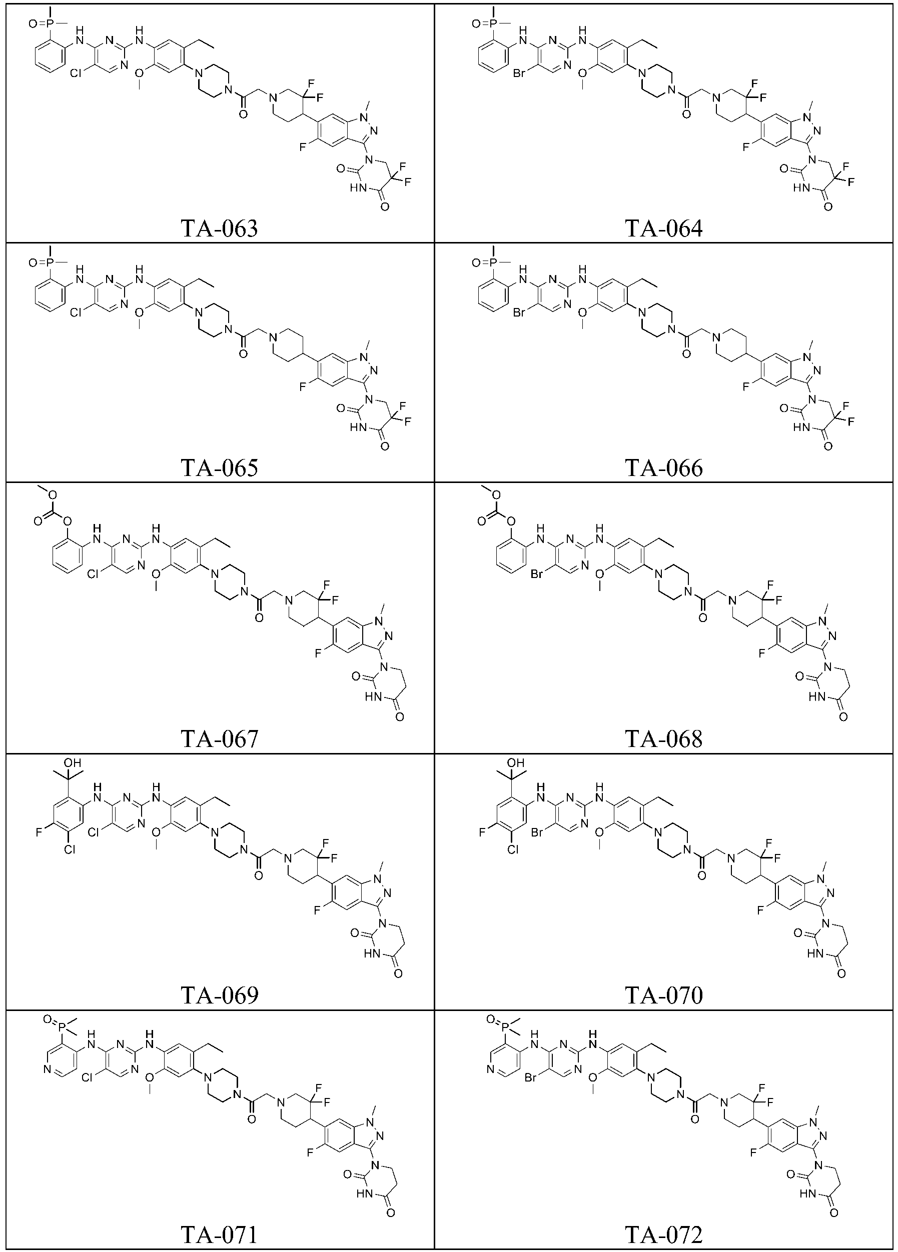

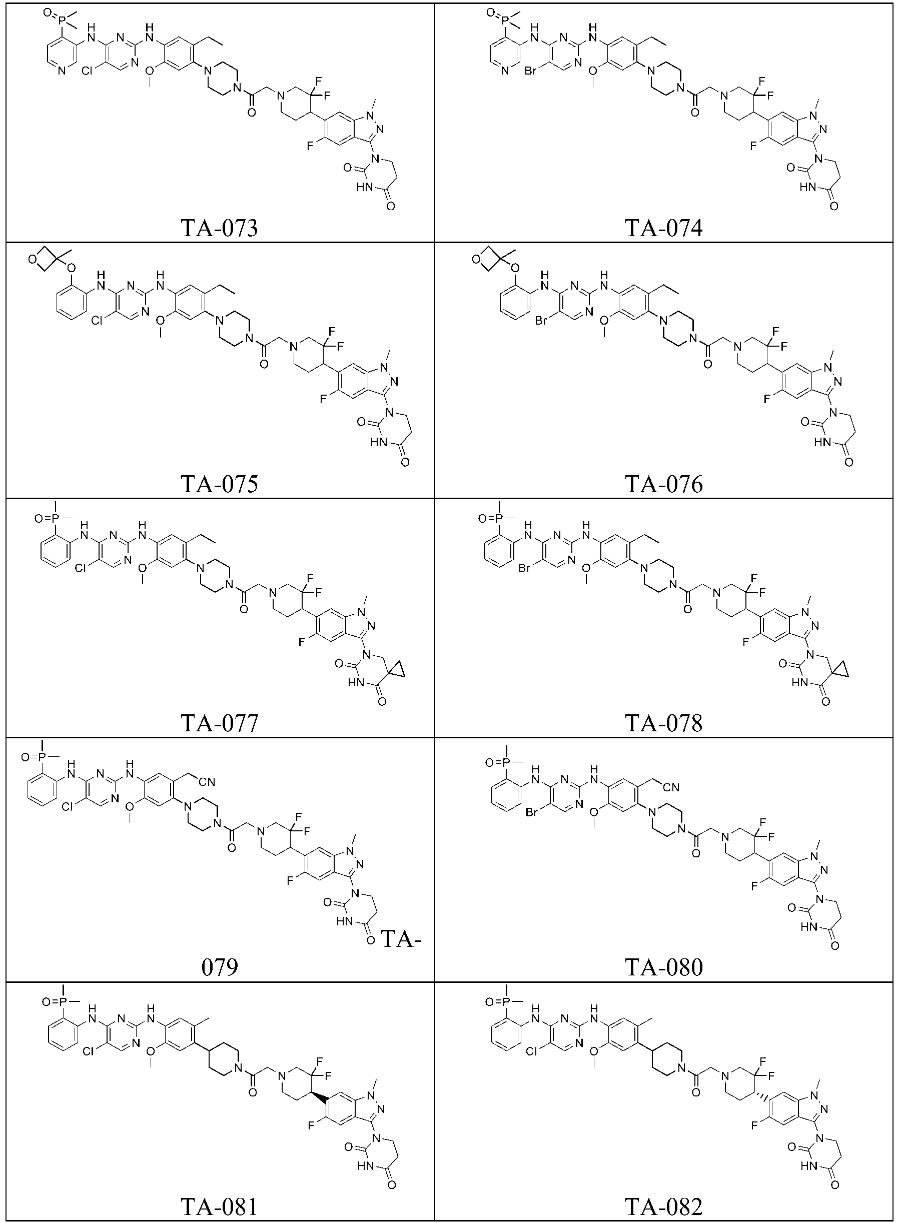

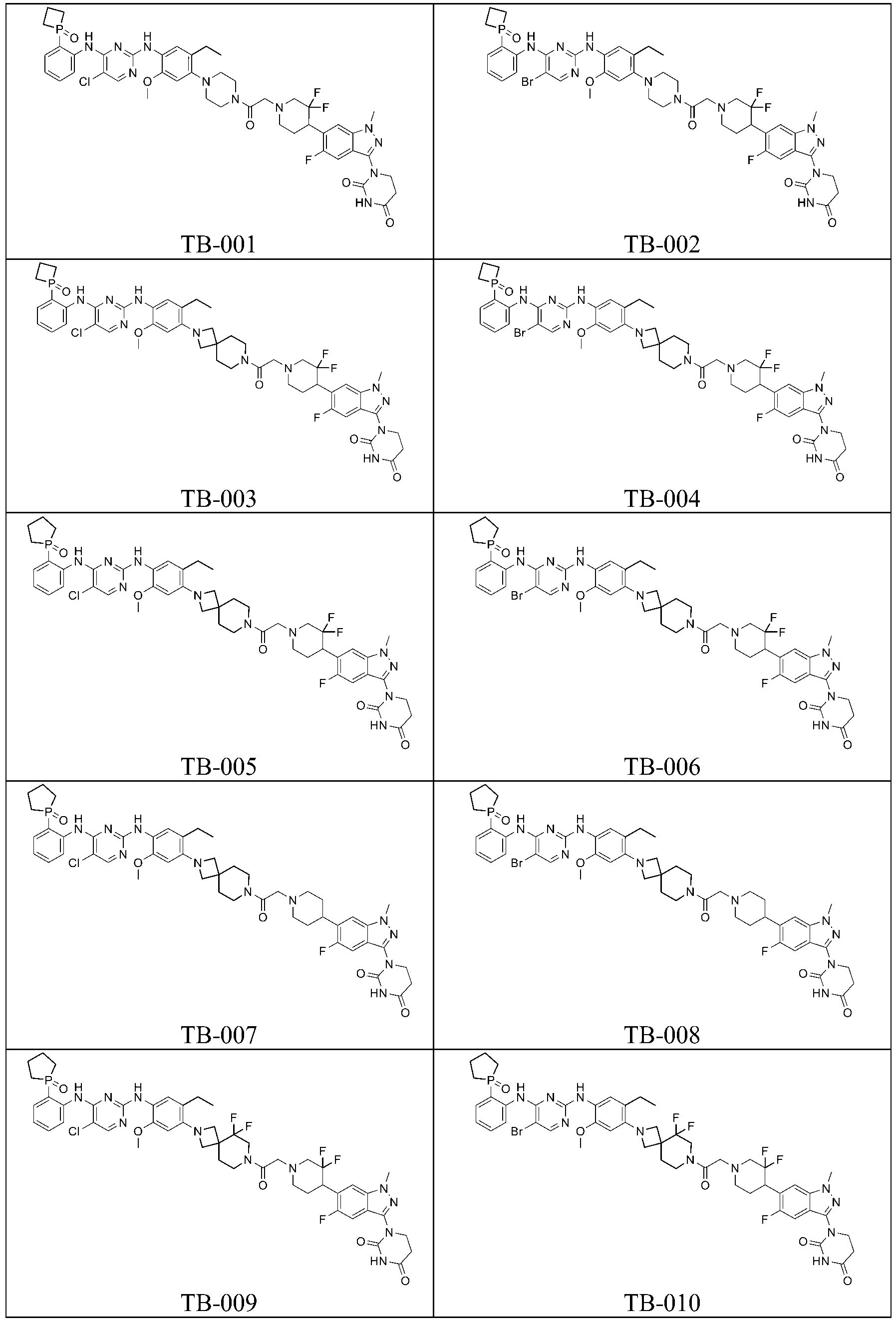

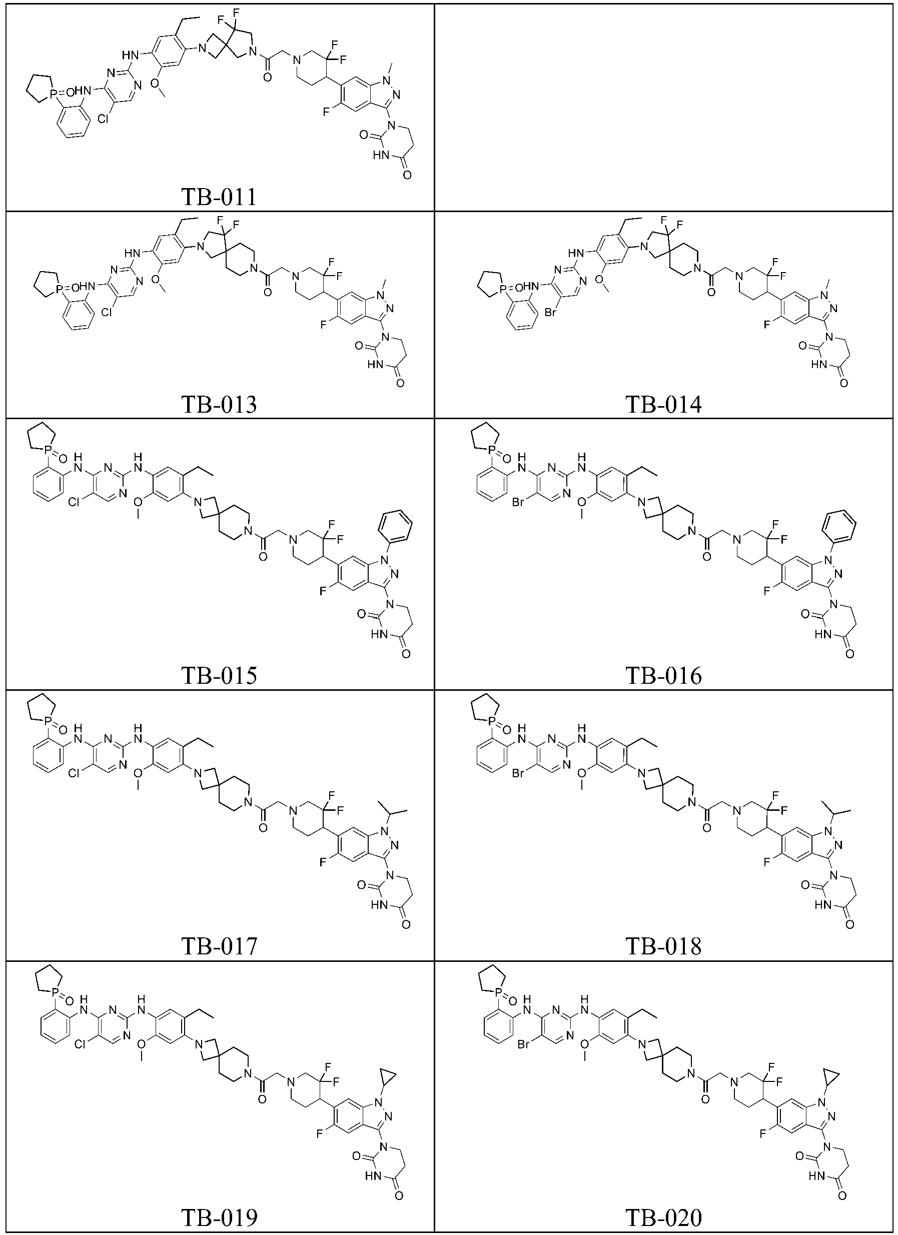

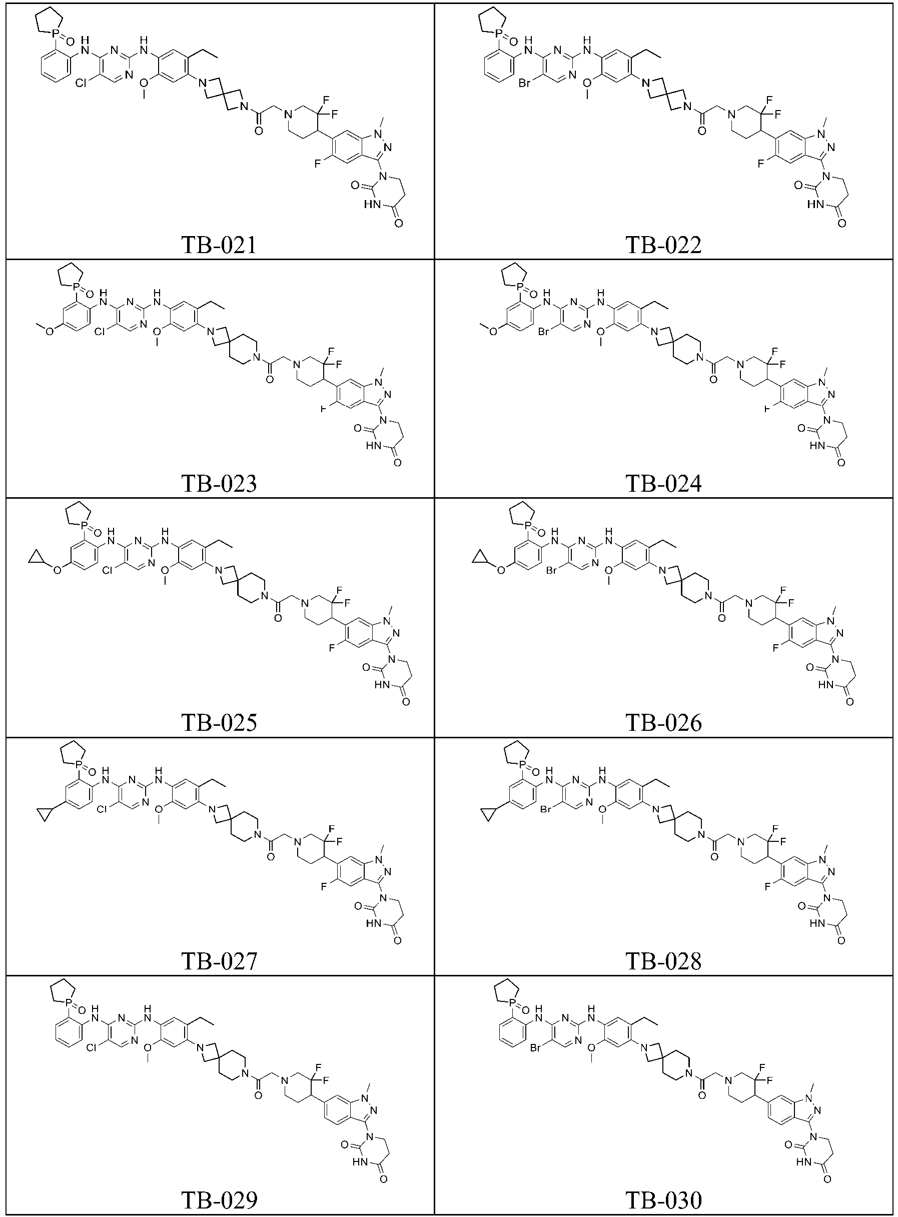

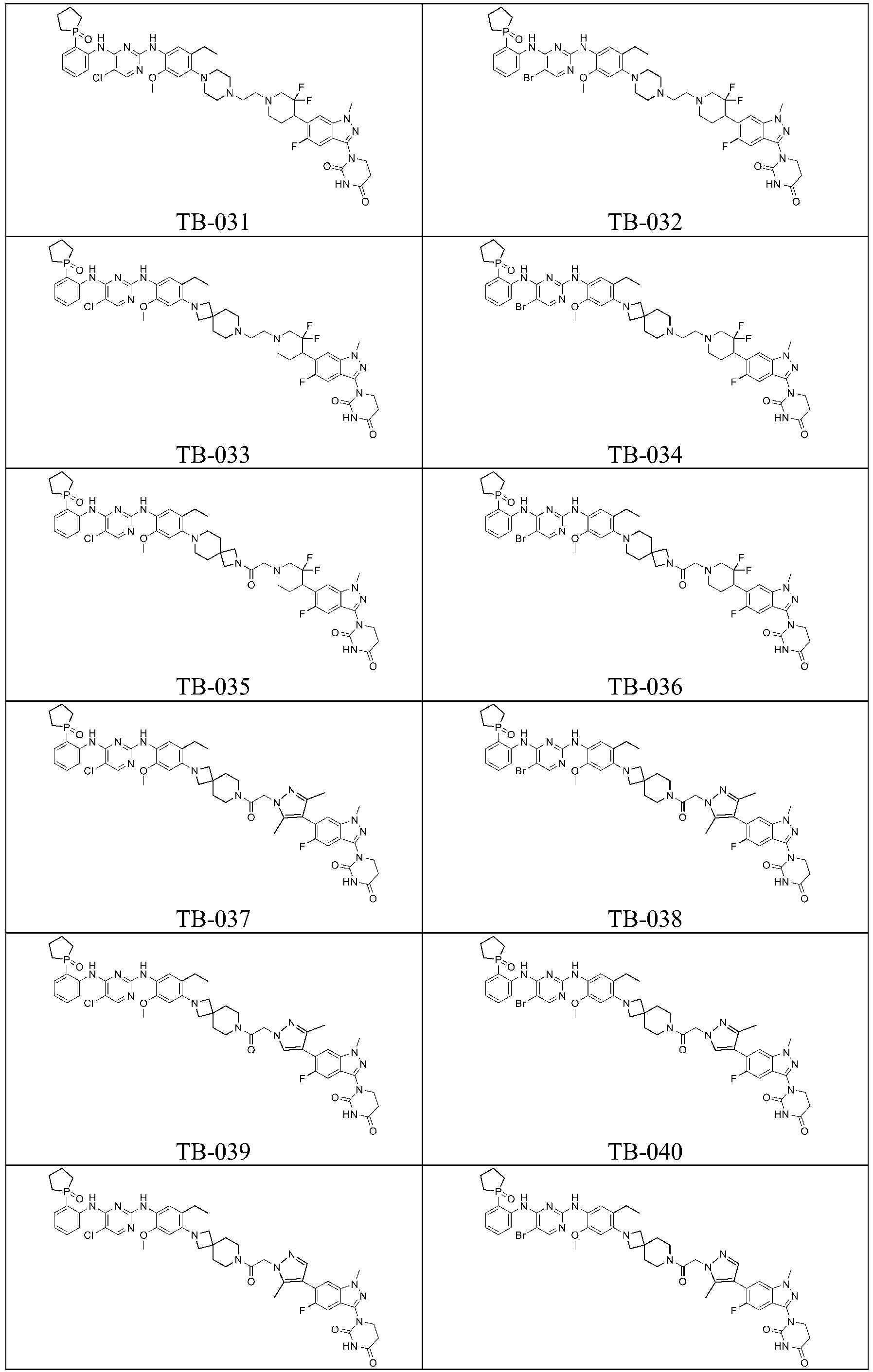

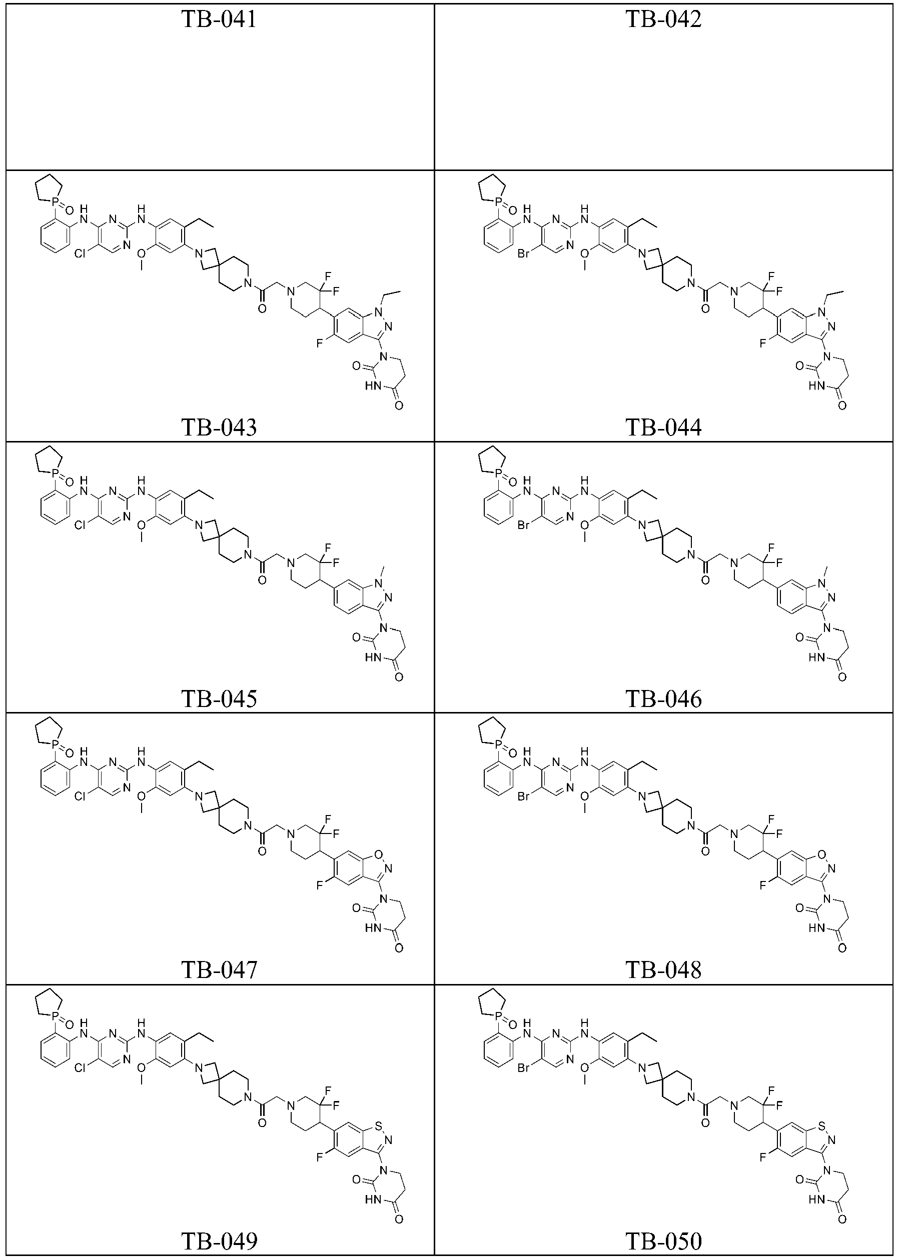

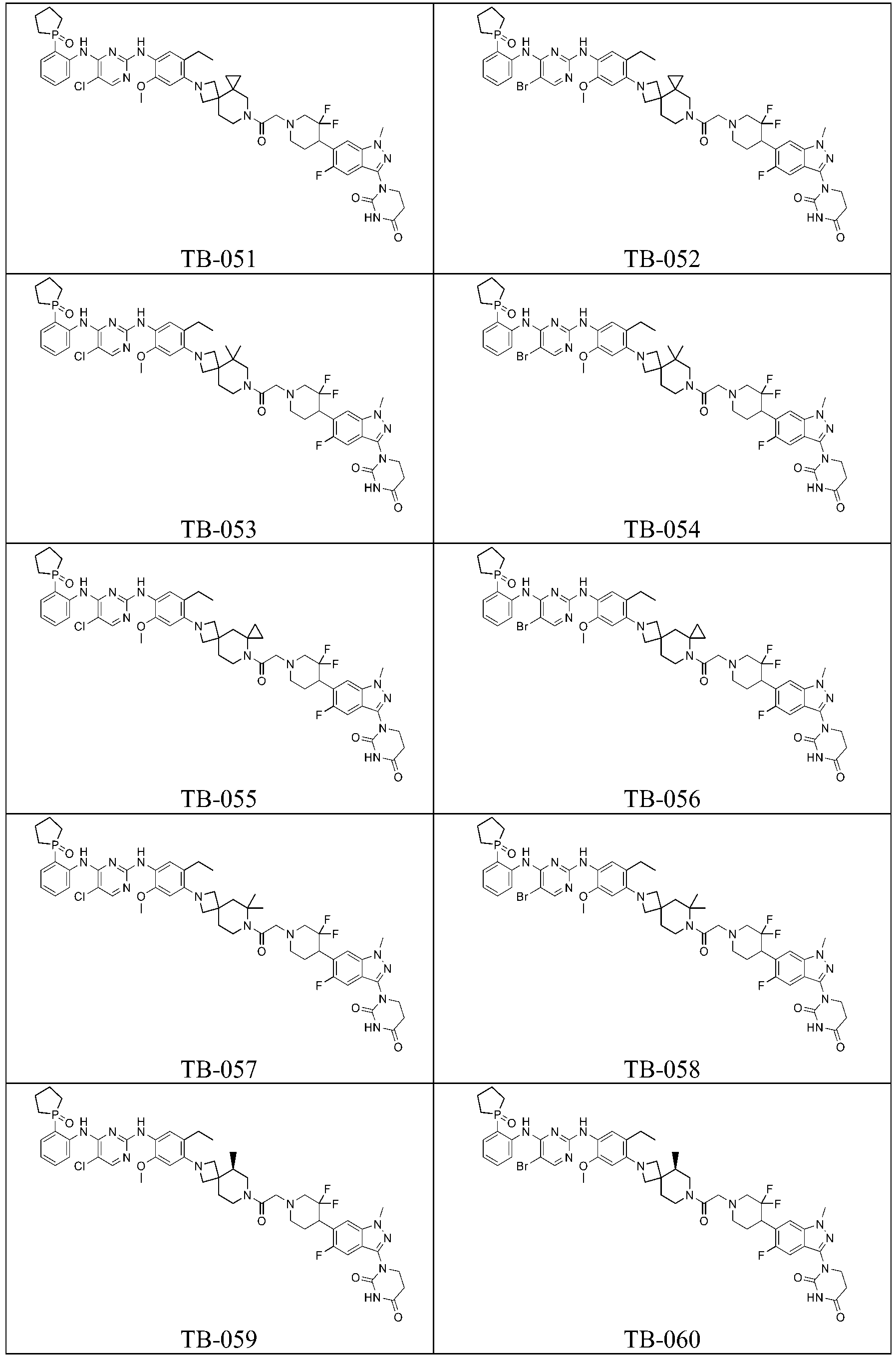

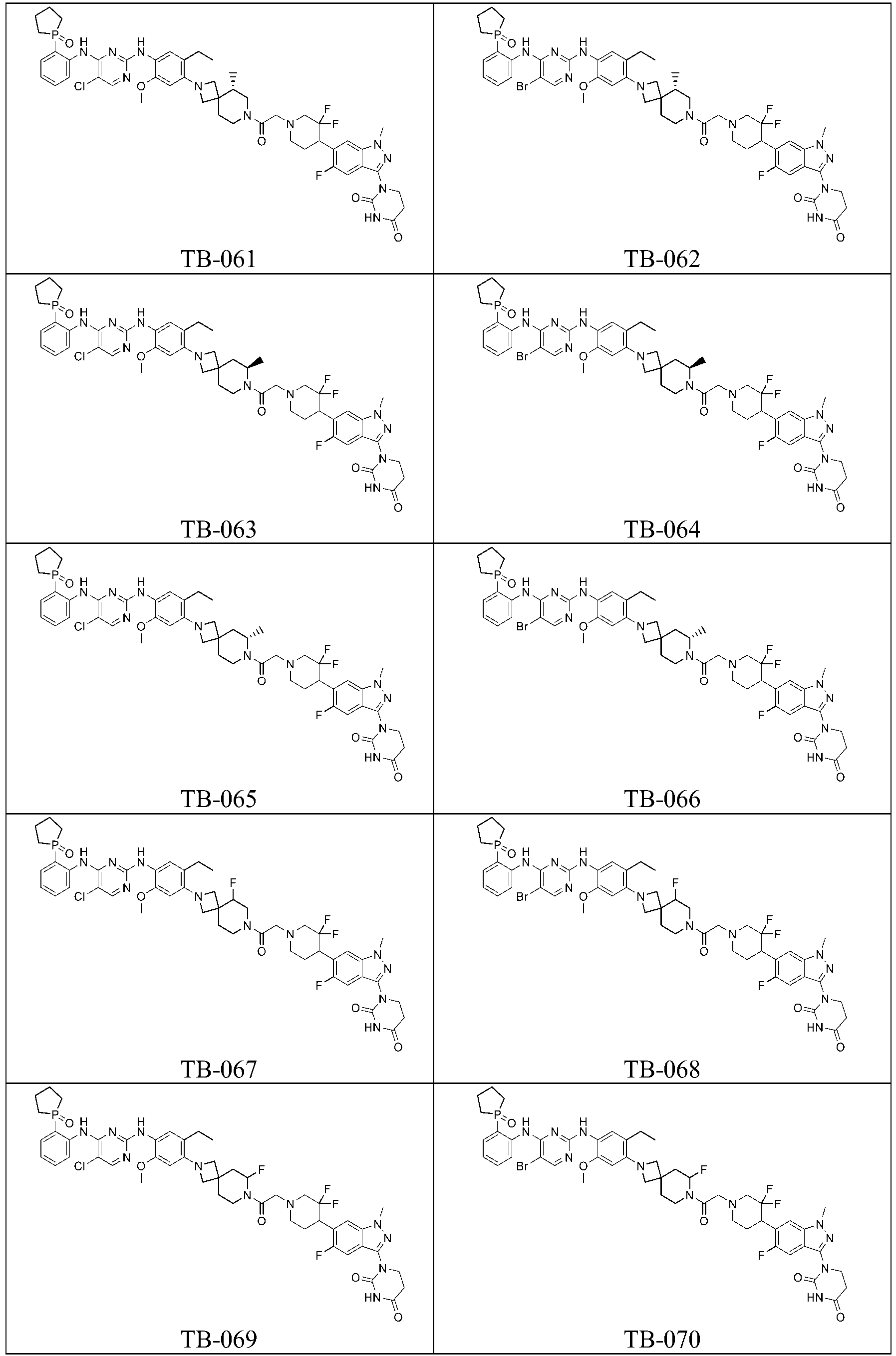

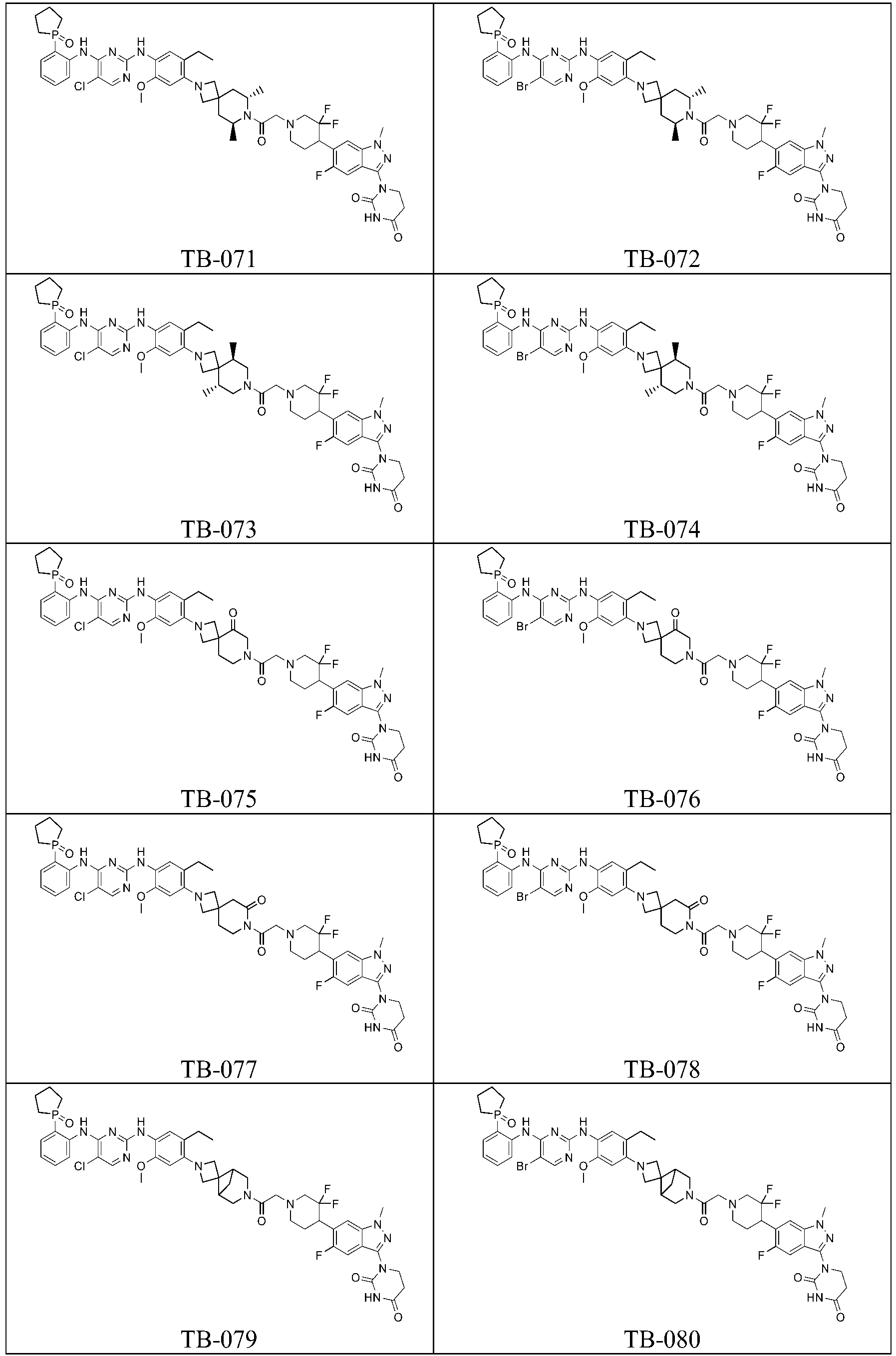

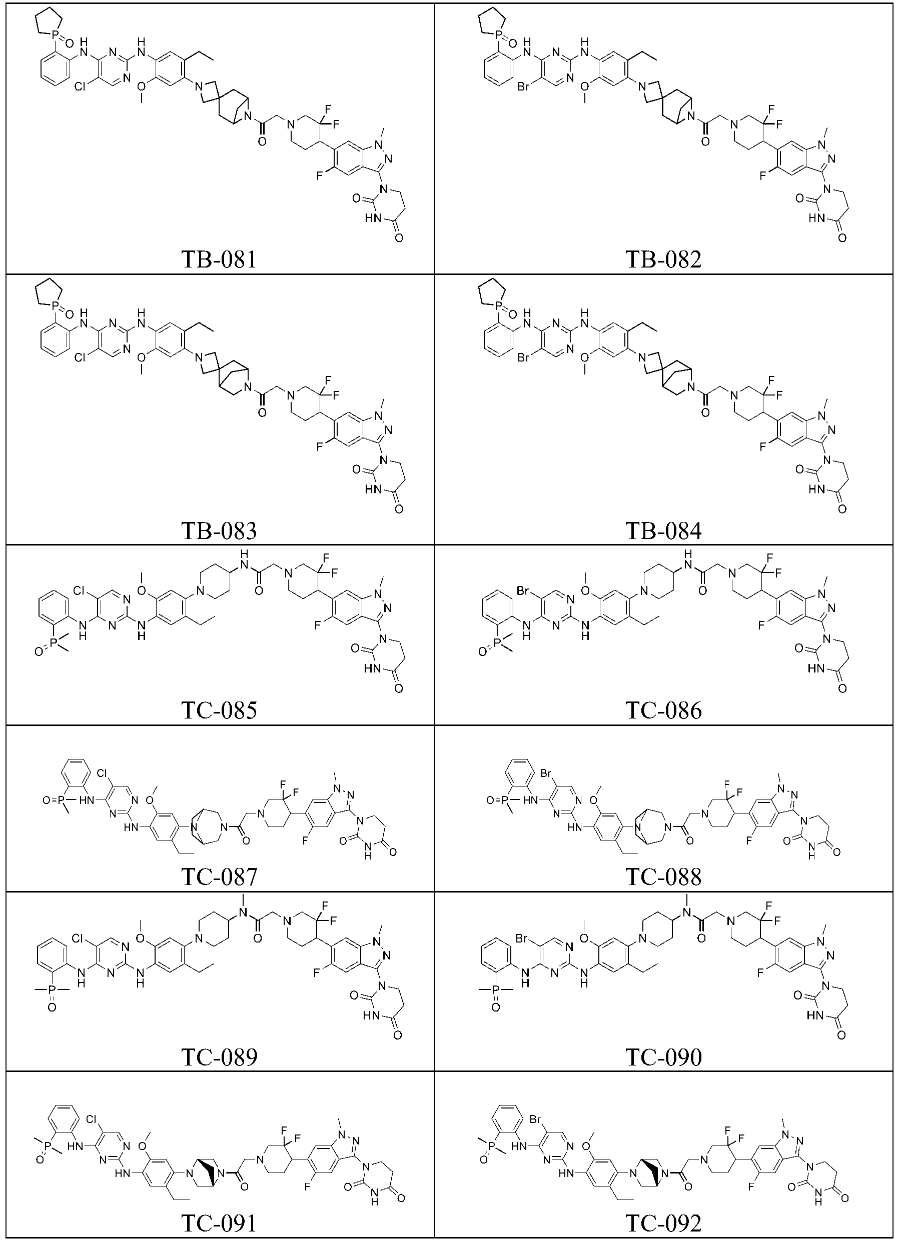

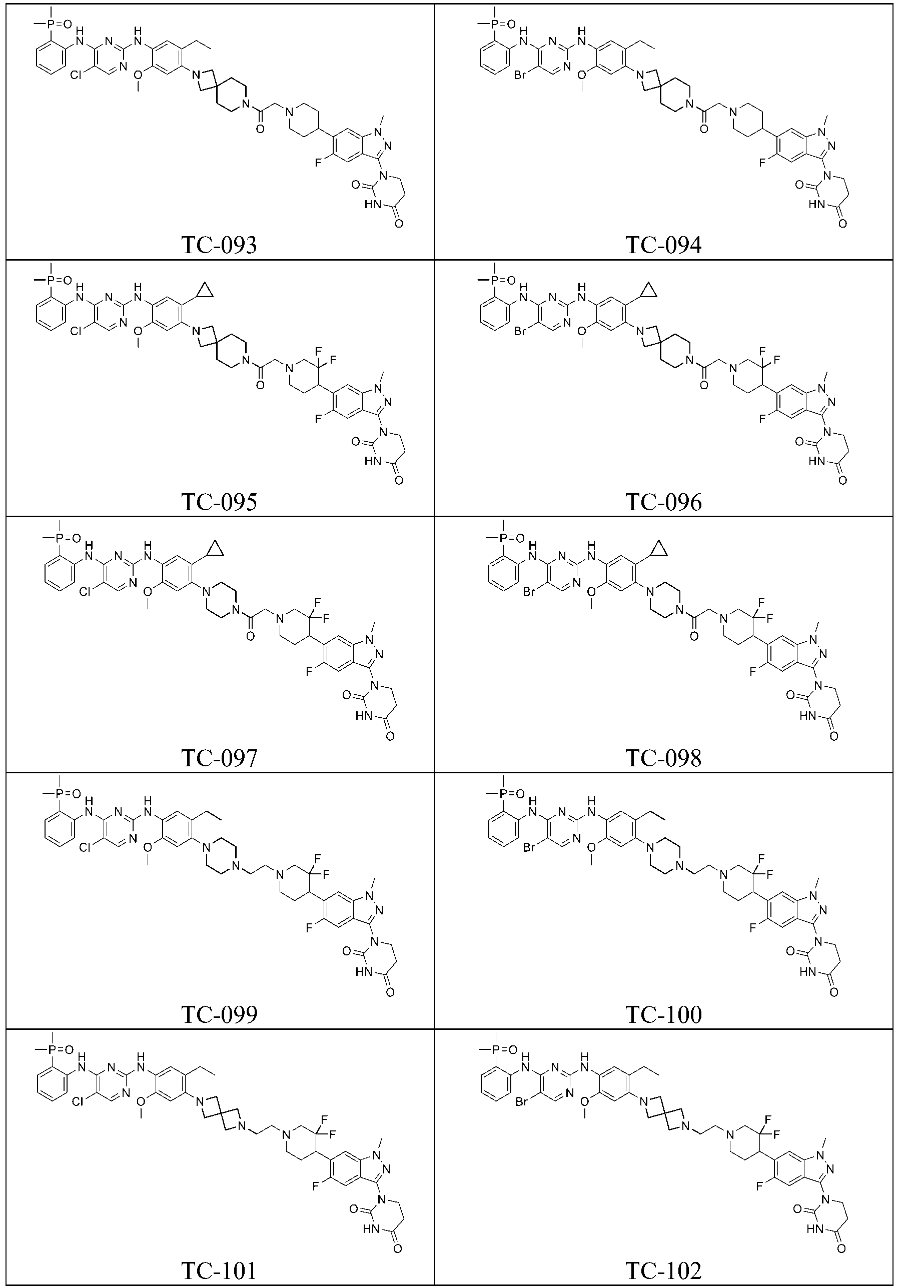

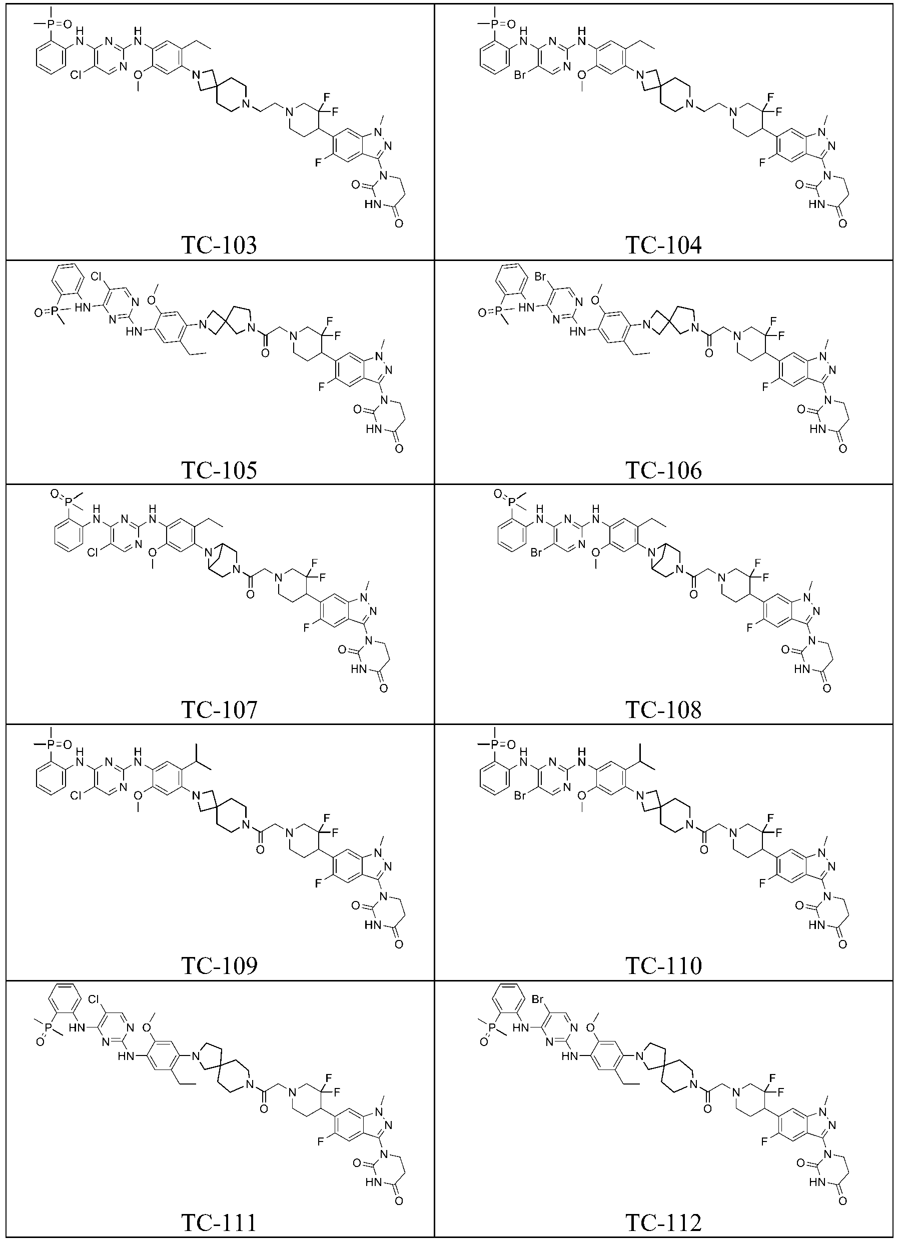

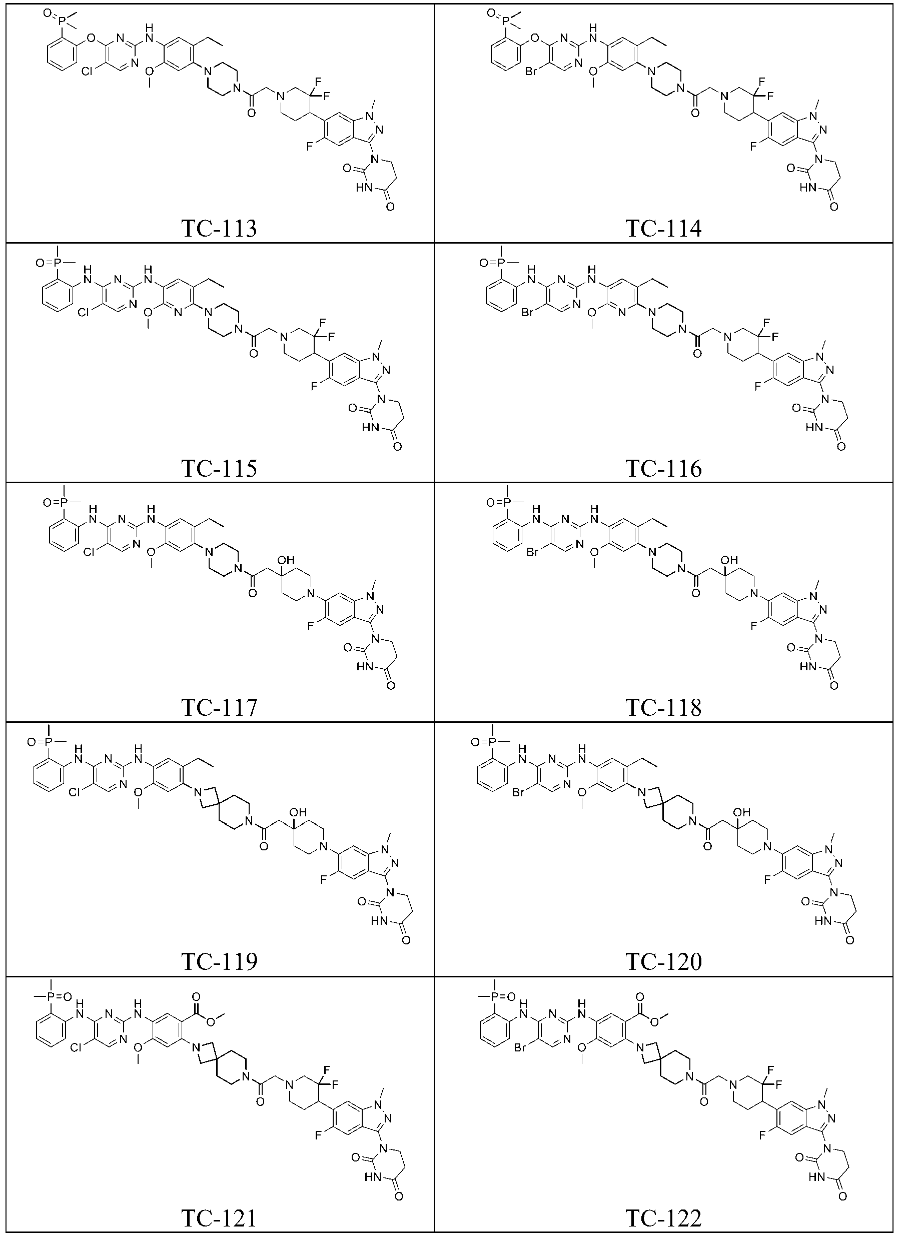

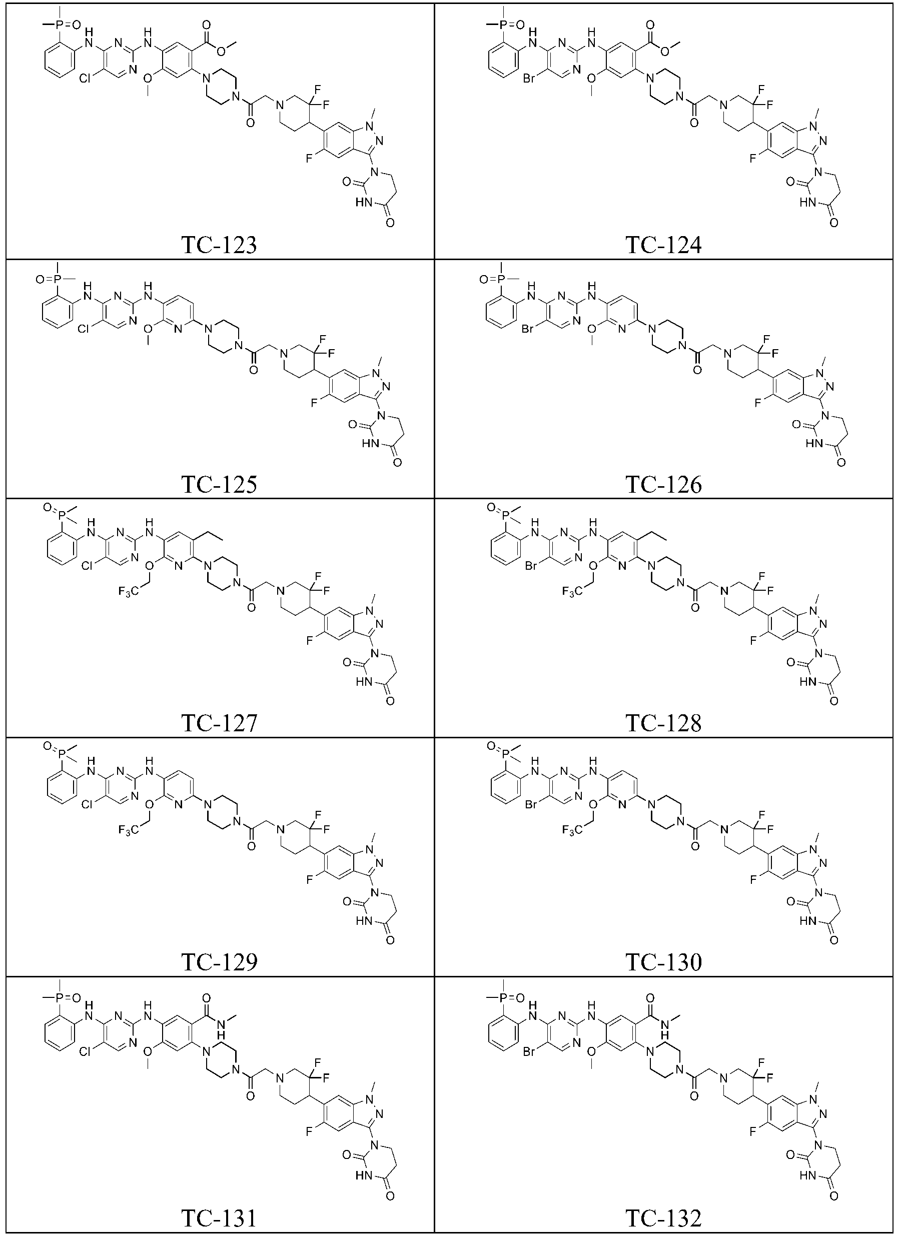

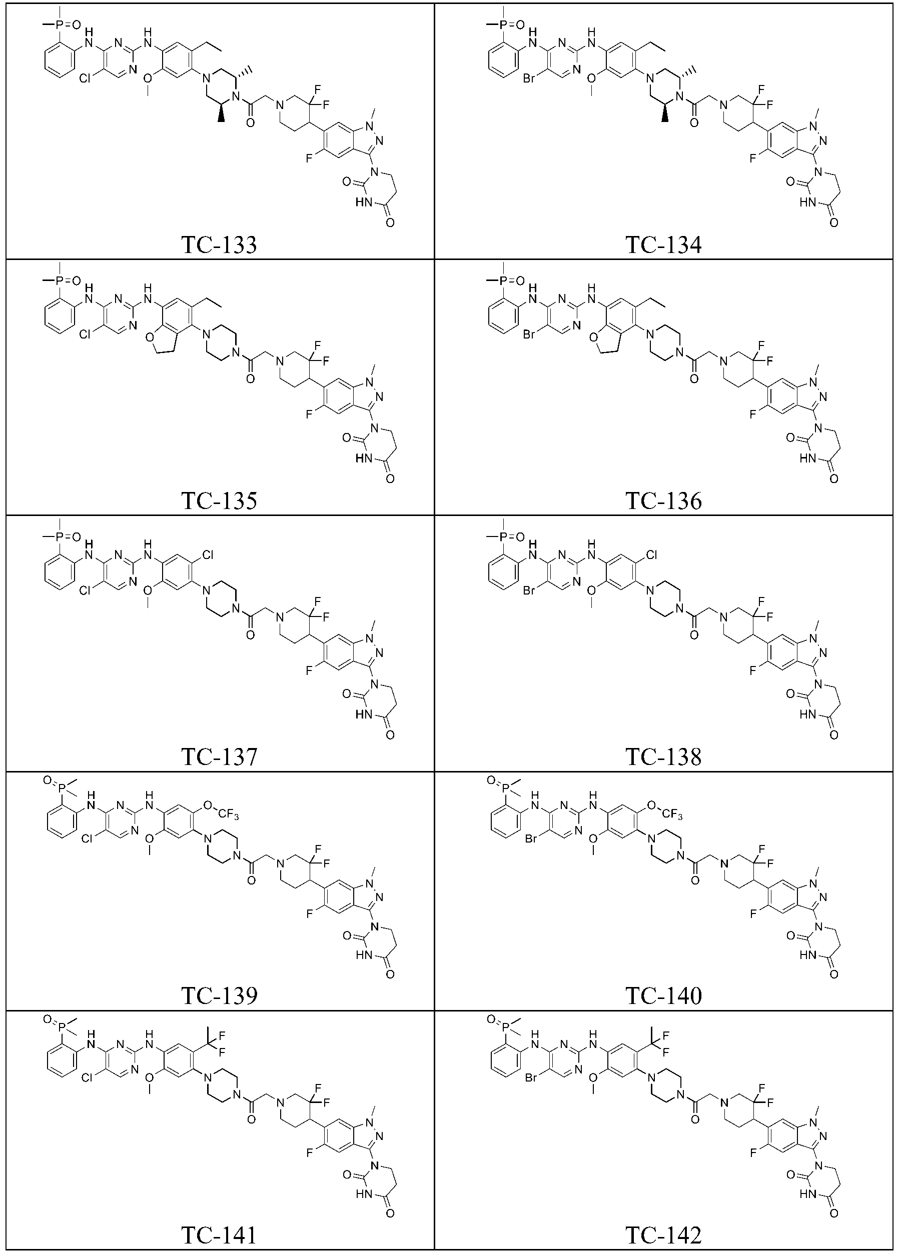

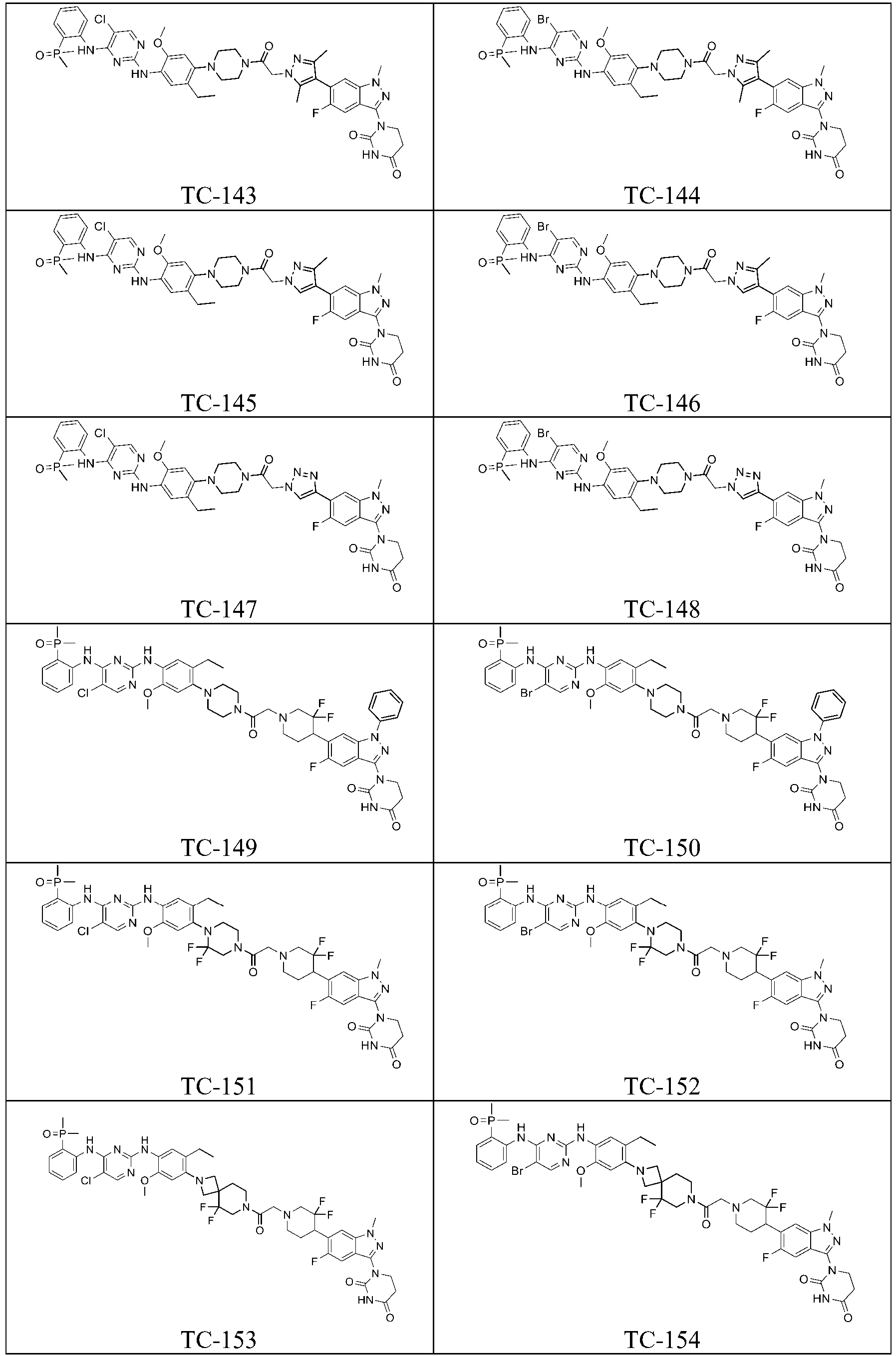

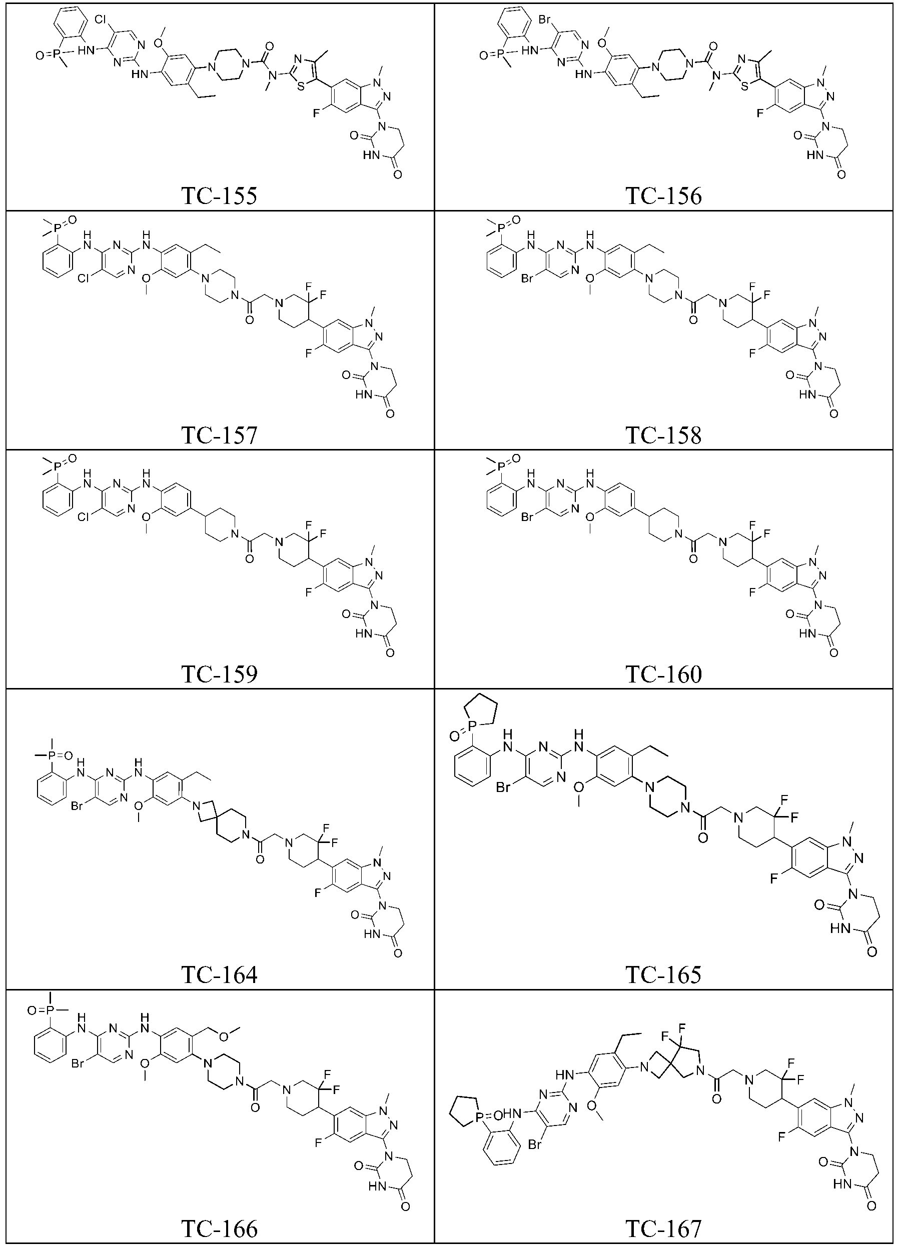

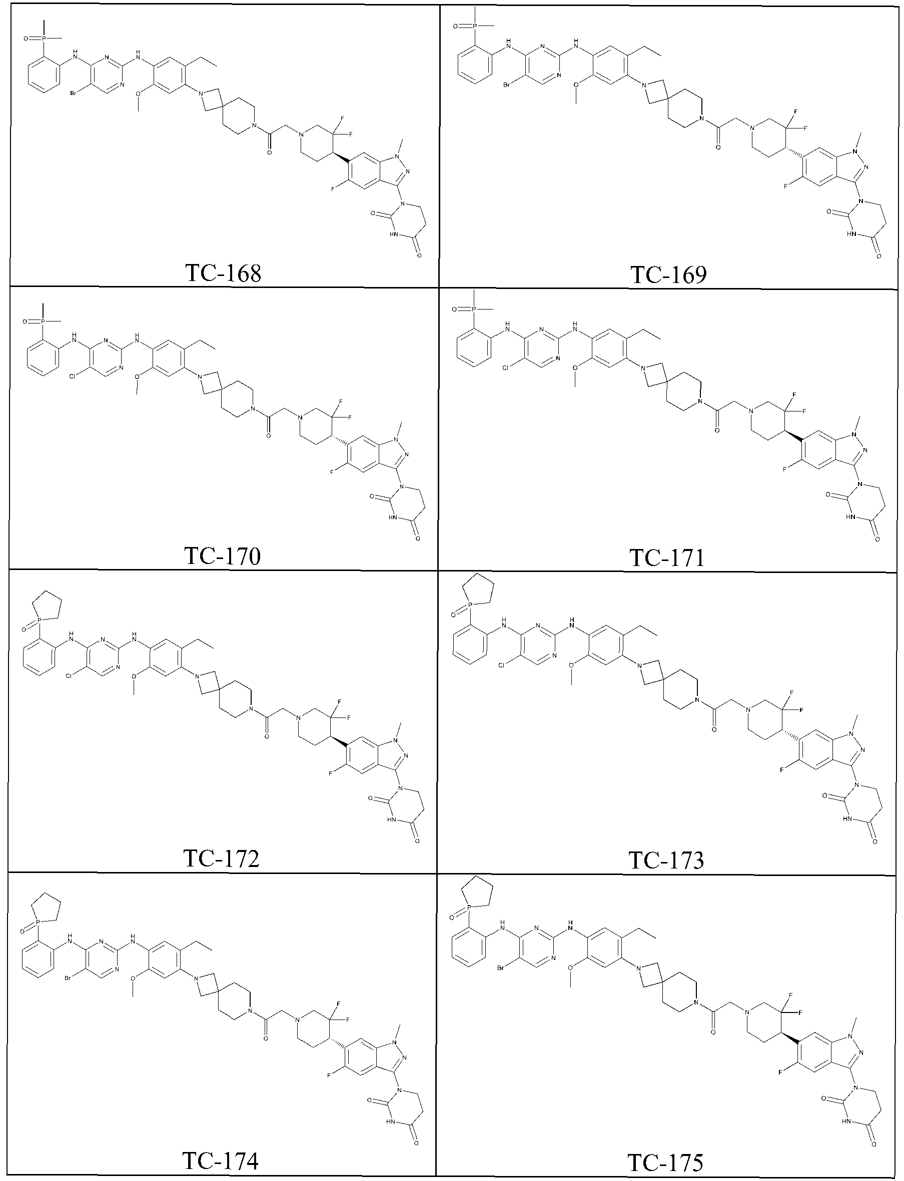

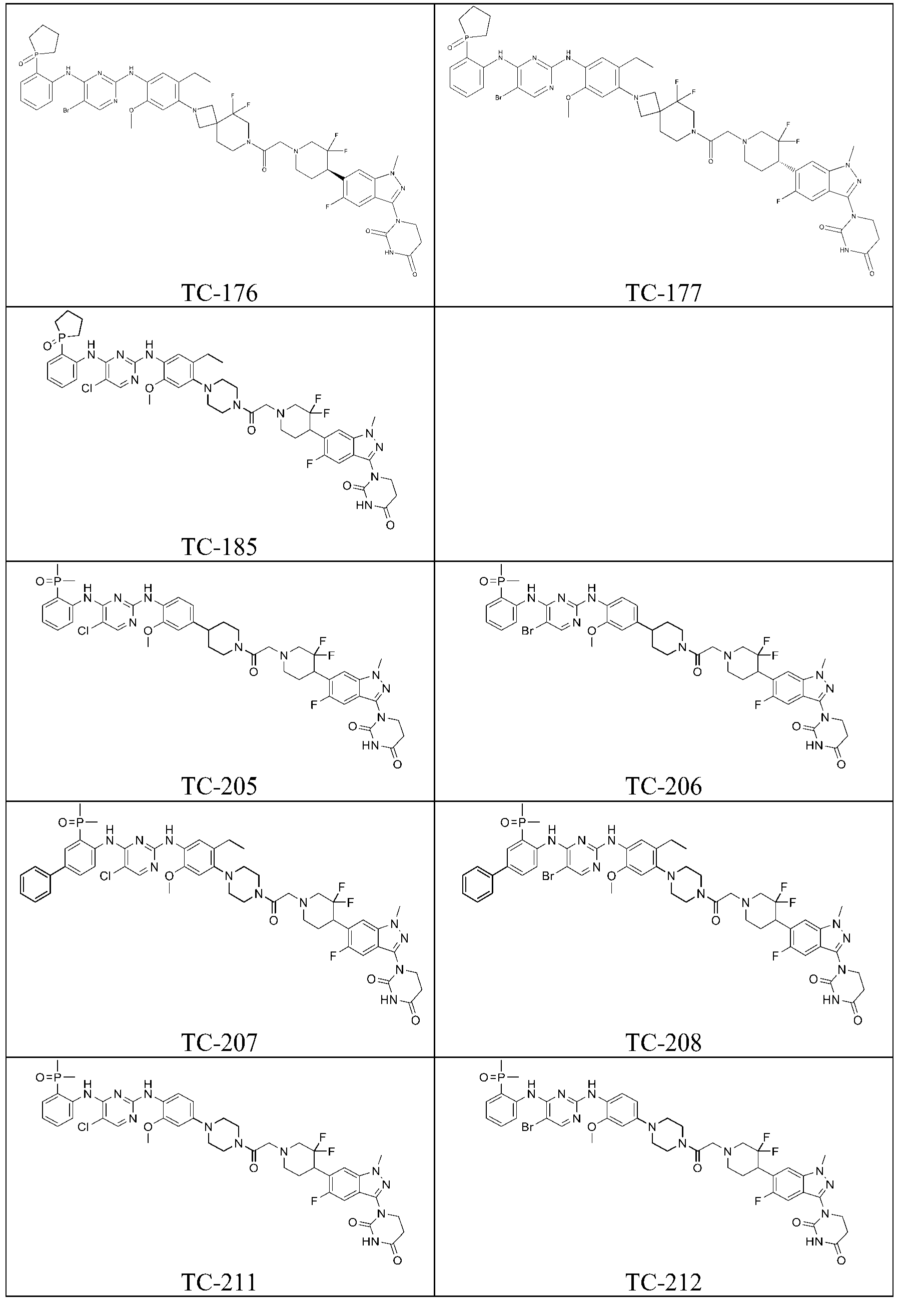

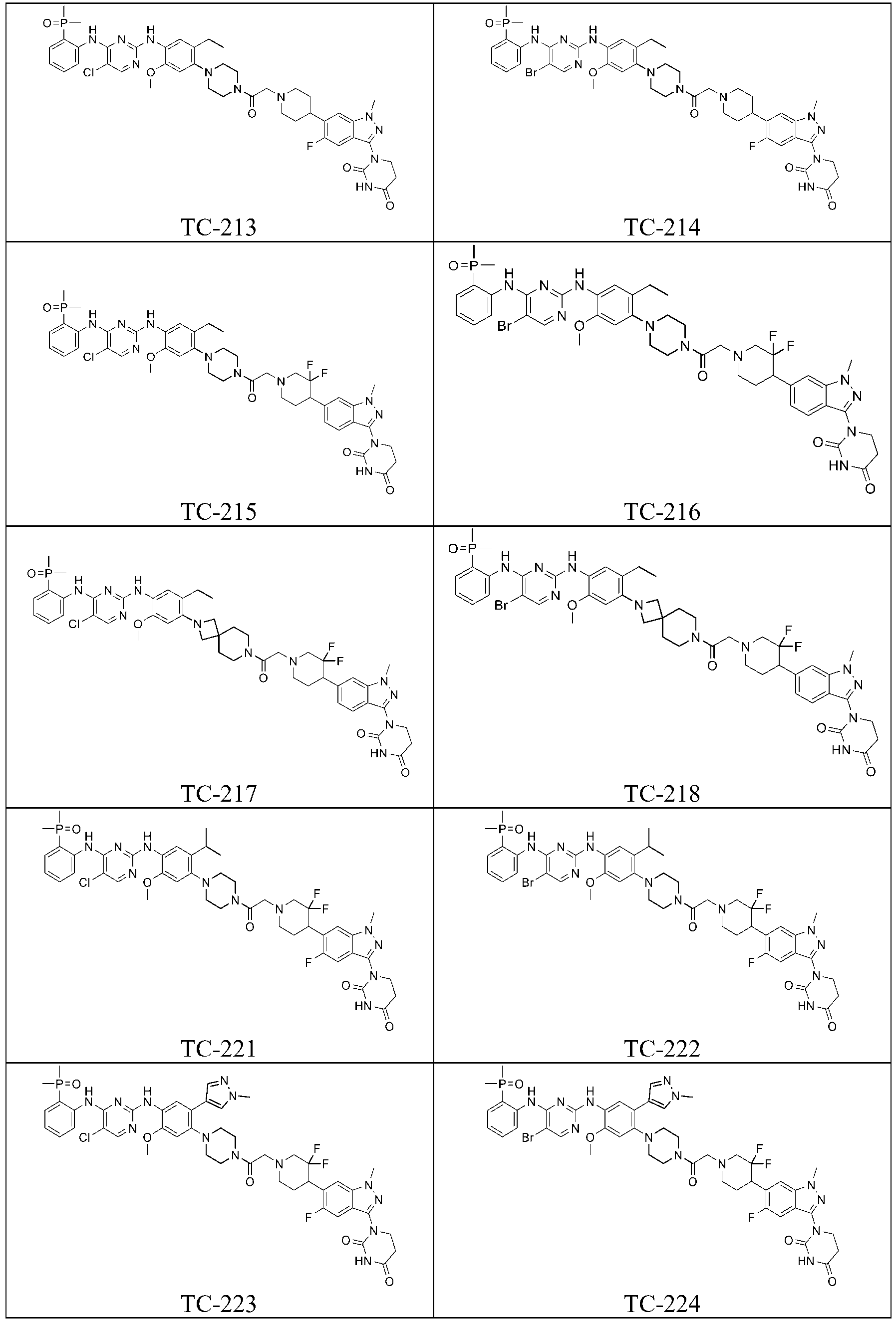

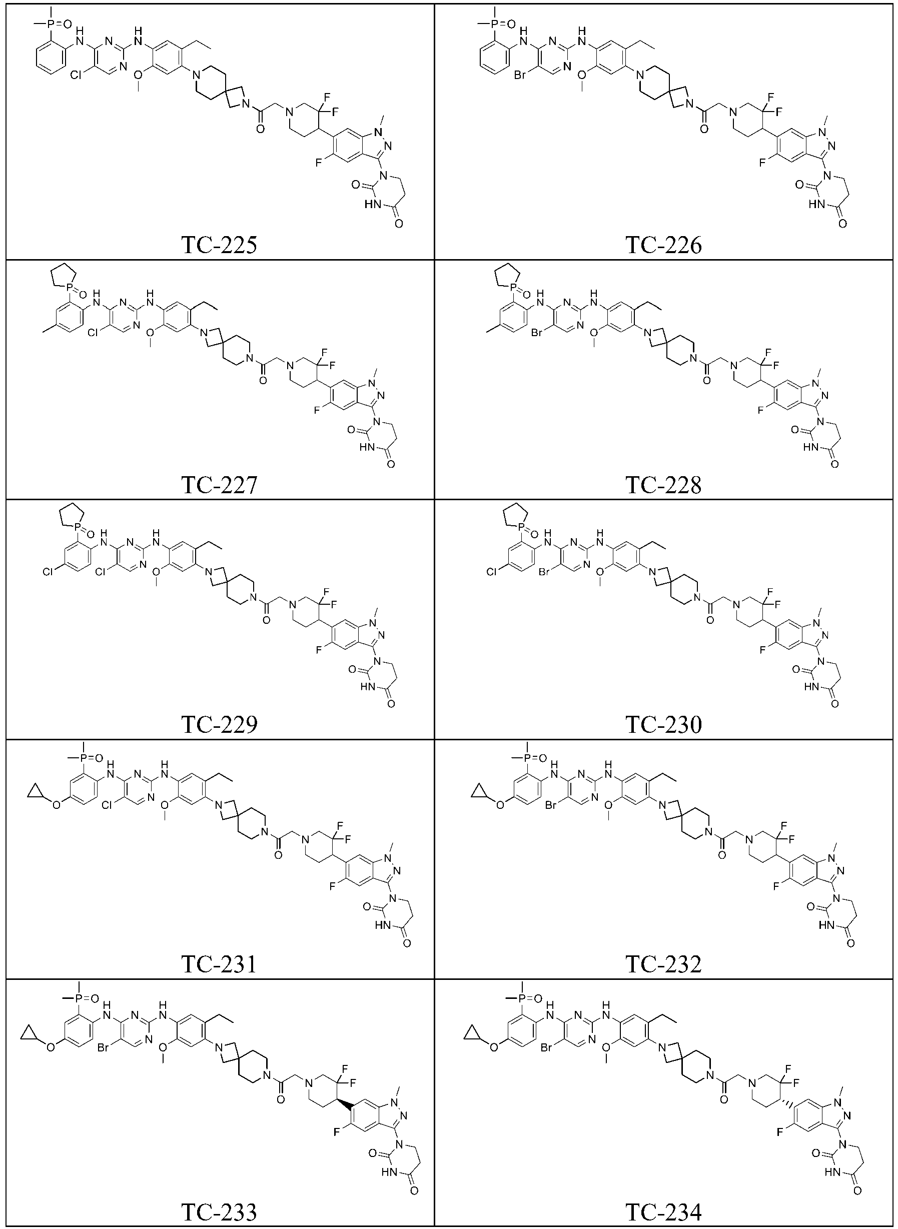

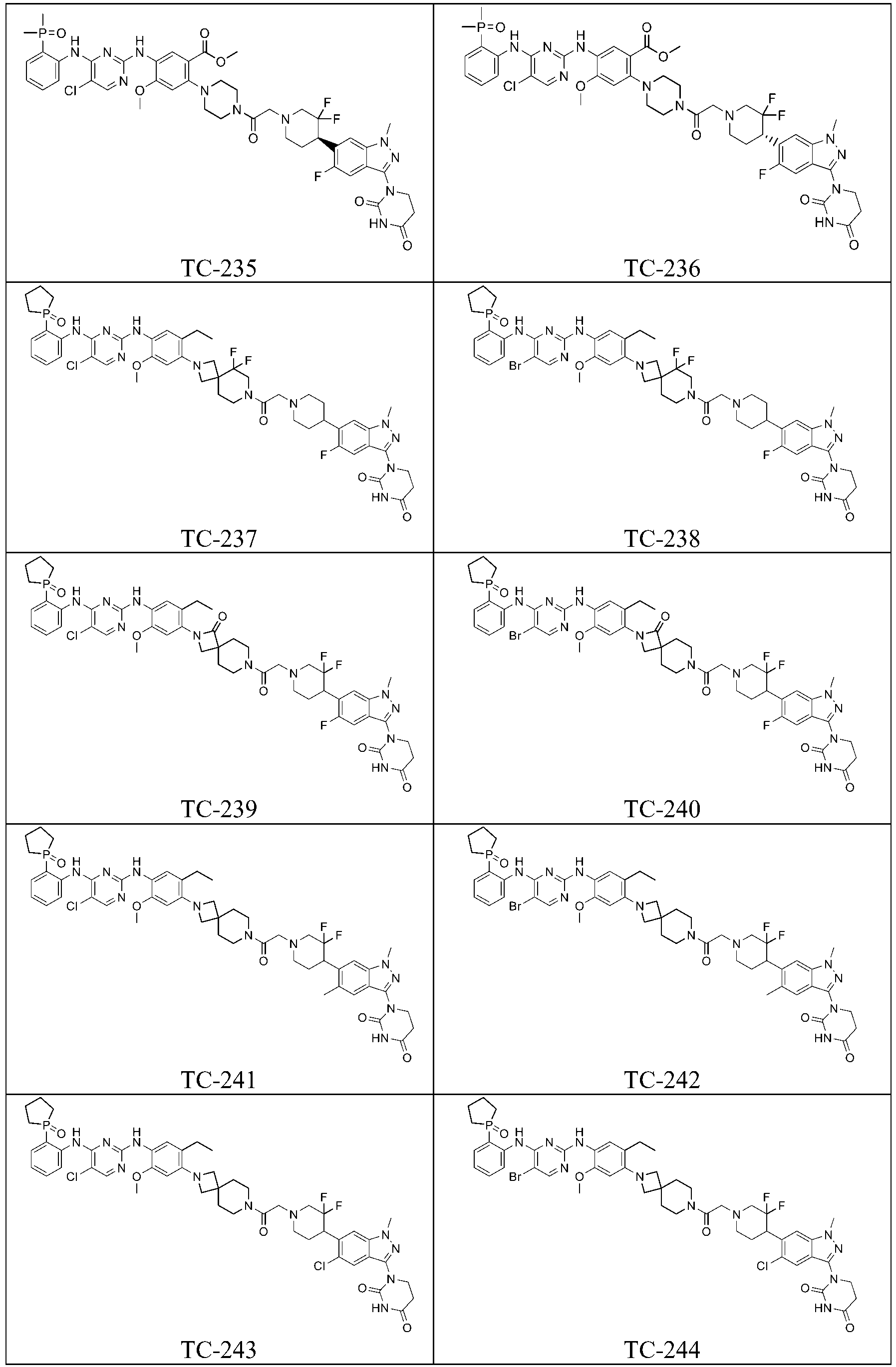

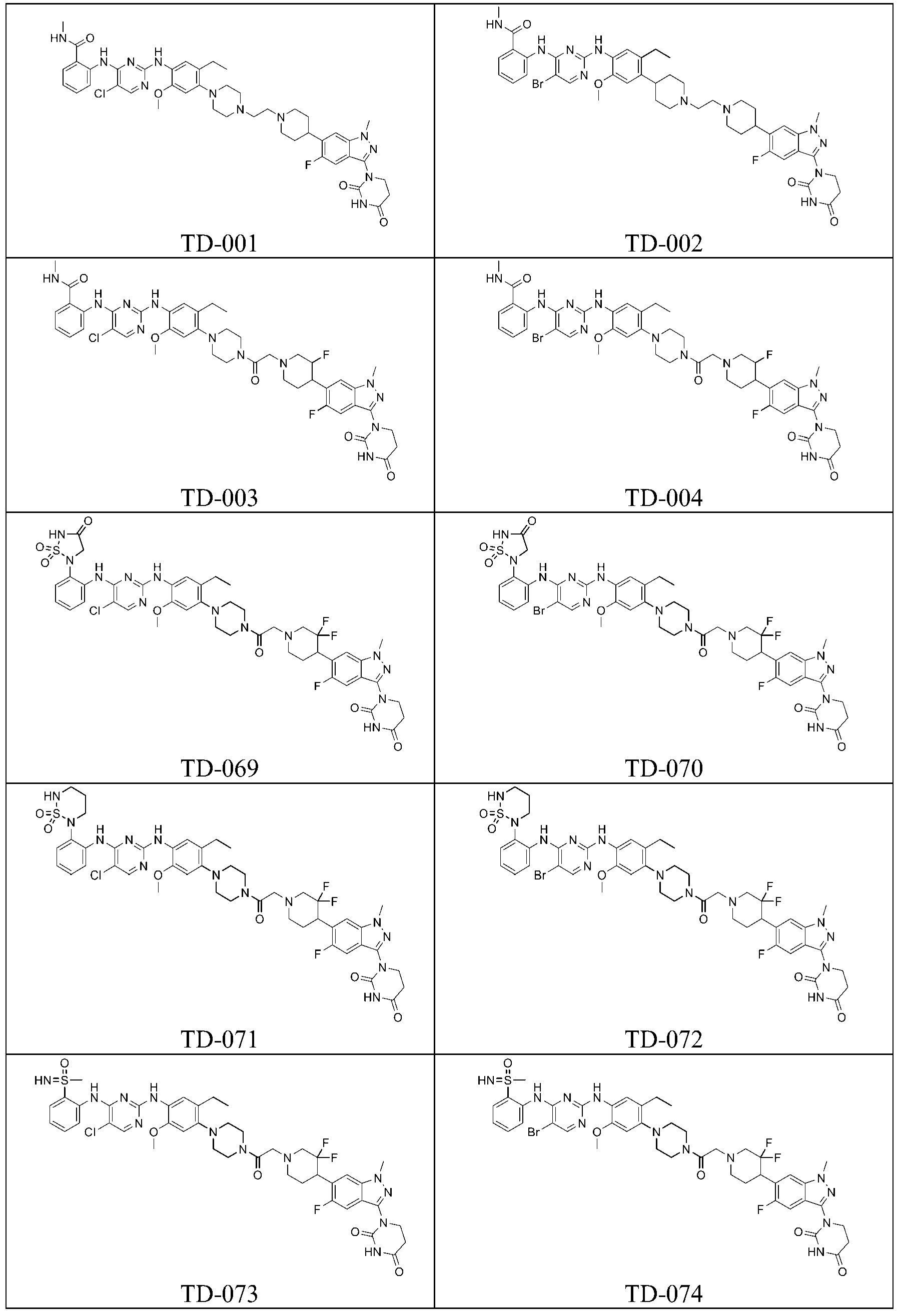

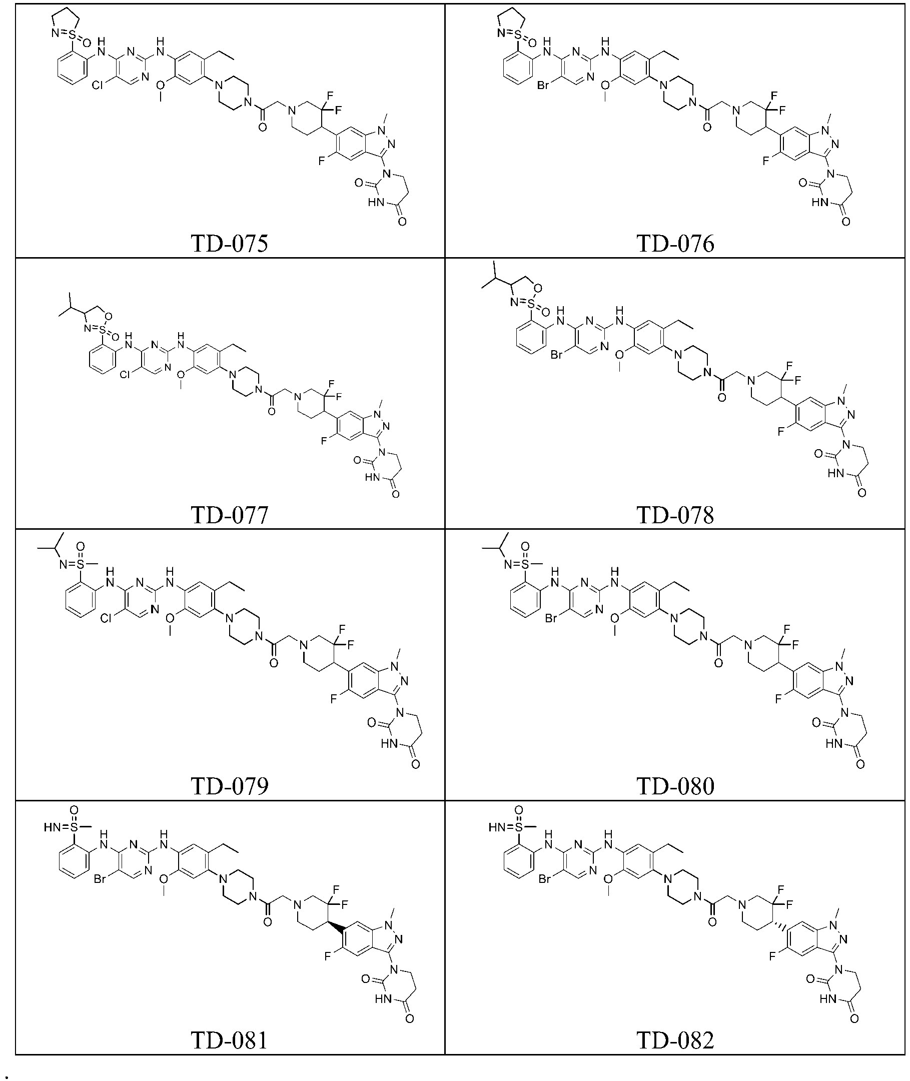

8. A pharmaceutical composition, wherein comprising (a) a therapeutically effective amount of the compound according to claim 1, 2, 3, or 7 as an active ingredient, and (b) a pharmaceutically acceptable carrier.

9. A use of the compound according to claim 1, 2, 3, or 7, wherein the use is selected from the group consisting of:
1) the preparation of a medicament for modulating abnormal EGFR kinase activity;
2) the preparation of a medicament for the prevention and/or treatment of diseases associated with abnormal EGFR kinase activity or mutations (preferably, diseases associated with EGFR resistance mutations); and/or
3) the preparation of a medicament for the degradation of the EGFR protein.

10. The use according to claim 9, wherein the disease associated with abnormal or mutated EGFR kinase activity is selected from the group consisting of inflammation, cancer, cardiovascular disease, infection, immune disorders, metabolic disorders, and combinations thereof.
